# EUROPEAN PATENT SPECIFICATION

(11) **EP 2 080 803 B1**
(45) Date of publication and mention of the grant of the patent: **25.05.2011**
(21) Application number: 08020922.4
(22) Date of filing: 28.02.2001
(51) Int. Cl.: C12N 15/14, C12N 15/16, C12N 15/17, C12N 15/20, C12N 15/21, C12N 15/31, C12N 15/32, C12N 15/62, C12N 15/82, C12P 21/02, A01H 5/00

(54) **Plastid transformation vectors for expressing proinsulin fused to the cholera toxin B-subunit in plants**
Plastidtransformationsvektoren zur Expression von Proinsulin, das an die Cholera Toxin B-Untereinheit fusioniert ist, in Pflanzen
Vecteurs de transformation de plastide pour exprimer une proinsuline fusionnée à la sous-unité B de la toxine du cholera dans des plantes

(30) Priority: 01.03.2000 US 185987 P; 23.01.2001 US 263424 P; 23.01.2001 US 263473 P; 23.01.2001 US 263668 P; 22.02.2001 US 270681 P
(43) Date of publication of application: 22.07.2009
(62) Divisional of application: 01954572.2
(73) Proprietor: Auburn University, Auburn University, AL 36849 (US); UNIVERSITY OF CENTRAL FLORIDA, Orlando, FL 32816 (US)
(72) Inventor: Daniell, Henry, Winter Park, FL 32792 (US)
(74) Representative: Predazzi, Valentina

(56) References cited:
- WO-A-99/10513
- US-A- 5 932 479
- ARAKAWA T ET AL: "A plant-based cholera toxin B subunit-insulin fusion protein protects against the development of autoimmune diabetes" NATURE BIOTECHNOLOGY, NATURE PUBLISHING GROUP, NEW YORK, NY, US, vol. 16, no. 10, 1 October 1998 (1998-10-01), pages 934-938, XP002093387 ISSN: 1087-0156
- BERGEROT I ET AL: "A CHOLERA TOXOID-INSULIN CONJUGATE AS AN ORAL VACCINE AGAINST SPONTANEOUS AUTOIMMUNE DIABETES" PROCEEDINGS OF THE NATIONAL ACADEMY OF SCIENCES OF USA, NATIONAL ACADEMY OF SCIENCE. WASHINGTON, US, vol. 94, April 1997 (1997-04), pages 4610-4614, XP002901456 ISSN: 0027-8424
- COSA DE B ET AL: "OVEREXPRESSION OF THE BT CRY2AA2 OPERON IN CHLOROPLASTS LEADS TO FORMATION OF INSECTICIDAL CRYSTALS" NATURE BIOTECHNOLOGY, NATURE PUBLISHING, US, vol. 19, January 2001 (2001-01), pages 71-74, XP002943533 ISSN: 1087-0156
- RUHLMAN TRACEY ET AL: "Expression of cholera toxin B-proinsulin fusion protein in lettuce and tobacco chloroplasts--oral administration protects against development of insulitis in non-obese diabetic mice." PLANT BIOTECHNOLOGY JOURNAL JUL 2007, vol. 5, no. 4, July 2007 (2007-07), pages 495-510, XP002530070 ISSN: 1467-7652

### SEQUENCE LISTING

<110> DANIELL, HENRY
<120> PRODUCTION OF PHARMACEUTICAL PROTEINS IN TRANSGENIC PLASTIDS
<130> 1465-PCT-US-00
<140> 09/807,742
   <141> 2001-04-18
<150> PCT/US01/06288
   <151> 2001-02-28
<160> 19
<170> PatentIn Ver. 2.1
<210> 1
   <211> 1250
   <212> PRT
   <213> Artificial Sequence
<220>
   <223> Description of Artificial Sequence: Synthetic peptide
<220>
   <223> This sequence may encompass 1-250 Gly Val Gly Val Pro repeats
<400> 1
   Gly Val Gly Val Pro Gly Val Gly Val Pro Gly Val Gly Val Pro Gly
   1               5                   10                  15
   Val Gly Val Pro Gly Val Gly Val Pro Gly Val Gly Val Pro Gly Val
               20                  25                  30
   Gly Val Pro Gly Val Gly Val Pro Gly Val Gly Val Pro Gly Val Gly
           35                  40                  45
   Val Pro Gly Val Gly Val Pro Gly Val Gly Val Pro Gly Val Gly Val
       50                  55                  60
   Pro Gly Val Gly Val Pro Gly Val Gly Val Pro Gly Val Gly Val Pro
   65                  70                  75                  80
   Gly Val Gly Val Pro Gly Val Gly Val Pro Gly Val Gly Val Pro Gly
   85 90 95
   Val Gly Val Pro Gly Val Gly Val Pro Gly Val Gly Val Pro Gly Val
   100 105 110
   Gly Val Pro Gly Val Gly Val Pro Gly Val Gly Val Pro Gly Val Gly
   115 120 125
   Val Pro Gly Val Gly Val Pro Gly Val Gly Val Pro Gly Val Gly Val
   130 135 140
   Pro Gly Val Gly Val Pro Gly Val Gly Val Pro Gly Val Gly Val Pro
   145 150 155 160
   Gly Val Gly Val Pro Gly Val Gly Val Pro Gly Val Gly Val Pro Gly
   165 170 175
   Val Gly Val Pro Gly Val Gly Val Pro Gly Val Gly Val Pro Gly Val
   180 185 190
   Gly Val Pro Gly Val Gly Val Pro Gly Val Gly Val Pro Gly Val Gly
   195 200 205
   Val Pro Gly Val Gly Val Pro Gly Val Gly Val Pro Gly Val Gly Val
   210 215 220
   Pro Gly Val Gly Val Pro Gly Val Gly Val Pro Gly Val Gly Val Pro
   225 230 235 240
   Gly Val Gly Val Pro Gly Val Gly Val Pro Gly Val Gly Val Pro Gly
   245 250 255
   Val Gly Val Pro Gly Val Gly Val Pro Gly Val Gly Val Pro Gly Val
   260 265 270
   Gly Val Pro Gly Val Gly Val Pro Gly Val Gly Val Pro Gly Val Gly
   275 280 285
   Val Pro Gly Val Gly Val Pro Gly Val Gly Val Pro Gly Val Gly Val
   290 295 300
   Pro Gly Val Gly Val Pro Gly Val Gly Val Pro Gly Val Gly Val Pro
   305 310 315 320
   Gly Val Gly Val Pro Gly Val Gly Val Pro Gly Val Gly Val Pro Gly
   325 330 335
   Val Gly Val Pro Gly Val Gly Val Pro Gly Val Gly Val Pro Gly Val
   340 345 350
   Gly Val Pro Gly Val Gly Val Pro Gly Val Gly Val Pro Gly Val Gly
   355 360 365
   Val Pro Gly Val Gly Val Pro Gly Val Gly Val Pro Gly Val Gly Val
   370 375 380
   Pro Gly Val Gly Val Pro Gly Val Gly Val Pro Gly Val Gly Val Pro
   385 390 395 400
   Gly Val Gly Val Pro Gly Val Gly Val Pro Gly Val Gly Val Pro Gly
   405 410 415
   Val Gly Val Pro Gly Val Gly Val Pro Gly Val Gly Val Pro Gly Val
   420 425 430
   Gly Val Pro Gly Val Gly Val Pro Gly Val Gly Val Pro Gly Val Gly
   435 440 445
   Val Pro Gly Val Gly Val Pro Gly Val Gly Val Pro Gly Val Gly Val
   450 455 460
   Pro Gly Val Gly Val Pro Gly Val Gly Val Pro Gly Val Gly Val Pro
   465 470 475 480
   Gly Val Gly Val Pro Gly Val Gly Val Pro Gly Val Gly Val Pro Gly
   485 490 495
   Val Gly Val Pro Gly Val Gly Val Pro Gly Val Gly Val Pro Gly Val
   500 505 510
   Gly Val Pro Gly Val Gly Val Pro Gly Val Gly Val Pro Gly Val Gly
   515 520 525
   Val Pro Gly Val Gly Val Pro Gly Val Gly Val Pro Gly Val Gly Val
   530 535 540
   Pro Gly Val Gly Val Pro Gly Val Gly Val Pro Gly Val Gly Val Pro
   545 550 555 560
   Gly Val Gly Val Pro Gly Val Gly Val Pro Gly Val Gly Val Pro Gly
   565 570 575
   Val Gly Val Pro Gly Val Gly Val Pro Gly Val Gly Val Pro Gly Val
   580 585 590
   Gly Val Pro Gly Val Gly Val Pro Gly Val Gly Val Pro Gly Val Gly
   595 600 605
   Val Pro Gly Val Gly Val Pro Gly Val Gly Val Pro Gly Val Gly Val
   610 615 620
   Pro Gly Val Gly Val Pro Gly Val Gly Val Pro Gly Val Gly Val Pro
   625 630 635 640
   Gly Val Gly Val Pro Gly Val Gly Val Pro Gly Val Gly Val Pro Gly
   645 650 655
   Val Gly Val Pro Gly Val Gly Val Pro Gly Val Gly Val Pro Gly Val
   660 665 670
   Gly Val Pro Gly Val Gly Val Pro Gly Val Gly Val Pro Gly Val Gly
   675 680 685
   Val Pro Gly Val Gly Val Pro Gly Val Gly Val Pro Gly Val Gly Val
   690 695 700
   Pro Gly Val Gly Val Pro Gly Val Gly Val Pro Gly Val Gly Val Pro
   705 710 715 720
   Gly Val Gly Val Pro Gly Val Gly Val Pro Gly Val Gly Val Pro Gly
   725 730 735
   Val Gly Val Pro Gly Val Gly Val Pro Gly Val Gly Val Pro Gly Val
   740 745 750
   Gly Val Pro Gly Val Gly Val Pro Gly Val Gly Val Pro Gly Val Gly
   755 760 765
   Val Pro Gly Val Gly Val Pro Gly Val Gly Val Pro Gly Val Gly Val
   770 775 780
   Pro Gly Val Gly Val Pro Gly Val Gly Val Pro Gly Val Gly Val Pro
   785 790 795 800
   Gly Val Gly Val Pro Gly Val Gly Val Pro Gly Val Gly Val Pro Gly
   805 810 815
   Val Gly Val Pro Gly Val Gly Val Pro Gly Val Gly Val Pro Gly Val
   820 825 830
   Gly Val Pro Gly Val Gly Val Pro Gly Val Gly Val Pro Gly Val Gly
   835 840 845
   Val Pro Gly Val Gly Val Pro Gly Val Gly Val Pro Gly Val Gly Val
   850 855 860
   Pro Gly Val Gly Val Pro Gly Val Gly Val Pro Gly Val Gly Val Pro
   865 870 875 880
   Gly Val Gly Val Pro Gly Val Gly Val Pro Gly Val Gly Val Pro Gly
   885 890 895
   Val Gly Val Pro Gly Val Gly Val Pro Gly Val Gly Val Pro Gly Val
   900 905 910
   Gly Val Pro Gly Val Gly Val Pro Gly Val Gly Val Pro Gly Val Gly
   915 920 925
   Val Pro Gly Val Gly Val Pro Gly Val Gly Val Pro Gly Val Gly Val
   930 935 940
   Pro Gly Val Gly Val Pro Gly Val Gly Val Pro Gly Val Gly Val Pro
   945 950 955 960
   Gly Val Gly Val Pro Gly Val Gly Val Pro Gly Val Gly Val Pro Gly
   965 970 975
   Val Gly Val Pro Gly Val Gly Val Pro Gly Val Gly Val Pro Gly Val
   980 985 990
   Gly Val Pro Gly Val Gly Val Pro Gly Val Gly Val Pro Gly Val Gly
   995 1000 1005
   Val Pro Gly Val Gly Val Pro Gly Val Gly Val Pro Gly Val Gly Val
   1010 1015 1020
   Pro Gly Val Gly Val Pro Gly Val Gly Val Pro Gly Val Gly Val Pro
   1025 1030 1035 1040
   Gly Val Gly Val Pro Gly Val Gly Val Pro Gly Val Gly Val Pro Gly
   1045 1050 1055
   Val Gly Val Pro Gly Val Gly Val Pro Gly Val Gly Val Pro Gly Val
   1060 1065 1070
   Gly Val Pro Gly Val Gly Val Pro Gly Val Gly Val Pro Gly Val Gly
   1075 1080 1085
   Val Pro Gly Val Gly Val Pro Gly Val Gly Val Pro Gly Val Gly Val
   1090 1095 1100
   Pro Gly Val Gly Val Pro Gly Val Gly Val Pro Gly Val Gly Val Pro
   1105 1110 1115 1120
   Gly Val Gly Val Pro Gly Val Gly Val Pro Gly Val Gly Val Pro Gly
   1125 1130 1135
   Val Gly Val Pro Gly Val Gly Val Pro Gly Val Gly Val Pro Gly Val
   1140 1145 1150
   Gly Val Pro Gly Val Gly Val Pro Gly Val Gly Val Pro Gly Val Gly
   1155 1160 1165
   Val Pro Gly Val Gly Val Pro Gly Val Gly Val Pro Gly Val Gly Val
   1170 1175 1180
   Pro Gly Val Gly Val Pro Gly Val Gly Val Pro Gly Val Gly Val Pro
   1185 1190 1195 1200
   Gly Val Gly Val Pro Gly Val Gly Val Pro Gly Val Gly Val Pro Gly
   1205 1210 1215
   Val Gly Val Pro Gly Val Gly Val Pro Gly Val Gly Val Pro Gly Val
   1220 1225 1230
   Gly Val Pro Gly Val Gly Val Pro Gly Val Gly Val Pro Gly Val Gly
   1235 1240 1245
   Val Pro
   1250
<210> 2
   <211> 6
   <212> PRT
   <213> Artificial Sequence
<220>
   <223> Description of Artificial Sequence: Illustrative endoplasmic reticulum retention signal
<400> 2
   Ser Glu Lys Asp Glu Leu
   1 5
<210> 3
   <211> 4
   <212> PRT
   <213> Artificial Sequence
<220>
   <223> Description of Artificial Sequence: Illustrative peptide
<400> 3
   Gly Pro Gly Pro
   1
<210> 4
   <211> 25
   <212> DNA
   <213> Artificial Sequence
<220>
   <223> Description of Artificial Sequence: Primer
<400> 4
   ccgtcgacgt agagaagtcc gtatt 25
<210> 5
   <211> 27
   <212> DNA
   <213> Artificial Sequence
<220>
   <223> Description of Artificial Sequence: Primer
<400> 5
   gcccatggta aaatcttggt ttattta 27
<210> 6
   <211> 28
   <212> DNA
   <213> Artificial Sequence
<220>
   <223> Description of Artificial Sequence: Primer
<400> 6
   cctttaaaaa gccttccatt ttctattt 28
<210> 7
   <211> 25
   <212> DNA
   <213> Artificial Sequence
<220>
   <223> Description of Artificial Sequence: Primer
<400> 7
   gccatggtaa aatcttggtt tatta 25
<210> 8
   <211> 12
   <212> DNA
   <213> Artificial Sequence
<220>
   <223> Description of Artificial Sequence: Illustrative preferred nucleotide sequence
<400> 8
   tttcgtttca gt 12
<210> 9
   <211> 5
   <212> PRT
   <213> Artificial Sequence
<220>
   <223> Description of Artificial Sequence: Synthetic peptide
<400> 9
   Ala Val Gly Val Pro
   1 5
<210> 10
   <211> 7
   <212> PRT
   <213> Artificial Sequence
<220>
   <223> Description of Artificial Sequence: Illustrative peptide
<400> 10
   Glu Asn Leu Tyr Phe Gln Gly
   1 5
<210> 11
   <211> 6
   <212> PRT
   <213> Artificial Sequence
<220>
   <223> Description of Artificial Sequence: Illustrative peptide
<400> 11
   Leu Val Pro Arg Gly Ser
   1 5
<210> 12
   <211> 6
   <212> PRT
   <213> Artificial Sequence
<220>
   <223> Description of Artificial Sequence: 6-His tag
<400> 12
   His His His His His His
   1 5
<210> 13
   <211> 25
   <212> DNA
   <213> Artificial Sequence
<220>
   <223> Description of Artificial Sequence: Primer
<400> 13
   aaaacccgtc ctcagttcgg attgc 25
<210> 14
   <211> 25
   <212> DNA
   <213> Artificial Sequence
<220>
   <223> Description of Artificial Sequence: Primer
<400> 14
   ccgcgttgtt tcatcaagcc ttacg 25
<210> 15
   <211> 119
   <212> PRT
   <213> Escherichia coli
<400> 15
   Gly Ile Val Pro Gly Val Gly Ile Val Pro Gly Val Gly Ile Val Pro
   1               5                   10                  15
   Gly Val Gly Ile Val Pro Gly Val Gly Ile Val Pro Gly Val Gly Ile
               20                  25                  30
   Val Pro Gly Val Gly Ile Val Pro Gly Val Gly Ile Val Pro Gly Val
           35                  40                  45
   Gly Ile Val Pro Gly Val Gly Ile Val Pro Gly Val Gly Ile Val Pro
       50                  55                  60
   Gly Val Gly Ile Val Pro Gly Val Gly Ile Val Pro Gly Val Gly Ile
   65                  70                  75                  80
   Val Pro Gly Val Gly Ile Val Pro Gly Val Gly Ile Val Pro Gly Val
                   85                  90                  95
   Gly Ile Val Pro Gly Val Gly Ile Val Pro Gly Val Gly Ile Val Pro
               100                 105                 110
   Gly Val Gly Val Pro Gly Val
           115
<210> 16
   <211> 260
   <212> DNA
   <213> Homo sapiens
<400> 16
   tttgtgaacc aacacctgtg cggctcacac ctggtggaag ctctctacct agtgtgcggg 60
   gaacgaggct tcttctacac acccaagacc cgccgggagg cagaggacct gcaggtgggg 120
   caggtggagc tgggcggggg ccctggtgca ggcagcctgc agcccttggc cctggagggg 180
   tccctgcaga agcgtggcat tgtggaacaa tgctgtacca gcatctgctc cctctaccag 240
   ctggagaact actgcaacta 260
<210> 17
   <211> 260
   <212> DNA
   <213> Artificial Sequence
<220>
   <223> Description of Artificial Sequence: Chloroplast modified proinsulin sequence
<400> 17
   ttcgtaaacc aacacttatg tggttctcac ctagtagaag ctttatactt agtatgtggt 60
   gaacgtggtt tcttctacac tcctaaaact cgtcgtgaag ctgaagattt acaagtaggt 120
   caagtagaat taggtggtgg tcctggtgct ggttctttac aacctttagc tttagaaggt 180
   tctttacaaa aacgtggtat tgtagaacaa tgttgtactt ctatttgttc tttataccaa 240
   ttagaaaact actgtaacta 260
<210> 18
   <211> 210
   <212> DNA
   <213> Homo sapiens
<400> 18
   ggaccggaga cgctctgcgg ggctgagctg gtggatgctc ttcagttcgt gtgtggagac 60
   aggggctttt atttcaacaa gcccacaggg tatggctcca gcagtcggag ggcgcctcag 120
   acaggcatcg tggatgagtg ctgcttccgg agctgtgatc taaggaggct ggagatgtat 180
   tgcgcacccc tcaagcctgc caagtcagct 210
<210> 19
   <211> 210
   <212> DNA
   <213> Homo sapiens
<400> 19
   ggtcctgaaa ctttatgtgg tgctgaatta gtagatgctt tacaattcgt atgtggtgat 60
   cgtggtttct atttcaacaa acctactggt tacggttctt cttctcgtcg tgctcctcaa 120
   actggtattg tagatgaatg ttgtttccgt tcttgtgatt tacgtcgttt agaaatgtac 180
   tgtgctcctt taaaacctgc taaatctgct                                  210

## Description

This invention relates to compositions and methods and products of Pharmaceutical Proteins, Human Therapeutics, Human Serum Albumin, Insulin, Native Cholera Toxic B Submitted On Transgenics Plastids, containing transformed plastids.

This invention relates to several embodiments which are disclosed herein in several specifications and corresponding figures titled as Pharmaceutical Proteins, Human Therapeutics, Human Serum Albumin, Insulin, Native Cholera Toxic B Submitted On Transgenics Plastids presented as one patent application and a set of claims thereof.

Despite the potential advantages of chloroplasts for biopharmaceutical production, it was not obvious that expressed pharmaceutical proteins in plastids would assemble in this organelle. Prior to this patent application there were no published reports of biopharmaceutical proteins expression in chloroplasts. Indeed, there were valid reasons to suggest that such expression would be problematic. Proinsulin contains both a and β chains and the C-peptide that connects them. It is synthesized as a pre-proinsulin by the pancreas. After proper folding, disulfide bridges are established between α and P chains. NIH reviewers noted that chloroplasts expression of high levels of properly assembled proinsulin was unanticipated. Nor was it obvious, as pointed out by NIH reviewers, that proinsulin expressed within plastids would be fully functional. Prior to this invention, there was no report of expression of proinsulin within plastids.

Similarly, prior to this invention, there was no report of expression of Human Serum Albumin within plastids. Human serum albumin is a globular protein of 66.5 kDa in size that should be properly folded and stabilized with seventeen disulfide bridges. Each human serum albumin consists of three structurally similar globular domains and the disulfides are positioned in repeated series of nine loop-link-loop structures centered around eight sequential Cys-Cys pairs. HSA is initially synthesized as pre-pro-albumin by the liver and released from the endoplasmatic reticulum after removal of the aminoterminal propeptide of 18 amino acids. The pro-albumin is further processed in the Golgi complex where the other 6 aminoterminal residues of the propeptide are cleaved by a serine proteinase. This results in the secretion of the mature polypeptide of 585 amino acids. It was likewise unanticipated that fully assembled HSA would be synthesized in large amount within plastids.

*Vibrio cholerae* causes diarrhea by colonizing the small intestine and producing enterotoxins, of which the cholera toxin (CT) is considered the main cause of toxicity. CT is a hermetic AB₅ protein having one 27KDa A subunit which has toxic ADP-ribosyl transferase activity and a non-toxic pentamer of 11.6 kDa B subunits that are non-covalently linked into a very stable doughnut like structure into which the toxic active (A) subunit is inserted. The A subunit of CT consists of two fragments - A1 and A2 which are linked by a disulfide bond The enzymatic activity of CT is located solely on the A1 fragment. The A2 fragment of the A subunit links the A1 fragment and the B pentamer. CT binds via specific interactions of the B-subunit pentamer with GM1 ganglioside, the membrane receptor, present on the intestinal epithelial cell surface of the host The A subunit is then translocated into the cell where it ADP-ribosylates the Gs subunit of adenylate cyclase bringing about the increased levels of cyclic AMP in effected cells that is associated with the electrolyte and fluid loss of clinical cholera. However, the B subunit, when administered orally, is a potent mucosal immunogen which can neutralize the toxicity of CT holotoxin by preventing its binding to intestinal cells. To achieve this effect, the B-subunit must be assembled in a pentameric form and disulfide bridges should be established among the subunit structures. It was not obvious that plastids could express and assemble pentameric CTB. There has been no prior report of expression of CTB or its assembly within plastids.

There was no certainty that biopharmaceuticals would assemble normally in chloroplasts or that they would retain their functionality in the prior art. Indeed, there might have been unforeseen deleterious effects of high-level expression of biopharmaceuticals in chloroplasts on plant growth or development that were not apparent from the experiences with other transgenes. The pH and oxidation state of the chloroplast differs from that of the human blood or bacterial cell in ways that might inhibit or prevent proper folding and assembly.

There are examples of protein complexes in the chloroplast in which all the subunits are native to the plant, the ribosome being an example. However, the expression and assembly in transformed chloroplasts of heterologous proteins into multi-protein complexes has not been reported until the present invention. There is a single example in the literature of an inter-chain disulfide bond in plant chloroplasts, and that is between neighboring large subunits of the enzyme ribulose-1, 5-biphosphase carboxylase/oxygenase. The expression and assembly in transformed chloroplasts of functional proteins consisting of different protein chains, including disulfide bonds between different subunits, as represented by expression and assembly of a mammalian protein has never been demonstrated until the present invention.

This invention relates to the production of pharmaceutical proteins in transgenic chloroplasts. More particularly, this invention relates to the production of human insulin in tobacco plants.

Research efforts have been made to synthesize high value pharmacologically active recombinant proteins in plants. Recombinant proteins such as vaccines, monoclonal antibodies, hormones, growth factors, neuropeptides, cytotoxins, serum proteins and enzymes have been expressed in nuclear transgenic plants (May et al., 1996). It has been estimated that one tobacco plant should be able to produce more recombinant protein than a 300-liter fermenter of *E. coli.* In addition, a tobacco plant produces a million seeds, thereby facilitating large-scale production. Tobacco is also an ideal choice because of its relative ease of genetic manipulation and an impending need to explore alternate uses for this hazardous crop.

A primary reas on for the high cost of production via fermentation is the cost of carbon source co-substances as well as maintenance of a large fermentation facility. In contrast, most estimates of plant production are a thousand-fold less expensive than fermentation. Tissue specific expression of high value proteins in leaves can enable the use of crop plants as renewable resources. Harvesting the cobs, tubers, seeds or fruits for food and feed and leaves for value added products should result in further economy with no additional investment.

However, one of the major limitations in producing pharmaceutical proteins in plants is their low level of foreign protein expression, despite reports of higher level expression of enzymes and certain proteins. May et at. (1998) discuss this problem using the following examples. Although plant derived recombinant hepatitis B surface antigen was as effective as a commercial recombinant vaccine, the levels of expression in transgenic tobacco were low (0.01% of total soluble protein). Even though Norwalk virus capsid protein expressed in potatoes caused oral immunization when consumed as food (edible vaccine), expression levels were low (0.3% of total soluble protein). A synthetic gene coding for the human epidermal growth factor was expressed only up to 0.001% of total soluble protein in transgenic tobacco. Human serum albumin has been expressed only up to 0.02% of the total soluble protein in transgenic plants.

Therefore, it is important to increase levels of expression of recombinant proteins in plants to exploit plant production of pharmacologically important proteins. An alternate approach is to express foreign proteins in chloroplasts of higher plant. Foreign genes (up to 10,000 copies per cell) have been incorporated into the tobacco chloroplast genome resulting in accumulation of recombinant proteins up to 30% of the total cellular protein (McBride et al., 1994).

The aforementioned approaches (except chloroplast transformation) are limited to eukaryotic gene expression because prokaryotic genes are expressed poorly in the nuclear compartment. However, several pharmacologically important proteins (such as insulin, human serum albumin, antibodies, enzymes etc.) are produced currently in *E. coli.* Also, several bacterial proteins (such as cholera toxin B subunit) are used as oral vaccines against diarrheal diseases. Therefore, it is important to develop a plant production system for expression of pharmacologically important proteins that are currently produced in prokaryotic systems (such as *E. coli)* via fermentation.

Chloroplasts are prokaryotic compartments inside eukaryotic cells. Since the transcriptional and translational machinery of the chloroplast is similar to *E. coli* (Brixey et al., 1997), it is possible to express prokaryotic genes at very high levels in plant chloroplasts than in the nucleus. In addition, plant cells contain up to 50,000 copies of the circular plastid genome (Bendich 1987) which may amplify the foreign gene like a "plasmid in the plant cell," thereby enabling higher levels of expression . Therefore, chloroplasts are an ideal choice for expression of recombinant proteins that are currently expressed in *E. coli* (such as insulin, human serum albumin, vaccines, antibodies, etc.). We exploited the chloroplast transformation approach to express a pharmacological protein that is of no value to the plant to demonstrate this concept, GVGVP gene has been synthesized with a codon preferred for prokaryotic (EG121) or eukaryotic (TG131) expression. Based on transcript levels, chloroplast expression of this polymer was a hundred-fold higher than nuclear expression in transgenic plants (Guda et al., 1999). Recently, we observed 16.966-fold more tps 1 transcripts in chloroplast transformants than the highly expressing nuclear transgenic plants (Lee et al. 2000, in review).

In our research, we use insulin as a model protein to demonstrate its production as a value added trait in transgenic tobacco. Most importantly, a significant advantage in the production of pharmaceutical proteins in chloroplasts is their ability to process eukaryotic protein, including folding and formation of disulfide bridges (Dreshcher et al., 1998). Chaperonin proteins are present in chloroplasts (Verling 1991; Roy 1989) that function in folding and assembly of prokaryotic/eukaryotic proteins. Also, proteins are activated by disulfide bond oxido/reduction cycles using the chloroplast inicredoxin system (Reulland and Miginiac-Maslow, 1999) or chloroplast protein disulfide isomerase (Kim and Mayfield, 1997). Accumulation of fully assembled, disulfide bonded form of antibody inside chloroplasts, even though plastics were not transformed (During et al. 1990), provides strong evidence for successful assembly of proinsulin inside chloroplasts. Indeed, we observed fully assembled heavy and light chains of humanized Guy's 13 antibody in transgenic tobacco chloroplasts (Panchal et al. 2000, in review). Such folding and assembly eliminates the need for post-purification processing of pharmaceutical proteins. Chloroplasts may also be isolated from crude homogenates by centrifugation (1500 X g). This fraction is free of other cellular proteins. Isolated chloroplasts are burst open by osmotic shock to release foreign proteins that are compartmentalized in this organelle along with few other native soluble proteins (Daniel and McFadden, 1987).

GVGVP is a PBP made from synthetic genes. At lower temperatures the polymers exist as more extended molecules which, on raising the temperature above the transition range, hydrophobically fold into dynamic structures called β-spirals that further aggregate by hydrophobic association to form twisted filaments (Urry, 1991; Urry, et al., 1994). Inverse temperature transition offers several advantages. Expense associated with chromatographic resins and equipment are eliminated. It also facilitates scale up of purification from grams to kilograms. Milder purification conditions use only a modest change in temperature and ionic strength. This also facilitates higher recovery, faster purification and high volume processing. Protein purification is generally the slow step (bottleneck) in pharmaceutical product development. Through exploitation of this reversible inverse temperature transition property, simple and inexpensive extraction and purification is performed. The temperature at which the aggregation takes place can be manipulated by engineering biopolymers containing varying numbers of repeats and changing salt concentration in solution (McPherson et al., 1996). Chloroplast mediated expression of insulin-polymer fusion protein eliminates the need for the expensive fermentation process as well as reagents needed for recombinant protein purification and downstream processing.

Large-scale production of insulin in plants in conjunction with an oral delivery system is a powerful approach to provide insulin to diabetes patients at an affordable cost and provide tobacco farmers alternate uses for this hazardous crop. For example, Sun et al. (1994) showed that feeding a small dose of antigens conjugated to the receptor binding non-toxic B subunit moiety of the cholera toxin (CTB) suppressed systemic T cell-mediated inflammatory reactions in animals. Oral administration of a myelin antigen conjugated to CTB has been shown to protect animals against encephalomyelitis, even when given after disease induction (Sun et al. 1996). Bergerot et al. (1997) reported that feeding small amounts of human insulin conjugated to CTB suppressed beta cell destruction and clinical diabetes in adult non-obese diabetic (NOD) mice. The protective effect could be transferred by T cells from CTB-insulin treated animals and was associated with reduced insulitis. These results demonstrate that protection against autoimmune diabetes can indeed be achieved by feeding small amounts of pancreas islet cell auto antigen linked to CTB (Bergerot, et al. 1997). Conjugation with CTB facilitates antigen delivery and presentation to the Gut Associated Lymphoid Tissues (GALT) due to its affinity for the cell surface receptor GM-ganglioside located on GALT cells, for increased uptake and immunologic recognition (Arakawa et al. 1998). Transgenic potato tubers expressed up to 0.1% CTB-insulin fusion protein of total soluble protein, which retained GM-ganglioside binding affinity and native autogenicty for both CTB and insulin. NOD mice fed with transgenic potato tubers containing microgram quantities of CTB-insulin fusion protein showed a substantial reduction in insulitis and a delay in the progression of diabetes (Arkawa et al., 1998). However, for commercial exploitation, the levels of expression need to be increased in transgenic plants. Therefore, we undertook the expression of CTB-insulin fusion in transgenic chloroplasts of nicotine free edible tobacco to increase levels of expression adequate for animal testing.

In accordance with one advantageous feature of this invention, we use poly(GVGVP) as a fusion protein to enable hyper-expression of insulin and accomplish rapid one step purification of fusion peptides utilizing the inverse temperature transition properties of this polymer. In another advantageous feature of this invention, we develop insulin-CTB fusion protein for oral delivery in nicotine free edible tobacco (LAMD 605). Both features are accomplished as follows:
a) Develop recombinant DNA vectors for enhanced expression of Proinsulin as fusion proteins with GVGVP or CTB via chloroplast genomes of tobacco,
b) Obtain transgenic tobacco (Petit Havana & LAMD 605) plants,
c) Characterize transgenic expression of proinsulin polymer or CTB fusion proteins using molecular and biochemical methods in chloroplasts,
d) Employ existing or modified methods of polymer purification from transgenic leaves,
e) Analyze Mendelian or maternal inheritance of transgenic plants,
f) Large scale purification of insulin and comparison of current insulin purification methods with polymer-based purification method in *E. coli* and tobacco,
g) Compare natural refolding chloroplasts with *in vitro* processing,
h) Characterization (yield and purity) of proinsulin produced in *E. coli* and transgenic tobacco, and
i) Assessment of diabetic symptoms in mice fed with edible tobacco expressing CTB. insulin fusion protein.
Diabetes and Insulin: Insulin lowers blood glucose (Oakly et al. 1973). This is a result of its immediate effect in increasing glucose uptake in tissues. In muscle, under the action of insulin, glucose is more readily taken up and either converted to glycogen and lactic acid or oxidized to carbon dioxide. Insulin also affects a number of important enzymes concerned with cellular metabolism. It increases the activity of glucokinase, which phosphoryiates glucose, thereby increasing the rate of glucose metabolism in the liver. Insulin also suppresses gluconeogenesis by depressing the function of liver enzymes, which operate the reverse pathway from proteins to glucose. Lack of insulin can restrict the transport of glucose into muscle and adipose tissue. This results in increases in blood glucose levels (hyperglycemia). In addition, the breakdown of natural fat to free fatty acids and glycerol is increased and there is a rise in the fatty acid content in the blood. Increased catabolism of fatty acids by the liver results in greater production ofketone bodies. They diffuse from the liver and pass to the muscles for further oxidation. Soon, ketone body production rate exceeds oxidation rate and ketosis results. Fewer amino acids are taken up by the tissues and protein degradation results. At the same time, gluconeogenesis is stimulated and protein is used to produce glucose. Obviously, lack of insulin has serious consequences.

Diabetes is classified into types I and II. Type I is also known as insulin dependent diabetes mellitus (IDDM). Usually this is causedbyacell-mediated autoimmune destruction of the pancreatic β-cells (Davidson, 1998). Those suffering from this type are dependent on external sources of insulin. Type II is known as noninsulin-dependent diabetes mellitus (NIDDM). This usually involved resistance to insulin in combination with its underproduction. These prominent diseases have led to extensive research into microbial production of recombinant human insulin (rHI).
**Expression of Recombinant Human Insulin in** ***E. coli:*** In 1978, two thousand kilograms of insulin were used in the world each year; half of this was used in the United States (Steiner et al., 1978). At that time, the number of diabetics in the US were increasing 6% every year (Gunby,1978). In 1997 - 98,10% increase in sales of diabetes care products and 19% increase in insulin products have been reported by Novo Nordisk (world's leading supplier of insulin), making it a 7.8 billion dollar industry. Annually, 160,000 Americans are killed by diabetes, making it the fourth leading cause of death. Many methods of production of rHI have been developed. Insulin genes were first chemically synthesized for expression in *Esherichia coli* (Crea et al., 1978). These genes encoded separate insulin A and B chains. The genes were each expressed in *E. coli* as fusion proteins with the β-galactosidase (Goeddel et al., 1979). The first documented production of rHI using this system was reported by David Goeddel from Genentech (Hall,1988). For reasons explained later, the genes were fused to the Trp synthase gene. This fusion protein was approved for commercial production by Eli Lilly in 1982 (Chance and Frank, 1993) with a product name of Humulin. As of 1986, Humulin was produced from proinsulin genes. Proinsulin contains both insulin chains and the C-peptide that connects them. Data concerning commercial production of Humulin and other insulin products is now considered proprietary information and is not available to the public.
**Delivery of Human Insulin:** Insulin has been delivered intravenously in the past several years. However, more recently, alternate methods such as nasal spray are also available. Oral delivery of insulin is yet another new approach (Mathiowitz et al., 1997). Engineered polymer microspheres made of biologically erodable polymers, which display strong interactions with gastrointestinal mucus and cellular linings, can traverse both mucosal absorptive epithelium and the follicle-associated epithelium, covering the lymphoid tissue of Peyer's patches. Polymers maintain contact with intestinal epithelium for extended periods of time and actually penetrate through and between cells. Animals fed with the poly(FA: PLGA)-encapsulated insulin preparation were able to regulate the glucose load better than controls, confirming that insulin crossed the intestinal barrier and was released from the microspheres in a biologically active form (Mathiowitz et al., 1997).
**Protein Based Polymers (PBP):** The synthetic gene that codes for a bioelastic PBP was designed after repeated amino acid sequences GVGVP, observed in all sequenced mammalian elastin proteins (Yeh et al. 1987). Elastin is one of the strongest known natural fibers and is present in skin, ligaments, and arterial walls. Bioelastic PBPs containing multiple repeats of this pentamer have remarkable elastic properties, enabling several medical and non-medical applications (Urry et al. 1993, Urry 1995, Daniell 1995). GVGVP polymers prevent adhesions following surgery, aid in reconstructing tissues and delivering drugs to the body over an extended period of time. North American Science Associates, Inc. reported that GVGVP polymer is non-toxic in mice, non-sensitizing and non-antigenic in guinea pigs, and non-pyrogenic in rabbits (Urryet al. 1993). Researchers have also observed that inserting sheets of GVGVP at the sites of contaminated wounds in rats reduces the number of adhesions that form as the wounds heal (Urry et al. 1993). In a similar manner, using the GVGVP to encase muscles that are cut during eye surgery in rabbits prevents scarring following the operation (Urry et al. 1993, Urry 1995). Other medical applications of bioelastic PBPs include tissue reconstruction (synthetic ligaments and arteries, bones), wound coverings, artificial pericardia, catheters and programmed drug delivery (Urry, 1995; Urry et al., 1993, 1996).

We have expressed the elastic PBP (GVGVP)₁₂₁ in *E. coli* (Guda et al. 1995, Brixey et al.1997), in the fungus *Aspergillus nidulans* (Herzog et al.1997), in cultured tobacco cells (Zhang et al. 1995), and in transgenic tobacco plants (Zhang et al. 1996). In particular, (GVGVP)₁₂₁ has been expressed to such high levels in *E. coli* that polymer inclusion bodies occupied up to about 90% of the cell volume. Also, inclusion bodies have been observed in chloroplasts of transgenic tobacco plants (see attached article, Daniell and Guda, 1997). Recently, we reported stable transformation of the tobacco chloroplasts by integration and expression the biopolymer gene (EG121), into the Large Single Copy region (5,000 copies per cell) or the Inverted Repeat region (10,000 copies per cell) of the chloroplast genome (Guda et al.,1999).
**PBP as Fusion Proteins:** Several systems are now available to simplify protein purification including the maltose binding protein (Marina et al. 1988), glutethione S-tranferase (Smith and Johnson 1988), biotinylated (Tsao et al. 1996), thioredoxin (Smith et al. 1998) and cellulose binding (Ong et al. 1989) proteins. Recombinant DNA vectors for fusion with short peptides are now available to effectively utilize aforementioned fusion proteins in the purification process (Smith et al. 1998; Kim and Raines, 1993; Su et al. 1992). Recombinant proteins are generally purified by affinity chromatography, using ligands specific to carrier proteins (Nilsson et al. 1997). While these are useful techniques for laboratory scale purification, affinity chromatography for large-scale purification is time consuming and cost prohibitive. Therefore, economical and non-chromatographic techniques are highly desirable. In addition, a common solution to N-terminal degradation of small peptides is to fuse foreign peptides to endogenous *E. coli* proteins. Early in the development of this technique, β-galactosidase (β-gal) was used as a fusion protein (Goldberg and Goff, 1986). A drawback of this method was that the β-gal protein is of relatively high molecular weight (MW 100,000). Therefore, the proportion of the peptide product in the total protein is low. Another problem associated with the large β-gal fusion is early termination of translation (Burnette, 1983; Hall, 1988). This occurred when β-gal was used to produce human insulin peptides because the fusion was detached from the ribosome during translation thus yielding incomplete peptides. Other proteins of lower molecular weight proteins have been used as fusion proteins to increase the peptide production. For example, better yields were obtained with the tryptophan synthase (190aa) fusion proteins (Hall, 1988; Burnett,1983).

Accordingly, one achievement according to this invention is to use poly(GVGVP) as a fusion protein to enable hyper-expression of insulin and accomplish rapid one step purification of the fusion peptide. At lower temperatures the polymers exist as more extended molecules which, on raising the temperature above the transition range, hydrophobically fold into dynamic structures called β-spirals that further aggregate by hydrophobic association to form twisted filaments (Urry, 1991). Through exploitation of this reversible property, simple and inexpensive extraction and purification is performed. The temperature at which aggregation takes place (T₁) is manipulated by engineering biopolymers containing varying numbers of repeats or changing salt concentration (McPherson et al., 1996). Another group has recently demonstrated purification of recombinant proteins by fusion with thermally responsive polypeptides (Meyer and Chilkoti, 1999). Polymers of different sizes have been synthesized and expressed in *E. coli*. This approach also eliminates the need for expensive reagents, equipment and time required for purification.
**Cholera Toxin β subunit as a fusion protein:** Vibrio cholerae causes diarrhea by colonizing the small intestine and producing enterotoxins, of which the cholera toxin (CT) is considered the main cause of toxicity. CT is a bexameric AB₅ protein having one 27KDa A subunit which has toxic ADP-ribosyl transferase activity and a non-toxic pentamer of 11.6 kDa B subunits that are non-covalently linked into a very stable doughnut like structure into which the toxic active (A) subunit is inserted. The A subunit of CT consists of two fragments -A1 and A2 which are linked by a disulfide bond. The enzymatic activity of CT is located solely on the A1 fragment (Gill, 1976). The A2 fragment of the A subunit links the A1 fragment and the B pentamer. CT binds via specific interactions of the B subunit pentamer with GM1 ganglioside, the membrane receptor, present on the intestinal epithelial cell surface of the host. The A subunit is then translocated into the cell where it ADP-ribosylates the Gs subunit of adenylate cyclase bringing about the increased levels of cyclic AMP in affected cells that is associated with the electrolyte and fluid loss of clinical cholera (Lebens et al. 1994). For optimal enzymatic activity, the A1 fragment needs to be separated from the A2 fragment by proteolytic cleavage of the main chain and by reduction of the disulfide bond linking them (Mekalanos et al., 1979).

The Expression and assembly of CTB in transgenic potato tubers has been reported (Arakawa et al. 1997). The CTB gene including the leader peptide was fused to an endoplasmic reticulum retention signal (SEKDEL) at the 3' end to sequester the CTB protein within the lumen of the ER. The DNA fragment encoding the 21-amino acid leader peptide of the CTB protein was retained to direct the newly synthesized CTB protein into the lumen of the ER. Immunoblot analysis indicated that the plant derived CTB protein was antigenically indistinguishable from the bacterial CTB protein and that oligomeric CTB molecules (Mr - 50 kDa) were the dominant molecular species isolated from transgenic potato leaf and tuber tissues. Similar to bacterial CTB, plant derived CTB dissociated into monomers (Mr-15 kDa) during heat acid treatment.

Enzyme linked immunosorbent assay methods indicated that plant synthesized CTB protein bound specifically to GM1 gangliosides, the natural membrane receptors of Cholera Toxin. The maximum amount of CTB protein detected in auxin induced transgenic potato leaf and tuber issues was approximately 0.3% of the total soluble protein. The oral immunization of CD-1 mice with transgenic potato tissues transformed with the CTB gene (administered at weekly intervals for a month with a final booster feeding on day 65) has also been reported. The levels of serum and mucosal anti-cholera toxin antibodies in mice were found to generate protective immunity against the cytopathic effects of CT holotoxin.

Following intraileal injection with CT, the plant immunized mice showed up to a 60% reduction in diarrheal fluid accumulation in the small intestine. Systemic and mucosal CTB-specific antibody titers were determined in both serum and feces collected from immunized mice by the class-specific chemiluminescent ELISA method and the endpoint titers for the three antibody isotypes (IgM, IgG and IgA) were determined.

The extent of CT neutralization in both Vero cell and ileal loop experiments suggested that anti-CTB antibodies prevent CT binding to cellular GM1-gangliosides. Also, mice fed with 3 g of transgenic potato exhibited similar intestinal protection as mice gavaged with 30 g of bacterial CTB. Recombinant LTB [rLTB] (the heat labile enterotoxin produced by Enterotoxigenic *E. coli*) which is structurally, functionally and immunologically similar to CTB was expressed in transgenic tobacco (Arntzen et al.1998; Haq et al.,1995). They have reported that the rLTB retained its antigenicity as shown by immunoprecipitation of rLTB with antibodies raised to rLTB from *E. coli.* The rLTB protein was of the right molecular weight and aggregated to form the pentamer as confirmed by gel permeation chromatography.

CTB has also been demonstrated to be an effective carrier molecule for induction of mucosal immunity to polypeptides to which it is chemically or genetically conjugated (McKenzie et al, 1984; Dertzbaugh et al, 1993). The production of immunomodulatory transmucosal carrier molecules, such as CTB, in plants may greatly improve the efficacy of edible plant vaccines (Haq et al, 1995; Thanavala et al, 1995; Mason et al, 1996) and may also provide novel oral tolerance agents for prevention of such autoimmune diseases as Type 1 diabetes (Zhang et al, 1991), Rheumatoid arthritis (Trentham et al, 1993), multiple sclerosis (Khoury et al, 1990; Miller et al, 1992; Weiner et al, 1993) as well as the prevention of allergic and allograft rejection reactions (Savegh et al, 1992; Hancock et al, 1993). Therefore, expressing a CTB-proinsulin fusion is an ideal approach for oral delivery of insulin.
**Chloroplast Genetic Engineering:** Several environmental problems related to plant genetic engineering now prohibit advancement of this technology and prevent realization of its full potential. One such common concern is the demonstrated escape of foreign genes through pollen dispersal from transgenic crop plants to their weedy relatives creating super weeds or causing gene pollution among other crops or toxicity of transgenic pollen to non-target insects such as butterflies. The high rates of gene flow from crops to wild relatives (as high as 38% in sunflower and 50% in strawberries) are certainly a serious concern. Clearly, maternal inheritance (lack of chloroplast DNA in pollen) of the herbicide resistance gene via chloroplast genetic engineering has been shown to be a practical solution to these problems (Daniell et al, 1998). Another common concern is the sub-optimal production of *Bacillus thuringiensis* (B.t.) insecticidal protein or reliance on a single (or similar) B.t. protein in commercial transgenic crops resulting in B.t. resistance among target pests. Clearly, different insecticidal proteins should be produced in lethal quantities to decrease the development of resistance. Such hyper-expression ofa novel B.t. protein in chloroplasts has resulted in 100% mortality of insects that are up to 40,000- fold resistant to other B.t. proteins (Kota et al. 1999). Therefore, chloroplast genome is an attractive target for expression of foreign genes due to its ability to express extraordinarily high levels of foreign proteins and efficient containment of foreign genes through maternal inheritance.

When we developed the concept of chloroplast genetic engineering (Daniell and McFadden, 1988 U.S. Patents; Daniell, World Patent, 1999). It was possible to introduce isolated intact chloroplasts into protoplasts and regenerate transgenic plants (Carlson, 1973). Therefore, early investigations on chloroplast transformation focused on the development of *in organello* systems using intact chloroplasts capable of efficient and prolonged transcription and translation (Daniell and Rebeiz, 1982; Daniell et al., 1983, 1986) and expression of foreign *genes* in isolated chloroplasts (Daniell and McFadden, 1987). However, after the discovery of the gene gun as a transformation device (Daniell, 1993), it was possible to transform plant chloroplasts without the use of isolated plastids and protoplasts. Chloroplast genetic engineering was accomplished in several phases. Transient expression of foreign genes in plastids of dicots (Daniell et al., 1990; Ye et al., 1990) was followed by such studies in monocots (Daniell et al., 1991). Unique to the chloroplast genetic engineering is the development of a foreign gene expression system using autonomously replicating chloroplast expression vectors (Daniell et al., 1990). Stable integration of a selectable marker gene into the tobacco chloroplast genome (Svab and Maliga, 1993) was also accomplished using the gene gun. However, useful genes conferring valuable traits via chloroplast genetic engineering have been demonstrated only recently. For example, plants resistant to B.t. sensitive insects were obtained by integrating the *crylAc gene* into the tobacco chloroplast genome (McBride et al., 1995). Plants resistant to B.t. resistant insects (up to 40,000 fold) were obtained by hyper-expression of the *cryilA* gene within the tobacco chloroplast genome (Kota et al., 1999). Plants have also been genetically engineered via the chloroplast genome to confer herbicide resistance and the introduced foreign genes were maternally inherited, overcoming the problem of cut-cross with weeds (Daniell et al., 1998). Chloroplast genetic engineering has also been used to produce pharmaceutical products that are not used by plants (Guda ct al., 2000). Chloroplast genetic engineering technology is currently being applied to other useful crops (Sidorov et al. 1999; Daniell. 1999).
**Polymer-proinsulin Recombinant DNA Vectors:** First we developed independent chloroplast vectors for the expression of insulin chains A and B as polymer fusion peptides, as it has been produced in *E. coli* for commercial purposes in the past. The disadvantage of this method is that *E. coli* does not form disulfide bridges in the cell unless the protein is targeted to the periplasm. Expensive *in vitro* assembly after purification is necessary for this approach. Therefore, a better approach is to express the human proinsulin as a polymer fusion protein. This method is better because chloroplasts are capable of forming disulfide bridges. Using a single gene, as opposed to the individual chains, eliminates the necessity of conducting two parallel vector construction processes, as is needed for individual chains. In addition, the need for individual fermentations and purification procedures is eliminated by the single gene method. Further, proinsulin products require less processing following extraction. Another benefit of using the proinsulin is that the C-peptide, which is an essential part the proinsulin protein, has recently been shown to play a positive role in diabetic patients (Ido et al, 1997).

Recently, the human pre-proinsulin gene was obtained from Genentech, Inc. First, the pre-proinsulin was sub-cloned into pUC19 to facilitate further manipulations. The next step was to design primers to make chloroplast expression vectors. Since we are interested in proinsulin expression, the 5' primer was designed to land on the proinsulin sequence. This FW primer eluded the 69 bases or 23 coded amino acids of the leader or pre-sequence of preproinsulin. Also, the forward primer included the enzymatic cleavage site for the protease factor Xa to avoid the use of cyanogen bromide. Beside the Xa-factor, a Smal site was introduced to facilitate subsequent subcloning. The order of the FW primer sequence is Smal - Xa-factor - Proinsulin gene. The reverse primer includes BamHI and XbaI sites, plus a short sequence with homolgy with the pUC19 sequence following the proinsulin gene. The 297bp PCR product (Xa Pris) includes three restriction sites, which are the Smal site at the 5'-end and XbaI/BamHI sites at the 3' end of the proinsulin gene. The Xa-Pris was cloned into pCR2.1 resulting in pCR2.1 - Xa- Pris (4.2kb). Insertion of Xa-Pris into the multiple cloning site of pCR2.1, resulted in additional flanking restriction enzyme sites that will be used in subsequent sub-cloning steps. A GVGVP 50-mer was generated as described previously (Daniell et al. 1997). The ribosome binding sequence was introduced by digesting pUCs-10, which contains the RBS sequence GAAGGAG, with Nool and Hind III flanking sites. The plasmid pUC19-50 was also digested with the same enzymes. The 50mer gene was eluted from the gel and ligated to pUCs-10 to produce pUCs-10-50mer. The ligation step inserted into the 50mer gene a RBS sequence and a Smal site outside the gene to facilitate subsequent fusion to proinsulin.

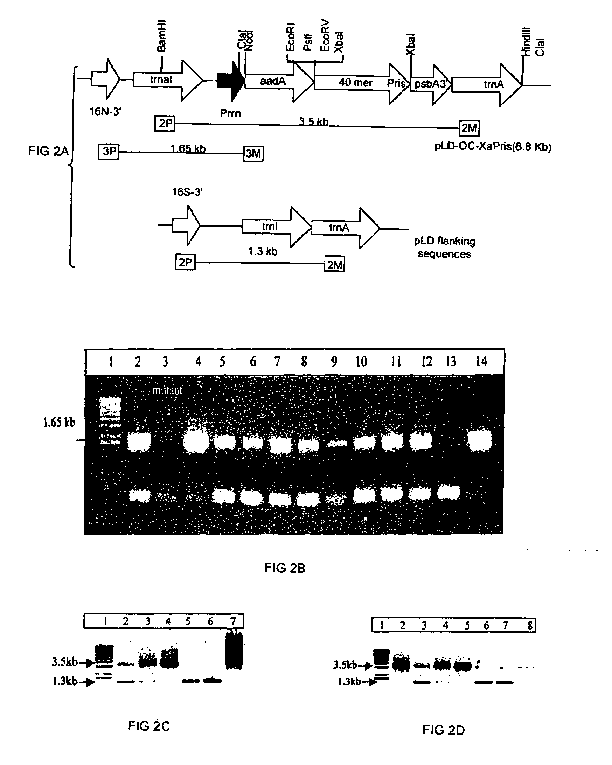

Another Smal partial digestion was performed to eliminate the stop codon of the biopolymer, transform the 50mer to a 40mer, and fuse the 40mer to the A-proinsulin sequence. The conditions for this partial digestion needed a decrease in DNA concentration and the 1:15 dilution of Smal. Once the correct fragment was obtained by the partial digestion of Smal (eliminating the stop codon but include the RBS site), it was ligated to the Xa-proinsulin fusion gene resulting in the construct pCR2.1-40-XaPris. Finally, the biopolymer (40mer) - proinsulin fusion gene was subcloned into pSBL-CtV2 (chloroplast vector) by digesting both vectors with Xbal. Then the fusion gene was ligated to the pSBL-CtV2 and the final vector was called pSBL-OC-XaPris. The orientation of the insert was checked with Nool: one the five colonies chosen had the correct orientation of the gene. The fusion gene was also subcloned into pLD-CtV vector and the orientation was checked with EooRI and Pvuil. One of the four colonies had the correct orientation of the insert. This vector was called pLD-OC-XaPris (Fig.2A).

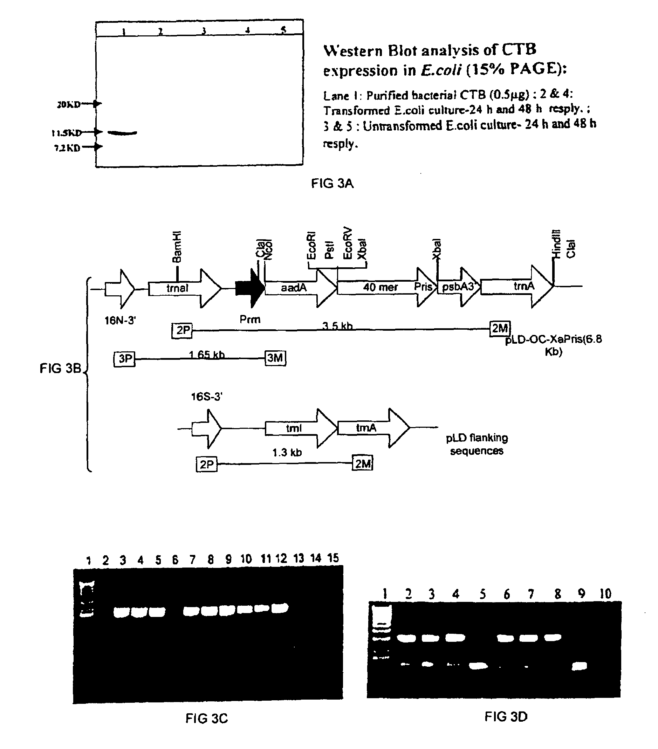

Both chloroplast vectors contain the 16S rRNA promoter (Prm) driving the selectable marker gene *aadA* (aminoglycoside adenyl transferase conferring resistance to spectinomycin) followed by the *psbA* 3' region (the terminator from a gene coding for photosystem II reaction center components) from the tobacco chloroplast genome. The only difference between these two chloroplast vectors (pSBL and pLD) is the origin of DNA fragments. Both pSBL andpLD are universal chloroplast expression/integration vectors and can be used to transform chloroplast genomes of several other plant species (Daniell et al. 1998) because these flanking sequences are highly conserved among higher plants. The universal vector uses *trnA.* and *trnl* genes (chloroplast transfer RNAs coding for Alanine and Isoleucine) from the inverted repeat region of the tobacco chloroplast genome as flanking sequences for homologous recombination as shown in Figs. 2A and 3B. Because the universal vector integrates foreign genes within the Inverted Repeat region of the chloroplast genome, it should double the copy number of insulin genes (from 5000 to 10,000 copies per cell in tobacco). Furthermore, it has been demonstrated that homoplasmy is achieved even in the first round of selection in tobacco probably because of the presence of a chloroplasts origin of replication within the flanking sequence in the universal vector (thereby providing more templates for integration). Because of these and several other reasons, foreign gene expression was shown to be much higher when the universal vector was used instead of the tobacco specific vector (Guda et al., 2000).

DNA sequence of the polymer-proinsulin fusion was determined to confirm the correct orientation of genes, in frame fusion and lack of stop codons in the recombinant DNA constructs. DNA sequencing was performed using a Perkin Elmer ABI prism 373 DNA sequencing system using a ABI Prism Dye Termination Cycle Sequencing Kit. The kit uses AmpliTaq DNA polymerase. Insertion sites at both ends were sequenced using primers for each strand. Expression of all chloroplast vectors was first tested in *E. coli* before their use in tobacco transformation because of the similarity of protein synthetic machinery (Brisey et al. 1997). For *Escherichia coli* expression XL-1 Blue strain was used. *E. coli* was transformed by standard CaCl₂ transformation procedure.
**Expression and Purification, of the Biopolymer-proinsulin fusion protein:** Terrific broth growth medium was inoculated with 40µl of Ampicillin (1 00mg/ml) and 40µl of the XL-1 Blue MRF To strain of *E*. *coli* containing pSBL-OC-XaPris plasmid. Similar inoculations were made for pLD-OC-XaPris and the negative controls, which included both plasmids containing the gene in the reverse orientation and the *E. coli* strain without any plasmid. Then, 24hr cultures were centrifuged at 13,000 rpm for 3 min. The pellets were resuspended in 500µl of autoclaved dH₂O and transferred to 6ml Falcon tubes. The resuspended pellet was sonicated, using a High Intensity Ultrasonic processor, for 15 sec at an amplitude of 40 and then 15 sec on ice to extract the fusion protein from cells. This sonication cycle was repeated 15 times. The sonicated samples were transferred to microcentrifuge tubes and centrifuged at 4°C at 10,000g for 10 min to purify the fusion protein. After centrifugation, the supernatant were transferred to microcentrifudge tubes and an equal volume of 2XTN buffer (100mM TrisHCI, pH 8, 100 mM NaCl) was added. Tubes were warmed at 42°C for 25 min to induce biopolymer aggregation. Then the fusion protein was recovered by centrifuging at 2,500 rpm at 42°C for 3 min. The recovered fusion protein was resuspended in 100µl of cold water. The purification process was repeated twice. Also, the fusion protein was recovered by using 6M Guanidine hydrochloride phosphate buffer, pH 7.0 (instead of water), to facilitate stability of insulin. New cultures were incubated for this step following the same procedure as described above, except that the pSBL-OC-XaPris expressing cells were incubated for 24,48 and 72 hrs, Cultures were centrifuged at 4,000 rpm for 12 min and the pellet was resuspended in 6M Guanidine hydrochloride phosphate buffer, pH 7.0, and then sonicated as described above. After sonication, samples were run in a 16.5% Tricine gel, transferred to the nitrocellulose membrane, and immunoblotting was performed the following day.

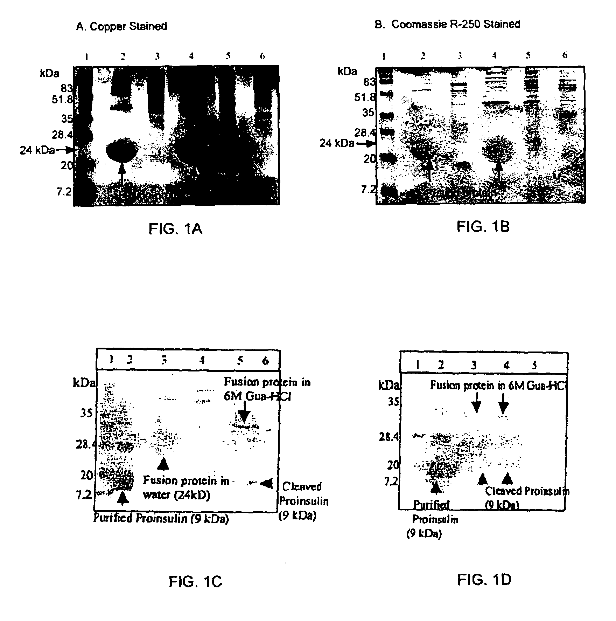

A 15°/ glycine gel was run for 6h at recommended voltage as shown in Fig.1. Two different methods of extraction were used. It was observed that when the sonic extract is in 6M Guanicine Hydrochloride Phosphate Buffer, pH7.0, the molecular weight changes from its original and correct MW 24 kD to a higher MW of approximately 30 kDa (FIG. 1C). This is probably due to the conformation that the biopolymer takes under this kind of buffer, which is used to maximize the extraction of proinsulin.

The gel was first stained with 0.3M CuCl₂, and then the same gel was stained with Commassie R-250 Staining Solution for an hour and then destained for 15 min first, and then overnight. CaCl₂ creates a negative stain (Lee et al. 1987). Polymer proteins (without fusion) appear as clear bands against a blue background in color or dark against a light semiopaque background (Fig. 1A). This stain was used because other protein stains such as Coomassie Blue R250 does not stain the polymer protein due to the lack of aromatic side chains (McPherson et aL, 1992). Therefore, the observation of the 24 kDa protein in R250 stained gel (Fig. 1B) is due to the insulin fusion with the polymer. This observation was further confirmed by probing these blots with the antihuman proinsulin antibody. As anticipated, the polymer insulin fusion protein was observed in western blots as shown in Fig. 1C, even though the binding of antibody was less efficient (probably due to concealment of insulin epitopes by the polymer). Larger proteins observed as shown in FIG. 1D are tetramer and hexamer complexes of proinsulin.

It is evident that the insulin-polyer fusion proteins are stable in *E. coli.* Confirming this observation, recently another lab has shown that the PBP polymer protein conjugates (with thioredoxin and tendamistat) undergo thermally reversible phase transition, retaining the transition behavior of the free polymer (Meyer and Chikoti, 1999). These results clearly demonstrate that insulin fusion has not affected the inverse temperature transition property of the polymer. One of the concerns is the stability of insulin at temperatures used for thermally reversible purification. Temperature induced production of human insulin has been in commercial use (Schmidt et al. 1999). Also, the temperature transition can be lowered by increasing the ionic strength of the solution during purification of this PSP (McPherson et al, 1996). Thus, GVGVP-fusion could be used to purify a multitude of economically important proteins in a simple inexpensive step.
**Biopolymer-proinsulin fusion gene expression in chloroplast:** As described in section d, pSBL-OC.R40XaPris vector and pLD-OGR40XaPris vectors were bombarded into the tobacco chloroplasts genome via particle bombardment (Daniell., 1997). PCR was performed to confirm biopolymer-proinsulin fusion gene integration into chloroplast genome. The PCR products were examined in 0.8% agarose gels. Fig. 2A shows primers landing sites and expected PCR products. Fig. 2B shows the 1.6 kbp PCR product, confirming integration of the aadA gene into the chloroplast genome. This 1.6kb product is seen in all clones except L9, which is a mutant. We used primers 2P and 2M to confirm integration of both the aadA and biopolymer-proinsulin fusion gene. The 1.3 kbp product corresponds to the native chloroplast fragment and the 3.5 kbp product corresponds to the chloroplast genome that has integrated all three genes as shown in Figs. 2C amd D. All the clones examined at this time show heteroplasmy, exce[t c;pmes :8d om Fog/ 2C, and S41b in Fig. 2D, which show almost homoplasmy.
**Protease Xa Digestion of the Biopolymer-proinsulin fusion protein and Purification of Proinsulin:** Factor Xa was purchased from New England Biolabs at a concentration of 1.0 mg/ml. The Factor Xa is supplied in 20mM HEPES, 500mM, NaCl, 2mM CaCl₂, 50% glycerol, (pH 8.0). The reaction was carried out in a 1:1 ratio of fusion protein to reaction buffer. The reaction buffer was made with 20mM Tris-HCl, 100mM NaCl, 2mM CaCl₂, (pH 8.0). The enzymatic cleavage of the fusion protein to release the proinsulin protein from the (GVVP)₄₀ was initiated by adding the protease to the purified fusion protein at a ratio (ww) of approximately 1,500. This digestion was continued for 5 days with mild stirring at 4°C. Cleavage of the fusion protein was monitored by SDS-PAGE analysis. After the cleavage, the same conditions are used for purification of the proinsulin protein. The purification steps are the same as for the purification of the fusion protein, except that instead of recovering the pellet, the supernatant is saved. We detected cleaved proinsulin in the extracts isolated in 6M guanidine hydrochloride buffer as shown in FIG. 1D. Conditions can be estimized for complete cleavage. The Xa protease has been successfully used to cleave (GVGVP)₂₀-GST fusion (McPherson et al. 1992). Therefore, cleavage of proinsulin from GVGVP using the Xa protease does not pose problems.
**Vector for CTB expression in chloroplasts**: The leader sequence (63 bp) of the native CTB gene (372 bp) was deleted and a start codon (ATC) introduced at the 5' end of the remaining CTB gene (309 bp). Primers were designed to introduce a rbs site 5 bases upstream of the start codon. The 5' primer (38mer) was designed to and on the start codon and the 5'-end of the CTB gene. This primer had an Xbal site at the 5'-end, the rbs site [GGAGG], a 5 bp breathing space followed by the first 20 bp of the CTB gene. The 3' primer (32mer) was designed to land on the 3' end of the CTB gene and it introduced restriction sites at the 3' end to facilitate subcloning. The 347 bp rCTB PCR product was subcloned into pCR2.1 resulting in pcCR2.1-rCTB. The final step was insertion of rCTB into the Xbal site of the universal or tobacco vector (pLB-CtV2) that allows the expression of the constructs in *E. coli* and chloroplasts. Restriction enzyme digestion of the pLD-LH-rCTB vector with BamH1 was performed to confirm the correct orientation of the inserted fragment in the vector.

Because of the similarity of protein synthetic machinery, expression of the chloroplast vector was tested in *E. coli* before its use in tobacco transformation. For *Escherichia coli* expression the XL-1 Blue MRFᵣₒ strain was used. *E. coli* was transformed by standard CaCl₂ transformation procedures. Transformed *E. coli* (24 hrs cultures and 48 hrs culture in 100ml TB with 100mg/ml ampicillin) and untransformed *E. coli* (24 hrs culture and 48 hrs culture in 100ml TB with 12.5mg/ml tetracycline) was then centrifuged at 10000 x g in a Beckman GS-15R centrifuge for 15 min. The pellet was washed with 200mM Tris-Cl twice and resuspended in 500µl extraction buffer (200mM Tris-Cl, pH8.0, 100mM NaCl; 10mM EDTA, 2mM PMSF) and then sonicated using the Autotune Series High Intensity Ultrasonic Processor. Then, 100µl aliquots of the sonicated transformed and untransformed cells [containing 50 - 100µg of crude protein extract as determined by Bradford protein assay (Bio-Rad Inc)] and purified CTB (Sigma C-9903) were boiled with 2X SDS sample buffer, and separated an a 15% SDS-PAGE gel in Tris-glycine buffer (25mM Tris, 250 mM glycine. pH8.3, 0.1% SDS). The separated protein was then transferred to a nitrocellulose membrane by electro blotting using the Trans-Blot Electrophoretic Transfer Cell (Bio-Rad Inc.). **Immunoblot detection of CTB expression in** *E. coli*: Nonspecific antibody reactions were blocked by incubation of the membrane in 25ml of 5% non-fat dry milk in TBS buffer for 1 - 3 hrs on a rotary shaker (40 rpm), followed by washing in TBS buffer for 5 min. The membrane was then incubated for an hour with gentle agitation in 30 ml of a 1:5000 dilution of rabbit anti-cholera antiserum (Sigma C-3062) in TBS with Tween-20 [TBST] (containing 1% non-fat dry milk) followed by washing 3 times in TBST buffer. The membrane was incubated for an hour at room temperature with gentle agitation in 30 ml of a 1:10000 dilution of mouse anti-rabbit 1gG conjugated with alkaline phosphatase in TBST. It was then washed thrice with TBST and once with TBS followed by incubation in the Alkaline Phosphatase Color Development Reagents, BCIP/NBT in AP color development Buffer (Bio-Rad, Inc.) for an hour. Immunoblot analysis snows the presence of 11.5 kDa polypeptide for purified bacterial CTB and transformed 24h/48h cultures (Fig. 3A, lanes 2, 3 and 5). The 48h culture appears to express more CTB than that of the 24h culture indicating the accumulation of the CTB protein over time. The purified bacterial CTB (45 Kda) dissociated into monomers (11.5 KDa each) due to boiling prior to SDS PAGE. These results indicate that the pLD-LH-CTB vector is expressed in *E. coli.* Because of the similarity of the *E. coli* protein synthetic machinery to that of chloroplasts, chloroplast expression of the above vector should be possible.
**CTB expression In chloroplasts:** As described below, pLD-LH-CTB was integrated into the tobacco chloroplast genome via particle bombardment (Daniell, 1997). PCR analysis was performed to confirm chloroplast integration. Fig. 3B shows primer landing sites and size of expected products. PCR analysis of clones obtained after the first round of selection was carried out as described below. PCR products were examined on 0.8% agarose gels. The PCR results (Fig. 3C) show that clones I and 5 that do not show any product are mutants while clones 2, 3, 4, 6, 7, 8, 9, 10 and 11 that gave a 1.65 kbp product are transgenic. As expected, lanes 13 - 15 did not give any PCR product, confirming that the PCR reaction was not contaminated. Because primers 3P & 3M land on the aadA gene and on the chloroplast genome, all clones that show PCR products have integrated the CTB gene and the selectable marker into the chloroplast genome. Clones that showed chloroplast integration of the CTB gene were moved to the second round of selection to increase copy number. PCR analysis of clones obtained after the second round of selection was also carried out. PCR results shown in Fig. 3D indicate that clone 5 does not give a 3 kbp product indicating that it is a mutant as observed earlier. Other clones give a strong 3 kbp product and a faint 1.3 kbp (similar to the 1.3 kbp untransformed plant product) product, indicating that they are transgenic but not yet homoplasmic. Complete homoplasmy can be accomplished by several more rounds of selection or by germinating seeds from transgenic plants on 500 µg/ml of spectinomycin.
**CTB-Proinsulin Vector Construction:** The chloroplast expression vectorpLD-CTB-Proins was constructed as follows. First, both proinsulin and cholera toxin B-subunit genes were amplified from suitable DNA using primer sequences. Primer 1 contains the GGAGG chloroplast preferred ribosome binding site five nucleotides upstream of the start codon (ATG) for the CTB gene and a suitable restriction enzyme site (Spel) for insertion into the chloroplast vector. Primer 2 eliminates the stop codon and adds the first two amino acids of a flexible hinge tetrapeptide GPGP as reported by Bergerot et al. (1997), in order to facilitate folding of the CTB-proinsulin fusion protein. Primer 3 adds the remaining two amino acids for the hinge tetra-peptide and eliminates the pre-sequence of the pre-proinsulin. Primer 4 adds a suitable restriction site (Spel) for subcloning into the chloroplast vector. Amplified PCR products were inserted into the TA cloning vector. Both the CTB and proinsulin PCR fragments were excised at the Smal and Xbal restriction sites. Eluted fragments were ligated into the TA cloning vector. Interestingly, all white colonies showed the wrong orientation for CTB insert while three of the five blue colonies examined showed the right orientation of the CTB insert. The CTB-proinsulin fragment was excised at the EcoR1 sites and inserted into EcoR1 digested dephosphorolated pLD vector. Resultant onicroplast integration expression vector, pLD-CTB-Proins will be tested for expression in *E. coli* by western blots, After confirmation of expression of CTB-proinsulin fusion in *E. coli,* pLD-CTB-Proins will be bombarded into tobacco cells as described below.
**Optimization of fusion gene expression:** It has been reported that foreign genes are expressed between 5% (crylAC, cryllA) and 30% (uldA) in transgenic chloroplasts (Daniell, 1999). If the expression levels of the CTB-Proinsulin or polymer-proinsulin fusion proteins are low, several approaches will be used to enhance translation of these proteins. In chloroplast, transcriptional regulation of gene expression is less important, although some modulations by light and developmental conditions are observed (Cohen and Mayfield, 1997). RNA and protein stability appear to be less important because of observation of large accumulation of foreign proteins (e.g. GUS up to 30% of total protein) and tps1 transcripts 16,966-fold higher than the highly expressing nuclear transgenic plants. Chloroplast gene expression is regulated to a large extent at the post-transcriptional level. For example, 5' UTRs are used for optional translation of chloroplast mRNAs. Shine-Delgamo (GGAGG) sequences as well as a stem-loop structure located 5' adjacent to the SD sequence are used for efficient translation. A recent study has shown that insertion of the psbA 5' UTR downstream of the 16S rRNA promoter enhanced translation of a foreign gene (GUS) hundred-fold (Eibl et al. 1999). Therefore, the 85-bp tobacco chloroplast DNA fragment (1595 - 1680) containing 5' psbA UTR will be amplified using the following primers cctttaaaaagccttccattttctattt, gccatggtaaaatctfggtttatta This PCR product will be inserted downstream of the 16S rRNA promoter to enhance translation of the proinsulin fusion proteins.

Yet another approach for enhancement of translation is to optimize codon compositions of these fusion protein. Since both fusion proteins are expressed well in *E. coli,* we expected efficient expression in chloroplasts. However, optimizing codon compositions of proinsulin and CTB genes to march the psbA gene could further enhance the level of translation. Although rbcL (RuBisCO) is the most abundant protein on earth, it is not translated as frequently as the psbA gene due to the extremely high turnover of the psbA gene product. The psbA gene is under stronger selection for increased translation efficiency and is the most abundant thylakoid protein. In addition, codon usage in higher plant chloroplasts is biased towards the NNC codon of 2-fold degenerate groups (i.e. TTC over TTT, GAC over GAT, CAC over CAT, AAC over AAT, ATC over ATT, ATA etc.). This is in addition to a strong bias towards T at third position of 4-fold degenerate groups. There is also a context effect that should be taken into consideration while modifying specific codons. The 2-fold degenerate sites immediately upstream from a GNN codon do not show this bias towards NNC, (TTT GGA is preferred to TTC GGA while TTC CGT is preferred to TTT CGT TTC AGT to TTT AGT and TTC TCT to TTT TCT). In addition, highly expressed chloroplast genes use GNN more frequently than other genes. The web site may be used optimize codon composition by comparing different species. Abundance of amino acids in chloroplasts can be taken into consideration (pathways compartmentalized in plastids as opposed to those that are imported into plastids).

As far as the biopolymer gene is concerned, we observed incomplete translation products in plastids when we expressed the 120mer gene (Guda et al. 2000). Therefore, while expressing the polymer-proinsulin fusion protein, we decreased the length of the polymer protein to 40mer, without losing the thermal responsive property. In addition, optimal codons for glycine (GGT) and valine (GTA), which constitute 80% of the total amino acids of the polymer, have been used. In all nuclear encoded genes glycine make up 147/1000 amino acids while in tobacco chloroplasts it is 129/1000. Highly expressing genes like psbA and rbcL of tobacco make up 192 and 190 gly/1000. Therefore, glycine may not be a limiting factor. Nuclear genes use 52/1000 proline as opposed to 42/1000 in chloroplasts. However, currently used codon for proline (CCG) can be modified to CCA or CCT to further enhance translation. It is know that pathways for proline and valine are compartmentalized in chloroplasts (Guda et al. 2000). Also, proline is known to accumulate in chloroplasts as an osmoprotectant (Daniell et al. 1994).
**Bombardment and Regeneration of Chloroplast Transgenic Plants:** Tobacco (*Nicotiana tabacum* var. Petit Havana) and nicotine free edible tobacco (LAMD 665, gift from Dr. Keith Wycoff. Planet Biotechnology) plants are grown aseptically by germination of seeds on MSO medium. THis medium contains MS salts (4.3 g/liter), B5 vitamin mixture (myo-inositol, 100mg/liter, thiamine-HCl. 10mg/liter nicotinic acid 1 mg/liter; pyridoxine-HCL. 1 mg/liter), sucrose (30 g/liter) and phytagar (6 g/liter) at pH 5.8. Fully expanded, dark green leaves of about two month old plants are used for bombardment

Leaves are placed abaxial side up on a Whatman No. 1 filter paper laying on the RMOP medium (Daniell, 1993) in standard petri plates (100x15 mm) for bombardment Tungsten (1 µm) or Gold (0.6 µm) microprojectiles are coated with plasmid DNA (chloroplast vectors) and bombardments carried out with the biolistic device PDS1000/He (Bio-Rad) as described by Daniell (1997). Following bombardment, petri plates are sealed with parafilm and incubated at 24°C under 12 h photoperiod. Two days after bombardment, leaves are chopped into small pieces of~5 mm² in size and placed on the selection medium (RMOP containing 500 µg/ml of spectinomycin dihydrochloride) with abaxial side touching the medium in deep (100x25 mm) petri plates (∼10 pieces per plate). The regenerated spectinomycin resistant shoots are chopped into small pieces (~2mm²) and subcloned into fresh deep petri plates (~5 pieces per plate) containing the same selection medium. Resistant shoots from the second culture cycle arbe transferred to the rooting medium (MSO medium supplemented with IBA. 1 mg/liter and spectinomycin dihydrochloride, 500 mg/liter). Rooted plants are transferred to soil and grown at 26°C under continuous lighting conditions for further analysis.
**Polymerase Chain Reaction:** PCR is performed using DNA solated from control and transgenic plants to distinguish a) true chloroplast transformants from mutants and b) chloroplast transformants from nuclear transformants. Primers for testing the presence of the aadA gene (that confers spectinomycin resistance) in transgenic pants are landed on the aadA coding sequence and 16S rRNA gene (primers 1P&1M.). To test chloroplast integration of the insulin gene, one primer lands on the aadA gene, while another lands on the native chloroplast genome (primers 3P&3M) as shown in Figs. 2A and 3B. No PCR product is obtained with nuclear transgenic plants using this set of primers. The primer set (2P & 2M, in Figs. 2A and 3B) is used to test integration of the entire gene cassette without internal deletion or looping out during homologous recombination. A similar strategy has been used successfully to confirm chloroplast integration of foreign genes (Daniell et al., 1998; Kota et al, 1999; Guda et al., 1999). This screening is essential to eliminate mutants and nuclear transformants.

Total DNA from unbombarded and transgenic plants is isolated as described by Edwards et al., (1991) to conduct PCR analyses in transgenic plants. PCR reactions are performed in a total volume of 50 µl containing approximately 10 ng of template DNA and 1 µM of each primer in a mixture of 300 µM of each deoxynucleotide (dNTPs), 200 mM Tris (pH 8.8), 100 mM KCl, 100 mM (NH₄)₂SO₄, 20 mM MgSO₄, 1% Triton X-100, 1 mg/ml nuclease-free BSA and 1 or 2 units of Taq Plus polymerase (Stratagene, La Jolla, CA). PCR is carried out in the Perkin Elmer's GeneAmp PCR system 2400, by subjecting the samples to 94°C for 5 min and 30 cycles of 94°C for 1 min, 55°C for 1.5 min, 72°C for 1.5 or 2 min followed by a 72°C step for 7 min. PCR products are analyzed by electrophoresis on 0.8% agarose gels. Chloroplast transgenic plants containing the proinsulin gene are then moved to second round of selection to achieve homoplasmy. Southern Blot Analysis: Southern blots are performed to determine the copy number of the introduced foreign gene per cell as well as to test homoplasmy. There are several thousand copies of the chloroplast genome present in each plant cell. Therefore, when foreign genes are inserted into the chloroplast genome, it is possible that some of the chloroplast genomes have foreign genes integrated while others remain as the wild type (heteroplasmy). Therefore, to ensure that only the transformed genome exists in cells of transgenic plants (homoplasmy), the selection process is continued. To confirm that the wild type genome does not exist at the end of the selection cycle, total DNA from transgenic plants should be probed with the chloroplast border (flanking) sequences (the trnl-trnA fragment, Figs. 2A and 3B). If wild type genomes are present (heteroplasmy), the native fragment size is observed along with transformed genomes. Presence of a large fragment (due to insertion of foreign genes within the flanking sequences) and absence of the native small fragment confirms homoplasmy (Daniell et al., 1998; Kota et al., 1999; Guda et al., 1999).

The copy number of the integrated gene is determined by establishing homoplasmy form the transgenic chloroplast genome. Tobacco chloroplasts contain 5000~10,000 copies of their genome per cell (Daniell et al., 1998). If only a fraction of the genomes are actually transformed, the copy number, by default, must be less than 10,000. By establishing that in the trangenics the insulin inserted transformed genome is the only one present, one can establish that the copy number is 5000~10,000 per cell. This is usually achieved by digesting the total DNA with a suitable restriction enzyme and probing with the flanking sequences that enable homologous recombination into the chloroplast genome. The native fragment present in the control should be absent in the transgenics. The absence of native fragment proves that only the Transgenic chloroplast genome is present in the cell and there is no native, untransformed, chloroplast genome, without the insulin gene present. This establishes the homoplasmic nature of the transformants, simultaneously, thereby providing an estimate of 5000~10,000 copies of the foreign genes per cell.

Total DNA is extracted from leaves of transformed and wild type plants using the CTAB procedure outlined by Rogers and Bendich (1988). Total DNA is digested with suitable restriction enzymes, electrophoresed on 0.7% agarose gels and transferred to nylon membranes (Micron Separation Inc., Westboro, MA). Probes are labeled with ³²P-dCTP using the random-primed procedure (Promega). Pre-hybridization and hybridization steps are carried out at 42°C for 2 h and 16 h, respectively. Blots are soaked in a solution containing 2X SSC and 0.5% SDS for 5 min followed by transfer to 2X SSC and 0.1% SDS solution for 15 min at room temperature. Then, blots are incubated in hybridization bottles containing 0.1X SSC and 0.5% SDS solution for 30 min at 37°C followed by another step at 68°C for 30 min, with gentle agitation. Finally, blots are briefly rinsed in 0.1X SSC solution, dried and exposed to X-ray film in the dark.
**Northern Blot Analysis:** Northern blots are performed to test the efficiency of transcription of the proinsulin gene fused with CTB or polymer genes. Total RNA is isolated from 150 mg of frozen leaves by using the "Rneasy Plant Total RNA Isolation Kit" (Qiagen Inc., Chatsworth, CA). RNA (10 - 40 mg) is denatured by formaldehyde treatment, separated on a 1.2% agarose gel in the presence of formaldehyde and transferred to a nitrocellulose membrane (MSI) as described in Sambrook et al. (1989). Probe DNA (proinsulin gene coding region) is labeled by the random-primed method (Promega) with ³²P-dCT isotope. The blot is pre-hybridized, hybridized and washed as described above for southern blot analysis. Transcript levels are quantified by the Molecular Analyst Program using the GS-700 Imaging Densitometer (Bio-Rad, Hercules, CA).
**Polymer-insulin fusion protein purification, quantitation and characterization:** Because polymer insulin fusion proteins exhibit inverse temperature transition properties as shown in Figs. 1A and B, they are purified from transgenic plants essentially following the same method for polymer purification from transgenic tobacco plants (Zhang et al., 1996). However, an additional step is introduced to take advantage of the compartmentalization of insulin polymer fusion protein within chloroplasts. Chloroplasts are first isolated from crude homogenate of leaves by a simple centrifugation step at 1500Xg. This eliminates most of the cellular organelles and proteins (Daniell at al., 1983, 1986). Then, chloroplasts are burst open by resuspending them in a hypotonic buffer (osmotic shock). This is a significant advantage because there are fewer soluble proteins inside chloroplasts when compared to hundreds of soluble proteins in the cytosoL Polymer extraction buffer contains 50 mMTris-HCl, pH 7.5, 1% 2-mecaptoethanol, 5mM EDTA and 2mM PMSF and 0.8 M NaCl. The homogenate is then centrifuged at 10,000 g for 10 min (4°C), and the pellet discarded. Then supernatant is incubated at 42°C for 30 minutes and then centrifuged immediately for 3 minutes at 5,000 g (room temperature). If insulin is found to be sensitive to this temperature, T₁ is lowered by increasing salt concentration (McPherson et al., 1996). The pellet containing the insulin-polymer fusion protein is resuspended in the extraction buffer and incubated on ice for 10 minutes. The mixture is centrifuged at 12,000 g for 10 minute (4°C). The supernatant is then collected and stored at -20°C. The purified polymer insulin fusion-protein is electrophoresed in a SDS-PAGE gel according to Laemml (1970) and visualized by either staining with 0.3 M CuCl₂ (Lee et al., 1987) or transferred to nitrocellulose membrane and probed with antiserum raised against the polymer or insulin protein as described below. Quantification of purified polymer proteins may then be carried out by densitometry.

After electrophoresis, proteins are transferred to a nitrocellulose membrane electrophoretically in 25 mM Tris, 192mM glycine, 5% methanol (pH 8.3). The filter is blocked with 2% dry milk in Tris-buffered saline for two hours at room temperature and stained with antiserum raised against the polymer AVGVP (kindly provided by the University of Alabama at Birmingham, monoclonal facility) overnight in 2% dry milk/Tris buffered saline. The protein bands reacting to the antibodies are visualized using alkaline phosphatase-linked secondary antibody and the substrates nitroblue tetrazolium and 5-bromo-4-chloro-3-indolyl-phosphate (Bio-Rad). Alternatively, for insulin-polymer fusion proteins, a Mouse anti-human proinsulin (IgGl) monoclonal antibody is used as a primary antibody. To detect the binding of the primary antibody to the recombinant proinsulin, a Goat anti-mouse IgG Horseradish Peroxidase Labeled monoclonal antibody (HPR) is used. The substrate used for conjugation with HPR is 3,3', 5,5'-Tetramethylbenzidine. All products are available from American Qualex Antibodies, San Clemente, CA. As a positive control, human recombinant proinsulin from Sigma may be used. This human recombinant proinsulin was expressed in *E. coli* by a synthetic proinsulin gene. Quantification of purified polymer fusion proteins is carried out by densitometry using Scanning Analysis software (BioSoft, Ferguson, MO) installed on a Macintosh LC III computer (Apple Computer, Cupertino, USA) with a 160-Mb hard disk operating on a System 7.1, connected by SCSI interface to a Relisys RELI 2412 Scanner (Relisys, Milpitas, CA). Total protein contents is then determined by the dye-binding assay using reagents supplied in kit fro Bio-Rad, with bovine serum albumin as a standard.
**Characterization of CTB expression:** CTB protein levels in transgenic plants are determined using quantitative ELISA assays. A standard curve is generated using known concentrations of bacterial CTB. A 96-well microtiter plate padded with 100 µl/well of bacterial CTB (concentrations in the range of 10 - 1000 ng) is incubated overnight at 4°C. The plate is washed thrice with PBST (phosphate buffered saline containing 0.05% Tween-20). The background is blocked by incubation in 1% bovine serum albumin (BSA) in PBS (300 l/well) at 37°C for 2 h followed by washing 3 times with PBST. The plate is incubated in a 1:8,000 dilution of rabbit anti-cholera toxin antibody (Sigma C-3062) (100 µl/well) for 2 h at 37°C, followed by washing the wells three times with PBST. The plate is incubated with a 1:80,000 dilution of anti-rabbit IgG conjugated with alkaline phoshatase (100 µl/well) for 2 h at 37°C and washed thrice with PBST. Then, 100 µl alkaline phosphatase substrate (Sigma Fast p-nitrophenyl phosphate tablet in 5 ml of water is added and the reaction stopped with 1M NaOH (50 µl/well) when absorbancies in the mid-range of the nitration reach about 2.0, or after 1 hour, whichever comes first. The plate is then read at 405nm. These results are used to generate a standard curve from which concentrations of plant protein can be extrapolated. Thus, total soluble plant protein (concentration previously determined using the Bradford assay) in bicarbonate buffer, pH 9.6 (15 nM Na₂Co₃, 35mM NaHCO₃) is loaded at 100 plant µl/well and the same procedure as above can be repeated. The absorbance values are used to determine the ratio of CTB protein to total soluble plant protein, using the standard curve generated previously and the Bradford assay results. **Inheritance of Introduced Foreign Genes:** In initial tobacco transformants, some are allowed to self-pollinate, whereas others are used in reciprocal crosses with control tobacco (transgenics as female acceptors and pollen donors: testing for maternal inheritance). Harvested seeds (T1) are germinated on media containing spectinomycin. Achievement of homoplasmy and mode of inheritance can be classified by looking at germination results. Homoplasmy is indicated by totally green seedlings (Daniell et al., 1998) while heteroplasmy is displayed by variegated leaves (lack of pigmentation, Svab & Maliga, 1993). Lack of variation in chlorophyll pigmentation among progeny also underscores the absence of position effect, an artifact of nuclear transformation. Maternal inheritance may be demonstrated by scie transmission of introduced genes via seed generated on transgenic plants, regardless of pollen source (green seedlings on selective media). When transgenic pollen is used for pollination of control plants, resultant progeny does not contain resistance to chemical in selective media (will appear bleached; Svab and Maliga, 1993). Molecular analyses confirms transmission and expression of introduced genes, and T2 seed is generated from those confirmed plants by the analyses described above.
**Comparison of Current Purification with Polymer-based Purification Methods:** It is important to compare purification methods to test yield and purity of insulin produced in *E. coli* and tobacco. One liter of pSBL containing bacteria is grown in LB/ampicillin (100 µg/ml) overnight and the fusion protein expressed. Cells are harvested by centrifugation at 5000 X g for 10 min at 4°C, and the bacterial pellets resuspended in 5 ml/g (wet wt. Bacteria) of 100 mM Tris-HCl, pH 7.3. Lysozyme is added at a concentration of 1 mg/ml and placed on a rotating shaker at room temperature for 15 min. The lysate is subjected to probe sonication for two cycles of 30 s on/30 s off at 4°C. Cellular debris is removed by centrifugation at 1000 X g for 5 min at 4°C. Insulin polymer fusion protein is purified by inverse temperature transition properties (Daniell et al., 1997). Alternatively, the fusion protein is purified according to Cowley and Mackin (1997). The supernatant is retained and centrifuged again at 27000 X g for 15 min at 4°C to pellet the inclusion bodies. The supernatant is discarded and the pellet resuspended in 1 ml/g (original wt. Bacteria) of dH₂O, aliquoted into microcentrifuge tubes as 1 ml fractions, and then centrifuged at 16000 X g for 5 min at 4°C. The pellets are individually washed with 1 ml of 100 mM Tris-HCl, pH 8.5, 1M urea, 1-1 Triton X-100 and again washed with 100 mM Tris HCl pH8.5,2 M urea, 2% Trinton X-100. The pellets are resuspended in 1 ml of dH₂O and transferred to a pre-weighted 30 ml Corex centrifuge tube. The sample is centrifuged at 15000 X g for 5 min at 4°C, and the pellet resuspended in 10 ml/g (wet wt. pellet) of 70% formic acid. Cyanogen bromide is added to a final concentration of 400 mM and the sample incubated at room temperature in the dark for 16 h. The reaction is stopped by transferring the sample to a round bottom flask and removing the solvent by rotary evaporation at 50°C. The residue is resuspended in 20 ml/g (wet wt. pellet) of dH₂O, shell frozen in a dry ice ethanol bath, and then lyophilized. The lyophilized protein is dissolved in 20 ml/g (wet wt. pellet) of 500 mM Tris-HCl, pH 8.2, 7 M urea. Oxidative sulfitolysis is performed by adding sodium sulfite and sodium tetrathionate to final concentrations of 100 and 10 mM, respectively, and incubating at room temperature for 3 h. This reaction is then stopped by freezing on dry ice.
**Purification and folding of Human Proinsulin:** The S-sulfonated material is applied to a 2 ml bed of Sephadex G-25 equilibrated in 20 mM Tris-HCl, pH 8.2, 7 M urea, and then washed with 9 vols of 7 M urea. The collected fraction is then applied to a Pharmacia Mono Q HR 5/5 column equilibrated in 20 mM Tris-HCl, pH 8.2, 7 M urea at a flow rate of 1 ml/min. A linear gradient leading to final concentration of 0.5 M NaCl is used to elute the bound material. 2 min (2 ml) fractions are collected during the gradient, and protein concentration in each fraction determined. Purity and molecular mass of fractions are estimated by Tricine SDS-PAGE (as shown in Fig. 2), where Tricine is used as the trailing ion to allow better resolution of peptides in the range of 1-1000 kDa. Appropriate fractions are pooled and applied to a 1.6 X 20 cm column of Sephadex G-25 (superfine) equilibrated in 5 mM ammonium acetate pH 6.8. The sample is collected based on UV absorbance and freeze-dried. The partially purified S-sulfonated material is resuspended in 50 mM glycine/NaOH, pH 10.5 at a final concentration of 2 mg/ml. β-mer-captoethanol is added at a ratio of 1.5 mol per mol of cysteine S-sulfonate and the sample stirred at 4°C in an open container for 16 h. The sample is then analyzed by reversed-phase high-performance liquid chromatography (RP-HPLC) using a Vydac C₄ column (2.2 X 150 mm) equilibrated in 4% acetonitrile and 0.1% TFA. Adsorbed peptides are eluted with a linear gradient of increasing acetonitrite concentration (0.88% per min up to a maximum of 48%). The remaining refolded proinsulin are centrifuged at 16000 X g to remove insoluble material, and loaded onto a semi-preparative Vydad C₄ column (10 X 250 mm). The bound material is then eluted as described above, and the proinsulin collected and lyophilized.
Analysis and characterization of insulin expressed in *E. coli* and Tobacco: The purified expressed proinsulin is subjected to matrix-assisted laser desorption/ionization-time of flight (MALDI-TCF) analysis (as described by Cowley and Mackin, 1997), using proinsulin from Eli Lilly as both an internal and external standard. A proteolytic digestion is performed using *Staphylococcus aureus* protease V8 to determine if the disulfide bridges have formed correctly naturally inside chloroplasts or by *in vitro* processing. Five µg of both the expressed proinsulin and Eli Lilly's proinsulin are lyophilized and resuspended in 50 µl of 250 mM NaPO₄ pH 7.8. Protease V8 is added at a ratio of 1:50 (w/w) in experimental samples and no enzyme added to the controls. All samples are then incubated overnight at 37°C, the reactions stopped by freezing on dry ice, and samples stored at -20°C until analyzed. The samples are analyzed by RP-HPLC using a Vydac C₄ column (2.2 X 150 mm) equilibrated in 4% acetonitrite and 0.1% TFA. Bound material is then eluted using a linear gradient of increasing acetonitrile concentration (0.88% per min up to a maximum of 48%).
**CTB-GM1 ganglioside binding assay:** A GM1-ELISA assay is performed as described by Arakawa et al. (1997) to determine the affinity of plant-derived CTB for GM1-ganglioside. The microtiter plate is coated with monosialogangliosice-GM1 (Sigma G-7641) by incubating the plate with 100 µl/well of GM1 (3.0 µg/ml) in bicarbonate buffer, pH 9.6 at 4°C overnight. Alternatively, the wells are coated with 100 µl/well of BSA (3.0 µg/ml) as control. The plates are incubated with transformed plant total soluble protein and bacterial CTB (Sigma C-9903) in PBS (100 µl/well) overnight at 4°C. The remainder of the procedure is then identical to the USA described above.
**Mouse feeding assays for CTB:** This is performed as described by Haq et al. (1995). BALB/c mice, divided into groups of five animals each, are fasted overnight before feeding them transformed edible tobacco (that tastes like spinach) expressing CTB, untransformed edible tobacco and purified bacterial CTB. Feedings are performed at weekly intervals (0, 7, 14 days) for three weeks. Animals are observed to confirm complete consumption of material. On day 20, fecal and serum samples are collected from each animal for analysis of anti-CTB antibodies. Mice are bled retro-orbitally and the samples stored at -20°C until assayed. Fecal samples are collected and frozen overnight at -70°C, lyophilized, resuspended in 0.8 ml PBS (pH7.2) containing 0.05% sodium azide per 15 fecal pellets, centrifuged at 1400xg for 5 min and the supernatant stored at -20°C until assayed. Samples are then serially diluted in PBS containing 0.05% Tween-20 (PBST) and assayed for anti-CTB IgG in serum and anti-CTB IgA in fecal pellets by the ELISA method, as described earlier.
Assessment of diabetic symptoms in NOD mice: The incidence of diabetic symptoms is compared among mice fed with control nicotine free edible tobacco and those that express the CTB-proinsulin fusion protein. Four week old female NOD mice are divided into two groups, each group consisting of ten mice. Each group is fed with control or transgenic edible tobacco (nicotine free) expressing the CTB-proinsulin fusion gene. The feeding dosage is determined based on the level of expression. Starting at 10 weeks of age, the mice are monitored on a biweekly basis with urinary glucose test strips (Clinistix and Diastix, Bayer) for development of diabetes. Glycosuric mice are bled from the tail vein to check for glycemia using a glucose analyzer (Accu-Check, Boehringer Mannheim). Diabetes is confirmed by hyperglycemia (>250 mg/dl) for two consecutive weeks (Ma et al., 1997).

### References to Project Description

Arakawa T. YuJ. Chong, DKX, Hough J, Engen PC, Langridge WHR (1998) A plant-based cholera toxin B subunit-insulin fusion protein protects against the development of autoimmune diabetes. Nature Biotechnology. 16:934-938.
Arakawa T. Chong DKX. Merritt JL. Langridge WHR (1997) Expression of cholera toxin B subunit oligomers in transgenic potato plants. Transgenic Research 6:403-413.
Arntzen CJ., Mason H.S., et al (1998) Edible vaccine protects mice against E. coli heat labile enterotoxin: potatoes expressing a synthetic LTB gene. Vaccine. 16(13):133 6-1343
Bendich AJ (1987) Why do chloroplasts and mitochondria contain so many copies of their genome? Bioassays 6:279-282.
Bergerot I, Ploix C, Peterson J, Moulin V, Rask C, Fabien N, et al. (1997) A cholera toxoid-insulin conjugate as an oral vaccine against spontaneous autoimmune diabetes. Proc. Natl. Acad. Sci. USA. 94:4610-4614.
Burnette JP (1983) Experimental Manipulation of Gene Expression. Oxender, D.L., Fox, C.F., ets. Pp. 71-82. Alan R. Liss, Inc., New York, NY
Brixey J, Guda C, Daniell H (1997) The chloroplast psbA promoter is more efficient in E. coli than the T7 promoter for hyper expression of a foreign protein. Biotechnology Letters 19:395-400.
Carlson PS (1973) The use of protoplasts for genetic research. Proc. Natl. Acad. Sci. USA 70:598-602
Chance R.E. Frank BH (1993) Research, development, production and safety of biosynthetic human insulin. Diabetes Care. 16(3):133-142.
Cohen A. Mayfield (1997) Translational regulation of gene expression in plants. Current Opinion in Biotechnology 8: 189-194.
Cowley DJ. Mackin RB (1997) Expression, purification and characterization of recombinant human proinsulin. FEBS Letts. 402: 124-130.
Crea R. Kraszewski A. Kirose T, Itakura K (1978) Chemical synthesis of genes for human insulin. Proc. Natl. Acad. Sci. 75(12): 5765-5769.
Daniell H. (1993) Foreign gene expression in chloroplasts of higher plants mediated by rungsten particle bombardment. Methods Enzymol 217:536-556.
Daniell H. (1995) Producing polymers in plants and bacteria. Inform 6-1365-1370.
Daniell H. (1997) Transformation and foreign gene expression in plants mediated by microprojectile bombardment. Meth. Mol. Biol 62:453-488
Daniell H. (1999) Universal chloroplast integration and expression vectors, transformed plants and products thereof, World Intellectual Property Organization WO 99:10513.
Daniell H. Datta R. Varma S. Gray S. Lee SB (1998) Containment of herbicide resistance through genetic engineering of the chloroplast genome. Nature Biotechnology 16:345-348.
Daniell H. Guda C. (1997) Biopolymer production in microorganisms and plants. Chemistry and Industry, 14:555-560.
Daniell H. Guda C. McPherson DT, XU J. Zhang X. Urry DW (1997) Hyper expression of an environmentally friendly synthetic polymer gene. Meth Mol Biol 63:359-371.
Daniell H. Krishnan M. McFadden DA (1991) Expression of H-glucuronidase gene in different cellular compartments following biolistic delivery of foreign DNA into wheat leaves and calli. Plant Cell Reports 9:615-619.
Daniell H. Krishnan M. Umabai U. Gnanam A (1986) An efficient and prolonged in vitro translational system from cucumber ecoplasts. Biochem. Biophys. Res. Comun 135:48-255.
Daniell H. McFadden BA (1987) Uptake and expression of bacteria and cyanobacterial genes by isolated cucumber etioplasts. Proc. Natl. Acad. Sci. USA 84_49-6353.
Daniell H. McFadden BA (1988) Genetic Engineering of plant chloroplasts. United States Patents 5,932,479; 5,693,507
Daniell H. Ramanujan P. Krishnan M. Gnanam A. Rebeiz CA (1983) in vitro synthesis of photosynthetic membranes: I. Development of photosystem I activity and cyclic phosphorylation. Biochem. Biophys. Res. Comun. 111:740-749.
Daniell H. Rebeiz CA (1982) Chloroplast culture IX: Chlorophyl(ide): A biosynthesis in vitro at rates higher than in vivo. Biochem. Biophys. Res. Comun 106:466-471.
Daniell H. Vivekananda J. Neilsen B. Ye GN: Tewari KK. Sanford JC (1990) Transient foreign gene expression in chloroplasts of cultured tobacco cells following biolistic delivery of chloroplast vectors. Proc. Natl. Acad. Sci USA 87:88-92.
Davidson, MB (1998) Diagnosis and classification of diabetes mellitus in "Diabetes Mellitus-Diagnosis and Treatment. Pp. 1-16. 4th edition., W.B. Saunders Co., Philadelphia, PA.
Dertzbaugh MT, Elson CO (1993) Comparative effectiveness of the cholera toxin B subunit and alkaline phosphatase as carriers for oral vaccines. Infect. Immun. 61: 48-55.
Drescher DF, Follmann H. Haberlein I (1998: Sulfitolysis and thioredoxin-dependent reduction reveal the presence of a structural disulfide bridge in spinach chloroplast fructose-1.6-bisphospate. FEBS Letters 424:109-112.
During K. Hippe S. Kreuzaler F. Schell J (1990) Synthesis and self-assembly of a functional monoclonal antibody in transgenic Nicotiana tabacum. Plant Molecular Biology. 15:281-293.
Edwards K. Johnstone C. Thompson C (1991) A simple and rapid method for preparation of plant genomic DNA for PCR analysis. Nucleic Acids Res. 19:1349.
Eibl C. Zou Z. Beck A. Kim M. Mullet J. Koop UH 91999) In vivo analysis of plastid psbA, rbcL and rp132 UTR elements by chloroplast transformation: tobacco plastid gene expression is controlled by modulation of transcript levels and translation efficiency. The Plant Journal 19: 333-345.
Gill DM (1976) The arrangement os subunits in cholera toxin. Biochemistry 15:1242-1248.
Goeddel DV, Kleid DG, Bolivar F, Heyneker HL. Yansura DG, Crea R, Hirose T, Kraszewski A. Italkura K. Riggs AD (1979) Expression in Escherichia coli of chemically synthesized genes for human insulin. Proc. Natl. Acad. Sci. 76: 106-110.
Goldberg AL, Goff SA (1986) Maximizing Gene Expression. Reznikoff and Gold, eds.pp. 287 311, Butterworth Publishers, Stoneham, MD.
Guda C. Zhang X. McPherson DT, XU J. Cherry J. Urry DW, Daniell H. 91995) Hyperexpression of an environmentally friendly synthetic gene. Biotechnol Lett 17:745-750.
Guda C. Lee SB, Daniell H 92000) Stable expression of biodegradable protein based polymer in tobacco chloroplasts. Plant Cell Rep. 19:257-262.
Gunby P (1978) Bacteria directed to produce insulin in test application of genetic code. J. AM. Med. Assoc. 240(16): 1697-1698.
Hall SS (1988) Invisible Frontiers - The Race to Synthesize a Human Gene. Atlantic Monthly Press, New York, NY.
Hancock WW, Sayegh MH, Weiner HL et al. (1993) Oral, but not intravenous, alloantigen prevents accelerated allograft rejection by selective intragraft Th2 cell cativation. Transplanation 55:1112-18.
Haq T.A. Mason HS. Clements JD. Arntzen C. et al (1995 Oral immunization with a recombinant bacterial antigen produced in transgenic plants. Science 268:714-716
Herzog RW Singh NK Urry DW Daniell H (1997) Synthesis of a protein based polymer (Elastomer) gene in Aspergillus nidulans. Applied Microbiology & Biotechnology 47:368-372.
Itakura K. Hirose T. Crea R. Riggs A.D. Heyneker HL Bolivar F. Boyer HW (1977) Expression in Escherichia coli of a chemically synthesized gene for the hormone somatostatin. Science. 198:1056-1063.
Ido Y. Vindigni A. Chang K. Stramm L. Chance R. Heath WF, DiMarchi RD, DiCera E. Williamson JR (1997)Prevention of vascular and neural dysfunction in diabetic rats by C-peptide. Science 277:563-566.
Jones JN (1990) Production of human calcitonin by recombinant DNA technology. "Fundamentals of Protein Technology" (Stein, S. ed), pp. 171-180, Xoma Corp.,
Berkely, CA. Khoury SJ Lider O. Weiner HL et al. (1990) Suppression of experimental auto immune encephalomyelitis by oral administration of myelin basic protein. Cell Immunol. 131:302-10.
Kim J-S, Raines RT (1993) Ribonuclease S-peptide as a carrier in fusion proteins. Protein. Sci. 2:348-356.
Kota M. Daniell H. Varma S. Garczynski F. Gould F. Moar WJ (1999) Overexpression of the Bacillus thuringiensis Cry2A protein in chloroplasts confers resistance to plants against susceptible and Bt-resistant insects. Proc. Natl. Acad. Sci. USA 96:1840-1845.
Laemmli UK (1970) Cleavage of structural proteins during the assembly of the head of bacteriophage T4. Nature 227:680-685.
Lebens M. Holmgren J. (1994) Mucosal vaccines based on the use of Cholera Toxin B subunit as immunogen and antigen carrier. Recombinant Vectors in Vaccine Development [Brown F (ed)], 82: 215-227.
Lee C. Levin A. Branton D, (1987) Copper staining: A five-minute protein stain for sodium dodecyl sulfate-polyacrylamide gels. Anal Biochem 166:308-312.
McBride KE Schaaf DJ. Daley M. Stalker DM (1994) Controlled expression of plastid transgenes in plants based on a nuclear encoded and plastid targeted T7 RNA polymerase. Proc. Natl. Acad. Sci. USA 91:7301-7305
McBride KE Svab Z, Schaaf DJ, Hogen PS, Stalker DM, Maliga P 91995) Amplification of a chimeric Bacillus gene in chloroplasts leads to extraordinary level of an insecticidal protein in tobacco. Bio/technology 13:362-365.
McKenzie SJ and Halsey JF (1984) Cholera toxin B subunit as a carrier protein to stimulate a mucosal immune response. Journal of Immunology. 133:1818-24.
McPherson DT, Morrow C. Mineham DJ, Wu J. Hunter E. Urry DW 91992) Production and purification of a recombinant elastomeric polypeptide. G (IVPGVG) 19-VPGV from Escherichia coli. Biotechnology Prog. 8:317-322.
McPherson DT Xu J Urry DW (1996) Product purification by reversible phase transition following Escherichia coli expression of genes encoding up to 251 repeats of the elastomeric pentapeptide GVGVP. Protein Expression and Purification 7:51-57.
Ma S-W, Zhao D-L. Yin Z-Q. Mukherjee R. Singa B. Qin H-Y et al. (1997) Transgenic plants expressing autoantigens fed to mice to induce oral tolerance. Transgenic Res. 3:793-796.
Marina CV et al. (1988) An Escherichia coli vector to express and purify foreign proteins by fusion to and separation from maltose binding protein. Gene 74:365-373.
Mason HS Ball JM Anrtzen CJ et al. (1996). Expression of Norwalk virus capsid protein in transgenic tobacco and potato and its oral immunogenicity in mice. Proc. Nat. Acad. Sci. USA 93:5335-40.
Mathiowitz E. Jacob JS Jong YS Carino GP. Chickering DE. Chaturvedi P. Santos CA Vijayarahauau K. Montgomery S. Bassett M. Morrell C. (1997) Biologically erodible microspheres as potential oral drug delivery systems. Nature 386:410-414.
May GD. Mason HS Lyons PC (1996) Application of transgenic plants as production systems for pharmaceuticals in ACS symposium series 647. Fuller et al eds. Chapter 13. 196-204.
Mekalanos JJ SadoffFC (1979) Cholera vaccines: Fighting an ancient scourge. Science 265: 1387-1389
Meyer DE Chilkoti A (1999) Purification of recombinant proteins by fusion with thermally-responsive polypeptides. Nature Biotechnology 17:1112-1115.
Miller A. Weiner HL et al. (1992) Suppressor T cells generated by oral tolerization to myelin basic protein suppress both in vitro and in vivo immune responses by the release of transforming growth factor B after antigen specific triggering. Proc Nat. Acad. Sci. USA 89:421-5.
Mor TS, Palmer KE et al. (1998) Perspective: edible vaccines - a concept coming of age. Trends In microbiology 6:449-453
Nilsson J. Stahl S. Lundeberg J. Uhlen M. Nygren PA (1997) Affinity fusion strategies for detection, purification, and immobilization of recombinant proteins. Protein Expr. Purif. 11:1-16.
Oakly WG, Pyke DA, Taylor KW 91973) Biochemical basis of Diabetes. In "Diabetes and It's Management", pp. 1-14, 2nd edition, Blackwell Scientific Publications. Osney Mead, Oxford.
Ong E et al. (1989) The cellulose-binding domains of cellulases: tools for biotechnology. Trends Biotechnol. 7:239-243.
Reulland E. Miginiac-Maslow M (1999) Regulation of chloroplast enzyme activities by thioredoxins; activation or relief from inhibition. Trends in Plant science 4:136-141.
Richter L. Mason HS, Arntzen CJ (1996) Transgenic plants created for oral immunization against diarrheal diseases. Journal of travel medicine. 3:52-56.
Rogers SO, Bendich AJ (1988) In: Gelvin SB Schilperoot RA (eds) Plant molecular biology manual, Kluwer Academis Publishers, Dordrecht, Netherlands, pp. A6:1-10.
Roy H (1989) Rubisco assembly; a model system for studying the mechanism of chaperonin action. Plant Cell. 1:1035-1042
Sambrook J. Fritch EF, Maniatis T (1989) Molecular cloning. Cold Spring Harbor Press, cold Spring Harbor, New York.
Sayegh MH, Khoury SJ, Weiner HL et al (1992) Induction of immunity and oral tolerance with polymorphic class II major histocompatibility complex allopeptides in the rat. Proc. Nat. Acad. Sci. USA: 89:7762-6
Schmidt M. Babu KR Khanna N. Marten S. Rimas U. (1999) Temperature induced production of recombinant human insulin in high density cultures of recombinant E.coli. Biotechnology 68: 71-83.
Schonberger O. Hirst T. R and Pines O (1991) Targeting and assembly of an oligomeric bacterial enterotoxcid in the endoplasmic reticulum of Sacharomyces cervisiae. Molecular Microbiology 5:2663-2671.
Smith DB, Johnson KS (1988) Single-step purification of polypeptides expressed in Escherichia coli as fusion with glutathione S-transferase. Gene 67:31-40.
Smith PA et al. (1998) A plasmid expression system for quantitative in vivo biotinylation of thioredoxin fusion proteins in Escherichia coli. Nucleic Acids Res. 26:1414-1420.
Smith MC Furman TC, Ingolia TD, Pidgeon C. (1988) Chelating peptide-immobilized metal ion affinity chromatography. J. Biol. Chem. 263:7211-7215.
Sidorov VA, Kasten D, Pang SZ, Hajcukiewicz PTJ, Staub JM, Nehra NS (1999) Stable chloroplast transformation in potato use of green fluorescent protein as a plastid marker. Plant Journal 19:209-216.
Steiner DF, Arquila ER, LemerJ, MartinDB (1978) Recombinant DNA Research, Diabetes. 27:877-878
Su X, Prestwood AK, McGraw RA (1992) Production of recombinant percine tumor necrosis factor alpha in a novel E. coli expression system. Biotechniques 13:756-62.
Sun JB, Holmgren J, Czerkinsky C (1994) Cholera toxin B subunit: an efficient transmucosal carrier-delivery system for induction of peripheral immunological tolerance. Proc. Natl. Acad. Sci. USA. 9:10795-10799.
Sun JB, Rask C. Olsson T, Holmgren J, Czerkinsky C (1996) Treatment of experimental autoimmune encephalomyelitis by feeding myelin basic protein conjugated to cholera toxin B subunit. Proc. Natl. Acad. Sci. US 93:7196-9201.
Svab Z. Maliga P (1993) High frequency plastid transformation in tobacco by selection for a chimeric aadA gene. Proc. Natl. Acad. Sci. USA 90:913-917.
Tsao KW, deBarbieri B. Hanspeter M. Waugh DW (1996) A versatile plasmid expression vector of the production of biotinylated proteins by site-specific enzymatic modification in Escherichia coli. Gene 69:59-64.
Thanavala, y, Yang Y, Lyons P. et al (1995) Immunogenicity of transgenic plant derived hepatitis B surface antigen. Proc. Nat. Acad. Sci. USA 92:3358-3361.
Trentham DE., Weiner HL et al (1993) Effects of oral administration of Type II collagen on rheumatoid arthritis. Science 261:1727-30.
Urry DW (1995) Elastic biomolecular machines. Scientific American. 272: 64-69.
Urry DW (1991) Thermally Driven Self-assembly. Molecular Structuring and Entropic Mechanisms in Elastomeric Polypeptides in "Molecular Conformation and Biological Interactions" (Balaram, P., and Ramasashan S., Eds.) Pp. 555-583. Indian Acad. Of Sci., Bangalore, India.
Urry DW, McPherson J., Xu J., Gowda DC, Jing N. Parker TM, Daniell H. Guca C. (1996) Protein Based Polymeric Materials (Synthesis and Properties) in "Polymeric Materials Encyclopedia". (Solomone ed.) Vol. 9. Pp 2645-2699, CRC Press.

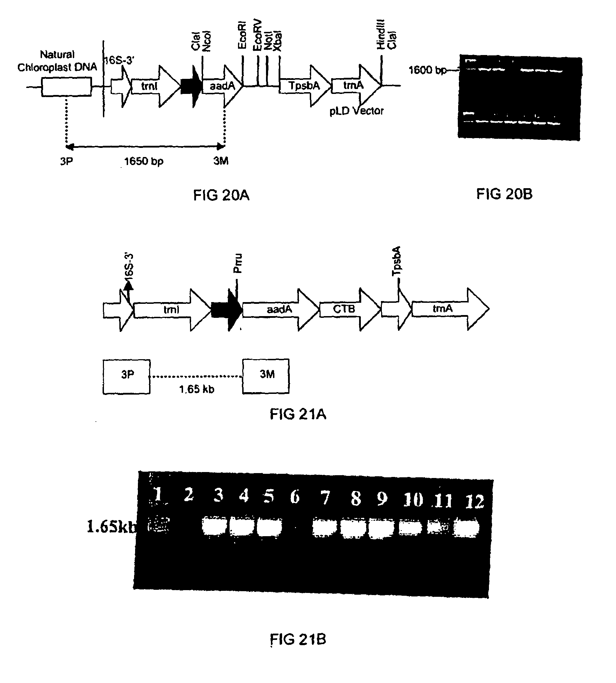

**Selection and Regeneration of Transgenic Plants:** Bombarded leaf pieces when placed on selection medium continued to grow but were bleached. Green shoots emerged from the part of the leaf in contact with the medium. Five rounds of bombardment (5 leaves each) resulted in 68 independent transformation events. Each such transgenic line was subjected to a second round of antibiotic selection. These putative transformants were subjected to PCR analysis to distinguish from nuclear transformants and mutants.
**Determination of Chloroplast Integration and Homoplasmy:** PCR and Southern hybridization were used to determine integration of the CTB gene into the chloroplast genome. Primers, 3P and 3M, designed to confirm incorporation of the gene cassette into the chloroplast genome were used to screen putative transgenics initially. The primer, 3P, landed on the chloroplast genome outside of the chloroplast flanking sequence used for homologous recombination as shown in FIG. 21A. The primer, 3M, landed on the aadA gene. No PCR product should be obtained if foreign genes are integrated into the nuclear genome or in mutants lacking the aadA gene. The presence of the 1.6kb PCR product in 9 of the 10 putative transgenics screened, confirmed the site-specific integration of the gene cassette into the chloroplast genome. Database searches showed that no random priming took place as both the 3P and 3M primers showed no homology with other gene sequences. This is confirmed by the absence of PCR product in untransformed plants (FIG. 21B). Similar strategy has been used successfully by us in order to confirm chloroplast integration of foreign genes (13,14,24,25). This screening is essential to eliminate mutants and nuclear transformants and saves space and labor of maintaining hundreds of transgenic lines.

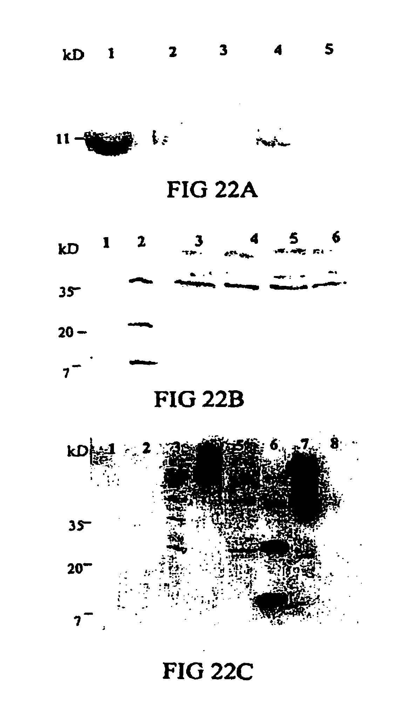

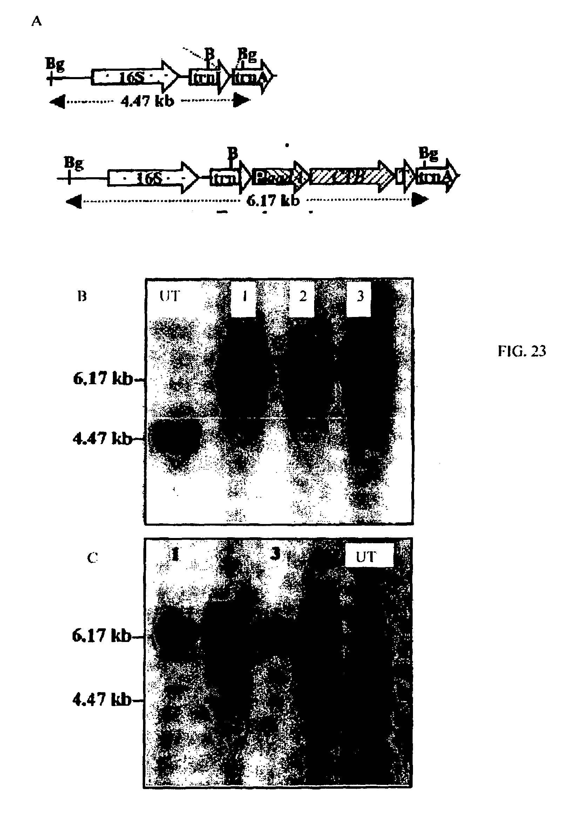

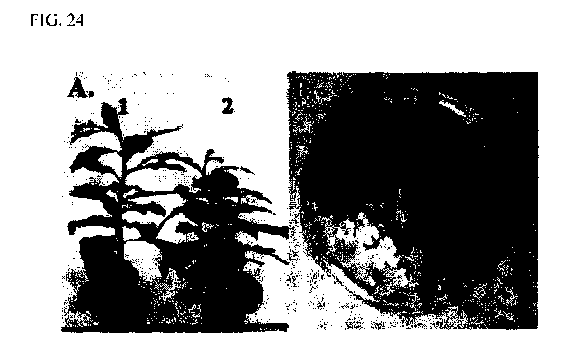

Southern blot analysis of three of the PCR positive transgenic lines was done to further confirm site specific integration and to establish copy number. In the chloroplast genome, BgIII sites flank the chloroplast border sequences 5' of 16S rRNA and 3'of the trnA region as shown in FIG. 23A. A 6.17kb fragment from a transformed plant and a 4.47 kb fragment from an untransformed plant were obtained when total plant DNA from transformed and untransformed plants was digested with BglII. The blot of the digested products was probed with a ³²P random primer-labeled 0.81 kb trnl-trnA fragment The probe hybridized with the control giving a 4.47 kb fragment as expected, while for the transgenic lines a 6.17 kb fragment was observed, indicating that all plastid genomes had the gene cassette, inserted between the tml and trnA regions. The absence of a 4.47 kb fragment in transgenic lines indicates that hornoplasmy has been achieved, to the detection level of a Southern blot. These results explain the high levels of CTB observed in transgenic tobacco plants. Southern blot confirmed plants transferred to pots were seen to have no adverse pleiotropic effects when compared to untransformed plants as shown in FIG. 24A. Southern blot analysis of T₁ plants in FIG. 23C shows that all 4 transgenic lines analyzed maintained homoplasmy.
**Immunoblot Analysis of Chloroplast Synthesized CTB:** Anti-cholera toxin antibodies did not show significant cross-reaction with tobacco plant protein as can be seen in FIG. 22C, lanes 1 & 2. Boiled and unboiled leaf homogenates were run on 12% SDS PAGE gels. Unboiled chloroplast synthesized CTB protein appeared as contact 45 kDa oligomers as shown in FIG. 22C, lane 4 similar to the unboiled, pentameric bacterial CTB which appeared to have partially dissociated into tetramers, trimers and monomers upon storage at 4°C over a period of several months from FIG. 22C, lane7.

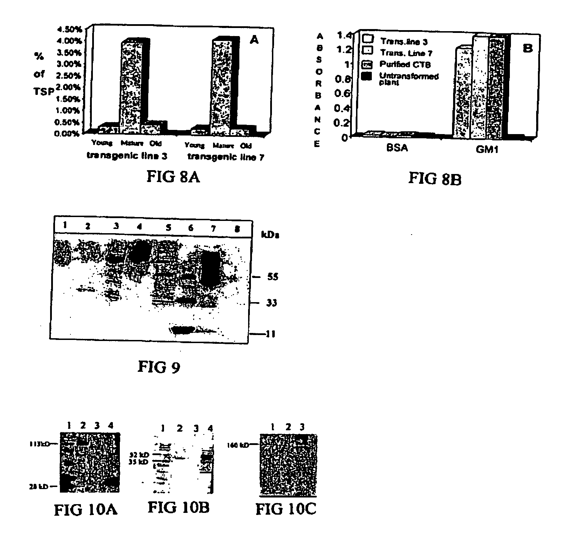

While heat treatment (4 min. boiling) prior to SDS PAGE of pentameric bacterial CTB, gave CTB monomers predominantly, with some protein in the dimeric and trimeric form as shown in Fig. 22C, lane 6, chloroplast synthesized CTB dissociated into dimers and trimers only, when subjected to similar heat treatment as in Fig. 22C, lanes 3 & 5. These results are different from the heat induced dissociation of potato plant nucleus synthesized CTB; oligomers into monomers (8). A probable reason for this stability could be a more stable conformation of chloroplast synthesized CTB which maybe an added advantage in storage and administration of edible vaccines. Leaf homogenates from four different transgenic plants showed almost similar expression levels of CTB protein (see Fig. 22B). This suggests very little clonal variation of CTB expression, as was confinned later by ELISA quantification assays. Consistent expression levels of recombinantproteins in plants (as obtained for CTB in this research) may be essential for production of edible vaccines in plants.
**GM₁ Gauglioside ELISA Binding Assays:** Both chloroplast synthesized and bacterial CTB demonstrated a strong affinity for GM1, - gangliosides (see FIG. 8B) indicating that chloroplast synthesized CTB conserved the antigenic sites necessary for binding of the CTB pentamer to the pentasaccharide GM₁I. The GM₁ binding ability also suggests proper folding of CTB molecules resulting in the pentameric structure. Since oxidation of cysteine residues in the B subunits is a prerequisite for in vivo formation of CTB pentamers (20), proper folding is a further confirmation of the ability of chloroplasts to form disulfide bonds.

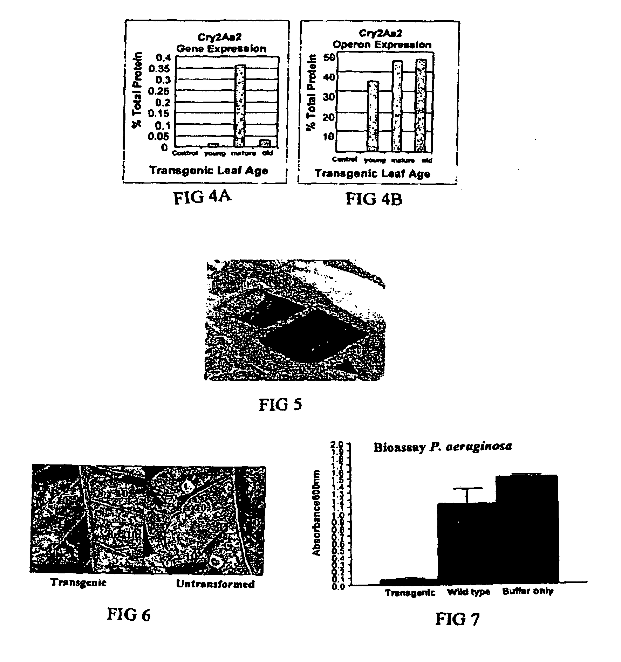

High levels of expression of CTB in transgenic tobacco did not affect growth rates, flowering or seed setting as has been observed in this laboratory, unlike previously reported for the synthetic LTB gene, constitutively expressed via the nuclear genome (7). Transformed plant seedlings were green in color while untransformed seedlings larking the aadA gene were bleached white as shown in Fig. 4B when germinated on antibiotic medium.

The potential use of this technology is three-fold. While, it can be used for large scale production of purified GTB, it can also be used as an odible vaccine if expressed in an edible plant or as a transmucosal carrier of peptides to which it is fused to, so as to either enhance mucosal immunity or to induce oral tolerance to the products of these peptides (5). Large-scale production of purified CTB in bacteria involves the use of expansive fermentation techniques and stringent purification protocols (26) making this a prohibitively expensive technology for developing countries. The cost of producing 1kg of recombinant protein in transgenic crops has been estimated to be 50 times lower than the cost of producing the same amount by *E. coli* fermentation, assuming that recombinant protein is 20% of total *E.coli* protein (27). Thus, isolation and lysis of CTB producing chloroplasts from chloroplast transformed plants could serve as a cost-effective means of mass production of purified CTB. If used as an edible vaccine, a selection scheme eliminating the use of antibiotic resistant genes should be developed. One such scheme uses the betaine aldehyde dehydogenase (BADH) gene, which converts toxic betaine aldehyde to nontoxic glycine betaine, an osmoprotectant (28). Also, several other strategies have been proposed to eliminate antibiotic-resistant genes from transgenic plants (29).

Transgenic potato plants that synthesize a CTB-insulin fusion protein at levels of up to 0.1% of the total soluble tuber protein have been found to show a substantial reduction in pancreatic islet inflammation and a delay in the progression of clinical diabetes (30). This may prove to be an effective clinical approach for prevention of spontaneous autoimmune diabetes. Since, increased CTB expression levels have been shown to be achievable via the chloroplast genome through this research, expression of a CTB-proinsulin fusion protein in the chloroplasts of edible tobacco (LAMD) is currently being tested in our laboratory. While existing expression levels of CTB via the chloroplast genome are adequate for commercial exploitation, levels can be increased further (about 10 fold) by insertion of a putative chaperonin, as in the case of the Bt Cry2aA2 operon, (13) which likely aids in the subsequent purification of recombinant CTB due to crystallization.

### REFERENCES

5. Sun, J. B., Rask, C., Olsson, T., Holmgren, J., Czerkinsky, C. (1996) Proc. Natl. Acad. Sci. USA 94,4610 - 4614.
7. Mason, H. S., Haq, T. A., Clements, J. D. and Arritzen, C. J., (1998) Vaccine 16, 1336 - 1343.
13. DeCosa, B., Lee, S. B., Moar, W., Miller, M., Daniell, H. Nature Biotech. in press.
20. Sixma, T. K., Pronk, S. E., Kalk, K. H., Wartna, E. S., Van Zanten, B. A. M., Witholt, B. B., Hol, W. G. H. (1991) E. coli. Nature 351,371- 377.
26. Lebens, M., Johansson, S., Osek, J., Lindblad, M., Holingren, J. (1993) Biotechnology 11, 1574 - 1578.
27. Petridis, D., Sapidou,E., Calandranis, J. (1995) Biotechnol. BioEng. 48,529 - 541.
28. Daniell, H., B. Muthulcurnar, Lee, S. B., Current genetics in press.
30. Arakawa, T., Yu, J., Chong, D. K., Hough, J., Engen, P. C., Langridge, W. H. R. (1998) Nature Biotech. 16,934 - 938.

**Engineering novel pathways via the chloroplast genome:** In plant and animal cells, nuclear mRNAs are translated monocistronically. This poses a serious problem when engineering multiple genes in plants (32). Therefore, to express the polyhydroxybutyrate polymer or Guy's 13 antibody, single genes were first introduced into individual transgenic plants. Then, these plants were back-crossed to reconstitute the entire pathway or the complex protein (33,34). Similarly, in a seven year long effort, Ye et al. (22) recently introduced a set of three genes for a short biosynthetic pathway that resulted in β-carotene expression in rice. In contrast, most chloroplast genes of higher plants are cotranscribed (32). Expression of polycistrons via the chloroplast genome provides a unique opportunity to express entire pathways in a single transformation event. We have recently used the *Bacillus thuringiensis* (Bt) cry2Aa2 operon as a model system to demonstrate operon expression and crystal formation via the chloroplast genome (29). *Cry*2Aa2 is the distal gene of a three-gene operon. The *orf* immediately upstream of cry2Aa2 codes for a putative chaperonin that facilitates the folding of cry2Aa2 (and other proteins) to form protcolytically stable cuboidal crystals (35).

Therefore, the cry2Aa2 bacterial operon was expressed in tobacco chloroplasts to test the resultant transgenic plants for increased expression and improved persistence of the accumulated insecticidal protein(s). Stable foreign gene integration was confirmed by PCR and Southern blot analysis in T₀ and T₁ transgenic plants. *Cry2Aa2* operon derived protein accumulated at 45.3% of the total soluble protein in mature leaves and remained stable even in old bleached leaves (46.1%)(see figure number 4 in attached article De Cosa et al. 2001, 29). This is the highest level of foreign gene expression ever reported in transgenic plants. Exceedingly difficult to control insects (10-day old cotton bollworm, beetarmy worm) were killed 100% after consuming transgenic leaves. Electron micrographs showed the presence of the insecticidal protein folded into cuboidal crystals similar in shape to *Cry2Aa2* crystals observed in *Bacillus thuringiensis* (see figure number 6 in attached article De Cosa et al. 2001, 29).

In contrast to currently marketed transgenic plants with soluble CRY proteins, folded protoxin crystals are processed only by target insects that have alkaline gut pH. This approach should improve safety of Bt transgenic plants. Absence of insecticidal proteins in transgenic pollen eliminates toxicity to non-target insects via pollen. In addition to these environmentally friendly approaches, this observation should serve as a model system for large-scale production of foreign proteins within chloroplasts in a folded configuration enhancing their stability and facilitating single step purification. This is the first demonstration of expression of a bacterial operon in transgenic plants and opens the door to engineer novel pathways in plants in a single transformation event.
**Expressing small peptides via the chloroplast genome:** It is common knowledge that the medical community has been fighting a vigorous battle against drug resistant pathogenic bacteria for years. Cationic antibacterial peptides from mammals, amphibians and insects have gained more attention over the last decade (3 6). Key features of these cationic peptides are anet positive charge, an affinity for negatively-charged prokaryotic membrane phospholipids over neutral-charged eukaryotic membranes and the ability to form aggregates that disrupt the bacterial membrane (37).

There are three majorpeptides with a-helicalstructures, cecropin from *Hyalophora cecropia* (giant silk moth), magainins from *Xenopus laevis* (African frog) and defensins from mammalian neutrophils. Magainin and its analogues have been studied as a broad-spectrum topical agent, a systemic antibiotic; a wound-healing stimulant; and an anticancer agent (38). We have recently observed that a synthetic lytic peptide (MSI-99, 22 amino acids) can be successfully expressed in tobaccochloroplast (39). The peptide retained its lytic activity against the phytopathogenic bacteria *Pseudomonas syringae* and multidrug resistant human pathogen, *Pseudomonas aeruginosa.* The anti-microbialpeptide (AMP) used in this study was an amphipathic alpha-helixmolecule that has an affinity for negatively charged phospholipids commonly found in the outer-membrane of bacteria.

Upon contact with these membranes, individual peptides aggregate to form pores in the membrane, resulting in bacterial lysis. Because of the concentration dependent action of the AMP, it was expressedvia the chloroplast genome to accomplish high dose delivery at the point of infection. PCR products and Southern blots confirmed chloroplast integration of the foreign genes and homoplasmy. Growth and development of the transgenic plants was unaffected by hyper-expression of the **AMP** within chloroplasts. *In vitro* assays with T₀ and T, plants confirmed that the AMP was expressed at high levels (21.5 to 43% of the total soluble protein) and retained biological activity against *Pseudomonas syringae,* a major plant pathogen. *In situ* assays resulted in intense areas of necrosis around the point of infection in control leaves, while transformed leaves showed no signs of necrosis (200-800 µg of AMP at the site of infection) as shown in Fig. 6. T₁ *in vitro* assays against *Pseudomonas aeruginosa* (a multi-drug resistant human pathogen) displayed a 96% inhibition of growth as shown in Fig. 7. These results give a new option in the battle againstphytopathogenicand drug-resistant human pathogenic bacteria. Small peptides (like insulin) are degraded in most organisms. However, stability of this AMP in chloroplasts opens up this compartment for expression of hormones and other small peptides.

The use of microorganisms such as bacteria permits manufacture on a larger scale, but introduces the disadvantage of producing products, which differ appreciably from the products of natural origin. For example, proteins that are usually glycosylatedin humans are not glycosylated by bacteria, Furthermore, human proteins that are expressed at high levels in E. *coli* frequently acquire an unnatural conformation, accompanied by intracellular precipitation due to lack of proper folding and disulfide bridges. Production of recombinant proteins in plants has many potential advantages for generating biopharmaceuticals relevant to clinical medicine. These include the following: (i)plant systems are more economical than industrial facilities using fermentation systems; (ii) technology is available for harvesting and processing plants/plant products on a large scale; (iii) elimination of the purification requirement when the plant tissue containing the recombinant protein is used as a food (edible vaccines); (iv) plants can be directed to target proteins into stable, intracellularcompartments as chloroplasts, or expressed directly in chloroplasts; (v) the amount of recombinant product that can be produced approaches industrial-scale levels; and (vi) health risks due to contamination with potential human pathogens/toxins are minimized.

It has been estimated that one tobacco plant should be able to produce more recombinant protein than a 300-liter fermenter of E. *coli*. In addition, a tobacco plant produces a million seeds, facilitating large-scale production. Tobacco is also an ideal choice because of its relative ease of genetic manipulation and an impending need to explore alternate uses for this hazardous crop. However, with the exception of enzymes (e.g. phytase), levels of foreign proteins produced in nuclear transgenic plants are generally low, mostly less than 1 % of the total soluble protein (52). May et aL (53) discuss this problem using the following examples. Although plant derived recombinant hepatitis B surface antigen was as effective as a commercial recombinant vaccine, the levels of expression in transgenic tobacco were low (0.0066% of total soluble protein). Even though Norwalk virus capsid protein expressed in potatoes caused oral immunization when consumed as food (edible vaccine), expression levels were low (0.3% of total soluble protein). In particular, expression of human proteins in nuclear Transgenic plants has been disappointingly low: e.g. human Interferon-β 0.000017% of fresh weight, human serum albumin 0.02% and crythropoietin 0.0026% of total soluble protein (see Table1 in ref. 52). A synthetic gene coding for the human epidermal growth factor was expressed only up to 0.001% of total soluble protein in transgenic tobacco (53).

The cost of producing recombinant proteins in alfalfa leaves was estimated to be 12-fold lower than in potato tubers and comparable with seeds (52). However, tobacco leaves are much larger and have much higher biomass than alfalfa. The cost of production of recombinant proteins will be 50-fold lower than that of *E.coli* fermentation (with 20% expression levels, 52). A decrease in insulin expression from 20% to 5% of biomass doubled the cost of production (54). Expression level less than 1% of total soluble protein in plants has been found to be not commercially feasible (52). Therefore, it is important to increase levels of expression of recombinant proteins in plants to exploit plant production of pharmacologically important proteins.

An alternate approach is to express foreign proteins in chloroplasts of higher plants. We have recently integrated foreign genes (up to 10,000 copies per cell) into the tobacco chloroplast genome resulting in accumulation of recombinant proteins up to 46% of the total cellular protein (29). Chloroplast transformation utilizes two flanking sequences that, through homologous recombination, insert foreign DNA into the spacer region between the functional genes of the chloroplast genome, thus targeting the foreign genes to a precise location. This eliminates the "position effect" and gene silencing frequently observed in nuclear transgenic plants. Chloroplast genetic engineering is an environmentally friendly approach, minimizing concerns of out-cross of introduced traits via pollen to weeds or other crops. Most importantly, a significant advantage in the production of Pharmaceutical proteins in chloroplasts is their ability to process eukaryotic proteins, including folding and formation of disulfide bridges (55). Chaperonin proteins are present in chloroplasts (56,57) that function in folding and assembly of prokaryotic/eukaryotic proteins. Also, proteins are activated by disulfide bond oxido/reduction cycles using the chloroplast thioredoxin system (58) or chloroplast protein disulfide isomerase (59). Accumulation of fully assembled, disulfide bonded form of human somatotropin via chloroplast transformation (31) and oligomeric form of CTB (47) and assembly of heavy and light chains of humanized Guy's 13 antibody in transgenic chloroplasts (50) provide strong evidence for successful processing of pharmaceutical proteins inside chloroplasts. Such folding and assembly should eliminate the need for highly expensive *in vitro* processing of pharmaceutical proteins. For example, 60% of the total operating cost in the production of human insulin is associated with in vitro processing (formation of disufide bridges and cleavage of methionine)(54).

Taken together, low levels of expressionofhuman proteins in nuclear transgenic plants, and difficulty in folding, assembly/processing of human proteins in *E. coli* should make chloroplasts an ideal compartment for expression of recombinant proteins. Production of human proteins in transgenic chloroplasts also dramatically lowers the production cost. Large-scale production of human serum albumin in plants is a powerful approach to provide safe treatment to patients at an affordable cost and provide tobacco farmers alternate uses for this hazardous crop. Therefore, it would be highly advantageous to provide for expression of human serum albumin in transgenic tobacco chloroplasts to increase levels of expression and accomplish in vivo processing.
**Use of chemical or enzymatic cleavage:** The strategy of fusing a protein to a tag with affinity for a certain ligand has been used to enable affinity purification of recombinant products (73 - 75). However, scaleup of this technology is usuallyquite expensive. Avast numberofcleavage methods, both chemical and enzymatic, have been investigated for this purpose (75). Chemical cleavage methods have low specificity and the relatively harsh cleavage conditions can result in chemical modifications of the released products (75). Some of the enzymatic methods offer significantly higher cleavage specificities together with high efficiency, e.g. H64A subtilisin, IgA protease and factor Xa (74,75), but these enzymes have the drawback of being quite expensive.

Trypsin, which cleaves C-terminal of basic amino-acid residues, has been used for along time to cleave fusion proteins (3,76). Despite expected low specificity, trypsin has been shown to be useful for specific cleavage of fusion proteins, leaving basic residues within foldedprotein domains uncleavaged (76). The use of trypsin only requires that the N-terminus of the mature protein be accessible to the protease and that the potentialintemal sites are protected in the native conformation. Trypsinhas the additionaladvantageofbeing inexpensive and readily available. In the case of HSA, when it was expressed in E. *coli* with *6* additional codons coding for a trypsin cleavage site, HSA was processed successfully into the mature protein after treatment with the protease. In addition, the N-terminal sequence was found to be unique and identical to the sequence of natural HSA, the conversion was complete and no degradation products were observed (3). This *in vitro* maturation is selective because correctly folded albumin is highly resistant to trypsin cleavage at inner sites (3). This system could be tested for chloroplasts HSA vectors using protein expressed in E. *coli.*

Staub et al. (31) demonstrated that the chloroplast methionine aminopeptidase is active and they found 95% of removal of the first methionine of an ATG-somatotropinprotein that was expressed via the chloroplast genome. There are several investigations that have shown a very strict pattern of cleavage by this peptidase (77). Methionine is only removed when second residues are glycine, alanine,serine, cysteine, threonine, proline or valine, but if the third amino acid is proline the cleavage is inhibited. For HSA the second aminoacid is aspartic acid, so the cleavage may not be possible.

**3) Optimization of gene expression:** We have reported that foreign genes are expressed between 3% (*cry2Aa2*) and 46% (*cry2Aa2* operon) in transgenic chloroplasts (26,29). Based on the outcome of the evaluation of HSA chloroplast transgenic plants, several approaches will be used to enhance translation of the recombinant proteins. In chloroplasts, transcriptional regulation of gene expression is less important, although some modulations by light and developmental conditions are observed (78). RNA stability appears to be one among the least problems because of observation of excessive accumulation of foreign transcripts, at times 16,966-fold higher than the highly expressing nuclear transgenic plants (79). Chloroplast gene expression is regulated to a large extent at the post-transcriptional level. For example, 5' UTRs are necessary for optimal translation of chloroplast naNAs. Shine-Dalgarno (GGAGG) sequences as well as a stem-loop structure located 5' adjacent to the SD sequence are required for efficient translation. A recent study has shown that insertion of the psbA 5' UTR downstream of the 16S rRNA promoter enhanced translation of a foreign gene (GUS) hundred-fold (80). Therefore, the 200-bp tobacco chloroplast DNA fragment (1680-1480) containing 5' psbA UTR is used. This PCR product is then inserted downstream of the 16S rRNA promoter to enhance translation of the recombinant proteins.

Yet another approach for enhancement of translation can be the codon composition optimization. It is reasonable to expect efficient expression in chloroplasts since the protein is translated in E. *coli.* Although rbcL (RuBisCO) is the most abundant protein on earth, it is not translated as highly as the psbA gene due to the extremely high turnover of the psbA gene product. The psbA gene is under stronger selection for increased translation efficiency and is the most abundant thylakoid protein. In addition, the codon usage in higher plant chloroplasts is biased towards the NNC codon of 2-fold degenerate groups (i.e. TTC over TTT, GAC over GAT, CAC over CAT, AAC over AAT, ATC over ATT, ATA etc.). This is in addition to a strong bias towards T at third position of 4-fold degenerate groups. There is also a context effect that should be taken into consideration while modifying specific codons. The 2-fold degenerate sites immediately upstream from a GNN codon do not show this bias towards NNC. (TTT GGA is preferred to TTC GGA while TTC CGT is preferred to TTT CGT, TTC AGT to TTT AGT and TTC TCT to TTT TCT)(81,82).

In addition, highly expressed chloroplast genes use GNN more frequently than other genes. The disclosure found in web site bttp:/fwww.kazusa.or.ip/codon was used to analyze codon composition by comparing different species. Abundance of amino acids in chloroplasts and tRNA anticodons present in chloroplast was taken into consideration. We also compared A+T% content of all foreign genes that had been expressed in transgenic chloroplasts in our laboratory with the percentage of chloroplast expression. We found that higher levels of A+T always correlated with high expression levels (see Table 1). The HSA sequence showed 57% of A+T content and 40% of the total codons matched with the psbA most translated codons. According to the data of the Table and taking into consideration all these factors, good chloroplast expression of the HSA gene without modifications in its codon composition can be expected.

| **Open reading % Frame** | **TSP** | **%A+ % T** | **psbA** | **% c p tRNA** |
|---|---|---|---|---|
| CTB | 4 | 66 | 47 | 34 |
| Cry2A operon | 46 | 65 | 37 | 37 |
| Antimicrobial peptide | 21 - 43 | 63 | 35 | 35 |
| HSA | ? | 57 | 57 | 47 |
| RUBISCOss TP | ? | 50 | 32 | 42 |
| Guy's light chain | <1% | 49 | 31 | 44 |
| Guy's heavy chain | <1% | 40 | 25 | 44 |

**4) Vector constructions:** pLD vector is used for the constructs. This vector was developed for chloroplast transformation. It contains the 16S rRNA promoter (Prm) driving the selectable marker gene *aadA* (aminoglycoside adenyl transferase conferring resistance to spectinomycin) followed by the *psbA* 3' region (the terminator from a gene coding for photosystem II reaction center components) from the tobacco chloroplast genome. The pLD vector is a universal chloroplast expression/integration vector and can be used to transform chloroplast genomes of several other plant species (14,27) because these flanking sequences are highly conserved among higher plants. The universal vector uses *trnA* and *trnI* genes (chloroplast transfer RNAs coding for Alanine and Isoleucine) from the inverted repeat region of the tobacco chloroplast genome as flanking sequences for homologous recombination. Because the universal vector integrates foreign genes within the Inverted Repeat region of the chloroplast genome, it doubles the copy number of the transgene (from 5000 to 10,000 copies per cell in tobacco). Furthermore, it has been demonstrated that homoplasmy is achieved even in the first round of selection in tobacco probably because of the presence of a chloroplast origin of replication within the flanking sequence in the universal vector (thereby providing more templates for integration). Because of these and several other reasons, foreign gene expression was shown to be much higher when the universal vector was used instead of the tobacco specific vector (30).

The following vectors can be used to optimize protein expression, purification and production of USA with the same amino acid composition as the human protein.
a) We increase translation using the psbA 5'UTR to optimize expression. The 200 bp tobacco chloroplast DNA fragment containing 5' psbA is amplified by PCR using tobacco chloroplast. DNA as template. This fragment is cloned directly in the pLD vector multiple cloning site (EcoRI-NcoI) downstream of the promoter and the aadA gene. The cloned sequence is the same as in the psbA gene.
b) For enhancing protein stability and facilitating purification, the *cry2Aa2 Bacillus thuringiensis* operon derived putative chaperonin is used. Expression of the *cry2Aa2* operon in chloroplasts provides a model system for hyper-expression of foreign proteins (46% of total soluble protein) in a folded configuration enhancing their stability and facilitating purification (29). This justifies inclusion of the putative chaperonin from the *cry2Aa2* operon in one of the newly designed constructs. In this region there are two open reading frames (ORF1 and ORF2) and a ribosomal binding site (rbs). This sequence contains elements necessary for *Cry2Aa2* crystallization which help to fold or crystallize the HSA protein helping in the subsequent purification. Successful crystallization of other proteins using this putative chaperonin has been demonstrated (35). We amplify the ORF1 and ORP2 of the Bt *Cry2Aa2* operon by PCR using the complete operon as template. The fragment is cloned into a PCR 2.1 vector and excised as an EcoRI-EcoRV product. This fragment is then cloned directly into the pLD vector multiple cloning site (EcoRI-EcoRV) downstream of the promoter and the aadA gene.
c) To obtain HSA with the same amino acid composition as the mature human protein (without the extra methionine), we first fuse HSA with the RuBisCo small subunit transit peptide. Also, other constructions are performed to allow cleavage of the protein after isolation from chloroplast.

The first set of constructs includes the sequence of HSA beginning with an ATG, introduced by PCR using primers. Once optimal expression levels are achieved, and when the ATG is shown to be a problem (determined by mice immunological assays), processing to produce the mature protein is addressed. The first attempt is the use of the RuBisCo small subunit transit peptide. This transit peptide is amplified by PCR using tobacco DNA as a template and cloned into the PCR 2.1 vector. The HSA gene is fused with the transit peptide using a MluI restriction site that is introduced in the PCR primers for amplification of the transit peptide and the HSA coding sequence. The gene fusion is then inserted into the pLD vector downstream of the 5'region that gives optimal expression of HSA (RBS, 5'UTRpsbA, ORF1+2, ORF1+2-5'U'fRpsbA). Another approach to eliminate the ATG of the coding region is the use of the ATG before a protease recognition sequence, like trypsin, and remove in vitro such extra sequence to obtain the mature protein. Such sequences will be introduced by primers in a PCR After completing vector constructions, the vectors are sequenced to confirm correct nucleotide sequence and in frame fusion. DNA sequencing is performed using a Perkin Elmer ABI prism 373 DNA sequencing system or the like.

Because of the similarity of protein synthetic machinery (64), expression of chloroplast vectors is first tested in *E.coli* before their use in tobacco transformation. For *Escherichia coli* expression XL-1 Blue strain is used. Purification and cleavage assays is performed using *E. coli* expressed protein.

**5) Bombardment, Regeneration and Characterization of Chloroplast Transgenic Plants:** Tobacco (*Nicotiana tabacum* var. PetitHavana) plants are grown aseptically by germination of seeds on MSO medium, This medium contains MS salts (4.3 g/liter), B5 vitamin mixture (myo-inositol, 100 mg/liter, thiamine-HCl, 10 mg/liter, nicotinic acid, 1 mg/liter; pyridoxine-HCl, 1 mg/liter), sucrose (30 g/liter) and phytagar (6 g/liter) at pH 5.8. Fully expanded, dark green leaves of about two month old plants are used for bombardment.

Leaves are placed abaxial side up on a Whatman No. 1 filter paper laying on the RMOP medium (20) in standard petri plates (100x15mm) for bombardment. Gold (0.6 µm) microprojectiles are coated with plasmid DNA (chloroplast vectors) and bombardments are carried out with the biolistic device PDS 1000/He (Bio-Rad) as described by Daniell (65). Following bombardment, petri plates are sealed with parafilm and incubated at 24°C under 12 h photoperiod. Two days after bombardment, leaves are chopped into small pieces of ∼5 mm² in size and placed on the selection medium (RMOP containing 500 µg/ml of spectinomycin dihydrochloride) with abaxial side touching the medium in deep (100x25 mm) petri plates (∼10 pieces perplate). The regenerated spectinomycin resistant shoots are chopped into small pieces (∼2mm²) and subcloned into fresh deep petri plates (∼5 pieces per plate) containing the same selection medium. Resistant shoots from the second culture cycle are transferred to the rooting medium (MSO medium and spectinomycin dihydrochloride, 500 mg/liter). Rooted plants are transferred to soil and grown at 26°C under 16 hour photoperiod conditions for further analysis.
**PCR analysis of putative transformants:** PCR is performed using DNA isolated from control and transgenic plants to distinguish a) true chloroplast transformants from mutants and b) chloroplast transformants from nuclear transformants. Primers for testing the presence of the aadA gene (that confers spectinomycin resistance) in transgenic plants are landed on the aadA coding sequence and 168 rRNA gene. One primer lands on the aadA *gene* while another lands on the native chloroplast genome as shown in FIG. 20A to test chloroplast integration of the genes. No PCR product is obtained with nuclear transgenic plants using this set of primers. The primer set is used to test integration of the entire gene cassette without any internal deletion or looping out during homologous recombination. A similar strategy has been used successfully by us to confirm chloroplast integration of foreign genes (26-30). This screening is essential to eliminate mutants and nuclear transformants. Total DNA from unbombarded and transgenic plants is isolated as described by Edwards a al. (83) to conduct PCR analyses in transgenic plants. Chloroplast transgenic plants containing the desired gene are moved to second round of selection to achieve homoplasmy.
**Southern Analysis for homoplasmy and copy number:** Southern blots are performed to determine the copy number of the introduced foreign gene per cell as well as to test homoplasmy. There are several thousand copies of the chloroplast genome present in each plant cell. Therefore, when foreign genes are inserted into the chloroplast genome, it is possible that some of the chloroplast genomes have foreign genes integrated while others remain as the wild type (heteroplasmy). Therefore, to ensure that only the transformed genome exists in cells of transgenic plants (homoplasmy), the selection process is continued Total DNA from transgenic plants are probed with the chloroplast border (flanking) sequences (the trnI-trnA fragment) to confirm that the wild type genome does not exist at the end of the selection cycle. If wild type genomes are present (heteroplasmy), the native fragment size is observed along with transformed genomes. Presence of a large fragment (due to insertion of foreign genes within the flanking sequences) and absence of the native small fragment confirms homoplasmy (26,27,30).

The copy number of the integrated gene is determined by establishing homoplasmy for the transgenic chloroplast genome. Tobacco chloroplasts contain 5000∼10,000 copies of their genome per cell (27). When only a fraction of the genomes are actually transformed, the copy number, by default, must be less than 10,000. By establishing that in the transgenics the gene inserted transformed genome is the only one present, it can be established that the copy number is about 5000∼10,000 per cell. This is usually done by digesting the total DNA with a suitable restriction enzyme and probing with the flanking sequences that enable homologous recombination into the chloroplast genome. The native fragment present in the control should be absent in the transgenics. The absence of native fragment proves that only the transgenic chloroplasts genome is present in the cell and there is no native, untransformed, chloroplast genome, without the foreign gene present. This establishes the homoplasmic nature of our transformants, simultaneously providing us with an estimate of about 5000∼10,000 copies of the foreign genes per cell.
**Northern Analysis for transcript stability:** Northern blots arc performed to test the efficiency of transcription of the genes. Total RNA is isolated from 150 mg of frozen leaves by using the"Rneasy Plant Total RNA Isolation Kit" (Qiagen Inc., Chatsworth, CA). RNA (10-40 µg) is denatured by formaldehyde treatment, separated on a 1,2% agarose gel in the presence of formaldehyde and transferred to a nitrocellulose membrane (MSI) as described in Sambrook et al. (84). Probe DNA (HSA gene coding region) is labeled by the random-primed method (Promega) with ³²P-dCTP isotope. The blot is pre-hybridized, hybridized and washed as described above for southern blot analysis. Transcript levels are quantified by the Molecular Analyst Program using the GS-700 Imaging Densitometer (Bio-Rad, Hercules, CA) or the like.
**Expression and quantification of the total protein expressed in chloroplast:** Chloroplast expression assays are performed by Western Blot Recombinant protein levels in transgenic plants of first and second generation (To and T1) are determined using quantitative ELISA assays. A standard curve is generated using known concentrations and serial dilutions ofrecombinant and native proteins. Different tissues are analyzed using young, mature and old leaves against goat anti-HSA (Nordic Immunology) antibodies. Bound IgG is measured using horseradish peroxidase-labelled anti-goat IgG (Sigma).
**Inheritance of Introduced Foreign Genes:** While it is unlikely that introduced DNA moves from the chloroplast genome to nuclear genome, it is possible that the gene can be integrated in the nuclear genome during bombardment and remain undetected in Southern analysis. Therefore, in initial tobacco transformants, some is allowed to self-pollinate, whereas others are used in reciprocal crosses with control tobacco (transgenic as female accepters and pollen donors; testing for maternal inheritance). Harvested seeds (T1) are germinated on media containing spectinomycin. Achievement of homoplasmy and mode of inheritance can be classified by looking at germination results. Homoplasmy can be indicated by totally green seedlings (27) while heteroplasmy is displayed by variegated leaves (lack of pigmentation, 24). Lack of variation in chlorophyll pigmentation among progeny also underscores the absence of position effect, an artifact of nuclear transformation. Maternal inheritance is demonstrated by sole transmission of introduced genes via seed generated on transgenic plants, regardless of pollen source (green seedlings on selective media). When transgenic pollen is used for pollination of control plants, resultant progeny do not contain resistance to chemical in selective media (will appear bleached; 24). Molecular analyses confirm transmission and expression of introduced genes, and T2 seed is generated from those confirmed plants by the analyses described above.

**6) Purification methods:** The standard method of purification employs classical biochemical techniques with the crystallized proteins inside the chloroplast. In this case, the homogenates are passed through miracloth to remove cell debris. Centrifugation at 10,000 xg pellets all foreign proteins (29). Proteins are solubilized using pH, temperature gradient, etc. This is possible if the ORF1 and 2 of the *cry2Aa2* operon can fold and crystallize the recombinant protein. When there is no crystal formation, other purification methods are applied (classical biochemistry techniques). Albumin is typically administered in tens of gram quantities. At a purity level of 99.999% (a level considered sufficient for other recombinant protein preparations), recombinant HSA (rHSA) impurities on the order of one mg is still injected into patients. Hence, impurities from the host organism must be reduced to a minimum. Furthermore, purified rHSA must be identical to human HSA. Despite these stringent requirements, purification costs must be kept low. It is not appropriate to apply conventional processes for purifying HSA originating in plasma to purify the HSA obtained by gene manipulation. This is because the impurities to be eliminated from rHSA differ from those contained in the HSA originating in plasma. Namely, rHSA is contaminated with, for example, coloring matters characteristic to recombinant HSA, proteins originating in the host cells, polysaccharides, etc. In particular, it is necessaryto sufficiently eliminate components originating in the host cells, since they are foreign matters for living organisms including human and can cause the problem of antigenicity.

In plants, two different methods ofHSA purification have been performed at laboratory scale. Sijmons et al. (12) transformed potato and tobacco plants with *Agrobacterium tumefaciens.* For the extraction and purification of HSA,1000 g of stem and leaf tissue was homogenized in 1000 ml cold PBS, 0.6% PVP, 0.1 mM PMSF and 1 mM EDTA. The homogenate was clarified by filtration, centrifuged and the supernatant incubated for 4 h with 1.5 ml polyclonal antiHSA coupled to Reactigel spheres (Pierce Chem) in the presence of 0.5% Tween 80. The complex HSA-anti HSA-. Reactigel was collected and washed with 5 ml 0.5% Tween 80 in PBS. HSA was desorbed from the reactigel complex with 2.5 ml of 0.1 M glycine pH 2.5,10% dioxane, immediately followed by a buffer exchange with Sephadex G25 to 50 mM Tris pH 8. The sample was then loaded on a HR5/5 MonoQ anion exchange column (Pharmacia) and eluted with a linear NaCl gradient (0 - 350 mM NaCl in 50 mM Tris pH 8 in 20 min at 1ml/min). Fractions containing the concentrated HSA (at 290 mM NaCl) were lyophilized and applied to a HR 10/30 Sepharose 6 column (Pharmacia) in PBS at 0.3 ml/min. However, this method uses affinity columns (polyclonal anti-HSA) that are very expensive to scale-up. Also, the protein is released from the column with 0.1 M glycine pH 2.5 that typically denatures the protein. Therefore, this method can be suitably modified.

The second method is used for HSA extraction and purification from potato tubers (Dr. Mingo-Castel's laboratory). After grinding the tuber in phosphate buffer pH 7.4 (1 mg/2ml), the homogenate is filtered in miracloth and centrifuged at 14,000 rpm 15 minutes. After this step, another filtration of the supernatant in 0.45 µm filters is necessary. Then, chromatography of ionic exchange in FPLC using a DEAE Sepharose Fast Flow column (Amersham) is required Fractions recovered are passed through an affinity column (Blue Sepharose fast flow Amersham) resulting in a product of high purity. HSA purification based on both methods can then be investigated.

**7) Characterization of the Recombinant proteins:** For the safe use of recombinant proteins as a replacement in any of the current applications, these proteins must be structurally equivalent and must not contain abnormal host-derived modifications. To confirm compliance with these criteria human and recombinant proteins can be compared using the currently highly sensitive and highly resolving techniques expected by the regulatory authorities to characterize recombinant products (85).
1- Amino acid analysis: N-terminal sequence analysis is performed by Edman degradation using AB1477 A protein sequencer witt an on-line 120A phenylthiohydantoin-amino acid analyzer. Automated C-terminal sequence analysis uses a Hewlett-Packard G 1009A protein sequencer. The C-terminal tryptic peptide is isolated from tryptic digests by reverse-phase HPLC to confirm the C-terminal sequence to a greater number of residues.
2- Protein folding and disulfide bridges formation: Western blots with reducing and non-reducing gels is performed to check protein folding. Protein standards (Sigma) are loaded to compare the mobility of the recombinant protein. PAGE is performed on PhastGels (Pharmacia Biotech). Proteins are blotted and then probed with goat anti-HSA antibodies. Bound IgG is detected with horseradish peroxidase-labelled anti goat IgG and visualized on X-ray film using ECL detection reagents (Amersham).
3- Chromatographic techniques: For HSA, analytical gel-permeation HPLC is performed using a TSK G3000 SWx1 column. Preparative gel permeation chromatography of HSA is performed using a Sephacryl S200 HR column. The monomer fraction, identified by absorbance at 280 nm, is dialyzed and reconcentrated to its starting concentration.
4- Viscosity: This is a classical assay for recombinant HSA. Viscosity is a characteristic of proteins related directly to their size, shape, and conformation. The viscosities of HSA and recombinant HSA are measured at 100 mgMl-1 in 0.15 M NaCl using a U-tube viscosimeter (M2 type, Poulton, Selfe and Lee Ltd, Essex, UK) at 25°C.
5- Glycosylation: Chloroplast proteins are not known to be glycosylated. However there are no publications to confirm or refute this assumption. Therefore, glycosylation will be measured using a scaled-up version of the method of Ahmed and Furth (86).

8) **Animal testing and Pre-Clinical Trials:** When albumin is produced at adequate levels in tobacco and the physicochemical properties of the product correspond to those of the natural protein, toxicology studies need to be done in mice. To avoid mice response to the human protein, transgenic mice carrying HSA genomic sequences is used (87). After injection of none, 1,10,50 and 100 mg of purified recombinant protein, classical toxicology studies are carried out (body weight and food intake, animal behavior, piloerection, etc.). Albumin can be tested for blood volume replacement after paracentesis to eliminate the fluid from the peritoneal cavity in patients with liver cirrhosis. It has been shown that albumin infusion after this maneuver is essential to preserve effective circulatory volume and renal function (88).

### References

1. Brennan S, Owen M, Boswell D, Lewis J, Carrell R (1984). Circulating proalbumin associated with a variant proteinase inhibitor. Biochim. Biophys. Acta 802: 24 - 28.
2. Lawn RM, Adelman J, Bock SC, Franks AE, Houck CM, Najarian RC, Seeburg PH, Wion KL (1981). The sequence of human serum albumin cDNA and its expression in E. coli. Nucleic Acids Res. 9(22): 6103 - 114.
3. Latta M, Knapp M, Sarmientos P, Brefort G, Becquart J, Guerrier L, Jung G and Mayaux J (1987). Synthesis and purification of mature human serum albumin from E. coli. Bio/Technology 5: 1309 - 1314.
4. Saunders C, Schmidt B, Mallonee R, Guyer M (1987). Secretion of human serum albumin from Bacillus subtilis. J. Bact. 169: 2917 - 2925.
5. Sumi, et al. (1993). Biotechnology of Bloods proteins 227. Rivat and Stoltz eds. Pp 293 - 298.
6. Okabayashi K, NakagawaY, Hayasuke N, Ohi H, Miura M, Ishida Y, Shimizu M, Murakami K, Hirabayashi K, Minamino H, et al. (1991). Secretory expression of the human serum albumin gene in the yeast Saccharomyces cerevisiae. J. Biochem. (Tokyo) 110(1): 103 - 10.
7. Quirk AV, Geisow MJ, Woodrow JR, Burton SJ, Wood PC, Sutton AD, Johnson RA, DodsworthN(1989). Production of recombinant human serum albumin from Saccharomyces cerevisiae. Biotechnol. Appl. Biochem. 11(3): 273 - 87.
8. Sleep D, Belfield GP, Goodey AR (1990). The secretion of human serum albumin from the yeast Saccharomyces cerevisiae using five different leader sequences. Biotechnology (NY) 8(1): 42-6.
9. Dodsworth N, Harris R, Denton K, Woodrow J, Wood PC, Quirk A (1996). Comparative studies of recombinant human albumin and human serum albumin derived by blood fractionation. Biotechnol Appl. Biochem. 24( Pt 2): 171-6.
10. Saliola M, Mazzoni C, Solimando N, Crisa A, Falcone C, Jung G, Fleer R (1999). Use of the KIADH4 promoter for ethanol-dependent production of recombinant human serum albumin in Kluyveromyces lactis. Appl. Environ. Microbiol. 65(1): 53 - 60.
11. Ohtani W, Nawa Y, Takeshima K, Kamuro H, Kobayashi K, Ohmura T (1998). Physicochemical and immunochemical properties of recombinant human serum albumin from Pichia pastoris. Anal. Biochem. 256(1): 56 - 62.
12. Sijmons PC, Dekker BM, Schrammeijer B, Verwoerd TC, van den Elzen PJ, Hockema A (1990). Production of correctly processed human serum albumin in transgenic plants. Biotechnology (NY) 8(3): 217-21.
13. Daniell H, McFadden BA (1988). Genetic Engineering of plant chloroplasts. United States Patents 5,932,479; 5,693,507.
14. Daniell H (1999). Universal chloroplast integration and expression vectors, transformed plants and products thereof. World Intellectual Property Organization WO 99/10513.
15. Carlson PS (1973). The use of protoplasts for genetic research. Proc. Natl. Acad Sci. USA 70: 598 - 602.
16. Daniell H, Rebeiz CA (1982). Chloroplast culture IX: Chlorphyll(ide) A bio-synthesis in vitro at rates higher than in vivo. Biochem. Biophys. Res. Comun. 106: 466 - 471.
17. Daniell H, Ramanujan P, Krishnan M, Gnanam A, Rebeiz CA (1983). In vitro synthesis of photosynthetic membranes: I. Development of photosystem I activity and cyclic phosphorylation. Biochem. Biophys. Res. Comun. 111: 740 - 749.
18. Daniell H, Krishnan M, Umabai U, Gnanam A (1986). An efficient and prolonged in vitro translational system from cucumber etioplasts. Biochem. Biophys. Res. Comun. 135: 48 - 255.
19. Daniell H, McFadden BA (1987). Uptake and expression of bacterial and cyanobacterial genes by isolated cucumber ctioplasts. Proc. Natl. Acad. Sci. USA 84: 6349 - 6353.
20. Daniell H (1993). Foreign gene expression in chloroplasts of higher plants mediated by tungsten particle bombardment. Methods Enzymol 217: 536 - 556.
21. Daniell H, Vivekananda J, Neilsen B, Ye GN, Tewari KK, Sanford JC (1990). Transient foreign gene expression in chloroplasts of cultured tobacco cells following biolistic delivery of chloroplast vectors. Proc. Natl. Acad. Sci. USA 87: 88-92.
22. Ye X, et al. (2000). Engineering the provitamin A (β-carotene) biosynthetic pathway into (carotenoid-free) rice endosperm. Science 287: 303 - 305.
23. Daniell H, Krishnan M, McFadden BA (1991). Expression of B-glucuronidase gene in different cellular compartments following biolistic delivery of foreign DNA into wheat leaves and calli. Plant Cell Reports 9: 615 - 619.
24. Svab Z, Maliga P (1993). High frequency plastid transformation in tobacco by selection for a chimeric aadA gene. Proc. Natl. Acad. Sci. USA 90: 913-917.
25. McBride KE, Svab Z, Schaaf DJ, Hogen PS, Stalker DM, Maliga P (1995). Amplification of a chimeric Bacillus gene in chloroplasts leads to extraordinary level of an insecticidal protein in tobacco. Bio/technology 13: 362 - 365.
26. Kota M, Daniell H, Varma S, Garczynski F, Gould F, Moar WJ (1999). Overexpression of the Bacillus thuringiensis Cry2A protein in chloroplasts confers resistance to plants against susceptible and Bt-resistant insects. Proc. Natl. Acad. Sci. USA 96: 1840 - 1845.
27. Daniell H, Datta R, Varma S, Gray S, Lee SB (1998). Containment of herbicide resistance through genetic engineering of the chloroplast genome. Nature Biotechnology 16: 345 - 348.
28. Sidorov VA, Kasten D, Pang SZ, Hajdukiewicz PTJ, Staub JM, Nehra NS (1999). Stable chloroplast transformation in potato: use of green fluorescent protein as a plastid marker. Plant Journal 19: 209 - 216.
29. DeCosa B, Moar W, Lee SB, Miller M, Daniell H (2001). Hyper-expression of the Bt Cry2Aa2 operon in chloroplasts leads to formation of insecticidal crystals. Nature Biotechnology, In press.
30. Guda C, Lee SB, Daniell H (2000). Stable expression of biodegradable protein based polymer in tobacco chloroplasts. Plant Cell Rep. 19: 257 - 262.
31. Staub JM, Garcia B, Graves J, Hajdukiewicz PT, Hunter P, Nehra N, Paradkar V, Schlittler M, Carroll JA, Spatola L, Ward D, Ye G, Russell DA (2000). High-yield production of a human therapeutic protein in tobacco chloroplasts. Nat. Bio-technol.18(3): 333 - 338.
32. Bogorad L (2000). Engineering chloroplasts: an alternative site for foreign genes, proteins, reactions and products. Trends in Biotechnology 18: 257 - 263.
33. Navrath C, Poirier Y, Somerville C (1994). Targeting of the polyhydroxybutyrate biosynthetic pathway to the plastids of Arabidopsis thaliana results in high levels of polymer accumulation. Proc. Natl. Acad. Sci. 91: 12760 - 12764.
34. Ma JK, Hiatt A, Hein M, Vine ND, Wang F, Stabila P, van Dolleweerd C, Mostov K, Lehner T(1995). Generation and assembly of secretory antibodies in plants. Science 268: 716-719.
35. Ge B et al. (1998). Differential effects of helper proteins encoded by the cry2A and cry11A operons on the formation of Cry2A inclusions in Bacillus thuringiensis. FEMS Microbiol. Lett 165: 35 - 41.
36. Hancock R, LehrerR(1998). Cationic peptides: a new source of antibiotics. TIBTECH 16: 82 - 88.
37. Biggin P, Sansom M (1999). Interactions of α-helices with lipid bilayers: a review of simulation studies. Biophysical Chemistry 76: 161 - 183.
38. Jacob L, Zasloff M (1994). Potential therapeutic applications of magainins and other antimicrobial agents of animal origin. Ciba Foundation Symposium 186: 197 - 223.
39. DeGray G, Smith F, Sanford J, Daniell H (2001). Hyper-expression of an antimicrobial peptide via the chloroplast genome to confer resistance against phytopathogenic bacteria. In review.
40. Lebens M, Holmgren J (1994). Mucosal vaccines based on the use of Cholera Toxin B subunit as immunogen and antigen carrier. Recombinant Vectors in Vaccine Development [Brown F (ed )]. 82: 215 - 227.
41. Mor TS, Palmer KE, et al. (1998). Perspective: edible vaccines- a concept coming of age. Trends in Microbiology 6: 449 - 453.
42. Lipscombe M, Charles IG, Roberts M, Dougan G, Tite J, Fairweather NF (1991). Intranasal immunization using the B subunit of the Escherichia coli heat-labile toxin fused to an epitope of the Bordetella pertussis P.69 antigen. Mol. Microbiol. 5(6): 1385 - 1392.
43. Dertzbaugh MT, Elson CO (1993). Comparitive effectiveness of the cholera toxin B subunit and alkaline phosphatase as carriers for oral vaccines. Infect. Immun. 61: 48 - 55.
44. Holmgren J, Lycke N, Czerkinsky C (1993). Cholera toxin and cholera B subunit as oral-mucosal adjuvant and antigen vector systems. Vaccine. 11(12): 1179-84. Review.
45. Nashar TO, Amin T, Marcello A, Hirst TR (1993). Current progress in the development of the B subunits of cholera toxin and Escherichia coli beat-labile enterotoxin as carriers for the oral delivery of heterologous antigens and epitopes. Vaccine. 11(2): 235 - 40.
46. Sun JB, Holmgren J, Czerkinsky C (1994). Cholera toxin B subunit: an efficient transmucosal carrier-delivery system for induction of peripheral immunological tolerance. Proc. Natl. Acad. Sci. USA 91: 10795 -10799..
47. Henriques L and Daniell H (2001). Expression of cholera toxin B subunit oligomers in transgenic tobacco chloroplasts. In review.
48. Hotz P, Guggenheim B, Schmid R (1972). Carbohydrates in pooled dental plaque. Caries Res. 6(2): 103-21.
49. Ma J, Hitmak B, Wycoff K, Vine N, Charlegue D, Yu L1, Hein M, Lehner T (1998). Characterization of a recombinant plant monoclonal secretory antibody and preventive immunotherapy in humans. Nature Medicine. 4(5): 601 - 606.
50. Panchal T, WycoffK and Daniell H (2001). Expression of humanized antibody in transgenic tobacco chloroplasts. In review.
51. Daniell H, Streatfield S and WycoffK (2001). Medical molecular pharming: production of antibodies, biopharmaceuticals and edible vaccines in plants. Trends in Plant Science. In press.
52. Kusnadi A, Nikolov Z, Howad J (1997). Production of Recombinant proteins in Transgenic plants: Practical considerations. Biotechnology and Bioengineering. 56 (5): 473 - 484.
53. May GD, Mason HS, Lyons PC (1996). Application of transgenic plants as production systems for pharmaceuticals in ACS Symposium series 647. Fuller et al. eds., chapter 13,196 - 204.
54. Petridis D, Sapidou E, Calandranis J (1995). Computer-Aided process analysis and economic evaluation for biosynthetic human insulin production-A case Study. Biotechnology and Bioengineering 48: 529 - 541.
55. Drescher DF, Follmann H, Haberlein I (1998). Sulfitolysis and thioredoxin-dependent reduction reveal the presence of a structural disulfide bridge in spinach chloroplast fructose-1, 6-bisphosphate. FEBS Letters 424: 109-112.
56. RoyH(1989). Rubisco assembly: a model system for studying the mechanism of chaperonin action. Plant Cell. 1: 1035 - 1042.
57. Vierling E (1991). The roles of heat shock proteins in plants. Annu. Rev. Plant Physiol. Plant Mol. Biol. 42: 579 - 620.
58. Reulland E, Miginiac-Maslow M (1999). Regulation of chloroplast enzyme activities by thioredoxins: activation or relief from inhibition. Trends in Plant Science 4: 136-141.
59. Kim J, Mayfield PS (1997). Protein disulfide isomerase as a regulator of chloroplast translational activation. Science 278: 1954 - 1957.
60. Twell D, y Ooms G (1987). The 5' flanking DNA of a patatin gene directs tuber specific expression of a chimaeric gene in potato. Plant Mol. Biol. 9: 365 - 375.
61. Sánchez-Serrano JJ, Schmidt R, Schell J, y Willmitzer L (1986). Nucleotide sequence of proteinase inhibitor II encoding cDNA of potato (Solanum tuberosum) and its mode of expression. Mol. Gen. Genet 203: 15 - 20.
62. Vaucheret H, Beclin C, Elmayan T, Feuerbach F, Godon C, Morel JB, Mourrain P, Palauqui JC, Vernhettes S (1998). Transgene induced gene silencing in plants. Plant J.16: 651- 659.
63. De Neve M, De Buck S, De Wilde C, Van Houdt H, Strobbe I, Jacobs A, Van Montagu, DepickerA(1999). Gene silencing results in instability of antibody production in transgenic plants. Mol. Gen. Genetics 260: 582 - 592.
64. Brixey J, Guda C, Daniell H (1997). The chloroplast psbA promoter is more efficient in E. coli than the T7 promoter for hyper expression of a foreign protein. Biotechnology Letters 19: 395 - 400.
65. Daniell H (1997). Transformation and foreign gene expression in plants mediated by microprojectile bombardment Meth. Mol. Biol. 62: 453 - 488.
66. Berry-Lowe S and Schmidt G (1991). In The Molecular Biology of Plastids. Bogorad & Vasil Eds., Academic Press 10: 257 - 302.
67. Keegstra K and Cline K (1999). Protein Import and Routing System of Chloroplasts. The Plant Cell 11: 557 - 570.
68. Gregory WS and Mishkind ML (1986). The transport of proteins into chloroplasts. Ann. Rev. Biochem. 55: 879 - 912.
69. Keegstra K (1989). Transport and routing of proteins into chloroplasts. Cell 56: 247 - 253. Intranasal immunization using the B subunit of the Escherichia coli heat-labile toxin fused to an epitope of the Bordetella pertussis P.69 antigen. Mol. Microbiol. 5(6): 1385-1392.
70. Smeekens S, Weisbeek P, Robinson C (1990). Protein transport into and within chloroplasts. Trends Biochem. Sci. 15(2): 73 - 6.
71. Rangasamy D, Ratledge C, Woolston C (1997). Plastid targeting and transient expression of rat liver ATP: citrate lyase in pea protoplasts. Plant Cell Reports 16: 700 - 704.
72. Rangasamy D and Ratledge C (2000). Genetic enhancement of Fatty acid synthesis by targeting Rat Liver ATP: Cytrate Lyase into plastids of tobacco. Plant Physiology 122: 1231-1238.
73. Uhlen M, Nilsson B, Guss B, Lindberg M, Gatenbeck S, Philipson L (1983). Gene fusion vectors based on the gene for staphylococcal protein A, Gene. 23(3): 369 -378.
74. Uhlen M, Forsberg G, Moks T, Hartmanis M, Nilsson B (1992). Fusion proteins in biotechnology. Curr. Opin. Biotechnol. 3(4): 363 - 369.
75. Nygren PA, Stahl S, Uhlen M (1994). Engineering proteins to facilitate bio-processing. Trends in Biotechnol. 12(5): 184 - 8.
76. M Wang, WA Scott, KR Rao, J Udey, GE Conner and Brew K (1989). Recombinant bovine alpha-lactalbumin obtained by limited proteolysis of a fusion protein expressed at high levels in Escherichia coli. J. Biol. Chem. 264: 21116 - 21121.
77. Gonzales T, Robert-Baudouy J (1996). Bacterial aminopeptidases: properties and functions. FEMS Microbiol Rev. 18(4): 319 - 44.
78. Cohen A, Mayfield (1997). Translational regulation of gene expression in plants. Current Opinion in Biotechnology 8: 189 -194.
79. Lee SB, Kwon H, Kwon S, Park S, Jeong M, Han S, Daniell H, Byun H (2001). Drought tolerance conferred by the yeast trehalose-6 phosphate synthase gene engineered via the chloroplast genome In review.
80. Eibl C, Zou Z, BeckA, Kim M, Mullet J, Koop UH (1999). In vivo analysis ofplastid psbA, rbcL and rp132 UTR elements by chloroplast transformation: tobacco plastid gene expression is controlled by modulation of transcript levels and translation efficiency. The Plant Journal 19: 333 - 345.
81. Morton B (1993). Chloroplast DNA Codon Use: Evidence for Selection at the psbA Locus . Based on tRNA Availability. J Mol Evol 37: 273 - 280.
82. Morton B and Bernadette G (2000). Codon usage in plastid genes is correlated with context, position within the gene, and amino acid content. J Mol Evol. 50: 184 -193.
83. Edwards K, Johnstone C, Thompson C (1991). A simple and rapid method for preparation of plant genomic DNA for PCR analysis. Nucleic Acids Res. 19: 1349.
84. Sambrook J, Fritch EF, Maniatis T (1989). Molecular cloning. Cold Spring Harbor Press, Cold Spring Harbor, New York.
85. Federici M (1994). The quality control of biotechnology products. Biologicals 22(2): 151 - 9.
86. Ahmed N, Furth AJ (1991). A microassay for protein glycation based on the periodate method. Anal Biochem 192(1): 109-11.
87. Shani M, Barash I,Nathan M, Ricca G, Searfoss GH, Dekel I, Faerman A, Givol D, Hurwitz DR. (1992). Expression of human serum albumin in the milk of transgenic mice. Transgenic Res. Sep; 1(5): 195 - 208.
88. Gines P, Arroyo V, Vargas V, Planas R, Casafont F, Panes-J, Hoyos M, Viladomiu L, Rimola A, Morillas R, et al. (1991). Paracentesis with intravenous infusion of albumin as compared with pentoneovenous shunting in cirrhosis with refractory ascites. N Engl J Med. 19; 325(12): 829 35.

The present invention is directed to the expression of a gene coding for cholera toxin B-subunit fused to proinsulin in plants to produce recombinant proteins.

Research on human proteins in the past years has revolutionized the use of these therapeutically valuable proteins in a variety of clinical situations. Since the demand for these proteins is expected to increase considerably in the coming years, it would be wise to ensure that in the future they will be available in significantly larger amounts, preferably on a cost-effective basis. Because most genes can be expressed in many different systems, it is essential to determine which system offers the most advantages for the manufacture of the recombinant protein. The ideal expression system would be one that produces a maximum amount of safe, biologically active material at a minimum cost. The use of modified mammalian cells with recombinant DNA techniques has the advantage of resulting in products which are closely related to those of natural origin; however, culturing of these cells is intricate and can only be carried out on limited scale. The use of microorganisms such as bacteria permits manufacture on a larger scale, but introduces the disadvantage of producing products, which differ appreciably from the products of natural origin. For example, proteins that are usually glycosylated in humans are not glycosylated by bacteria. Furthermore, human proteins that are expressed at high levels in E. *coli* frequently acquire an unnatural conformation, accompanied by intracellular precipitation due to lack of proper folding and disulfide bridges. Production of recombinant proteins in plants has many potential advantages for generating biopharmaceuticals relevant to clinical medicine. These include the following: (I) plant systems are more economical than industrial facilities using fermentation systems; (ii) technology is available for harvesting and processing plants/ plant products on a large scale; (iii) elimination of the purification requirement when the plant tissue containing the recombinant protein is used as a food (edible vaccines); (iv) plants can be directed to target proteins into stable, intracellular compartments as chloroplasts, or expressed directly in chloroplasts; (v) the amount of recombinant product that can be produced approaches industrial-scale levels; and (vi) health risks due to contamination with potential human pathogens/toxins are minimized.

It has been estimated that one tobacco plant should be able to produce more recombinant protein than a 300-liter fennenter of E *coli.* In addition, a tobacco plant produces a million ing large-scale production. Tobacco is also an ideal choice because of its relative ease of genetic manipulation and an impending need to explore alternate uses for this hazardous crop. However, with the exception of enzymes (e.g. phytase), levels of foreign proteins produced in nuclear transgenic plants are generally low, mostly less than 1% of the total soluble protein (1). May et al. (2a) discuss this problem using the following examples. Although plant derived recombinant hepatitis B surface antigen was as effective as a commercial recombinant vaccine, the levels of expression in transgenic tobacco were low (0.0066% of total soluble protein). Even though Norwalk virus capsid protein expressed in potatoes caused oral immunization when consumed as food (edible vaccine), expression levels were low (0.3°.b of total soluble protein). In particular, expression of human proteins in nuclear transgenic plants has been disappointingly low: e.g. human Interferan-□ 0.000017% of fresh weight, human serum albumin 0.02% and erythropoietin 0.0026% of total soluble protein (see table1 in refl). A synthetic gene coding for the human epidermal growth factor was expressed only up to 0.0012A of total soluble protein in transgenic tobacco (2a). The cost of producing recombinant proteins in alfalfa leaves was estimated to be 12-fold lower than in potato tubers and comparable with seeds (1). However, tobacco leaves are much larger and have much higher biomass than alfalfa. The cost of production of recombinant proteins will be 50-fold lower than that of *E.coli* fermentation (with 20% expression levels, 1). A decrease in insulin expression from 20% to 5% of biomass doubled the cost of production (2b). Expression level less than 1% of total soluble protein in plants has been found to be not commercially feasible (1). Therefore, it is important to increases levels of expression of recombinant proteins in plants in order to exploit plant production of pharmacologically important proteins.

An alternate approach is to express foreign proteins in chloroplasts of higher plants. We have recently integrated foreign genes (up to 10,000 copies per cell) into the tobacco chloroplast genome resulting in accumulation of recombinant proteins up to 47% of the total cellular protein (3). Chloroplast transformation utilizes two flanking sequences that, through homologous recombination, insert foreign DNA into the spacer region between the functional genes of the chloroplast genome, thus targeting the foreign genes to a precise location. This eliminates the "position effect" and gene silencing frequently observed in nuclear transgenic plants. Chloroplast genetic engineering is an environmentally friendly approach, minimizing concerns of out-cross of introduced traits via pollen to weeds or other crops. Also, the concerns of insects developing resistance to biopesticides are minimized by hyper-expression of single insecticidal proteins (high dosage) or expression of different types of insecticides in a single transformation event (gene pyramiding). Concerns of insecticidal proteins on non-target insects are minimized by lack of expression in transgenic pollen. Most importantly, a significant advantage in the pharmaceutical proteins in chloroplasts is their ability to process eukaryotic proteins, including folding and formation of disulfide bridges (4). Chaperonin proteins are present in chloroplasts (5,6) that function in folding and assembly of prokaryotic/eukaryotic proteins. Also, proteins are activated by disulfide bond oxido/reduction cycles using the chloroplast thioredoxin system (7) or chloroplast protein disulfide isomerase (8). Accumulation of fully assembled, disulfide bonded form of human somatotropin via chloroplast transformation (9) and oligomeric form of CTB (10) and assembly of heavy and light chains of humanized Guy's 13 antibody in transgenic chloroplasts (11) provide strong evidence for successful processing of pharmaceutical proteins inside chloroplasts. Such folding and assembly should eliminate the need for highly expensive *in vitro* processing of pharmaceutical proteins. For example, 60% of the total operating cost in the production of human insulin is associated with in *vitro* processing (formation of disufide bridges and cleavage of methionine)(2b).

Taken together, low levels of expression of human proteins in nuclear transgenic plants, and difficulty in folding, assembly/processing of human proteins in *E.coli* should make chloroplasts an ideal compartment for expression of these proteins; production of human proteins in transgenic chloroplasts should also dramatically lower the production cost. Large-scale production of these proteins in plants should be a powerful approach to provide treatment to patients at an affordable cost and provide tobacco farmers alternate uses for this hazardous crop. Therefore, we propose here expression of therapeutic proteins in transgenic tobacco chloroplasts to increase levels of expression and accomplish in *vivo* processing.

### BRIEF DESCRIPTION OF THE FIGURES

- Figure 4: is a graphical representation of total protein versus leaf age in transgenic tobacco plants.
- Figure 5: is an electron micrograph showingCry2Aa2 crystals in a transgenic tobacco leaf.
- Figure 6: is a photograph of leaves infected with P. *syringae* 5 days after inoculation.
- Figure 7: is a graph showing the results of an in vitro assay of *P. aeruginosa.*
- Figure 8: is two graphs showing oligomeric CTB expression levels as Total Soluble Protein.
- Figure 9: is a Western Blot Analysis of transgenic chloroplast expressed CTB and commercially available purified CTB- antigen.
- Figure 10: is a Western Blot Analysis of heavy and light chains of Guy's 13 monoclonal antibody from plant chloroplasts.
- Figure 16: is a Western Blot of transgenic potato tubers, cv Desiree.
- Figure 17: is a frequency histogram including percentage Kennebec and Désirée transgenci plants expressing different HAS levels.
- Figure 18: is a Western Blot of E.*coli* protein extracts.

When the concept of chloroplast genetic engineering was developed (72,73), it was possible to introduce isolated intact chloroplasts into protoplasts and regenerate transgenic plants (74). Therefore, early investigations on chloroplast transformation focused on the development of in *organello* systems using intact chloroplasts capable of efficient and prolonged transcription and translation (75-77) and expression of foreign *genes* in isolated chloroplasts (78). However, after the discovery of the gene gun as a transformation device (79), it was possible to transform plant chloroplasts without the use of isolated plastids and protoplasts. Chloroplast genetic engineering was accomplished in several phases. Transient expression of foreign genes in plastids of dicots (80,81) was followed by such studies in monocots (82). Unique to the chloroplast genetic engineering is the development of a foreign gene expression system using autonomously replicating chloroplast expression vectors (80). Stable integration of a selectable marker gene into the tobacco chloroplast genome (83) was also accomplished using the gene gun. However, useful genes conferring valuable traits via chloroplast genetic engineering have been demonstrated only recently. For example, plants resistant to B.t. sensitive insects were obtained by integrating the *cryIac* gene into the tobacco chloroplast genome (84). Plants resistant to B.t. resistant insects (up to 40,000 fold) were obtained by hyper-expression of the *cry2A* gene within the tobacco chloroplast genome (85). Plants have also been genetically engineered via the chloroplast genome to confer herbicide resistance and the introduced foreign genes were maternally inherited, overcoming the problem of out-cross with weeds (86). Chloroplast genetic engineering technology is currently being applied to other useful crops (73,87).

A remarkable feature of chloroplast genetic engineering is the observation of exceptionally large accumulation of foreign proteins in transgenic plants, as much as 46% of n total soluble protein, even in bleached old leaves (3). Stable expression of a pharmaceutical protein in chloroplasts was first reported for GVGVP, a protein based polymer with varied medical applications (such as the prevention of post-surgical adhesions and scars, wound coverings, artificial pericardia, tissue reconstruction and programmed drug delivery (88)). Subsequently, Expression of the human somatotropin via the tobacco chloroplast genome (9) to high levels (7% of total soluble protein) was observed. The following investigations that are in progress in the Daniell laboratory illustrate the power of this technology to express small peptides, entire operons, vaccines that require oligomeric proteins with stable disulfide bridges and monoclonals that require assembly of heavy/light chains via chaperonins.

In plant and animal cells, nuclear mRNAs are translated monocistronically. This poses a serious problem when engineering multiple genes in plants (91). Therefore, in order to express the polyhydroxybutyrate polymers or Guy's 13 antibody, single genes were first introduced into individual transgenic plants, then these plants were beck-crossed to reconstitute the entire pathway or the complete protein (92,93). Similarly, in a seven year long effort, Ye et al. (81) recently introduced a set of three genes for a short biosynthetic pathway that resulted in β-carotene expression in rice. In contrast, most chloroplast genes of higher plants are cotranscribed (91). Expression of polycistrons via the chloroplast genome provides a unique opportunity to express entire pathways in a single transformation event. The *Bacillus thuringiensis* (Bt) *cry*2Aa2 operon has recently been used as a model system to demonstrate operon expression and crystal formation via the chloroplast genome (3). Cry2Aa2 is the distal gene of a three-gene operon. The orf immediately upstream of *cry*2Aa2 codes for a putative chaperonin that facilitates the folding of *cry*2Aa2 (and other proteins) to form proteolytically stable cuboidal crystals (94).

Therefore, the *cry*2Aa2 bacterial operon was expressed in tobacco chloroplasts to test the resultant transgenic plants for increased expression and improved persistence of the accumulated insecticidal protein(s). Stable foreign gene integration was confirmed by PCR and Southern blot analysis in T₀ and T₁ transgenic plants. *Cry2Aa2* operon derived protein accumulated at 45.3% of the total soluble protein in mature leaves and remained stable even in old bleached leaves (46.1%) (FIG. 4). This is the highest level of foreign gene expression ever reported in transgenic plants. Exceedingly difficulty to control insects (10-day old cotton bollworm, beetarmy worm) were killed 100% after consuming transgenic leaves. Electron micrographs showed the presence of the insecticidal protein folded into cuboidal crystals similar in shape to *Cry2Aa2* crystals observed in *Bacillus thuringiensis* (FIG. 5)*.* In contrast to currently marketed Transgenic plants with soluble CRY proteins, folded protoxin crystals will be processed only by target insects that have alkaline gut pH; this approach should improve safety of Bt transgenic plants. Absence of insecticidal proteins in transgenic pollen eliminates toxicity to non-target Ilen. In addition to these environmentally friendly approaches, this observation should serve as a model system for large-scale production of foreign proteins within chloroplasts in a folded configuration enhancing their stability and facilitating single step purification. This is the fast demonstration of expression of a bacterial operon in transgenic plants and opens the door to engineer novel pathways in plants in a single transformation event.

It is common knowledge that the medical community has been fighting a vigorous battle against drug resistant pathogenic bacteria for years. Cationic antibacterial peptides from mammals, amphibians and insects have gained more attention over the last decade (95). Key features of these cationic peptides are a net positive charged, an affinity for negatively-charged prokaryotic membrane phospholipids over neutral-charged eukaryotic membranes and the ability to form aggregates that disrupt the bacterial membrane (96).

There are three major peptides with α-helical structures, cecropin from *Hyalophora cecropia* (giant- silk moth), magainins from *Xenopus laevis* (African frog) and defensins from mammalian neutrophils. Magainin and its analogues have been studied as a broad-spectrum topical agent, a systemic antibiotic, a wound-healing stimulant; and an anticancer agent (97). We have recently observed that a synthetic lytic peptide (MSI-99, 22 amino acids) can be successfully expressed in tobacco chloroplast (98). The peptide retained its lytic activity against the phytopathogenic bacteria *Pseudomonas syringae* and multidrug resistant human pathogen. *Pseudomonas aeruginosa.* The anti-microbial peptide (AMP) used in this study was an amphipathic alpha-helix molecule that has an affinity for negatively charged phospholipids commonly found in the outer-membrane of bacteria. Upon contact with these membranes, individual peptides aggregate to form pores in the membrane, resulting in bacterial lysis. Because of the concentration dependent action of the AMP, it was expressed via the chloroplast genome to accomplish high dose delivery at the point of infection. PCR products and Southern blots confirmed chloroplast integration of the foreign genes and homoplasmy. Growth and development of the transgenic plants was unaffected by hyper-expression of the AMP within chloroplasts. *In vitro* assays with T₀ and T₁ plants confirmed that the AMP was expressed at high levels (21.5 to 43% of the total soluble protein) and remained biological activity against *Pseudomonas syringae*, a major plant pathogen. *In situ* assays resulted in intense areas of necrosis around the point of infection in control leaves, while transformed leaves showed no signs of necrosis (200-800 µg of AMP at the site of infection)(FIG. 6). T₁ *in vitro* assays against *Pseudomonas aeruginosa* (a multi-drug resistant human pathogen) displayed a 96% inhibition of growth (FIG. 7). These results give a new option in the battle against phytopathogenic and drug-resistant human pathogenic bacteria. Small peptides (like insulin) are lost organisms. However, stability of this AMP in chloroplasts opens up this compartment for expression of hormones and other small peptides.

### Experimental

### Example 1

### Evaluation of chloroplast gene expression

A systematic approach is used to identify and overcome potential limitations of foreign gene expression in chloroplasts of transgenic plants. This experiment increases the utility of chloroplast transformation system by scientists interested in expressing other foreign proteins. Therefore, it is important to systematically analyze transcription, RNA abundance, RNA stability, rate of protein synthesis and degradation, proper folding and biological activity. The rate of transcription of the introduced HSA gene is compared with the highly expressing endogenous chloroplast genes (rbcL, psbA, 16S rRNA), using run on transcription assays to le 16SrRNA promoter is operating as expected. The transcription efficiency of transgenic chloroplast containing each of the three constructs with different 5' regions is tested. Similarly, transgene RNA levels are monitored by northerns, dot blots and primer extension relative to endogenous rbcL, 16S rRNA or psbA. These results, along with run on transcription assays, provide valuable information of RNA stability, processing, etc. RNA appears to be extremely stable based on northern blot analysis. This systematic study is valuable to advance utility of this system by other scientists. Most importantly, the efficiency of translation is tested in isolated chloroplasts and compared with the highly translated chloroplast protein (psbA). Pulse chase experiments help assess if translational pausing, premature termination occurs. Evaluation of percent RNA loaded on polysomes or in constructs with or without 5'UTRs helps to determine the efficiency of the ribosome binding site and 5' stem-loop translational enhancers. Codon optimized genes (IGF-I, IFN) are compared with unmodified genes to investigate the rate of translation, pausing and termination. A 200-fold difference in accumulation of foreign proteins due to decreases in proteolysis conferred by a putative chaperonin (3) was observed. Therefore, proteins from constructs expressing or not expressing the putative chaperonin (with or without ORF1+2) provide valuable information on protein stability.

### Example 2

### Expression of the mature protein

HSA, Interferon and IGF-I are pre-proteins that need to be cleaved to secrete mature proteins. The codon for translation initiation is in the presequence. In chloroplasts, the necessity of expressing the mature protein forces introduction of this additional amino acid in coding sequences. In order to optimize expression levels, we first subclone the sequence of the mature proteins beginning with an ATG. Subsequent immunological assays in mice demonstrates the extra-methionine causes immunogenic response and low bioactivity. Alternatively, systems may also produce the mature protein. These systems can include the synthesis of a protein fused to a peptide that is cleaved intracellular (processed) by chloroplast enzymes or the use of chemical or enzymatic cleavage after partial purification of proteins from plant cells.

### Use of peptides that are cleaved in chloroplast

Staub et al. (9) reported chloroplast expression, of human somatotropin similar to the native human protein by using ubiquitin fusions that were cleaved in the stroma by an ubiquitin protease. However, the processing efficiency ranged from 30-80% and the cleavage site was not accurate. In order to process chloroplast expressed proteins a peptide which is cleaved in the stroma is essential. The transit peptide sequence of the RuBisCo (ribulose 1,5-bisphosphate mall subunit is an ideal choice. This transit peptide has been studied in depth (111). RuBisCo is one of the proteins that is synthesized in cytoplasm and transported postranslationally into the chloroplasts in an energy dependent process. The transit peptide is proteolytically removed upon transport in the stroma by the stromal processing peptidase (112). There are several sequences described for different species (113). A transit peptide consensus sequence for the RuBisCo small subunit of vascular plants is published by Keegstra et al. (114). The amino acids that are proximal to the C-terminal (41-59) are highly conserved in the higher plant transit sequences and belong to the domain which is involved in enzymatic cleavage (111). The RuBisCo small subunit transit peptide has been fused with various marker proteins (114,115), even with animal proteins (116,117), to target proteins to the chloroplast. Prior to transformation studies, the cleavage efficiency and accuracy are tested by in vitro translation of the fusion proteins and *in organello* import studies using intact chloroplasts. Thereafter, knowing the correct fusion sequence for producing the mature protein, such sequence encoding the amino terminal portion of tobacco chloroplast transit peptide is linked with the mature sequence of each protein. Codon composition of the tobacco RuBisCo small subunit transit peptide is compatible with chloroplast optimal translation (see section d3 and table 1 on page 30). Additional transit peptide sequences for targeting and cleavage in the chloroplast have been described (111). The lumen of thylakoids could also be a good target because thylakoids are readily purified. Lumenal proteins can be freed either by sonication or with a very low triton X100 concentration, although this requires insertion of additional amino acid sequences for efficient import (111).

### Example 3

### Use of chemical or enzymatic cleavage

The strategy of fusing a protein to a tag with affinity for a certain ligand has been used extensively for more than a decade to enable affinity purification of recombinant products (118-120). A vast number of cleavage methods, both chemical and enzymatic, have been investigated for this purpose (120). Chemical cleavage methods have low specificity and the relatively harsh cleavage conditions can result in chemical modifications of the released products (120). Some of the enzymatic methods offer significantly higher cleavage specificities together with high efficiency, e. g. H64A subtilisin, IgA protease and factor Xa (119,120), but these enzymes have the drawback of being quite expensive.

Trypsin, which cleaves C-terminal of basic amino-acid residues, has been used for a long time to cleave fusion proteins (14,121). Despite expected low specificity, trypsin has been shown to be useful for specific cleavage of fusion proteins, leaving basic residues within folded protein avaged (121). The use of trypsin only requires that the N-terminus of the mature protein be accessible to the protease and that the potential internal sites are protected in the native conformation. Trypsin has the additional advantage of being inexpensive and readily available. In the case of HSA, when it was expressed in *E*. *coli* with 6 additional codons coding for a trypsin cleavage site, HSA was processed successfully into the mature protein after treatment with the protease. In addition, the N-terminal sequence was found to be unique and identical to the sequence of natural HSA, the conversion was complete and no degradation products were observed (14). This in vitro maturation is selective because correctly folded albumin is highly resistant to trypsin cleavage at inner sites (14). This system could be tested for chloroplasts HSA vectors using protein expressed in *E. coli.*

Staub et al. (9) demonstrated that the chloroplast methionine aminopeptidase is active and they found 95% of removal of the first methionine of an ATG-somatotropin protein that was expressed via the chloroplast genome. There are several investigations that have shown a very strict pattern of cleavage by this peptidase (122). Methionine is only removed when second residues arc glycine, alanine, serine, cysteine, threonine, proline or valine, but if the third amino acid is proline the cleavage is inhibited. In the expression of our three proteins we use this approach to obtain the mature protein in the case of Interferon because the penultimate aminoacid is cystein followed by aspartic acid. For HSA the second aminoacid is aspartic acid and for IGF-I glycine but it is followed by proline, so the cleavage is not dependable.

For IGF-I expression, the use of the TEV protease (Gibco cat n 10127-017) would be ideal. The cleavage site that is recognized for this protease is Gln-Asn-Leu-Tyr-Phe-Gln-Gly and it cuts between Gln-Gly. This strategy allows the release of the mature protein by incubation with TEV protease leaving a glycine as the first amino acid consistent with human mature IGF-I protein.

The purification system of the *E. coli* Interferon-□5 expression method was based on 6 Histidine-tags that bind to a nickel column and biotinylated thrombin to eliminate the tag on IFN-□5. Thrombin recognizes Leu-Val-Pro-Arg-Gly-Ser and cuts between Arg and Gly. This leaves two extra amino acids in the mature protein, but antiviral activity studies have shown that this protein is at least as active as commercial IFN-□2.

### Example 4

### Optimization of gene expression

Foreign genes are expressed between 3% (*cry2Aa2*) and 47% (*cry2Aa2* operon) in transgenic chloroplasts (3,85). Based on the outcome of the evaluation of USA chloroplast transgenic plants, several approaches can be used to enhance translation of the recombinant loroplasts, transcriptional regulation of gene expression is less important, although some modulations by light and developmental conditions are observed (123). RNA stability appears to be one among the least problems because of observation of excessive accumulation of foreign transcripts, at times 16,966-fold higher than the highly expressing nuclear transgenic plants (124). Chloroplast gene expression is regulated to a large extent at the post-transcriptional level. For example, 5' UTRs are necessary for optimal translation of chloroplast mRNAs. Shine-Dalgarno (GGAGG) sequences, as well as a stem-loop structure located 5' adjacent to the SD sequence, are required for efficient translation. A recent study has shown that insertion of the psbA 5' UTR downstream of the 16S rRNA promoter enhanced translation of a foreign gene (GUS) hundred-fold (125a). Therefore, the 200-bp tobacco chloroplast DNA fragment (1680-1480) containing 5' psbA UTR should be used. This PCR product is inserted downstream of the 16S rRNA promoter to enhance translation of the recombinant proteins.

Yet another approach for enhancement of translation is to optimize codon compositions. Since all the three proteins are translated in *E. coli* (see section b), it would be reasonable to expect efficient expression in chloroplasts. However, optimizing codon compositions to match the psbA gene could further enhance the level of translation. Although rbcL (RuBisCO) is the most abundant protein on earth, it is not translated as highly as the psbA gene due to the extremely high turnover of the psbA gene product. The psbA gene is under stronger selection for increased translation efficiency and is the most abundant thylakoid protein. In addition, the codon usage in higher plant chloroplasts is biased towards the NNC codon of 2-fold degenerate groups (i.e. TTC over TTT, OAC over GAT, CAC over CAT, AAC over AAT, ATC over ATT, ATA etc.). This is in addition to a strong bias towards T at third position of 4-fold degenerate groups. There is also a context effect that should be taken into consideration while modifying specific codons. The 2-fold degenerate sites immediately upstream from a GNN codon do not show this bias towards NNC. (TTT GGA is preferred to TTC GGA while TTC CGT is preferred to TTT CGT, TTC AGT to TTT AGT and TTC TCT to TTT TCT)(125b,126). In addition, highly expressed chloroplast genes use GNN more frequently that other genes. Codon composition was optimized by comparing different species. Abundance of amino acids in chloroplasts and tRNA anticodons present in chloroplast must be taken into consideration. We also compared A+T% content of all foreign genes that had been expressed in transgenic chloroplasts in our laboratory with the percentage of chloroplast expression. We found that higher levels of A+T always correlated with high expression levels (see table I). It is also possible to modify chloroplast protease recognition sites while modifying codons, without affecting their biological functions.

The study of the sequences of HSA, IGF-I and Interferon-□5 was done. The HSA ed 57% of A+T content and 40% of the total codons matched with the psbA most translated codons. According to the data of table 1, we expected good chloroplast expression of the HSA gene without any modifications in its codon compositions. IFN-□5 has 54% of A+T content and 40% of matching with psbA codons. The composition seems to be good but this protein is small (166 amino acids) and the sequence was optimized to achieve A+T levels close to 65%. Finally, the analysis of the IGF-I sequence showed that the A+T content was 40% and only 20% of the codons are the most translated in psbA. Therefore, this gene needed to be optimized. Optimization of these two genes is done using a novel PCR approach (127,128) which has been successfully used to optimize codon composition of other human proteins.

### Example 5

### Vector constructions

For all the constructs pLD vector is used. This vector was developed in this laboratory for chloroplast Transformation. It contains the 16S rRNA promoter (Prm) driving the selectable marker gene *aadA* (aminoglycoside adenyl transferase conferring resistance to spectinomycin) followed by the *psbA* 3' region (the terminator from a gene coding for photosystem II reaction center components) from the tobacco chloroplast genome. The pLD vector is a universal chloroplast expression /integration vector and can be used to transform chloroplast genomes of several other plant species (73,86) because these flanking sequences are highly conserved among higher plants. The universal vector uses *trnA* and *trnI* genes (chloroplast transfer RNAs coding for Alanine and Isoleucine) from the inverted repeat region of the tobacco chloroplast genome as flanking sequences for homologous recombination. Because the universal vector integrates foreign genes within the Inverted Repeat region of the chloroplast genome, it should double the copy number of the transgene (from 5000 to 10,000 copies per cell in tobacco). Furthermore, it has been demonstrated that homoplasmy is achieved even in the first round of selection in tobacco probably because of the presence of a chloroplast origin of replication within the flanking sequence in the universal vector (thereby providing more templates for integration). Because of these and several other reasons, foreign gene expression was shown to be much higher when the universal vector was used instead of the tobacco specific vector (88).

The following vectors are used to optimize protein expression, purification and production of proteins with the same amino acid composition as in human proteins.
a) In order to optimize expression, translation is increased using the psbA 5'UTR and optimizing the codon composition for protein expression in chloroplasts according to criteria previously. The 200' bp tobacco chloroplast DNA fragment containing 5' psbA UTR is amplified by PCR using tobacco chloroplast DNA as template. This fragment is cloned directly in the pLD vector multiple cloning site (EcoRI-NcoI) downstream of the promoter and the aadA gene. The cloned sequence is exactly the same as in the psbA gene.
b) For enhancing protein stability and facilitating purification, the *cry2Aa2 Bacillus thuringiensis* operon derived putative chaperonin is used. Expression of the *cry2Aa2* operon in chloroplasts provides a model system for hyper-expression of foreign proteins (46% of total soluble protein) in a folded configuration enhancing their stability and facilitating purification (3). This justifies inclusion of the putative chaperonin from the *cry2Aa2* operon in one of the newly designed constructs. In this region there are two open reading frames (ORF1 and ORF2) and a ribosomal binding site (rbs). This sequence contains elements necessary for *Cry2Aa2* crystallization which help to crystallize the HSA, IGF-I and EFN-□ proteins aiding in the subsequent purification. Successful crystallization of other proteins using this putative chaperonin has been demonstrated (94). We amplify the ORF1 and ORF2 of the Bt *Cry2Aa2* operon by PCR using the complete operon as template. The fragment is cloned into a PCR 2.1 vector and excised as an EcoRI-EcoRV product. This fragment is then cloned directly into the pLD vector multiple cloning site (EcoRI-EcoRV) downstream of the promoter and the aadA gene.
c) To obtain proteins with the same amino acid composition as mature human proteins, we first fuse all three genes (codon optimized and native sequence) with the RuBisCo small subunit transit peptide. Also other constructions are done to allow cleavage of the protein after isolation from chloroplast. These strategies also allow affinity purification of the proteins.

The first set of constructs includes the sequence of each protein beginning with an ATG, introduced by PCR using primers. Processing to get the mature protein may be performed where the ATG is shown to be a problem (determined by mice immunological assays). First, we use the RuBisCo small subunit transit peptide. This transit peptide is amplified by PCR using tobacco DNA as template and cloned into the PCR 2.1 vector. All genes are fused with the transit peptide using a MIuI restriction site that is introduced in the PCR primers for amplification of the transit peptide and genes coding for three proteins. The gene fusions are inserted into the pLD vectors downstream of the 5'UTR or ORFI+2 using the restriction sites NcoI and EcoRV respectively. If use of tags or protease sequences is necessary, such sequences can be introduced by designing primers including these sequences and amplifying the gene with completing vector constructions, all the vectors are sequenced to confirm correct nucleotide sequence and in frame fusion. DNA sequencing is done using a Perkin Elmer ABI prism 373 DNA sequencing system.

Because of the similarity of protein synthetic machinery (109), expression of all chloroplast vectors is first tested in *E.coli* before their use in tobacco transformation. For *Escherichia coli* expression XL-1 Blue strain is used. *E. coli* can be transformed by standard CaCl₂ transformation procedures and grown in TB culture media. Purification, biological and immunogenic assays are done using *E. coli* expressed proteins.

### Example 6

### Bombardment, Regeneration and Characterization of Chloroplast Transgenic Plants

Tobacco (*Nicotiana tabacum* var. Petit Havana) plants are grown aseptically by germination of seeds on MSO medium. This medium contains MS salts (4.3 g/liter), B5 vitamin mixture (myo-inositol, 100 mg/liter, thiamino-HCl, 10 mg/liter; nicotinic acid, 1 mg/liter; pyridoxine-HCl, 1 mg/liter), sucrose (30 g/liter) and phytagar (6 g/liter) at pH 5.8. Fully expanded, dark green leaves of about two month old plants are used for bombardment.

Leaves are placed abaxial side up on a Whatman No. 1 filter paper laying on the RMOP medium (79) in standard petri plates (100x15 mm) for bombardment. Gold (0.6 µm) microprojectiles are coated with plasmid DNA (chloroplast vectors) and bombardments are carried out with the biolistic device PDS1000/He (Bio-Rad) as described by Daniell (110). Following bombardment, petri plates are sealed with parafilm and incubated at 24°C under 12 h photoperiod. Two days after bombardment, leaves are chopped into small pieces of ∼5 mm² in size and placed on the selection medium (RMOP containing 500 µg/ml of spectinomycin dihydrochloride) with abaxial side touching the medium in deep (100x25 mm) petri plates (∼10 pieces per plate). The regenerated spectinomycin resistant shoots are chopped into small pieces (∼2mm²) and subcloned into fresh deep petri plates (-5 pieces per plate) containing the same selection medium. Resistant shoots from the second culture cycle are then transferred to the rooting medium (MSO medium supplemented with IBA, 1mg/liter and spectinomycin dihydrochloride, 500 mg/liter). Rooted plants are transferred to soil and grown at 26°C under 16 hour photoperiod conditions for further analysis.

### PCR analysis of putative transformants

PCR is done using DNA isolated from control and transgenic plants in order to distinguish a) true chloroplast transformants from mutants and b) chloroplast transformants from nuclear transformants. Primers for testing the presence of the aadA gene (that confers resistance) in transgenic plants are landed on the aadA coding sequence and 16S rRNA gene. In order to test chloroplast integration of the genes, one primer lands on the aadA *gene* while another lands on the native chloroplast genome. No PCR product is obtained with nuclear transgenic plants using this set of primers. The primer set is used to test integration of the entire gene cassette without any internal deletion or looping out during homologous recombination. Similar strategy was used successfully to confirm chloroplast integration of foreign genes (3,85-88). This screening is essential to eliminate mutants and nuclear transformants. In order to conduct PCR analyses in transgenic plants, total DNA from unbombarded and transgenic plants is isolated as described by Edwards et al. (129). Chloroplast transgenic plants containing the desired gene are then moved to second round of selection in order to achieve homoplasmy.

### Southern Analysis for homoplasmy and copy number

Southern blots are done to determine the copy number of the introduced foreign gene per cell as well as to test homoplasmy. There are several thousand copies of the chloroplast genome present in each plant cell Therefore, when foreign genes are inserted into the chloroplast genome, some of the chloroplast genomes have foreign genes integrated while others remain as the wild type (heteroplasmy). Therefore, in order to ensure that only the transformed genome exists in cells of transgenic plants (homoplasmy), the selection process is continued. In order to confirm that the wild type genome does not exist at the end of the selection cycle, total DNA from transgenic plants are probed with the chloroplast border (flanking) sequences (the trnIf-trnA fragment). When wild type genomes are present (heteroplasmy), the native fragment size is observed along with transformed genomes. Presence of a large fragment (due to insertion of foreign genes within the flanking sequences) and absence of the native small fragment confirms homoplasmy (85,86,88).

The copy number of the integrated gene is determined by establishing homoplasmy for the transgenic chloroplast genome. Tobacco chloroplasts contain 5000∼10,000 copies of their genome per cell (86). If only a fraction of the genomes are actually transformed, the copy number, by default, must be less than 10,000. By establishing that in the transgenic the gene inserted transformed genome is the only one present, one can establish that the copy number is 5000∼10,000 per cell. This is usually done by digesting the total DNA with a suitable restriction enzyme and probing with the flanking sequences that enable homologous recombination into the chloroplast genome. The native fragment present in the control should be absent in the transgenics. The absence of native fragment proves that only the transgenic chloroplast genome is present in the cell and there is no native, untransformed, chloroplast genome, without the present. This establishes the homoplasmic nature of our transformants, simultaneously providing us with an estimate of 5000∼10,000 copies of the foreign genes per celL

### Northern Analysis for transcript stability

Northern blots are done to test the efficiency of transcription of the genes. Total RNA is isolated from 150 mg of frozen leaves by using the "Rneasy Plant Total RNA Isolation Kit" (Qiagen Inc., Chatsworth, CA). RNA (10-40 µg) is denatured by formaldehyde treatment, separated on a 1.2% agarose gel in the presence of formaldehyde and transferred to a nitrocellulose membrane (MSI) as described in Sambrook et al. (130). Probe DNA (proinsulin gene coding region) is labeled by the random-primed method (Promega) with ³²P-dCTP isotope. The blot is pro-hybridized, hybridized and washed as described above for southern blot analysis. Transcript levels are quantified by the Molecular Analyst Program using the GS-700 Imaging Densitometer (Bio-Rad, Hercules, CA).

### Expression and quantification of the total protein expressed in chloroplast

Chloroplast expression assays are done for each protein by Western Blot. Recombinant protein levels in transgenic plants are determined using quantitative ELISA assays. A standard curve is generated using known concentrations and serial dilutions of recombinant and native proteins. Different tissues are analyzed using young, mature and old leaves against these primary antibodies: goat anti-HSA (Nordic Immunology), anti-IGF-I and anti-Interferon alpha (Sigma). Bound IgG is measured using horseradish peroxidase-labelled anti-goat IgG.

### Inheritance of Introduced Foreign Genes

While it is unlikely that introduced DNA would move from the chloroplast genome to nuclear genome, it is possible that the gene could get integrated in the nuclear genome during bombardment and remain undetected in Southern analysis. Therefore, in initial tobacco transformants, some are allowed to self pollinate, whereas others are used in reciprocal crosses with control tobacco (transgenics as female accepters and pollen donors; testing for maternal inheritance). Harvested seeds (T1) will be germinated on media containing spectinomycin. Achievement of homoplasmy and mode of inheritance can be classified by looking at germination results. Homoplasmy is indicated by totally green seedlings (86) while heteroplasmy is displayed by variegated leaves (lack of pigmentation, 83). Lack of variation in chlorophyll among progeny also underscores the absence of position effect, an artifact of nuclear transformation. Maternal inheritance is be demonstrated by sole transmission of introduced genes via seed generated on transgenic plants, regardless of pollen source (green seedlings on selective media). When transgenic pollen is used for pollination of control plants, resultant progeny do not contain resistance to chemical in selective media (will appear bleached; 83). Molecular analyses confirm transmission and expression of introduced genes, and T2 seed is generated from those confirmed plants by the analyses described above.

### Example 7

### Purification methods

The standard method of purification employs classical biochemical techniques with the crystallized proteins inside the chloroplast. In this case, the homogenates are passed through miracloth to remove cell debris. Centrifugation at 10,000 xg pelletizes all foreign proteins (3). Proteins are solubilized using PH, temperature gradient, etc. This is possible if the ORF1 and 2 of the *cry2Aa2* operon (see section c) can fold and crystallize the recombinant proteins as expected. Were there is no crystal formation, other purification methods must be used (classical biochemistry techniques and affinity columns with protease cleavage).

HSA: Albumin is typically administered in tens of gram quantities. At a purity level of 99.999% (a level considered sufficient for other Recombinant protein preparations), recombinant HSA (rHSA) impurities on the order of one mg will still be injected into patients. So impurities from the host organism must be reduced to a minimum. Furthermore, purified rHSA must be identical to human HSA. Despite these stringent requirements, purification costs must be kept low. To purify the USA obtained by gene manipulation, it is not appropriate to apply the conventional processes for purifying HSA originating in plasma as such. This is because the impurities to be eliminated from rHSA completely differ from those contained in the HSA originating in plasma. Namely, rHSA is contaminated with, for example, coloring matters characteristic to recombinant HSA, proteins originating in the host cells, polysaccharides, *etc*. In particular, it is necessary to sufficiently eliminate components originating in the host cells, since they are foreign matters for living organisms including human and can cause the problem of antigenicity.

In plants two different methods of HSA purification have been done at laboratory scale. Sijmons et al. (23) transformed potato and tobacco plants with *Agrobacterium tumefaciens.* For the extraction and purification of HSA, 1000 g of stem and leaf tissue was homogenized in 1000 ml cold PBS, 0.6% PVP, 0.1 mM PMSF and 1 mM EDTA. The homogenate was clarified by centrifuged and the supernatant incubated for 4 h with 1.5 ml polyclonal antiHSA coupled to Reactigel spheres (Pierce Chem) in the presence of 0.5% Tween 80. The complex HSA-anti HSA-Reactigel was collected and washed with 5 ml 0.5% Tween 80 in PBS. HSA was desorbed from the reactigel complex with 2.5 ml of 0.1 M glycine pH 2.5, 10% dioxane, immediately followed by a buffer exchange with Sephadex G25 to 50 mM Tris pH 8. The sample was then loaded on a HR5/5 MonoQ anion exchange column (Pharmacia) and eluted with a linear NaCl gradient (0-350 mM NaCl in 50 mM Tris pH 8 in 20 min at 1ml/min). Fractions containing the concentrated HSA (at 290 mM NaCl) were lyophilized and applied to a HR 10/30 Sepharose 6 column (Pharmacia) in PBS at 0.3 ml/min. However, this method uses affinity columns (polyclonal anti-HSA) that are very expensive to scale-up. Also the protein is released from the column with 0.1M glycine pH 2.5 that will most probably, denature the protein. Therefore, this method can suitably modified to reduce these drawbacks.

The second method is for HSA extraction and purification from potato tubers (Dr. Mingo-Castel's laboratory). After grinding the tuber in phosphate buffer pH 7.4 (1 mg/2ml), the homogenate is filtered in miracloth and centrifuged at 14.000 rpm 15 minutes. After this step another filtration of the supernatant in 0.45 Dm filters is necessary. Then, chromatography of ionic exchange in FPLC using a DEAE Sepharose Fast Flow column (Amersham) is required. Fractions recovered are passed through an affinity column (Blue Sepharose fast flow Amersham) resulting in a product of high purity. HSA purification based on either method is acceptable.
**IGF-1**: All earlier attempts to produce IGF-I in *E*. *coli* or *Saccharomyces cerevisiae* have resulted in misfolded proteins. This has made it necessary to perform additional *in vitro* refolding or extensive separation techniques in order to recover the native and biological form of the molecule. In addition, IGF-I has been demonstrated to possess an intrinsic thermodynamic folding problem with regard to quantitatively folding into a native disulfide-bonded conformation *in vitro* (131). Samuelsson et al. (131) and Joly et al. (132) co-expressed IGF-I with specific proteins of *E*. *coli* that significantly improved the relative yields of correctly folded protein and consequently facilitating purification. Samuelsson et al. (132) fused the protein to affinity tags based on either the IgG-binding domain (Z) from Staphylococcal protein A or the two serum albumin domains (ABP) from Streptococcal protein G (134). The fusion protein concept allows the IGF-I molecules to be purified by IgG or HSA affinity chromatography. We also use this Z tags for protein purification including the double Z domain from *S. aureus* protein and a sequence recognized by TEV protease (see section d.2). The fusion protein is incubated with an IgG column where binding via the Z domain occurs. Z domain-IgG interaction is very as high affinity, so contaminant proteins can be easily washed off the column. Incubation of the column with TEV protease elutes mature IGF-I from the column. TEV protease is produced in bacteria in large quantities fused to a 6 histidine tag that is used for TEV purification. This tag can be also used to separate IGF-I from contaminant TEV protease.

IFN-□: In the *E*. *coli* expression method used, the purification system was based on using 6 Histidine-tags that bind to a nickel column and biotinylated thrombin to eliminate the tag on IFN-□5.

### Example 8

### Characterization of the recombinant proteins

For the safe use of recombinant proteins as a replacement in any of the current applications, these proteins must be structurally equivalent and must not contain abnormal host-derived modifications. To confirm compliance with these criteria we compare human and recombinant proteins using the currently highly sensitive and highly resolving techniques expected by the regulatory authorities to characterize recombinant products (135).

### Amino acid analysis

Amino acid analysis to confirm the correct sequence is performed following off-line vapour phase hydrolysis using ABI 420A amino acid derivatizer with an on line 130A phenylthiocarbamyl-amino acid analyzer (Applied Biosystems/ABI). N-terminal sequence analysis is performed by Edman degradation using ABI 477A protein sequencer with an on-line 120A phenylthiohydantoin-amino acid analyzer. Automated C-terminal sequence analysis uses a Hewlett-Packard G1009A protein sequencer. To confirm the C-terminal sequence to a greater numbers of residues, the C-terminal tryptic peptide is isolated from tryptic digests by reverse-phase HPLC.

### Protein folding and disulfide bridges formation

Western blots with reducing and non-reducing gels are done to check protein folding. PAGE to visualize small proteins will be done in the presence of tricine. Protein standards (Sigma) are loaded to compare the mobility of the recombinant proteins. PAGE is performed on PhastGels (Pharmacia Biotech). Proteins are blotted and then probed with goat anti-HSA, interferon alpha and IGF-I polyclonal antibodies. Bound IgG is detected with horseradish peroxidase-labelled anti goat IgG and visualized on X-ray film using ECL detection reagents (Amersham).

### Tryptic mapping

firm the presence of chloroplast expressed proteins with disulfide linkages identical to native human proteins, the samples are subjected to tryptic digestion followed by peptide mass mapping using matrix-assisted laser desorption ionization mass spectrometry (MALDI-MS). Samples are reduced with dithiothreitol, alkylated with iodoacetamide and then digested with trypsin comprising three additions of 1:100 enzyme/substrate over 48h at 37°C. Subsequently tryptic peptides are separated by reverse-phase HPLC on a Vydac C18 column.

### Mass analysis

Electrospray mass spectrometry (ESMS) is performed using a VG Quattro electrospray mass spectrometer. Samples are desalted prior to analysis by reverse-phase HPLC using an acetonitrile gradient containing trifluoroacetic acid.

### CD

Spectra are measured in a nitrogen atmosphere using a Jasco J600 spectropolarimeter.

### Chromatographic techniques

For HSA, analytical gel-permeation HPLC is performed using a TSK G3000 SWxl column. Preparative gel permeation chromatography of HSA is performed using a Sephacryl S200 HR column. The monomer fraction, identified by absorbance at 280 nm, is dialyzed and reconcentrated to its starting concentration. For IGF-I, the reversed-phase chromatography the SMART system (Pharmacia Biotech) is used with the mRPC C2/18 SC 2.1/10 column.

### Viscosity

This is a classical assay for recombinant HSA. Viscosity is a characteristic of proteins related directly to their size, shape, and conformation. The viscosities of HSA and recombinant HSA can be measured at 100 mg. MI-1 in 0.15 M NaCl using a U-tube viscosimeter (M2 type, Poulton, Selfe and Lee Ltd, Essex, UK) at 25°C.

### Glycosylation

Chloroplast proteins are not know to be glycosylated. However there are no publications to confirm or refute this assumption. Therefore glycosylation should be measured using a scaled-up version of the method of Ahmed and Furth (136).

### Example 9

### Biological Assays

ISA does not have enzymatic activity, it is not possible to run biological assays. However, three different techniques can be used to check IGF-I functionality. All of them are based on the proliferation of IGF-I responding cells. First, radioactive thymidine uptake can be measured in 3T3 fibroblasts, that express IGF-I receptor, as an estimate of DNA synthesis. Also, a human megakaryoblastic cell line, HU-3, can be used. As HU-3 grows in suspension, changes in cell number and stimulation of glucose uptake induced by IGF-I are assayed using AlamarBlue or glucose consumption, respectively. AlamarBlue (Accumed International, Westlak.OH) is reduced by mitochondrial enzyme activity. The reduced form of the reagent is fluorescent and can be quantitatively detected, with an excitation of 530 nm and an emission of 590 nm. AlamarBlue is added to the cells for 24 hours after 2 days induction with different doses of IGF-I and in the absence of serum. Glucose consumption by HU-3 cells is then measured using a colorimetric glucose oxidase procedure provided by Sigma. HU-3 cells are incubated in the absence of serum with different doses of IGF-I. Glucose is added for 8 hours and glucose concentration is then measured in the supernatant. All three methods to measure IGF-I functionality are precise, accurate and dose dependent, with a linear range between 0.5 and 50 ng/ml (137).

The method to determine IFN activity is based on their anti-viral properties. This procedure measures the ability of IFN to protect HeLa cells against the cytopathic effect of encephalomyocarditis virus (EMC). The assay is performed in 96-well microtitre plate. First, HeLa cells are seeded in the wells and allowed to grow to confluency. Then, the medium is removed, replaced with medium containing IFN dilutions, and incubated for 24 hours. EMC virus is added and 24 hours later the cytopathic effect is measured For that, the medium is removed and wells are rinsed two times with PBS and stained with methyl violet dye solution. The optical density is read at 540 nm. The values of optical density are proportional to the antiviral activity of IFN (138). Specific activity is determined with reference to standard IFN-□ (code 82/576) obtained from NIBSC.

### Example 10

### Animal testing and Pre-Clinical Trials

Once albumin is produced at adequate levels in tobacco and the physicochemical properties of the product correspond to those of the natural protein, toxicology studies need to be done in mice. To avoid mice response to the human protein, transgenic mice carrying HSA genomic sequences are used (139). After injection of none, 1, 10, 50 and 100 mg of purified recombinant protein, classical toxicology studies are carried out (body weigh and food intake, animal behavior, piloerection, etc). Albumin can be tested for blood volume replacement after eliminate the fluid from the peritoneal cavity in patients with liver cirrhosis. It has been shown that albumin infusion after this maneuver is essential to preserve effective circulatory volume and renal function (140).

IGF-1 and IFN□ are tested for biological effects *in vivo* in animal models. Specifically, woodchucks (*marmota monax*) infected with the woodchuck hepatitis virus (WHV), are widely considered as the best animal model of hepatitis B virus infection (141). Preliminary studies have shown a significant increase in 5' oligoadenylate synthase RNA levels by real time polymerase chain reaction (PCR) in woodchuck peripheral blood mononuclear cells upon incubation with human LFN□5, a proof of the biological activity of the human IFN□5 in woodchuck cells. For *in vivo* studies, a total of 7 woodchucks chronically infected with WHV (WHV surface antigen and WHV-DNA positive in serum) are used: 5 animals are injected subcutaneously with 500,000 units of human IFN□5 (the activity of human IFN□5 is determined as described previously) three times a week for 4 months; the remaining two woodchucks are injected with placebo and used as controls. Follow-up includes weekly serological (WHV surface antigen and anti-WHV surface antibodies by ELISA) and virological (WHV DNA in serum by real time quantitative PCR) as well as monthly immunological (T-helper responses against WHV surface and WHV core antigens measured by interleukin 2 production from PBMC incubated with those proteins) studies. Finally, basal and end of treatment liver biopsies should be performed to score liver inflammation and intrahepatic WHV-DNA levels. The final goal of treatment is decrease of viral replication by WHV-DNA in serum, with secondary end points being histological improvement and decrease in intrahepatic WHV-DNA levels.

For IGF-I, the *in vivo* therapeutic efficacy is tested in animals in situations of IGF-I deficiency such as liver cirrhosis in rats. Several reports (56-58) have been published showing that recombinant human IGF-I has marked beneficial effects in increasing bone and muscle mass, improving liver function and correcting hypogonadism. Briefly, the induction protocol is as follows: Liver cirrhosis is induced in rats by inhalation of carbon tetrachloride twice a week for 11 weeks, with a progressively increasing exposure time from 1 to 5 minutes per gassing session. After the 11^{th} week, animals continue receiving CCl₄ once a week (3 minutes per inhalation) to complete 30 weeks of CCl₄ administration. During the whole induction period, phenobarbital (400 mg/L) is added to drinking water. To test the therapeutic efficacy of tobacco-derived IGF-I; cirrhotic rats receive 2 µg/100 g body weight/day of this compound in two divided doses, during the last 21 days of the induction protocol (weeks 28, 29, and 30). On day 22, animals are sacrificed and liver and blood samples collected. The results are compared to those obtained in cirrhotic animals receiving placebo instead of tobacco-derived IGF-I, and to healthy control rats.

Taken together, low levels of expressionof human proteins in nuclear transgenic plants, and difficulty in folding, assembly/processing ofhuman proteins in *E.coli* should make chloroplasts an alternate compartment for expression of these proteins. Production of human proteins in transgenic chloroplasts should also dramatically lower the production cost. Large-scale production of insulin in tobacco in conjunction with an oral delivery system can be a powerful approach to provide treatment to diabetes patients at an affordable cost and provide tobacco farmers alternate uses for this hazardous crop. Therefore, it is first advantageous to use poly(GVGVP) as a fusion protein to enable hyper-expression of insulin and accomplish rapid one step purification of the fusion peptide utilizing the inverse temperature transition properties of this polymer. It is further advantageous to develop insulin-CTB fusion protein for oral delivery in nicotine free edible tobacco (LAMD 605).
Both achievements can be accomplished as follows:
a) Develop recombinant DNA vectors for enhanced expression of Proinsulin as fusion proteins with GVGVP or CTB via chloroplast genomes of tobacco;
b) obtain transgenic tobacco (Petit Havana & LAMD 605) plants;
c) Characterize Transgenic expression of proinsulin polymer or CTB fusion proteins using molecular and biochemical methods in chloroplasts;
d) Employ existing or modified methods of polymer purification from transgenic leaves;
e) Analyze Mendelian or maternal inheritance of transgenic plants;
f) Large scale purification of insulin and comparison of current insulin purification methods with polymer-based purification method in *E.coli* and tobacco;
g) Compare natural refolding in chloroplasts with *in vitro* processing;
h) Characterization (yield and purity) of proinsulin produced in *E.coli* and transgenic tobacco; and
i) Assessment of diabetic symptoms in mice fed with edible tobacco expressing CTB-insulin fusion protein.

**Diabetes and Insulin**: The most obvious action of insulin is to lower blood glucose (Oakly et al. 1973). This is a result of its immediate effect in increasing glucose uptake in tissues. In muscle, under the action of insulin, glucose is more readily taken up and either converted to glycogen and lactic acid or oxidized to carbon dioxide. Insulin also affects a number of important enzymes concerned with cellular metabolism. It increases the activity of glucokinase, which phosphorylates glucose, thereby increasing the rate of glucose metabolism in the liver. Insulin also suppresses gluconeogenesis by depressing the function of liver enzymes, which operate the reverse pathway from proteins to glucose. Lack of insulin can restrict the transport of glucose into muscle and adipose tissue. This results in increases in blood glucose levels (hyperglycemia). In addition, the breakdown of natural fat to free fatty acids and glycerol is increased and there is a rise in the fatty acid content in the blood. Increased catabolism of fatty acids by the liver results in greater production of ketone bodies. They diffuse from the liver and pass to the muscles for further oxidation. Soon, ketone body production rate exceeds oxidation rate and ketosis results. Fewer amino acids are taken up by the tissues and protein degradation results. At the same time, gluconeogenesis is stimulated and protein is used to produce glucose. Obviously, lack of insulin has serious consequences.

Diabetes is classified into types I and U. Type I is also known as insulin dependent diabetes mellitus (IDDM). Usually this is caused by a cell-mediated autoimmune destruction of the pancreatic β-cells (Davidson, 1998). Those suffering from this type are dependent on external sources of insulin. Type II is known as noninsulin-dependent diabetes mellitus (NIDDM). This usually involves resistance) to insulin in combination with its underproduction. These prominent diseases have led to extensive research into microbial production of recombinant human insulin (rHI).
**Expression of Recombinant Human Insulin in *E.coli***: In 1978, two thousand kilograms of insulin were used in the world each year, half of this was used in the United States (Steiner et al, 1978). At that time, the number of diabetics in the US was increasing 6% every year (Gunby, 1978). In 1997-98, 10% increase in sales of diabetes care products and 19% increase in insulin products have been reported by Novo Nordisk, making it a 7.8 billion dollar industry. Annually,160,000 Americans are killed by diabetes, making it the fourth leading cause of death. Many methods of production of rHI have been developed. Insulin genes were first chemically synthesized for expression in *Esherichia* coli (Crea et al., 1978). These genes encoded separate insulin A andB chains. The genes were each expressed in *E.coli* as fusion proteins with the β-galactosidase (Goeddel et al., 1979). The first documented production of rHI using this system was reported by David Goeddel from Genentech (Hall, 1988). The genes were fused to the Trp synthase gene, which resulted in increased insulin yield, due to the smaller fusion peptide. This fusion protein was approved for commercial production by Eli Lilly in 1982 (Chance and Frank, 1993) with a product name of Humulin. As of 1986, Humulin was produced from proinsulin genes. Proinsulin contains both insulin chains and the C-peptidethat connects them. Normal *in vitro* post-translational processing of proinsulin includes use of trypsin and carboxypeptidaseB for maturation to insulin. Other data concerning commercial production of Humulin and other insulin products is now considered proprietary information and is not available to the public.
**Cholera Toxin** β **subunit as a fusion protein**: *Vibrio cholerae* causes diarrhea by colonizing the small intestine and producing enterotoxins, of which the cholera toxin (CT) is considered the main cause of toxicity. CT is a hexameric AB₅ protein having one 27KDa A subunit which has toxic ADP-ribosyl transferase activity and a non-toxic pentamer of 11.6 kDa. B subunits that are non-covalently linked into a very stable doughnut like structure into which the toxic active (A) subunit is inserted. The A subunit of CT consists of two fragments - A1 and A2 which are linked by a disulfide bond. The enzymatic activity of CT is located solely on the A1 fragment (Gill, 1976). The A2 fragment of the A subunit links the A1 fragment and the B pentamer. CT binds via specific interactions of the B subunit pentamer with GM1 ganglioside, the membrane receptor, present on the intestinal epithelial cell surface of the host. The A subunit is then translocated into the cell where it ADP-ribosylates the Gs subunit of adenylate cyclase bringing about the increased levels of cyclic AMP in affected cells that is associated with the electrolyte and fluid loss of clinical cholera(Lebens et al. 1994). For optimal enzymatic activity, the A1 fragment needs to be separated from the A2 fragment by proteolytic cleavage of the main chain and by reduction of the disulfide bond linking them (Mekalanos et al. 1979).

Expression and assembly of CTB in transgenic potato tubers has been reported (Arakawa et al.1997). The CTB gene including the leader peptide was fused to an endoplasmic reticulum retention signal (SEKDEL) at the 3= end to sequester the CTB protein within the lumen of the ER. The DNA fragment encoding the 21 -amino acid leader peptide of the CTB protein was retained to direct the newly synthesized CTB protein into the lumen of the ER. Immunoblot analysis indicated that the plant derived CTB protein was antigenically indistinguishable from the bacterial CTB protein and that oligomeric CTB molecules (Mr⁻ 50 kDa) were the dominant molecular species isolated from transgenic potato leaf and tuber tissues. Similar to bacterial CTB, plant derived CTB dissociated into monomers (Mr-15 kDa) during heat/acid treatment.

Enzyme linked immunosorbentassay methods indicated that plant synthesized CTB protein bound specifically to GM1 gangliosides, the natural membrane receptors of Cholera Toxin. The maximum amount of CTB protein detected in auxin induced transgenic potato leaf and tuber tissues was approximately 0.3% of the total soluble protein. The oral immunization of CD-1 mice with transgenic potato tissues transformed with the CTB gene (administered at weekly intervals for a month with a final booster feeding on day 65) has also been reported. The levels of serum and mucosal anti-cholera toxin antibodies in mice were found to generate protective immunity against the cytopathic effects ofCT holotoxin. Following intraileal injection with CT, the plant immunized mice showed up to a 60% reduction in diarrheal fluid accumulation in the small intestine. Systemic and mucosal CTB- specific antibody titers were determined in both serum and feces collected from immunized mice by the class-specific chemiluminescent ELISA method and the endpoint titers for the three antibody isotypes (IgM,IgG and IgA) were determined. The extent of CT neutralization in both Vero cell and ileal loop experiments suggested that anti-CTB antibodies prevent CT binding to cellular GMI-gangliosides. Also, mice fed with 3 g of transgenic potato exhibited similar intestinal protection as mice gavaged with 30 g of bacterial CTB. Recombinant LTB [rLTB] (the heat labile enterotoxin produced by Enterotoxigenic *E.coli* which is structurally, functionally and immunologically similar to CTB was expressed in transgenic tobacco (Arntzen et al.1998; Haq et al. 1995). They have reported that, the rLTB retained its antigenicity as shown by immunoprecipitation of rLTB with antibodies raised to rLTB from *E.coli.* The rLTB protein was of the right molecular weight and aggregated to form the pentamer as confirmed by gel permeation chromatography. **Delivery of Human Insulin**: Insulin has been delivered intravenously in the past several years. However, more recently, alternate methods such as nasal spray, are also available. Oral delivery of insulin is yet another new approach (Mathiowitz et al.,1997). Engineered polymer microspheres made of biologically erodable polymers, which display strong interactions with gastrointestinal mucus and cellular linings, can traverse both mucosal absorptive epithelium and the follicle-associated epithelium, covering the lymphoid tissue of Peyer's patches. Polymers maintain contact with intestinal epithelium for extended periods of time and actually penetrate through and between cells. Animals fed with the poly (FA: PLGA)-encapsulated insulin preparation were able to regulate the glucose load better than controls, confirming that insulin crossed the intestinal barrier and was released from the microspheres in a biologically active form (Mathiowitz et al., 1997).

Besides, CTB has also been demonstrated to be an effective carrier molecule for the induction of mucosal immunity to polypeptides to which it is chemically or genetically conjugated (McKenzie et aL 1984; Dertzbaugh et aL 1993). The production of immunomodulatory transmucosal carrier molecules, such as CTB, in plants may greatly improve the efficacy of edible plant vaccines (Haq et al. 1995; Thanavala et al. 1995; Mason et al. 1996) and may also provide novel oral tolerance agents for prevention of such autoimmune diseases as Type I diabetes (Zhang et al. 1991), Rheumatoid arthritis (Trentham et al. 1993), multiple sclerosis (Khoury et al.1990; Miller et al. 1992; Weiner et al.1993) as well as the prevention of allergic and allograft rejection reactions (Sayegh et al.1992; Hancock et al. 1993). Therefore, expressing a CTB-proinsulin fusion would be an ideal approach for oral delivery of insulin.
**Chloroplast Genetic Engineering**: When we developed the concept of chloroplast genetic engineering (Daniell and McFadden, 1988 U.S. Patents; Daniell, World Patent, 1999), it was possible to introduce isolated intact chloroplasts into protoplasts and regenerate Transgenic plants (Carlson, 1973). Therefore, early investigations on chloroplast transformation focused on the development of *in organello* systems using intact chloroplasts capable of efficient and prolonged transcription and translation (Daniell and Rebeiz, 1982; Daniell et al., 1983,1986) and expression of foreign genes in isolated chloroplasts (Daniell and McFadden, 1987). However, after the discovery of the gene gun as a transformation device (Daniell, 1993), it was possible to transform plant chloroplasts without the use of isolated plastids and protoplasts. Chloroplast genetic engineering was accomplished in several phases. Transient expression of foreign genes in plastids of dicots (Daniell et al., 1990; Ye et al., 1990) was followed by such studies in monocots (Daniell et al., 1991). Unique to the chloroplast genetic engineering is the development of a foreign gene expression system using autonomously replicating chloroplast expression vectors (Daniell et al., 1990). Stable integration of a selectable marker gene into the tobacco chloroplast genome (Svab and Maliga, 1993) was also accomplished using the gene gun. However, useful genes conferring valuable traits via chloroplast genetic engineering have been demonstrated only recently. For example, plants resistant to B.t. sensitive insects were obtained by integrating the *cryIAc gene* into the tobacco chloroplast genome (McBride et al., 1995). Plants resistant to B.t. resistant insects (up to 40,000 fold) were obtained by hyper-expression of the *cryllA* gene within the tobacco chloroplast genome (Kota et al., 1999). Plants have also been genetically engineered via the chloroplast genome to confer herbicide resistance and the introduced foreign genes were maternally inherited, overcoming the problem of out-cross with weeds (Daniell et al., 1998). Chloroplast genetic engineering has also been used to produce pharmaceutical products that are not used by plants (Staub et al. 2000, Guda et al. 2000). Chloroplast genetic engineering technology is currently being applied to other useful crops (Sidorov et al. 1999; Daniell, 1999).

This invention synthesizes high value pharmaceutical proteinsin nuclear transgenic plants by chloroplast expression for pharmaceutical protein production. Chloroplasts are suitable for this purpose because of their ability to process eukaryotic proteins, including folding and formation of disulfide bridges, thereby eliminating the need for expensive post-purificationprocessing. Tobacco is an ideal choice for this purpose because of its large biomass, ease of scale-up (million seeds per plant) and genetic manipulation. We use poly(GVGVP) as a fusion protein to enable hyper-expression of insulin and accomplish rapid one step purification of fusion peptides utilizing the inverse temperature transition propertiesof this polymer. We also use insulin-CTB fusion protein in chloroplasts of nicotine free edible tobacco (LAMD 605) for oral delivery to NOD mice.

### BRIEF DESCRIPTION OF DRAWINGS

Fig. 4 shows graphs of Cry2A protein concentration determined by ELISA in transgenic leaves.
Fig. 5 is an inmunogold labeled electron microscopy of a mature transgenic leaf.
Fig. 6 contains photographsof leaves infected with 10 µl of 8x0⁵ 8x10⁴, 8x10³ and 8x10² cells of P. *syringae* five days after inoculation.
Fig. 7 is a graph of total plant protein mixed with µl of mid-log phase bacteria from overnight culture, incubated for two hours at 25□C at 125 rpm and grown in LB broth overnight.
Fig. 8A is a graph of CTB ELISA quantification shown as a percentage of total soluble plant protein.
Fig. 8B is a graph of CTB-GM1 Ganglioside binding ELISA assays.
Fig. 9 is a 12% reducingPAGE using Chemiluminescent detection with rabbit anti-cholera serum (1□) and AP labeled mouse anti-rabbitIgG (20) antibodies.
Figs. 10A and B show reducing gels of expression and assembly of disulfide bonded Guy=s 13 monoclonal antibody.
Fig. 10C or shows a non-reducing gel of expression and assembly of disulfide bonded Guy=s 13 monoclonal antibody.

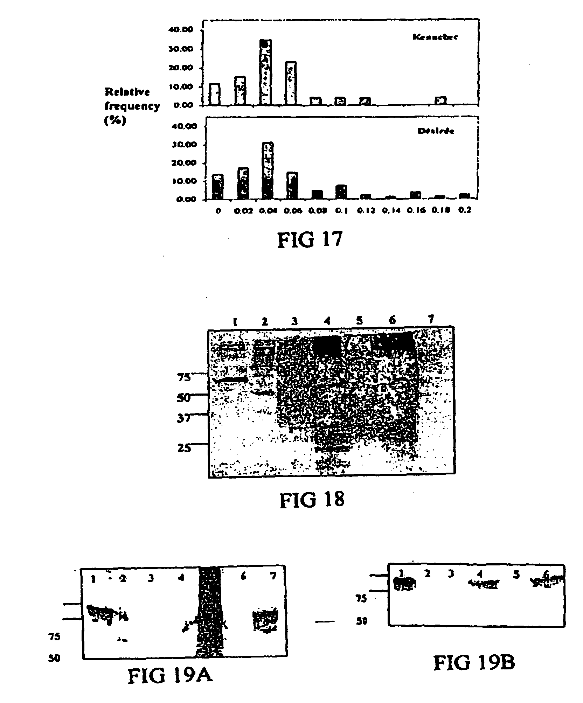

Figs. 19A-F show photographscomparingbetaine aldehyde and spectinomycin selection.
Figs. 1A and B show biopolymer-proinsulin fusion protein expression.
Fig. 1C shows western blots of biopolymer-proinsulin fusion protein after single step purification.
Fig. 1D shows western blots of another biopolymer-proinsul infusion protein after single step purification.

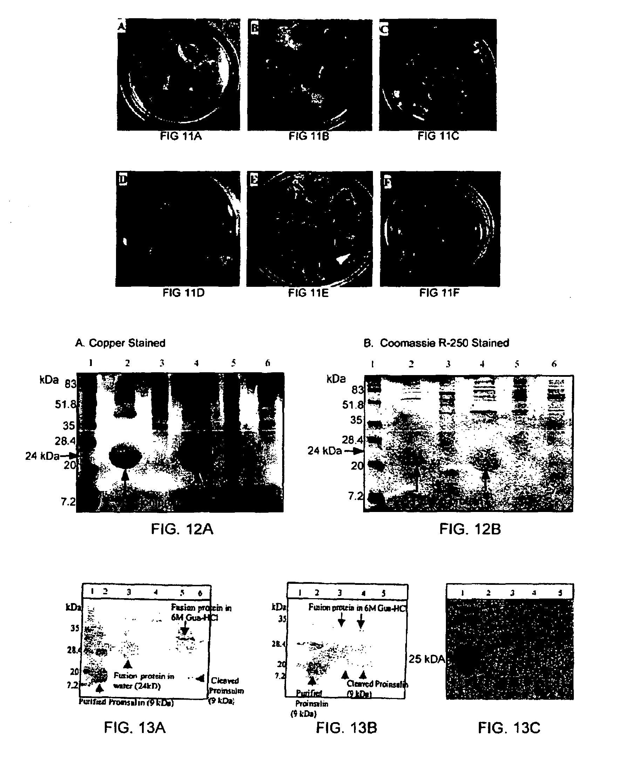

Fig. 13 shows western blots of yet another biopolymer-proinsulin fusion protein after single step purification.

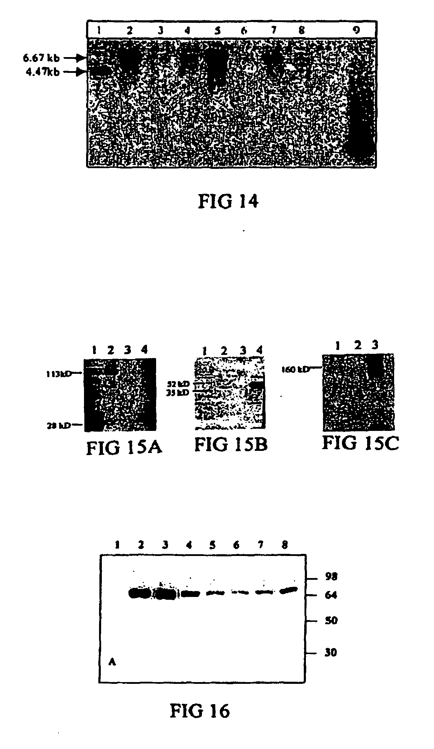

Fig. 14 shows biopolymer-proinsulinfusion gene integration into the chloroplast genome confirmed by Southern blot analysis.

A remarkable feature of chloroplast genetic engineering is the observationof exceptionally large accumulation of foreign proteins in transgenic plants. This can be as much as 46% of CRY protein in total soluble protein, even in bleached old leaves (DeCosa et al. 2001). Stable expression of a pharmaceutical protein in chloroplastswas first reported for GVGVP, a protein based polymer with varied medical applications (such as the prevention ofpost-surgicaladhesions and scars, wound coverings, artificial pericardia, tissue reconstruction and programmed drug delivery) (Guda et al. 2000). Subsequently, expression of the human somatotropin via the tobacco chloroplast genome (Staub et al. 2000) to high levels (7% of total soluble protein) was observed. The following investigations that are in progress illustrate the power of this technology to express small peptides, entire operons, vaccines that require oligomeric proteins with stable disulfide bridges and monoclonals that require assembly of heavy/light chains via chaperonins. It is essential to develop a selection system free of antibiotic resistant genes for the edible insulin approach to be successful. One such marker free chloroplast transformation system has been accomplished(Daniell et al. 2000). Experiments are in progress to develop chloroplast transformation of edible leaves (alfalfa and lettuce) for the practical applications of this approach.
Engineering novel **pathways via the chloroplast** genome: In plant and animal cells, nuclear mRNAs are translated monocistronically. This poses a serious problem when engineeringmultiple genes in plants (Bogorad, 2000). Therefore, single genes were first introduced into individual transgenic plants, then these plants were back-crossed to reconstitute the entire pathway or the complete protein to express the polyhydroxybutyrate polymer or Guy=s 13 antibody (Navrath et al. 1994; Ma et al. 1995). Similarly, in a seven year long effort, Ye et al. (2000) recently introduced a set of three genes for a short biosyntheticpathway that resulted in β-carotene expression in rice. In contrast, most chloroplast genes of higher plants are cotranscribed (Bogorad, 2000). Expression of polycistrons via the chloroplast genome provides a unique opportunity to express entire pathways in a single transformation event. We have recently used the *Bacillus thuringiensis* (Bt) *cry*2Aa2 operon as a model system to demonstrate operon expression and crystals formation via the chloroplast genome (De Cosa et al. 2001). *Cry*2Aa2 is the distal gene of a three-gene operon. The *orf* immediately upstream of *cry*2Aa2 codes for a putative chaperonin that facilitates the folding of *cry*2Aa2 (and other proteins) to form preteolytically stable cuboidal crystals (Ge et al. 1998).

Therefore, the *cry*Aa2 bacterial operon was expressed in tobacco chloroplasts to test the resultant transgenic plants for increased expression and improved persistence of the accumulated insecticidal protein(s). Stable foreign gene integration was confirmed by PCR and Southern blot analysis in T₀ and T₁ transgenic plants. *Cry2Aa2* operon derived protein accumulated at 45.3% of the total soluble protein in mature leaves and remained stable even in old bleached leaves (46.1%) as shown in FIG. 4. This is the highest level of foreign gene expression reported in transgenic plants. Exceedingly uncontrollable insects (10-day old cotton bollworm, beetarmy worm) were killed 100% after consuming transgenic leaves. Electron micrographs showed the presence of the insecticidal protein folded into cuboidal crystals similar in shape to *Cry2Aa2* crystals observed in *Bacillus thuringiensis* as shown in FIG. 5. In contrast to currently marketed transgenic plants with soluble CRY proteins, folded protoxin crystals are processed only by target insects that have alkaline gut pH. This approach improves safety of Bt transgenic plants. Absence of insecticidal proteins in transgenic pollen eliminates toxicity to non-target insects via pollen. In addition to these environmentally friendly approaches, this observation serves as a model system for large-scale production of foreign proteins within chloroplasts in a folded configuration enhancing their stability and facilitations single step purification. This is the first demonstration of expression of a bacterial operon in transgenic plants and opens the door to engineer novel pathways in plants in a single transformation event
**Expressing small peptides via the chloroplast genome**: It is common knowledge that the medical community has been fighting a vigorous battle against drug resistant pathogenic bacteria for years. Cationic antibacterial peptides from mammals, amphibians and insects have gained more attention over the last decade (Hancock and Lehrer,1998). Key features of these cationic peptides are a net positive charge, an affinity for negatively-charged prokaryotic membrane phospholipids over neutral-charged eukaryotic membranes and the ability to form aggregates that disrupt the bacterial membrane (Biggin and Sansom, 1999).

There are three major peptides with α-hetical structures, cecropin from *Hyalophora cecropia* (giant silk moth), magainins from *Xenopus laevis* (African frog) and defensins from mammalian neutrophils. Magainin and its analogues have been studied as a broad-spectrum topical agent, a systemic antibiotic; a wound-healing stimulant; and an anticancer agent (Jacob and Zasloff, 1994). We recently observed that a synthetic lytic peptide (MSI-99, 22 amino acids) can be successfully expressed in tobacco chloroplast (DeGray et al 2000). The peptide retained its lytic activity against the phytopathogenic bacteria *Pseudomonas syringae* and multidrug resistant human pathogen, *Pseudomonas aeruginosa.* The anti-microbial peptide (AMP) used in this study was an amphipathic alpha-helix molecule that has an affinity for negatively charged phospholipids commonly found in the outer-membrane of bacteria. Upon contact with these membranes, individual peptides aggregate to form pores in the membrane, resulting in bacterial lysis. Because of the concentration dependent action of the AMP, it was expressed via the chloroplast genome to accomplish high dose delivery at the point of infection. PCR products and Southern blots confirmed chloroplast integration of the foreign genes and homoplasmy. Growth and development of the transgenic plants was unaffected by hyper-expression of the AMP within chloroplasts. *In vitro* assays with T₁ and T₁ plants confirmed that the AMP was expressed at high levels (21.5 to 43% of the total soluble protein) and retained biological activity against *Pseudomonas syringae,* a major plant pathogen. *In situ* assays resulted in intense areas of necrosis around the point of infection in control leaves, while transformed, leaves showed no signs of necrosis (200-800 µg of AMP at the site of Infection) as shown in FIG. 6. T₁ *in vitro assays against Pseudomonas aeruginosa* (a multi-drug resistant human pathogen) displayed a 96% inhibition of growth as shown in FIG. 7. These results give a new option in the battle against phytopathogenic and drug-resistenant human pathogenic bacteria. Small peptides (like insulin) are degraded in most organisms. However, stability of this AMP in chloroplasts opens up this compartment for expression of hormones and other small peptides.
**Expression and assembly of monoclonals in transgenic chloroplasts:** Dental caries (cavities) is probably the most prevalent disease of humankind. Colonization of teeth by *S*. *mutans* is the single most important risk factor in the development of dental caries. *S. mutans* is a non-motile, gram positive coccus. It colonizes tooth surfaces and synthesizes glucans (insoluble polysaccharide) and fructans from sucrose using the enzymes glucosyltransferase and fructosyltransferase respectively (Hotz et al. 1972). The glucans play an important role by allowing the bacterium to adhere to the smooth tooth surfaces. The bacterium ferments sucrose and produces lactic acid after its adherence. Lactic acid dissolves the minerals of the tooth, thereby producing a cavity.

A topical monoclonal antibody therapy to prevent adherence of *S. mutans* to teeth has recently been developed The incidence of cariogenic bacteria (in humans and animals) and dental caries (in animals) was dramatically reduced for periods of up to two years after the cessation of the antibody therapy. No adverse events were detected either in the exposed animals or in human volunteers (Ma et al. 1998). The annual requirement for this antibody in the US alone may eventually exceed 1 metric ton. Therefore, this antibody was expressed via the chloroplast genome to achieve higher levels of expression and proper folding (Panchal et al. 2000). The integration of antibody genes into the chloroplast genome was confirmed by PCR and Southern blot analysis. The expression of both heavy and light chains was confirmed by western blot analysis under reducing conditions as shown in FIGs. 10A and B. The expression of fully assembled antibody was confirmed by western blot analysis under non-reducing conditions as shown in FIG. 10C. This is the first report of successful assembly of a multi-subunit human protein in transgenic chloroplasts. Production of monoclonal antibodies at agricultural level should reduce their cost and create new applications of monoclonal antibodies.
Marker free chloroplast transgenic plants: Most transformation techniques co-introduce a gene that confers antibiotic resistance, along with the gene of interest to impart a desired trait. Regenerating transformed cells in antibiotic containing growth media permits selection ofonly those cells that have incorporated the foreign genes. Once transgenic plants are regenerated, antibiotic resistance genes serve no useful purpose but they continued to produce their gene products. One among the primary concerns of genetically modified (GM) crops is the presence of clinically important antibiotic resistance gene products in transgenic plants that could inactivate oral doses of the antibiotic (reviewed by Puchta 2000; Daniell 1999A). Alternatively, the antibiotic resistant genes could be transferred to pathogenic microbes in the gastrointestinal tract or soil rendering them resistant to treatment with such antibiotics. Anabiotic resistant bacteria are one of the major challenges of modern medicine. In Germany, GM crops containing antibiotic resistant genes have been banned from release (Peerenboom 2000).

Chloroplast genetic engineering offers several advantages over nuclear transformation including high levels of gene expression and gene containment but utilizes thousands of copies of the most commonly used antibiotic resistance genes. Engineering genetically modified (GM) crops without the use of antibiotic resistance genes should eliminate potential risk of their transfer to the environment or gut microbes. Therefore, betaine aldehyde dehydrogenase (BADH) gene from spinach is used herein as a selectable marker (Daniell et al. 2000). The selection process involves conversion of toxic betaine aldehyde (BA) by the chloroplast BADH enzyme to nontoxic glycine betaine, which also serves as an osmoprotectant. Chloroplast transformation efficiency was 25 fold higher in BA selection than spectinomycin, in addition to rapid regeneration (Table 1). Transgenic shoots appeared within 12 days in 80% of leaf discs (up to 23 shoots per disc) in BA selection compared to 45 days in 15% of discs (1 or 2 shoots per disc) on spectinomycinselectionas shown in Fig. 8. Southern blots confirm stable integration of foreign genes into all of the chloroplast genomes (-10,000 copies percell) resulting in homoplasmy. Transgenic tobacco plants showed 1527-1816% higher BADH activity at different developmental stages than untransformed controls. Transgenic plants were morpho-logically indistinguishable from untransformed plants and the introduced trait was stably inherited in the subsequent generation. This is the first report of genetic engineering of the chloroplast genome without the use of antibiotic selection. Use of genes that are naturally present in spinach for selection, in addition to gene containment, should ease public concerns or perception of GM crops. Also, this should be very helpful in the development of edible insulin. Polymer-proinsulin Recombinant DNA Vectors: One possible insulin expression system involves independent expression of insulin chains A and B, as it has been produced in *E*.*coli* for commercial purposes in the past. The disadvantage of this method is that *E.coli* does not form disulfide bridges in the cell unless the protein is targeted to the periplasm. Expensive in *vitro* assembly after purification is necessary for this approach. Therefore, a better approach is to express the human proinsulin as a polymer fusion protein. This method is ideal because chloroplasts are capable of forming disulfide bridges. Using a single gene, as opposed to the individual chains, eliminates the need of conducting two parallel vector construction processes, as is the case for the individual chains. In addition, the need for individual fermentations and purification procedures is eliminated by the single gene method. In addition, proinsulin requires less processing following extraction.

Recently, the human pre-proinsulingene was obtained from Genentech, Inc. First the pre-proinsulinwas sub-cloned into pUC19 to facilitate further manipulations. The next step was to design primers to make chloroplast expression vectors. Since we are interested in proinsulin expression, the 5' primer was designed to land on the proinsulin sequence. This FW primer excluded the 69 bases or 23 coded amino acids of the leader or pre-sequence of preproinsulin. Also, the forward primer included the enzymatic cleavage site for the protease factor Xa to avoid the use of cyanogen bromide. Besides the Xa-factor, a SmaI site was introduced to facilitate subsequent subcloning. The order of the FW primer sequence is SmaI- Xa-factor - Proinsulin gene. The reverse primer included BamHI and XbaI sites, plus a short sequence with homology with the pUC 19 sequence following the proinsulin gene. The 297bp PCR product (Xa Pris) was cloned into pCR2.1. A GVGVP 50-mer was generated as described previously (Daniell et al. 1997) along with the RBS sequence GAAGGAG. Another SmaI partial digestion was performed to eliminate the stop codon of the biopolymer gene, decrease the 50mer to a 40mer, and fuse the 40mer to the Xa-proinsulin sequence. Once the correct fragment was obtained by the partial digestion of SmaI (eliminating the stop codon but including the RBS site), it was ligated to the Xa-proinsulin fusion gene resulting in the construct pCR2.1-40-XaPris. Finally, the biopolymer (40mer) B proinsulin fusion gene was subcloned into the chloro-plast vector pLD-CtV or pSBL-CtV and the orientation was checked in the final vector using suitable restriction sites.
Expression and Purification of the Biopolymer-proinsulin fusion protein: XL-1 Blue strain of *E.coli* containing pLD-OC-XaPris and the negative controls, which included a plasmid containing the gene in the reverse orientation and the *E. coli* strain without any plasmid were grown in TB broth. Cell pellets were resuspended in 500 µl of autoclaved dH₂O or 6M Guanidine hydrochloride phosphate buffer, pH 7.0 were sonicated and centrifuged at 4□C at 10,000 g for 10min. Afer centrifugation, the supernatants were mixed with an equal volume of 2XTN buffer (100 mM Tris-HCl, pH 8, 100 mM NaCl). Tubes were wormed at 42□C for 25 min to induce biopolymer aggregation. Then the fusion protein was recovered by centrifuging at 2,500 rpm at 42□C for 3 min. Samples were run in a 16.5% Tricine gel, transferred to the nitrocellulose membrane, and immunoblotting was performed. When the sonic extract is in 6M Guanidine Hydrochloride Phosphate Buffer, pH 7.0, the molecular weight changes from its original and correct MW 24 kD to a higher MW of approximately 30 kDa as shown in Figs. 9A and B. This is probably due to the conformation of the biopolymer in this buffer.

The gel was first stained with 0.3M CuCl₂ and then the same gel was stained with Commassie R-250 Staining Solution for an hour and then destained for 15 min first, and then overnight CuCl₂ creates a negative stain (Lee et al. 1987). Polymer proteins (without fusion) appear as clear bands against a blue background in color or dark against a light semiopaque background as shown in FIG. 12A. This stain was used because other protein stains such as Coomassie Blue R250 does not stain the polymer protein due to the lack of aromatic side chains (McPherson et al., 1992). Therefore, the observation of the 24 kDa protein in R250 stained gel as shown in FIG. 12B is due to the insulin fusion with the polymer. This observation was further confirmed by probing these blots with the anti-human proinsulin antibody. As anticipated, the polymer insulin fusion protein was observed in western blots as shown in FIG. 13A and B. Larger proteins observed in FIGs. 13A-C are tetramer and hexamer complexes of proinsulin. It is evident that the insulin-polymer fusion proteins are stable in *E*.*coli*. Confirming this observation, recently others have shown that the PBP polymer protein conjugates (with thioredoxin and tendamistat) undergo thermally reversible phase transition, retaining the transition behavior of the free polymer (Meyer and Chilkoti, 1999). These results clearly demonstrate that insulin fusion has not affected the inverse temperature transition property of the polymer. One of the concerns is the stability of insulin at temperatures used for thermally reversible purification. Temperature induced production of human insulin has been in commercial use (Schmidt et al. 1999). Also, the temperature transition can be lowered by increasing the ionic strength of the solution during purification of this PBP (McPherson et al. 1996). Thus, GVGVP-fusion could be used to purity a multitude of economically important proteins in a simple inexpensive step.
Biopolymer-proinsulin fusion gene expression in chloroplast: As described in section d, chloroplast vector was bombarded into the tobacco chloroplast genome via particle bombardment (Daniell, 1997). PCR and Southern Blots were performed to confirm biopolymer-proinsulin fusion gene integration into chloroplast genome. Southern blots show homoplasmy in most T₀ lines but a few showed some heteroplasmy as shown in FIG. 14. Western blots show the expression of polymer proinsulin fusion protein in all transgenic lines in FIG. 13C. Quantification is by ELISA.
Protease Xa Digestion of the Biopolymer-proinslin fusion protein and Purification of Proinsulin: The enzymatic cleavage of the fusion protein to release the proinsulin protein from the (GVGVP)₄₀ was initiated by adding the factor 10A protease to the purified fusion protein at a ratio (w/w) of approximately 1:500. Cleavage of the fusion protein was monitored by SDS-PAGE analysis. We detected cleaved proinsulin in the extracts isolated in 6M guanidine hydrochloride buffer as shown in FIG. 13A and B. Conditions are noweing optimized for complete cleavage. The Xa protease has been successfully used previously to cleave (GVGVP)₂₀-GST fusion (McPherson et al. 1992).
**Evaluation of chloroplast gene expression:** A systematic approach to identify and overcome potential limitations of foreign gene expression in chloroplasts of transgenic plants is essential. Information gained herein increases the utility of chloroplast transformation system by scientists interested in expressing other foreign proteins. Therefore, it is important to systematically analyze transcription, RNA abundance, RNA stability, rate of protein synthesis and degradation, proper folding and biological activity. For example, the rate of transcription of the introduced insulin gene may be compared with the highly expressing endogenous chloroplast genes (rbcL, psbA, 16S rRNA), using run on transcription assays to determine if the 16SrRNA promoter is operating as expected. Transgenic chloroplast containing each of the three constructs with different 5' regions is investigated to test their transcription efficiency. Similarly, transgene RNA levels is monitored by northerns, dot blots and primer extension relative to endogenous rbcL, 16S rRNA, or psbA. These results along with run on transcription assays should provide valuable information of RNA stability, processing, etc. With ourpast experience in expression of several foreign genes, foreign transcripts appear to be extremely stable based on northern blot analysis. However, a systematic study is valuable to advance utility of this system by other scientists.

Importantly, the efficiency of translation may be tested in isolated chloroplasts and compared with the highly translated chloroplast protein (psbA). Pulse chase experiments help assess if translational pausing, premature termination occurs. Evaluation of percent DNA loaded on polysomes or in constructs with or without 5'UTRs helps determine the efficiency of the ribosome binding site and 5' stem-loop translational enhancers: Codon optimized genes are also compared with unmodified genes to investigate the rate of translation, pausing and termination. In our recent experience, we observed a 200-fold difference in accumulation of foreign proteins due to decreases in proteolysis conferred by a putative chaperonin (De Cosa et al. 2001). Therefore, proteins from constructs expressing or not expressing the putative chaperonin (with or without ORF1+2) provide valuable information on protein stability. Thus, all of this information may be used to improve the next generation of chloroplast vectors.
Optimization of gene expression: We have reported that foreign genes are expressed between 3% (*cry2Aa2*) and 46% (*cry2Aa2* operon) in transgenic chloroplasts (Kota et al. 1999; De Cosa et al. 2001). Several approaches may be used to enhance translation of the recombinant proteins. In chloroplasts, transcriptional regulation as a bottle-neck in gene expression has been overcome by utilizing the strong constituitive promoter of the 16s rRNA (Prm). One advantage of Prm is that it is recognized by both the chloroplast encoded RNA polymerase and the nuclear encoded chloroplast RNA polymerase in tobacco (Allison et al. 1996). Several investigators have utilized Prm in their studies to overcome the initial hurdle of gene expression, transcription (De Cosa et al. 2001, Eibl et al. 1999, Staub et al. 2000). RNA stability appears to be one among the least problems because of observation of excessive accumulation of foreign transcripts, at times 16,966-fold higher than the highly expressing nuclear transgenic plants (Lee et al. 2000). Also, other investigations regarding RNA stability in chloroplasts suggest that efforts for optimizing gene expression need to be addressed at the post-transcriptional level (Higgs et al. 1999, Eibl et al. 1999). Our work focuses on addressing protein expression post-transcriptionally. For example, 5= and 3=UTRs are needed for optimal translation and mRNA stablility of chloroplast mRNAs (Zerges 2000). Optimal ribosomal binding sites (RBS=s) as well as a stem-loop structure located 5= adjacent to the RBS are needed for efficient translation. A recent study has shown that replacement of the Shine-Delgarno (GGAGG) with the psbA 5= UTR downstream of the 16S rRNA promoter enhanced translation of a foreign gene (GUS) hundred-fold (Eibl et al. 1999). Therefore, the 200-bp tobacco chloroplast DNA fragment (1680-1480) containing 5= psbA UTR may be used. This PCR product is inserted downstream of the 16S rRNA promoter to enhance translation of the recombinant proteins.

Yet another approach for enhancement of translation is to optimize codon compositions. We have compared A+T% content ofall foreign genes that had been expressed in transgenic chloroplasts with the percentage of chloroplast expression. We found that higher levels of A+T always correlated with high expression levels (see Table 2). It is also potentially possible to modify chloroplast protease recognition sites while modifying codons, without affecting their biological functions. Therefore, optimizing codon compositions of insulin and polymer genes to match the psbA gene should enhance the level of translation. Although rbcL (RuBisCO) is the most abundant protein on earth, it is not translated as highly as the psbA gene due to the extremely high turnover of the psbA gene product. The psbA gene is under stronger selection for increased translation efficiency and is the most abundant thylakoid protein. In addition, the codon usage in higher plant chloroplasts is biased towards the NNC codon of 2-fold degenerate groups (i.e. TTC over TTT, GAC over GAT, CAC over CAT, AAC over AAT, ATC over ATT, ATA etc.). This is in addition to a strong bias towards T at the third position of 4-fold degenerate groups. There is also a context effect that should be taken into consideration while modifying specific codons. The 2-fold degenerate sites immediately upstream from a GNN codon do not show this bias towards NNC. (TTT GGA is preferred to TTC GGA while TTC CGT is preferred to TTT CGT, TTC AGT to TTT AGT and TTC TCT to TTT TCT, Morton, 1993; Morton and Bernadette, 2000). In addition, highly expressed chloroplast genes use GNN more frequently that other genes. The disclosure of web site http://www.kazusa.or.jp/codon and http://www.ncbi.nlm.nih.gov may be used to optimize codon composition by comparing codon usage of different plant species= genomes and PsbA=s genes. Abundance of amino acids in chloroplasts and tRNA anticodons present in chloroplast may be taken into consideration. Optimization of polymer and proinsulin may be performed using a novel PCR approach (Prodromou and Pearl, 1992; Casimiro et al. 1997), which has been successfully used in our laboratory to optimize codon composition of other human proteins.
Vector constructions: pLD vector is used for all the constructs. This vector was developed for chloroplast transformation. It contains the 16S rRNA promoter (Prm) driving the selectable marker gene *aadA* (aminoglycoside adenyl transferase conferring resistance to spectinomycin) followed by the multiple cloning site and then the *psbA* 3' region (the terminator from a gene coding for photosystem II reaction center components) from the tobacco chloroplast genome. The pLD vector is a universal chloroplast expression /integration vector and can be used to transform chloroplast genomes of several other plant species (Daniell et al. 1998, Daniell 1999) because these flanking sequences are highly conserved among higher plants. The universal vector uses *trnA* and *trnI* genes (chloroplast transfer RNAs coding for Alanine and Isoleucine) from the inverted repeat region of the tobacco chloroplast genome as flanking sequences for homologous recombination. Because the universal vector integrates foreign genes within the Inverted Repeat region of the chloroplast genome, it should double the copy number of the transgene (from 5000 to 10,000 copies per cell in tobacco). Furthermore, it has been demonstrated that homoplasmy is achieved even in the first round of selection in tobacco probably because of the presence of a chloroplast origin of replication within the flanking sequence in the universal vector (thereby providing more templates for integration). These, and several other reasons, foreign gene expression was shown to be much higher when the universal vector was used instead of the tobacco specific vector (Guda et al. 2000).
**CTB-Proinsulin Vector Construction:** The chloroplast expression vector pLD-CTB-Proins may be constructed as follows. First, both proinsulin and cholera toxin B-subunit genes were amplified from suitable DNA using primer sequences. Primer 1 contains the GGAGG chloroplast preferred ribosome binding site five nucleotides upstream of the start codon (ATG) for the CTB gene and a suitable restriction enzyme site (SpeI) for insertion into the chloroplast vector. Primer 2 eliminates the stop codon and adds the first two amino acids of a flexible hinge tetrapeptide GPGP as reported by Bergerot et al. (1997), to facilitate folding of the CTB-proinsulin fusion protein. Primer 3 adds the remaining two amino acids for the hinge tetra-peptide and eliminates the pre-sequence of the native pre-proinsulin. Primer 4 adds a suitable restriction site (SpeI) for subcloning into the chloroplast vector. Amplified PCR products may be inserted into the TA cloning vector. Both the CTB and proinsulin PCR fragments may be excised at the SmaI and XbaI restriction sites. Eluted fragments are ligated into the TA cloning vector. The CTB-proinsulin fragment may be excised at the EcoRI sites and inserted into EcoRI digested dephosphorolated pLD vector.

The following vectors may be designed to optimize protein expression, purification and production of proteins with the same amino acid composition as in human insulin.
a) Using tobacco plants, Bibl(1999) demonstrated, *in vivo,* the differences in translation efficiency and mRNA stability of a GUS reporter gene due to various 5= and 3=untranslated regions (UTR=s). This already described systematic transcription and translation analysis can be used in a practical endeavor of insulin production. Consistent with Eibl=s (1999) data for increased translation efficiency and mRNA stability, the psbA 5= UTR can be used in addition with the psbA 3= UTR already in use. The 200 bp tobacco chloroplast DNA fragment containing 5= psbA UTR may be amplified by PCR using tobacco chloroplast DNA as template. This fragment may be cloned directly in the pLD vector multiple cloning site downstream of the promoter and the aadA gene. The cloned sequence may be exactly the same as in the psbA gene.
b) Another approach of protein production in chloroplasts involves potential insulin crystallization for facilitating purification. The *cry2Aa2 Bacillus thuringiensis* operon derived putative chaperonin may be used. Expression of the *cry2Aa2* operon in chloroplasts provides a model system for hyper-expression of foreign proteins (46% of total solublc protein) in a folded configuration enhancing their stability and facilitating purification (De Cosa et al. 2001). This justifies inclusion of the putative chaperonin from the *cry2Aa2* operon in one of the newly designed constructs. In this region there are two open reading frames (ORF1 and ORF2) and a ribosomal binding site (rbs). This sequence contains elements necessary for *Cry2Aa2* crystallization, which help to crystallize insulin and aid in subsequent purification. Successful crystallization of other proteins using this putative chaperonin has been demonstrated (Ge et al.1998). The ORF1 and ORF2 of the Bt *Cry2Aa2* operon may be amplified by PCR using the complete operon as a template. Subsequent cloning, using a novel PCR technique, allows for direct fusion of this sequence immediately upstream of the proinsulin fusion protein without altering the nucleotide sequence, which is normally necessary to provide a restriction enzyme site (Horton et al. 1988).
c) To address codon optimization the proinsulin gene may be subjected to certain modifications in subsequent constructs. The plastid modified proinsulin (PtPris) can have its nucleotide sequence modified such that the codons are optimized for plastid expression, yet its amino acid sequence remains identical to human proinsulin. PtPris is an ideal substitute for human proinsulin in the CTB fusion peptide. The expression of this construct can be compared to the native human proinsulin to determine the effects to codon optimization, which serve to address one relevant mechanistic parameter of translation. Analysis of human proinsulin gene showed that 48 of its 87 codons were the lowest frequency codons in the chloroplast for the amino acid for which they encode. For example, there are six different codons for leucine. Their frequency within the chloroplast genome ranges from 7.3 to 30.8 per thousand codons. There are 12 leucines in proinsulin, 8 have the lowest frequency codons (7.3), and none code for the highest frequency codons (30.8). In the plastid, optimized proinsulin gene all the codons code for the most frequent, whereas in human proinsulin over half of the codons are the least frequent. Human proinsulin nucleotide sequence contains 62% C+G, whereas plastid optimized proinsulin gene contain 24% C+G. Generally, lower C+G content of foreign genes correlates with higher levels of expression (Table 2).
d) Another version of the proinsulin gene, mini-proinsulin (Mpris), may also have its codons optimized for plastid expression, and its amino acid sequence does not differ from human proinsulin (Pris). Pris= sequence is B Chain-RR-C Chain-KR-A Chain, whereas MPris= sequence is B Chain-KR-A Chain. The MPris sequence excludes the RR-C Chain, which is normally excised in proinsulin maturation to insulin. The C chain of proinsulin is an unnecessary part of *in vitro* production of insulin. Proinsulin folds properly and forms the appropriate disulfide bonds in the absence of the C chain. The remaining KR motif that exists between the B chain and the A chain in MPris allows for mature insulin production upon cleavage with trypsin and carboxypeptidase B. This construct may be used for our biopolymer fusion protein. It=s codon optimization and amino acid sequence is ideal for mature insulin production.
e) Our current human proinsulin-biopolymer fusion protein contains a factor Xa proteolytic cut site, which serves as a cleavage point between the biopolymer and the proinsulin. Currently, cleavage of the polymer-proinsulin fusion protein with the factor Xa has been inefficient in our hands. Therefore, we replace this cut site with a trypsin cut site. This eliminates the need for the expensive factor Xa in processing proinsulin. Since proinsulin is currently processed by trypsin in the formation of mature insulin, insulin maturation and fusion peptide cleavage can be achieved in a single step with trypsin and carboxypeptidase B.
f) We observed incomplete translation products in plastids when we expressed the 120mer gene (Guda et al. 2000). Therefore, while expressing the polymer-proinsulin fusion protein, we decreased the length of the polymerprotein to 40mer, without losing the thermal responsive property. In addition, optimal codons for glycine (GGT) and valine (GTA), which constitute 80% of the total amino acids of the polymer, have been used. In all nuclear encoded genes, glycine makes up 147/1000 amino acids while in tobacco chloroplasts it is 129/1000. Highly expressing genes like psbA and rbcL of tobacco make up 192 and 190 gly/1000. Therefore, glycine may not be a limiting factor. Nuclear genes use 52/1000 proline as opposed to 42/1000 in chloroplasts. However, currently used codon for proline (CCG) can be modified to CCA or CCT to further enhance translation. It is known that pathways for proline and valine are compartmentalized in chloroplasts (Guda et al. 2000). Also, proline is known to accumulate in chloroplasts as an osmoprotectant (Daniell et al. 1994).
g) Codon comparison of the CTB gene with psbA, showed 47% homology with the most frequent codons of the psbA gene. Codon analysis showed that 34% of the codons of CTB are complimentary to the tRNA population in the chloroplasts in comparison with 51% of psbA codons that are complimentary to the chloroplast tRNA population. Because of the high levels of CTB expression in transgenic chloroplasts. (Henriques and Daniell, 2000), there will be no need to modify the CTB gene.

DNA sequence of all constructs may be determined to confirm the correct orientation of genes, in frame fusion, and accurate sequences in the recombinant DNA constructs. DNA sequencing may be performed using a Perkin Elmer ABI prism 373 DNA sequencing system using a ABI Prism Dye Termination Cycle Sequencing kit Insertion sites at both ends may be sequenced by using primers for each strand.

Expression of all chloroplast vectors are first tested in *E.coli* before their use in tobacco transformation because of the similarity of protein synthetic machinery (Brixley et al. 1997). For *Escherichia coli* expression XL-1 Blue strain was used. *E.coli* may be transformed by a standard CaCl₂ method.
**Bombardment and Regeneration of Chloroplast Transgenic Plants:** Tobacco (*Nicotiana tabacum* var. Petit Havana) and nicotine free edible tobacco (LAMD 605, gift from Dr. Keith Wycoff, Planet Biotechnology) plants may be grown aseptically by germination of seeds on MSO medium (Daniell 1993). Fully expanded, dark green leaves of about two month old plants may be used for bombardment.

Leaves may be placed abaxial side up on a Whatman No. 1 filter paper laying on the RMOP medium (Daniell, 1993) in standard petri plates (100 x 15 mm) for bombardment. Gold (0.6 µm) microprojectiles may be coated with plasmid DNA (chloroplast vectors) and bombardments carried out with the biolistic device PDS1000/He (Bio-Rad) as described by Daniell (1997). Following bombardment, petri plates are sealed with parafilm and incubated at 24 □ C under 12 h photoperiod. Two days after bombardment, leaves are chopped into small pieces of~5 mm² in size and placed on the selection medium (RMOP containing 500 µg/ml of spectinomycin dihydrochloride) with abaxial side touching the medium in deep (100 x 25 mm) petri plates (~10 pieces perplate). The regenerated spectinomycin resistant shoots are chopped into small pieces (~2mm²) and subcloned into fresh deep petri plates (~5 pieces per plate) containing the same selection medium. Resistant shoots from the second culture cycle are transferred to the rooting medium (MSO medium supplemented with IBA, 1 mg/liter and spectinomycin dihydrochloride, 500 mg/liter). Rooted plants may be transferred to soil and grown at 26□C under continuous lighting conditions for further analysis.
**Polymerase Chain Reaction:** PCR may be performed using DNA isolated from control and transgenic plants to distinguish a) true chloroplast transformants from mutants and b) chloroplast transformants from nuclear transformants. Primers for testing the presence of the aadA gene (that confers spectinomycin resistance) in transgenic plants may be landed on the aadA coding sequence and 16S rRNA gene (primers 1P&1M,). To test chloroplast integration of the insulin gene, one primer lands on the aadA *gene* while another lands on the native chloroplast genome (primers 3P&3M). No PCR product is obtained with nuclear transgenic plants using this set of primers. The primer set (2P & 2M) may be used to test integration of the entire gene cassette without any internal deletion or looping out during homologous recombination, by landing on the respective recombination sites. A similar strategy has been used successfully by us to confirm chloroplast integration of foreign genes (Daniell et al., 1998; Kota et al., 1999; Guda et al., 2000). This screening is essential to eliminate mutants and nuclear transformants. Total DNA from unbombarded and transgenic plants may be isolated as described by Edwards et al. (1991) to conduct PCR analyses in transgenic plants. Chloroplast transgenic plants containing the proinsulin gene may then be moved to second round of selection to achieve homoplasmy.
**Southern Blot Analysis:** Southern blots are performed to determine the copy number of the introduced foreign gene per cell as well as to test homoplasmy. There are several thousand copies of the chloroplast genome present in each plant cell. Therefore, when foreign genes are inserted into the chloroplast genome, it is possible that some of the chloroplast genomes have foreign genes integrated while others remain as the wild type (heteroplasmy). Therefore, to ensure that only the transformed genome exists in cells of transgenic plants (homoplasmy), the selection process is continued. To confirm that the wild type genome does not exist at the end of the selection cycle, total DNA from transgenic plants should be probed with the chloroplast border (flanking) sequences (the trnI-trnA fragment as shown in Figs. 2A and 3B. If wild type genomes are present (heteroplasmy), the native fragment size is observed along with transformed genomes. The presence of a large fragment (due to insertion of foreign genes within the flanking sequences) and absence of the native small fragment confirms homoplasmy (Daniell et al., 1998; Kota et al., 1999; Guda et al., 2000).

The copy number of the integrated gene is determined by establishing homoplasmy for the transgenic chloroplast genome. Tobacco Chloroplasts contain 5000~10,000 copies of their genome per cell (Daniell et al. 1998). If only a fraction of the genomes are actually transformed, the copy number, by default, must be less than 10,000. By establishing that in the transgenics the insulin inserted transformed genome is the only one present, one can establish that the copy number is 5000~10,000 per cell. This is usually done by digesting the total DNA with a suitable restriction enzyme and probing with the flanking sequences that enable homologous recombination into the chloroplast genome. The native fragment present in the control should be absent in the transgenics. The absence of native fragment proves that only the transgenic chloroplast genome is present in the cell and there is no native, untransformed, chloroplast genome, without the insulin gene present. This establishes the homoplasmic nature of the transformants, simultaneously providing an estimate of 5000~10,000 copies of the foreign genes per cell.
**Northern Blot Analysis:** Northern blots may be performed to test the efficiency of transcription of the proinsulin gene fused with CTB or polymer genes. Total RNA is isolated from 150 mg of frozen leaves by using the "Rneasy Plant Total RNA Isolation Kit" (Qiagen Inc., Chatsworth, CA). RNA (10-40 µg) is denatured by formaldehyde treatment, separated on a 1.2% agarose gel in the presence of formaldehyde and transferred to a nitrocellulose membrane (MSI) as described in Sambrook et al. (1989). Probe DNA (proinsulin gene coding region) may be labeled by the random-primed method (Promega) with ³²P-dCTP isotope. The blot is then pre-hybridized, hybridized and washed as described above for southern blot analysis. Transcript levels may be quantified by the Molecular Analyst Program using the GS-700 Imaging Densitometer (Bio-Rad, Hercules, CA) or the like.
**Polymer-insulin fusion protein purification, quantitation and characterization:** Because polymer insulin fusion proteins exhibit inverse temperature transition properties as shown in Figs. 9 and 10, they may be purified from transgenic plants essentially following the same method described for polymer purification from transgenic tobacco plants (Zhang et al.,1996). Polymer extraction buffer contains 50 mM Tris-HCl, pH, 7.5, 1% 2-mecaptoethanol, 5mM EDTA and 2mM PMSF and 0.8 MNaCl. The homogenate is then centrifuged at 10,000 g for 10 minutes (4□C), and the pellet discarded. The supernatant is incubated at 42□C for 30 minutes and then centrifuged immediately for 3 minutes at 5,000 g (room temperature). If insulin is found to be sensitive to this temperature, Tₜ is lowered by increasing salt concentration (McPherson et al., 1996). The pellet containing the insulin-polymer fusion protein is resuspended in the extraction buffer and incubated on ice for 10 minutes. The mixture is centrifuged at 12,000 g for 10 minutes (4□C). The supernatant is then collected and stored at -20□C. The purified polymer insulin fusion-protein is electrophoresed in a SDS-PAGE gel according to Laemmli (1970) and visualized by either staining with 0.3 M CuCl₂ (Lee et aL 1987) or transferred to nitrocellulose membrane and probed with antiserum raised against the polymer or insulin protein as described below. Quantification of purified polymer proteins may be carried out by ELISA in addition to densitometry.

After electrophoresis, proteins may be transferred to a nitrocellulose membrane electrophoretically in 25 mM Tris, 192 mM glycine, 5% methanol (pH 8.3). The filter is blocked with 2% dry milk in Tris-buffered saline for two hours at room temperature and stained with antiserum raised against the polymer AVGVP (kindly provided by the University of Alabama at Birmingham, monoclonal facility) overnight in 2% dry milk/Tris buffered saline. The protein bands reacting to the antibodies are visualized using alkaline phosphatase-linked secondary antibody and the substrates nitroblue tetrazolium and 5-bromo-4-chloro-3-indolyl-phosphate (Bio-Rad). Alternatively, for insulin-polymer fusion proteins, a Mouse anti-human proinsulin (IgG1) monoclonal antibody may be used as a primary antibody. To detect the binding of the primary antibody to the recombinant proinsulin, a Goat anti-mouse IgG Horseradish Peroxidase Labeled monoclonal antibody (HPR) may be used. The substrate to be used for conjugation with HRP may be 3,3=, 5,5=Tetramethylbenzidine. Products may be purchased from American Qualex Antibodies in San Clemente, CA. As a positive control, human recombinant proinsulin from Stigma may be used. This human recombinant proinsulin was expressed in *E.coli* by a synthetic proinsulin gene. Quantification of purified polymer fusion proteins may be carried out by densitometry using Scanning Analysis software (BioSoft, Ferguson, MO). Total protein contents may be determined by the dye-binding assay using reagents supplied in kit from Bio-Rad, with bovine serum albumin as a standard.
**Characterization of CTB expression:** CTB protein levels in transgenic plant crude extract can be determined using quantitative ELISA assays. A standard curve may be generated using known concentrations of bacterial CTB. A 96-well microtiter plate loaded with 100 µl/well of bacterial CTB (concentrations in the range of 10 -1000 ng) is incubated overnight at 4DC. The plate is then washed thrice with PBST (phosphate buffered saline containing 0.05% Tween-20). The background may be blocked by incubation in 1% bovine serum albumin (BSA) in PBS (300 µl/well) at 37□C for 2 h followed by washing 3 times with PBST. The plate may be incubated in a 1:8,000 dilution of rabbit anti-cholera toxin antibody (Sigma C-3062) (100 □l/well) for 2 h at 37□C, followed by washing the wells three times with PBST. The plate may be incubated with a 1:80,000 dilution of anti-rabbit IgG conjugated with alkaline phoshatase (100 □l/well) for 2 h at 37□C and washed thrice with PBST. Then, 100 □l alkaline phosphatase substrate (Sigma Fast p-nitrophenyl phosphate tablet in 5 ml of water is added and the reaction stopped with 1M NaOH (50 □lwell) when absorbancies in the mid-range of the titration reach about 2.0, or after 1 hour, whichever comes first. The plate is then be read at 405nm. These results are used to generate a standard curve from which concentrations of plant protein are extrapolated. Thus, total soluble plant protein (concentration previously determined using the Bradford assay) in bicarbonate buffer, pH 9.6 (15mM Na₂Co₃, 35mM NaHCO₃) may be loaded at 100 plant □1/well and the same procedure as above can be repeated. The absorbance values can be used to determine the ratio ofCTB protein to total soluble plant protein, using the standard curve generated previously and the Bradford assay results.
**Inheritance of Introduced Foreign Genes:** While if is unlikely that introduced DNA move from the chloroplast genome to nuclear genome, it is possible that the gene can be integrated in the nuclear genome during bombardment and remain undetected in Southern analysis. Therefore, in initial tobacco transformants, some are allowed to self-pollinate, whereas others are used in reciprocal crosses with control tobacco (transgenics as female accepters and pollen donors; testing for maternal inheritance). Harvested seeds (T1) are germinated on media containing spectinomycin. Achievement of homoplasmy and mode of inheritance can be classified by looking at germination results. Homoplasmy is indicated by totally green seedlings (Daniell et al., 1998) while heteroplasmy is displayed by variegated leaves (lack of pigmentation, Svab & Maliga, 1993). Lack of variation in chlorophyll pigmentation among progeny also underscores the absence of position effect, an artifact of nuclear transformation. Maternal inheritance is demonstrated by sole transmission of introduced genes via seed generated on transgenic plants, regardless of pollen source (green seedlings on selective media). When transgenic pollen is used for pollination of control plants, resultant progeny do not contain resistance to chemical in selective media (appears bleached; Svab and Maliga, 1993). Molecular analyses can confirm transmission and expression of introduced genes, and T2 seed is generated from those confirmed plants by the analyses described above.
**Comparison of Current Purification with Polymer-based Purification Methods**: It is important to compare purification methods by testing yield and purity of insulin produced in *E.coli* and tobacco. Three methods may be compared: a standard fusion protein in *E.coli,* polymer proinsulin fusion protein in *E.coli,* and polymer proinsulin fusion in tobacco. Polymer proinsulin fusion peptide from transgenic tobacco may be purified by methodology described in section c) and Daniell (1997). *E.coli* purification is performed as follows. One liter of each pLD containing bacteria is grown in LB/ampicillin (100 □g/ml) overnight and the fusion protein, either polymer-proinsulin or the control fusion protein (Cowley and Mackin 1997), expressed. Cells are harvested by centrifugation at 5000 X g for 10 min at 4□C, and the bacterial pellets resuspended in 5 ml/g (wet wt. Bacteria) of 100 mM Tris-HCl, pH 7.3. Lysozyme is added at a concentration of 1 mg/ml and placed on a rotating shaker at room temperature for 15 min. The lysate is subjected to probe sonication for two cycles of 30 s on/30 s off at 4□C. Cellular debris is removed by centrifugation at 1000 X g for 5 min at 4□C. The *E.coli* produced proinsulin polymer fusion protein is purified by inverse temperature transition properties (Daniell et al., 1997). After Factor Xa cleavage (as described in section c)) the proinsulin is isolated from the polymer using inverse temperature transition properties (Daneill et al., 1997) and subject to oxidative sulfitolysis as described below. Alternatively, the control fusion protein is purified according to Cowley and Mackin (1997) as follows. The supernatant is retained and centrifuged again at 27000 X g for 15 min at 4□C to pellet the inclusion bodies. The supernatant is then discarded and the pellet resuspended in I ml/g (original wt. Bacteria) of dH₂O, aliquoted into microcentrifuge tubes as 1 ml fractions, and then centrifuged at 16000 X g for 5 min at 4□C. The pellets are individually washed with 1 ml of 100 mM Tris-HCl, pH 8.5, 1M urea,1-1 Triton X-100 and again washed with100 mM Tris HCl pH8.5,2 M urea, 2 % Trinton X-100. The pellets are then resuspended in 1 ml of dH₂O and transferred to a pre-weighed 30 ml Corex centrifuge tube. The sample is centrifuged at 15000 X g for 5 min at 4□C, and the pellet resuspended in 10 ml/g (wet wt. pellet) of 70% formic acid. Cyanogen bromide is added to a final concentration of 400 mM and the sample incubated at room temperature in the dark for 16 h. The reaction is stopped by transferring the sample to a round bottom flask and removing the solvent by rotary evaporation at 50□OC. The residue is resuspended in 20 ml/g (wet wt. pellet) of dH₂O, shell frozen in a dry ice ethanol bath, and then lyophilized. The lyophilized protein is dissolved in 20 ml/g (wet wt. pellet) of 500 mM Tris-HCl, pH 8.2,7 M urea. Oxidative sulfitolysis may be performed by adding sodium sulfite and sodium tetrathionate to final concentrations of 100 and 10 mM, respectively, and incubating at room temperature for 3 h. This reaction is stopped by freezing on dry ice.
**Purification and folding of Human Proinsulin**: The S-sulfonated material may be applied to a 2 ml bed of Sephadex G-25 equilibrated in 20 mM Tris-HCl, pH 8.2, 7 M urea, and then washed with 9 vols of 7 M urea. The collected fraction is applied to a Pharmacia Mono Q HR 5/5 column equilibrated in 20 mM Tris HCl, pH 8.2, 7 M urea at a flow rate of 1 ml/min. A linear gradient leading to final concentration of 0.5 M NaCl is used to elute the bound material. 2 min (2 ml) fractions are collected during the gradient, and protein concentration in each fraction determined. Purity and molecular mass of fractions is estimated by Tricine SDS-PAGE (as shown in Fig. 2), where Tricine is used as the trailing ion to allow better resolution of peptides in the range of 1-1000 kDa. Appropriate fractions are pooled and applied to a 1.6 X 20 cm column of Sephadex G-25 (superfine) equilibrated in 5 mM ammonium acetate pH 6.8. The sample is collected based on UV absorbance and freeze-dried. The partially purified S-sulfonated material is then resuspended in 50 mM glycine/NaOH, pH 10.5 at a final concentration of 2 mg/ml. β-mercaptoethanol is added at a ratio of 1.5 mol per mol of cysteine S-sulfonate and the sample stirred at 40C in an open container for 16 h. The sample is then analyzed by reversed-phase high-performance liquid chromatography (RP-HPLC) using a Vydac C₄ column (2.2 X 150 mm) equilibrated in 4% acetonitrile and 0.1% ' TFA. Adsorbed peptides are eluted with a linear gradient of increasing acetonitrile concentration (0.88% per min up to a maximum of 48%). The remaining refolded proinsulin is centrifuged at 16000 Xg to remove insoluble material, and loaded onto a semi-preparative Vydac C₄ column (10 X 250 mm). The bound material is then eluted as described above, and the proinsulin collected and lyophilized.
**Analysis and characterization of insulin expressed in *E.coli* and Tobacco**: The purified expressed proinsulin is subjected to matrix-assisted laser desorption/ionization-time of flight (MALDI-TOF) analysis (as described by Cowley and Mackin, 1997), using proinsulin from Eli Lilly as both an internal and external standard. To determine if the disulfide bridges have formed correctly naturally inside chloroplasts or by *in vitro* processing, a proteolytic digestion id performed using *Staphylococcus aureus* protease V8. Five 0 g of both the expressed proinsulin and Eli Lilly=s proinsulin are lyophilized and resuspended in 50 □1 of 250 mM NaPO₄, pH 7.8. Protease V8 is added at a ratio of 1:50 (w/w) in experimental samples and no enzyme added to the controls. All samples are then incubated overnight at 37□C, the reactions stopped by freezing on dry ice, and samples stored at -20□OC until analyzed. The samples are analyzed by RP-HPLC using a Vydac C₄ column (2.2 X 150 mm) equilibrated in 4% acetonitrile and 0.1% TFA. Bound material is then eluted using a linear gradient of increasing acetonitrile concentration (0.88% per min up to a maximum of 48%).
**CTB-GM1 ganglioside binding assay:** A GM1-ELISA assay may be performed as described by Arakawa et al. (1997) to determine the affinity of plant-derived CTB for GM1-ganglioside. The microtiter plate is coated with monosialoganglioside-GM1 (Sigma G-7641) by incubating the plate with 100 µl/well of GM1 (3.0 µg/ml) in bicarbonate buffer, pH 9.6 at 40C overnight. Alternatively, the wells can be coated with 100 µl/well of BSA (3.0 µg/ml) as control. The plates are incubated with transformed plant total soluble protein and bacterial CTB (Sigma C-9903) in PBS (100 µl/well) overnight at 4□C. The remainder of the procedure is identical to the ELISA described above.
**Induction of oral tolerance:** Four week old female NOD mice may, for example, be purchased from Jackson Laboratory (Bar Harbor, ME) and housed at an animal care facility. The mice are divided into three groups, each group consisting often mice. Each group is fed one of the following nicotine free edible tobacco: untransformed, expressing CTB, or expressing CTB-proinsulin fusion protein. Beginning at 5 weeks of age, each mouse is fed 3 g of nicotine free edible tobacco once per week until reaching 9 weeks of age (a total of five feedings).
**Antibody titer:** At ten weeks of age, the serum and fecal material are assayed for anti-CTB and anti-proinsulin antibody isotypes using the ELISA method described above.
**Assessment of diabetic symptoms in NOD mice:** The incidence of diabetic symptoms can be compared among mice fed with control nicotine free edible tobacco that expresses CTB and those that express the CTB-proinsulin fusion protein. Starting at 10 weeks of age, the mice are monitored on a biweekly basis with urinary glucose test strips (Clinistix and Diastix, Bayer) for development of diabetes. Glycosuric mice are bled from the tail vein to check for glycemia using a glucose analyzer (Accu-Check, Boehringer Mannheim). Diabetes is confirmed by hyperglycemia (>250 mg/dl) for two consecutive weeks (Ma et al. 1997).

### Literature Cited

Allison LA, Maliga P (1995) Light-responsive and transcription-enhancing elements regulate the plastid psbD core promotor. EMBO J 14: 3721 - 3730.
Arakawa T, Yu J, Chong, DKX, Hough J, Engen PC, Langridge WHR (1998) A plant-based cholera toxin B subunit-insulin fusion protein protects against the development of autoimmune diabetes. Nature Biotechnology. 16: 934 - 938.
Arakawa T, Chong DKX, Merritt JL, Langridge WHR (1997) Expression of cholera toxin B subunit oligomers in transgenic potato plants. Transgenic Research 6: 403 - 413.
Arntzen CJ, Mason HS, et al (1998) Edible vaccine protects mice against E.coli heat labile enterotoxin: potatoes expressing a synthetic LTB gene. Vaccine. 16(13): 1336 - 1343.
Bergerot I, Ploix C, Peterson J, Moulin V, Rask C, Fabien N, et al. (1997) A cholera toxoid-insulin conjugate as an oral vaccine against spontaneous autoimmune diabetes. Proc. Natl. Acad. Sci. USA. 94: 4610 - 4614.
Biggin P, Sansom M (1999) Interactions of α-helices with lipid bilayers: a review of simulation studies. Biophysical Chemistry 76: 161 - 183.
Bock R, Hagemann R (2000) Extracellular inheritance: Plastid genomics: Manipulation of Plastid genomes and biotechnological applications. Progress in Botany 6: 76 - 90.
Bogorad L (2000) Engineering chloroplasts: an alternative site for foreign genes, proteins, reactions and products. Trends in Biotechnology 18: 257 - 263.
Brixey J, Guda C, Daniell H (1997) The chloroplast psbA promoter is more efficient in E.coli than the T7 promoter for hyper expression of a foreign protein. Biotechnology Letters 19: 395 - 400.
Burnette JP (1983) Experimental Manipulation of Gene Expression. Oxender, D.L., Fox, C.F. eds. pp. 71-82, Alan R, Liss, Inc., New York, NY.
Carlson PS (1973) The use of protoplasts for genetic research. Proc. Natl. Acad. Sci. USA 70: 598 - 602.
Casimiro DR, Wright PE and Dyson HJ (1997) PCR-based gene synthesis and protein NMR Spectroscopy. Structure 5 (11): 1407 - 1412.
Chance RE, Frank BH (1993) Research, development, production and safety of biosynthetic human insulin. Diabetes Care. 16(3): 133 - 142.
Cohen A, Mayfield (1997) Translational regulation of gene expression in plants. Current Opinion in Biotechnology 8: 189 - 194.
Cowley DJ, Mackin RB (1997) Expression, purification and characterization of recombinant human proinsulin. FEBS Letts. 402: 124 - 130.
Crea R, Kreszewski A, Hirose T, Itakura K (1978) Chemical synthesis of genes for human insulin. Proc. Natl. Acad. Sci. 75(12): 5765 - 5769.
Daniell H (1995) Producing polymers in plants and bacteria. Inform 6: 1365 - 1370.
Daniell H (1997) Transformation and foreign gene expression in plants mediated by microprojectile bombardment. Meth Mol Biol 62: 453 - 488.
**Daniell H (1999)** Universal chloroplast integration and expression vectors, transformed plants and products thereof, World Intellectual Property Organization WO 99/10513.
Daniell H, Datta R, Varma S, Gray S, Lee SB (1998) Containment of herbicide resistance through genetic engineering of the chloroplast genome. Nature Biotechnology 16: 345 - 348.
Daniell H, Guda C (1997) Biopolymer production in microorganisms and plants. Chemistry and Industry, 14: 555 - 560.
Daniell H, Guda C, McPherson DT, Xu J, Zhang X, Urry DW (1997) Hyper expression of an environmentally friendly synthetic polymer gene. Meth Mol Biol 63: 359 - 371.
Daniell H, Krishnan M, McFadden BA (1991) Expression of B-glucuronidase gene in different cellular compartments following biolistic delivery of foreign DNA into wheat leaves and calli. Plant Cell Reports 9: 615 - 619.
Daniell H, Krishnan M, Umabai U, Gnanam A (1986) An efficient and prolonged in vitro translational system from cucumber etioplasts. Biochem. Biophys. Res. Comun 135: 48 - 255.
Daniell H, McFadden BA (1987) Uptake and expression of bacterial and cyanobacterial genes by isolated cucumber etioplasts. Proc Natl Acad Sci USA 84: 6349 - 6353.
**Daniell H, McFadden BA (1988)** Genetic Engineering of plant chloroplasts. United States Patents 5,932,479; 5,693,507.
Daneill H, Muthukumar B, Lee SB (2000) Engineering chloroplast genome without the use of antibiotic resistance genes. Current Genetics, in press.
Daniell H, Ramanujan P, Krishnan M, Gnanam A, Rebeiz CA (1983) In vitro synthesis of photosynthetic membranes: I. Development ofphotosystem I activity and cyclic phosphorylation. Biochem. Biophys. Res. Comun 111: 740 - 749.
Daniell H, Rebeiz CA (1982) Chloroplast culture EX: Chlorphyll(ide) A biosynthesis in vitro at rates higher than in vivo. Biochem. Biophys. Res. Comun 106: 466 - 471.
Daniell H, Vivekananda J, Neilsen B, Ye GN, Tewarl KK, Sanford JC (1990) Transient foreign gene expression in chloroplasts of cultured tobacco cells following biolistic delivery of chloroplast vectors. Proc Natl Acad Sci USA 87: 88 - 92.
Daniell H, Wycoff K, Stratfield S (2000) Production of vaccines, monoclonals, and pharmaceutical proteins in plants. Trends in Plant Science, in press.
Davidson, MB (1998) Diagnosis and classification of diabetes mellituus in "Diabetes Mellitus-Diagnosis and Treatment" pp.1 - 16, 4th edition, W.B. Saunders Co., Philidelphia, PA.
De Cosa B, Moar W, Lee SB, Miller M, Daniell H (2001) Hyper-expression of the Bt Cry2Aa2 operon in chloroplasts leads to formation of insecticidal crystals. Nature Biotechnology, in press.
DeGray G, Smith F, Sanford J, Daniell H (2000) Hyper-expression of an antimicrobial peptide via the chloroplast genome to confer resistance against phytopathogenic bacteria. In review.
Dertzbaugh MT, Elson CO (1993) Comparitive effectiveness of the cholera toxin B subunit and alkaline phosphatase as carriers for oral vaccines. Infect.Immun. 61: 48 - 55.
Drescher DF, Follmann H, Haberlein I (1998) Sulfitolysis and thioredoxin-dependent reduction reveal the presence of a structural disulfide bridge in spinach chloroplast fructose-1, 6-bisphosphate. FEBS Letters 424: 109 - 112.
Edwards K, Johnstone C, Thompson C (1991) A simple and rapid method for preparation of plant genomic DNA for PCR analysis. Nucleic Acids Res 19: 1349.
Eibl C, Zou Z, Beck A, Kim M, Mullet J, Koop UH (1999) In vivo analysis of plastid psbA, rbcL and rp132 UTR elements by chloroplast transformation: tobacco plastid gene expression is controlled by modulation of transcript levels and translation efficiency. The Plant Journal 19: 333 - 345.
Gaskins HR, Prochazka M, Hamaguchi K, Serreze DV, Leiter EH. (1992) Beta cell expression of endogenous xenotropic retrovirus distinguishes diabetes-susceptible NOD/Lt from resistant NON/Lt mice. J Clin Invest. 90(6): 2220 - 7.
Ge B et al (1998) Differential effects of helper proteins encoded by the cry2A and cry11A operons on the formation of Cry2A inclusions in Bacillus thuringiensis. FEMS Microbiol. Lett. 165: 35 - 41.
Gill D M (1976) The arrangement of subunits in cholera toxin. Biochemistry.15; 1242 - 1248.
Goeddel DV, Kleid DG, Bolivar F, Heyneker HL, Yansura DG, Crea R, Hirose T, Kraszewski A, Italkura K, Riggs AD (1979) Expression in Escherichia coli of chemically synthesized genes for human insulin. Proc. Natl. Acad. Sci. 76: 106 - 110.
Goldberg AL, Goff SA (1986) Maximizing Gene Expression. Reznikoff and Gold, eds.pp. 287 - 311, Butterworth Publishers, Stoneham, MD.
Guda C, Zhang X, McPherson DT, Xu J, Cherry J, Urry DW, Daniell H (1995) Hyperexpression of an environmentally friendly synthetic gene. Biotechnol Lett 17: 745 - 750.
Guda C, Lee SB, Daniell H (2000) Stable expression of biodegradable protein based polymer in tobacco chloroplasts. Plant Cell Rep. 19: 257 - 262.
Gunby P (1978) Bacteria directed to produce insulin in test application of genetic code. J. Am. Med. Assoc. 240(16): 1697 - 1698.
Hall SS (1988) Invisible Frontiers - The Race to Synthesize a Human Gene. Atlantic Monthly Press, New York, NY.
Hancock R, Lehrer R (1998) Cationic peptides: a new source of antibiotics. TIBTECH 16: 82 - 88.
Hancock WW, Sayegh MH, Weiner HL et al. (1993) Oral,but not intravenous , alloantigen prevents accelerated allograft rejection by selective intragraft Th2 cell cativation. Transplantation 55: 1112 - 18.
Haq T A, Mason HS, Clements JD, Arntzen C et aL (1995) Oral immunization with a recombinant bacterial antigen produced in transgenic plants. Science .268: 714 - 716.
Heifetz P (2000) Genetic engineering of the chloroplast. Biochimie 82: 655 - 666.
Henriques L and Daniell H (2000) Expression of cholera toxin B subunit oligomers in transgenic tobacco chloroplasts. In review.
Herzog RW, Singh NK, Urry DW, Daniell H (1997) Synthesis of a protein based polymer (elastomer) gene in Aspergillus nidulans. Applied Microbiology & Biotechnology 47: 368 - 372.
Higgs DC, Shapiro RS, Kindle KL, Stern DB (1999) Small cis-acting sequences that specify secondary structures in chloroplast mRNA are essential for RNA stability and translation 19(12): 8479 - 8491.
Holmgren J, Lycke N, Czerkinsky C (1993) Cholera toxin and cholera B subunit as oral-mucosal adjuvant and antigen vector systems. Vaccine 11(12): 1179 - 84. Review.
Horton RM, Hunt HD, Ho SN, Pullen JK, Pease LR (1989) Engineering hybrid genes without the use of restriction enzymes: gene splicing by overlap extension. Gene 77: 61 - 68.
Hotz P, Guggenheim B, Schmid R (1972) Carbohydrates in pooled dental plaque. Caries Res. 6(2): 103 - 21.
Jacob L, Zasloff M (1994) Potential therapeutic applications of megainins and other antimicrobial agents of animal origin. Ciba Foundation Symposium 186: 197 - 223.
Khoury SJ, Lider O Weiner HL et al. (1990) Suppression of experimental auto immune encephalomyelitis by oral administration of myelin basic protein. Cell Immunol.131: 302 - 10.
Kim J, Mayfield PS (1997) Protein disulfide isomerase as a regulator of chloroplast translational activation. Science 278: 1954 - 1957.
Kim J-S, Raines RT (1993) Ribonuclease S-peptide as a carrier in fusion proteins. Protein. Sci. 2: 348 - 356.
Kota M, Daniell H, Varma S, Garczynski F, Gould F, Moar WJ (1999) Overexpression of the Bacillus thuringiensis Cry2A protein in chloroplasts confers resistance to plants against susceptible and Bt-resistant insects. Proc. Natl. Acad. Sci. USA, 96: 1840 - 1845.
Kusnadi A, Nikolov Z, Howard J (1997) Porduction of Recombinant proteins in Transgenic plants: Practical considerations. Biotechnology and Bioengineering. 56 (5): 473 - 484.
Laemmli UK (1970) Cleavage of structural proteins during the assembly of the head of bacteriophage T4. Nature 227: 680 - 685.
Lebens M, Holmgren J (1994) Mucosal vaccines based on the use of Cholera Toxin B subunit as immunogen and antigen carrier. Recombinant Vectors in Vaccine Development [Brown F (ed)]. 82: 215 - 227.
Lee C, Levin A, Branton D (1987) Copper staining: A five-minute protein stain for sodium dodecyl sulfate-polyacrylamide gels. Anal Biochem 166: 308 - 312.
Lee SB, Kwon H, Kwon S, Park S, Jeong M, Han S, Daniel H, Byun H (2000) Drought tolerance conferred by the yeast trehalose-6 phosphate synthase gene engineered via the chloroplast genome. In review.
Lipscombe M, Charles IG, Roberts M, Dougan G, Tite J, Fairweather NF (1991) intranasal immunization using the B subunit of the Escherichia coli heat-labile toxin fused to an epitope of the Bordetella pertussis P.69 antigen. Mol. Microbiol. 5(6): 1385 - 1392.
Ma J, et al (1995) Generation and assembly of secretory antibodies in plants. Science 268: 716 - 719.
Ma J, Hitmak B, Wycoff K, Vine N, Charlegue D, Yu LI, Hein M, Lehner T (1998) Charaterization of a recombinant plant monoclonal secretory antibody and preventive immunotherapy in humans. Nature Medicine. 4(5): 601 - 606.
Ma S-W, Zhao D-L, Yin Z-Q, Mukherjee R, Singh B, Qin H-Y et al. (1997) Transgenic plants expressing autoantigens fed to mice to induce oral tolerance. Transgenic Res. 3: 793 - 796.
Marina CV et al (1988) An Escherichia coli vector to express and purify foreign proteins by fusion to and separation from maltose binding protein. ene 74: 365 - 373.
Mason HS, Ball JM, Arntzen CJ et al. (1996) Expression of Norwalk virus capsid protein in transgenic tobacco and potato and its oral immunogenicity in mice. Proc. Nat. Acad. Sci. USA; 93: 5335 - 40.
May GD, Mason HS, Lyons PC (1996) Application of transgenic plants as production systems for pharmaceuticals in ACS symposium series 647. Fuller et al eds., chapter 13, 196 - 204.
Mathiowitz E, Jacob JS, Jong YS, Carino GP, Chickering DE, Chaturvedi P, Santos CA, Vijayarahauau K, Montgomery S, Bassett M, Morrell C (1997) Biologically erodable microspheres as potential oral drug delivery systems. Nature 386: 410 - 414.
McBride KE, Svab Z, Schaaf DJ, Hogen PS, Stalker DM, Maliga P (1995) Amplification of a chimeric Bacillus gene in chloroplasts leads to extraordinary level of an insecticidal protein in tobacco. Bio/technology 13: 362 - 365.
McKenzie SJ and Halsey JF (1984) Cholera toxin B subunit as a carrier protein to stimulate a mucosal immune response. Journal of Immunology.133: 1818 - 24.
McPherson DT, Morrow C., Mineham DJ, Wu J, Hunter E, Urry DW (1992) Production and purification of a recombinant elastomeric polypeptide, G-(VPGVG) 19-VPGV from Escherichia coli. Biotechnology Prog. 8: 317 - 322.
McPherson DT, Xu J, Urry DW (1996) Product purification by reversible phase transition following Escherichia coli expression of genes encoding up to 251 repeats of the elastomeric pentapeptide GVGVP. Protein Expression and Purification 7: 51 - 57.
Mekalanos JJ, Sadoff JC (1979) Cholera vaccines: Fighting an ancient scourge. Science 265: 1387 - 1389.
Meyer DE, Chilkoti A (1999) Purificaiton of recombinant protiens by fusion with thermally-responsive polypeptides. Nature Biotechnology 17: 1112 - 1115.
Miller A, Weiner HL et al. (1992) Suppressor T cells generated by oral tolerization to myelin basic protein suppress both in vitro and in vivo immune responses by the release of transforming growth factor B after antigen specific triggering. Proc. Nat. Acad. Sci. USA 89: 421 - 5.
Mor TS, Palmer KE et aL (1998) Perspective: edible vaccines- a concept coming of age. Trends in Microbiology.6: 449 - 453.
Morton B (1993) Chloroplast DNA Codon Use: Evidence for Selection at the psbA Locus Based on tRNA Availability. J Mol Evol 37: 273 - 280.
Morton B and Bernadette G (2000) Codon usage in plastid genes is correlated with context, position within the gene, amino acid content. J Mol Evol. 50: 184 - 193.
Nashar TO, Amin T, Marcello A, Hirst TR (1993) Current progress in the development of the B subunits of cholera toxin and Escherichia coli heat-labile enterotoxin as carriers for the oral delivery of heterologous antigens and epitopes. Vaccine. 11 (2): 235 - 40.
Navrath C, Poirier Y, Somerville C (1994) Targeting of the polyhydroxybutyrate biosynthetic pathway to the plastis of Arabidopsis thaliana results in high levels of polymer accumulation. Proc. Natl. Acad. Sci. 91: 12760 - 12764.
Nilsson J, Stahl S, Lundeberg J, Uhlen M, Nygren PA (1997) Affinity fusion strategies for detection, purification, and immobilization of recombinant proteins. Protein Expr. Purif. 11: 1-16.
Oakly WG, Pyke DA, Taylor KW (1973) Biochemical basis of Diabetes. In ADiabetes and It=s Management@, pp. 1 - 14, 2nd edition, Blackwell Scientific Publications, Osney Mead, Oxford.
Ong E et al. (1989) The cellulose-binding domains of celulases: tools for biotechnology. Trends Biotechnol. 7: 239 - 243.
Peerenboom E (2000) Geman health minister calls time out for B. T. maize. Nature Biotechnology. 18: 374.
Petridis D, Sapidou E, Calandrants J (1995) Computer-Aided process analysis and economic evaluation for biosynthetic human insulin production-A case study. Biotechnology and Bioengineering 48: 529 - 541.
Panchal T, Wycoff K and Daniell H (2000) Expression of humanized antibody in transgenic tobacco chloroplasts. In review.
Prodromou C and Pearl LH (1992) Recursive PCR: a novel technique for total gene synthesis. Protein Engineering 5(8): 827 - 829.
Puchta H (2000) Removing selectable marker genes: taking the shortcut. Trends in Plant Science 5: 273 - 274.
Reulland E, Miginiac-Maslow M (1999) Regulation of chloroplast enzyme activities by thioredoxins: activation or relief from inhibition. Trends in Plant Science 4: 136 - 141.
Roy H (1989) Rubisco assembly: a model system for studying the mechanism of chaperonin action. Plant Cell. 1: 1035 - 1042.
Sambrook J, Fritch EF, Maniatis T (1989) Molecular cloning. Cold Spring Harbor Press, Cold Spring Harbor, New York.
Sayegh MH, Khoury SJ,Weiner HL et al (1992) Induction of immunity and oral tolerance with polymorphic classII major histocompatibility complex allopeptides in the rat Proc. Nat. Acad. Sci. USA; 89: 7762 - 6.
Schmidt M, Babu KR, Khanna N, Marten S, Rinas U (1999) Temperature induced production of recombinant human insulin in high density cultures of recombinant E.coli. Biotechnology 68: 71 - 83.
Sidorov VA, Kasten D, Pang SZ, Hajdukiewicz PTJ, Staub JM, Nehra NS (1999) Stable chloroplast transformation in potato: use of green fluorescent protein as a plastid marker. Plant Journal 19: 209 - 216.
Smith DB, Johnson KS (1988) Single-step purification of polypeptides expressed in Escherichia coli as fusion with glutathione S-transferase. Gene 67: 31 - 40.
Smith PA et al. (1998) A plasmid expression system for quantitative in vivo biotinylation of thioredoxin fusion proteins in Escherichia coli. Nucleic Acids Res. 26: 1414 - 1420.
Smith MC, Furman TC, Ingolia TD, Pidgeon C (1988) Chelating peptide-immobilized metal ion affinity chromatography. J. Biol. Chem. 263: 7211 - 7215.
Staub JM, Garcia B, Graves J, Hajdukiewicz PT, Hunter P, Nehra N, Paradkar V, Schlittler M, Carroll JA, Spatola L, Ward D, Ye G; Russell DA (2000) High-yield production of a human therapeutic protein in tobacco chloroplasts. Nat. Biotechnol, 18(3): 333 - 338.
Steiner DF, Arquila ER, Lerner J, Martin DB (1978) Recombinant DNA Research. Diabetes. 27: 877 - 878.
Su X, Prestwood AK, McGraw RA (1992) Production of recombinant porcine tumor necrosis factor alpha in a novel E. coli expression system. Biotechniques 13: 756 - 762.
Sun JB, Holmgren J, Czerkinsky C (1994) Cholera toxin B subunit: an efficient transmucosal carrier-delivery system for induction of peripheral immunological tolerance. Proc. Natl. Acad. Sci. USA. 91: 10795 - 10799.
Sun JB, Rask C, Olsson T, Holmgren J, Czerkinsky C (1996) Treatment of experimental autoimmune encephalomyelitis by feeding myelin basic protien conjugated to cholera toxin B subunit. Proc. Natl. Acad. Sci. USA. 93: 7196 - 7201.
Svab Z, Maliga P (1993) High frequency plastid transformation in tobacco by selection for a chimeric aadA gene. Proc Natl Acad Sci USA 90: 913 - 917.
ThanavalaY,Yang Y, Lyons P et al. (1995) Immunogenicity of transgenic plant derived hepatitis B surface antigen. Proc. Nat. Acad. Sci. USA; 92: 3358 - 3361.
Trentham DE, Weiner HL et al. (1993) Effects of oral administration of Type II collagen on rheumatoid arthritis. Science 261: 1727 - 30.
Tsao KW, deBarbieri B, Hanspeter M, Waugh DW (1996) A versatile plasmid expression vector for the production of biotinylated proteins by site-specific enzymatic modification in Escherichia coli. Gene 69: 59 - 64.
Urry DW (1995) Elastic biomolecular machines. Scientific American. 272: 64 - 69.
Urry DW, Nicol A, Gowda DC, Hoban LD, McKee A, Williams T, Olsen DB, Cox BA (1993) Medical applications of bioelastic materials. In: Gebelein CG (ed), Biotechnological Polymers: Medical, Pharmaceutical and Industrial Applications, Technomic Publishing Co., Inc., Atlanta, GA, pp. 82 - 103.
Urry DW, McPherson J, Xu J, Gowda DC, Jing N, Parker TM, Daniell H, Guda C (1996) Protein Based Polymeric Materials (Synthesis and Properties) in APolymeric Materials Encyclopedia@, (Solomone ed.), vol. 9, pp. 2645 - 2699, CRC Press.
Urry DW, Nichol A, McPherson DT, Xu J, Shewry PR, Harris CM, Parker TM, Gowda DC (1994) Properties, preparations and applications of bioelastic materials in AHandbook of Biomaterials and Applications@, Mercel Dekker, New York, NY.
Urry DW (1991) Thermally Driven-Self assembly, Molecular Structuring and Entropic Mechanisms in Elastomeric Polypeptides in AMolecular Conformation and BiologicalInteractions@ (Balaram, P., and Ramasashan, S., Eds.), pp. 555 - 583, Indian Acad.of Sci., Bangalore, India.
Vierling E (1991) The roles of heat shock proteins in plants. Annu. Rev. Plant Physiol. Plant Mol. Biol. 42: 579 - 620.
Weiner HL, Mackin GA, Hafler DA et al. (1993) Double blind plot trial of oral tolerization with myelin antigens in multiple sclerosis. Science 259: 1321 - 4.
Ye GN, Daniell H, Sanford JC (1990) Optimization of delivery of foreign DNA into higher plant chloroplasts. Plant Mol. Biol. 15: 809 - 819.
Ye X, et al. (2000) Engineering the provitamin A (β-carotene) biosynthetic pathway into (carotenoid-free) rice endosperm. Science 287: 303 - 305.
Yeh H, Ornstein-Goldstein N, Indik Z, Sheppard P, Anderson N, Rosenbloom J, Cicilia G, Yoon K, Rosenbloom J (1987) Sequence variation of bovine elastin mRNA due to alternative splicing. Collagen Related Res 7: 235 - 247.
Zerges W (2000) Translation in chloroplasts. Biochimie 82: 583 - 601.
Zhang X, Guda C, Datta R, Dute R, Urry DW, Daniell H (1995) Nuclear expression of an environmentally friendly synthetic protein-based polymer gene in tobacco cells. Biotechnol Lett 17: 1279 - 1284.
Zhang X, Urry DW, Daniell H (1996) Expression of an environmentally friendly synthetic protein-based polymer gene in transgenic tobacco plants. Plant Cell Rep 16: 174 - 179.
Zhang ZJ, Davidson L, Weiner HLet aL(1991) Supression of diabetes in nonobese diabetic mice by oral administration of porcine insulin. Proc. Nat. Acad. Sci.USA 88: 10252 - 10256.

### References to Project Description

1. Kusnadi A, Nikolov Z, Howard J (1997). Production of Recombinant proteins in Transgenic plants: Practical considerations. Biotechnology and Bioengineering. 56 (5): 473-484.
2a.May GD, Mason HS, Lyons PC (1996). Application of transgenic plants as production systems for pharmaceuticals in ACS symposium series 647. Fuller et al eds., chapter 13, 196-204.
2b.Petridis D, Sapidou E, Calandranis J (1995). Computer-Aided process analysis and economic evaluation for biosynthetic human insulin production-A case Study. Biotechnology and Bioengineering 48: 529-541.
3. DeCosa B, Moar W, Lee SB, Miller M, Daniell H (2000). Hyper-expression of the Bt Cry2Aa2 operon in chloroplasts leads to formation of insecticidal crystals. Nature Biotechnology, In press.
4. Drescher DF, Follmann H, Haberlein I (1998). Sulfitolysis and thioredoxin-dependent reduction reveal the presence of a structural disulfide bridge in spinach chloroplast fructose-1, 6-bisphosphate. FEBS Letters 424: 109-112.
5. Roy H (1989). Rubisco assembly: a model system for studying the mechanism of chaperonin action Plant Cell. 1: 1035-1042.
6. Vierling E (1991). The roles of heat shock proteins in plants. Annu. Rev. Plant Physiol. Plant Mol. Biol. 42: 579-620.
7. Reulland E, Miginiac-Maslow M (1999). Regulation of chloroplast enzyme activities by thioredoxins: activation or relief from inhibition. Trends in Plant Science 4: 136-141.
8. Kim J, Mayfield PS (1997). Protein disulfide isomerase as a regulator of chloroplast translational activation. Science 278: 1954-1957.
9. Staub JM, Garcia B, Graves J, Hajdukiewicz PT, Hunter P, Nehra N, Paradkar V, Schlittler M, Carroll JA, Spatola L, Ward D, Ye G, Russell DA (2000). High-yield production of a human therapeutic protein in tobacco chloroplasts. Nat. Biotechnol.18(3): 333-338.
10. Henriques L and Daniell H. (2000) Expression of cholera toxin B subunit oligomers in transgenic tobacco chloroplasts. In review.
11. Panchal T, Wycoff K and Daniell H. (2000). Expression of humanized antibody in transgenic tobacco chloroplasts. In review.
12. Brennan S, Owen M, Boswell D, Lewis J, Carrell R (1984). Circulationg proalbumin associated with a variant proteinase inhibitor. Biochim. Biophys. Acta 802: 24-28.
1, Adelman J, Bock SC, Franke AE, Houck CM, Najarian RC, Seeburg PH, Wion KL (1981). The sequence of human serum albumin cDNA and its expression in E. coli. Nucleic Acids Res. 9(22): 6103-114.
14. Latta M, Knapp M. Sarmientos P, Brefort G, Becquart J, Guerrier L, Jung G, and Mayaux J (1987). Synthesis and purification of mature human serum albumin from E. coli. Bio/Technology 5: 1309-1314.
15. Saunders C, Schmidt B, Mallonee R, Guyer M (1987). Secretion of human serum albumin from Bacillus subtilis. J. Bact. 169: 2917-2925.
16. Sumi, et al. (1993). Biotechnology of Bloods proteins 227. Rivat and Stoltz eds. Pp 293-298.
17. Okabayashi K, Nakagawa Y, Hayasuke N, Ohi H, Miura M, Ishida Y, Shimizu M, Murakami K, Hirabayashi K, Minamino H, et al (1991). Secretory expression of the human serum albumin gene in the yeast Saccharomyces cerevisiae. J. Biochem. (Tokyo) 110(1): 103-10.
18. Quirk AV, Geisow MJ, Woodrow JR, Burton SJ, Wood PC, Sutton AD, Johnson RA, Dodsworth N (1989). Production of recombinant human serum albumin from Saccharomyces cerevisiae. Biotechnol. AppL Biochem. 11(3): 273-87.
19. Sleep D, Belfield GP, Goodey AR (1990). The secretion of human serum albumin from the yeast Saccharomyces cerevisiae using five different leader sequences. Biotechnology (NY) 8(1): 42-6.
20. Dodsworth N, Harris R, Denton K, Woodrow J, Wood PC, Quirk A (1996). Comparative studies of recombinant human albumin and human serum albumin derived by blood fractionation. Biotechnol. Appl. Biochem. 24(Pt 2): 171-6.
21. Saliola M, Mazzoni C, Solimando N, Crisa A, Falcone C, Jung G, Fleer R (1999). Use of the K1ADH4 promoter for ethanol-dependent production of recombinant human serum albumin in Kluyveromyces lactis. Appl. Environ. Microbiol. 65(1): 53-60.
22. Ohtani W, Nawa Y, Takeshima K, Kamuro H, Kobayashi K, Ohmura T (1998). Physicochemical and immunochemical properties of recombinant human serum albumin from Pichia pastoris. Anal. Biochem. 256(1): 56-62.
23. Sijmons PC, Dekker BM, Schrammeijer B, Verwoerd TC, van den Elzen PJ, Hoekema A (1990). Production of correctly processed human serum albumin in transgenic plants. Biotechnology (NY) 8(3): 217-21.
24. Samuel CE (1991). Antiviral actions of interferon. Interferon-regulated cellular proteins and their surprisingly selective antiviral activities. Virology 183: 1-11.
197). New insights into the mechanimss of interferon alpha: an immunoregulatory and anti-inflammatory cytokine. Gastroenterology 112: 1017-1021.
26. Malefyt RW (1997). The role of type I interferons in the differenciation and function of Th1 and Th2 cells. Semin. Oncol. 24: S94-S98.
27. Diaz M, Bohlander S and Allen G (1987). Nomenclature of human interferon genes. J. Interferon Res. Pp. 13243-13247.
28. Walter MR (1997). Three-dimensional models of interferon-a subtypes 1FN1-conI, IFN-□8, and IFN-□1 derived from the crystal Structure of IFN-□2b. Semin. Oncol. 24: S952-S962.
29. Tovey MG, Streuli M, Gresser I, Gugenheim J, Blanchard B, Guymarho J, Vignaux F, and Gigou M (1987). Interferon messenger RNA is produced constitutively in the organs of normal individuals. Proc. Natl. Acad. Sci. USA 84: 5038-5042.
30. Huang X, Yuan J, Goddard A, Foulis A, James RFL, Lemmark A, Pujol-Borreel R, Rabinovich A, Somoza N, and Stewart TA (1995). Interferon expression in the pancreases of patients with type I diabetes. Diabetes 44: 658-664.
31. Bisat F, Raj NB, and Pitha PM (1988). Differencial and cell type specific expression of murine alpha interferon genes is regulated on the transcriptional level. Nucleic Acids Res 16:6067-6083.
32. Stark GR, Kerr IM, Williams BRG, Silverman RH, and Schreiber RD (1998). How cells respond to interferons. Annu. Rev. Biochem. 67: 227-264.
33. Foster GR, Rodrigues O Ghouze F, Schulte-Frohlinde E, Testa D, Liao MJ, Stark GR, Leadbeater L, and Thomas HC (1996). Different relative activities of human cell-derived interferon-a subtypes: IFN-□8 has very high antiviral potency. J Interferon Citokine Res. 16: 1027-1033.
34. Castelruiz Y, Larrea E, Boya P, Civeira MP and Prieto J (1999). Interferon □ subtypes and levels of type I interferons in the liver and peripheral mononuclear cells in patients with chronic hepatitis C and controls. Hepatology 29: 1900-1904.
35. Larrea E, Alberdi A, Castelruiz Y, Boya P, Civeira MP and Prieto J. Expression of IFN-□ subtypes in peripheral mononuclear cells from patients with chronic hepatitis C. A role for IFN-α5. J. Viral Hepatitis (in press).
36. Prieto J, Civeira MP, and Larrea E. (2000). Use of IFN-□5 as a therapy for liver viral infections. Patent number 2138565 B1.
37a. Weissmann, et a1 (1980). Processes for preparing IFN □. Science 209: 1343-1349.
D, Greene AR, Heathcliffe GR, Moore VE, et al. (1983). Chemical synthesis of a human interferon-alpha2 gene and its expression in E. coli. Nucleic Acids Res. 11: 6419-6435.
38. Hitzeman RA, Hagie FE, Levine HL, Goeddel DV, Ammerer G, Hall BD. (1981). Expression of a human gene for interferon in yeast Nature 293: 717- 722.
39. Thatcher DR, Panayotatos N (1986). Related Purification of recombinant human IFN-□ 2. Methods Enzymol. 119: 166-177.
40. Swaminathan S, Khanna N. (1999). Affinity purification of recombinant interferon alpha on a mimetic ligand adsorbent. Protein Expre Purif. !5: 236-242.
41. Babu KR, Swaminathan S, Marten S, Khanna N, Rinas U (2000). Production of interferon-□ in high cell density cultures of recombinant Escherichia coli and its single step purification from refolded inclusion body proteins. Appl Microbiol Biotechnol 53(6): 655-60.
42. Smirnov SP, Teverovskaia EKh, Krasheninnikova LV, Pukhal'ski VA (1990). Design of an expression integrative vector and its application for introducing the human recombinant alfa interferon gene into plants. Genetika. 26(12): 2111-2121.
43. Elderbaum O, Stein D, Holland N, Gafni Y, Livneh O, Novick D, Rubinstein M, Sele 1. (1992). Expression of active human interferon beta in transgenic plants. J. Interferon Research. 12: 449-453.
44. Daughaday WH and Rotwein P (1989). Insulin-like growth Factor I and II. Peptide, Messenger Ribonucleic Acid and Gene Structures, Serum, and Tissue concentrations. Endocrine Reviews 10: 68-91.
45. Adamo ML, Neuenschwander S, LeRoith D, and Roberts CT(1994). Structure, expression, and regulation of the IGF-I gene. Current directions in Insulin growth factor research 343:1-11.
46. Condorelli G, Bueno R, and Smith RJ (1994). Two alternatively spliced forms of the human insulin-like growth factor I receptor have distinct biological activities and internalization kinetics. J. Biol. Chem. 269: 8510-8516.
47. Humbel RE and Rinderknecht E (1978). The amino acid sequence of human insuline-like growth factor I and its structural homology with proinsulin. J. Biol. Chem. 253: 2769-2776.
48. Lund PK (1994). Insuline-like Growth Factor I: Molecular Biology and Relevance to Tissue-Specific Expresion and Action. Recent Progress in hormone Research 49: 135-149.
J, Milewski WM, Young BD, Nakayama K and Steiner DF (1997). Processing of wild-type and mutant proinsulin-like growth factor-IA by subtilin related proprotein convertases. J. Biol. Chem. 272: 6663-6670.
50. Jones JL, D'ercole AJ, Camacho-Hubner C and Clemmons DR. (1991) Phosphorylation of insulin-like growth factor (IGF)-binding protein 1 in cell culture and in vivo: Effects on affinity for IGF-I. Proc. Natl. Acad. Sci. USA. 88: 7481-7485.
51. Heidenreich KI, Freidenberg GR, Figlewicz DT and Gilmore PR (1986). Evidence for a subtype of InsuIine-Likc Growth Factor I receptor in brain. Regulatory Peptides 15: 301-310.
52. Picardi A, Costa de Oliveira A, Muguerza B, Tosar A, Quiroga J, Castilla-Cortázar I, Santidrián S, and Prieto J (1997). Low doses of insulin-like growth factor-I improve nitrogen retention and food efficiency in rats with early cirrhosis. J. Hepatology 26: 191-202.
53. Castilla-Contázar I, Prieto J, Urdaneta E, Pascual M, Nuñez M, Zudaire E, Garcia M, Quiroga J, and Santidrian S (1997). Impaired Intestinal Sugar Transport in Cirrhotic Rats: Correction by low doses of Insulin-like Growth Factor I. Gastroenterology 113: 1180-1187.
54. Castilla-Cortázar I, Picardi A, Tosar A, Ainzúa J, Urdaneta E, Garcfa M, Pascual M, Quiroga J, and Prieto J (1999). Effect of insulin-like growth factor I on in vivo intestinal absorption of D-galactose in cirrhotic rats. American Journal Physiology 276(39): G37-G42.
55. Pascual M, Castilla-Cortazar I, Urdaneta E, Quiroga J, Garcia M, Picardi A, and Prieto J (2000). Altered intestinal transport of amino acids in cirrhotic rats: the effect of insulin-like growth factor-I. American J. Physiology (Gastrointestinal liver physiology)(in press).
56. Cemborain A, Castilla-Cortázar I, Garcia M, Quiroga J, Muguerza B, Picardi A, Santidrian S, and Prieto J (1998). Osteopenia in rats with liver cirrhosis: beneficial effects of IGF-I treatment. J. Hepatology 28: 122-131.
57. Castilla-Cortazar I, Garcia M, Quiroga J, Diez N, Diez-Caballero F, Calvo A, Diaz M, and Prieto J (2000). Insulin-like Growth Factor-I Reverts Testicular Atrophy in Rats With Advanced Cirrhosis. Hepatology 31: 592-600.
58. Castilla-Cortázar I, García M, Muguerza B, Quiroga J, Perez R, Santidrian S and Prieto J (1997). Hepatoprotective effects of Insulin-like Growth Factor I in Rats with Carbon Tetrachloride-induced Cirrhosis. Gastroenterology 113: 1682-1691.
Anin S, Klipper-Aurbach Y, and Klinger B (1992). Effects of insulin-like growth factor on linear growth, head circumference, and body hair in patients with Laron-type dwarfism. Lancet 339: 1258-1261.
60. Bach MA, Chin E, and Bondy CA (1993). The effects of recombinant insulin-like growth factor I (IGF-I) on growth hormone, IGF-II, IGF binding protein and blood glucose levels in normal and diabetic adolescents. Ped. Res 33:190-198.
61. Bondy CA (1994). Clinical uses of insulin-like growth factor I. Ann. Intern. Med 120:593-601.
62. Ebeling P, Jones JD, O'Fallon WM, Janes CH and Riggs BL (1993). Short-term effects of recombinant human insulin growth factor I on bone turnover in normal women. J. Clin. Endocrinol. Metab. 77: 1384-1387.
63. Kozakowski J, Papierska L, Krassowski J and Zgliczynski S (1998). The effect of growth hormone replacement therapy on markers of bone formation and bone mineral density in elderly men. Pol. Arch. Med. Wewn. 100: 306-312.
64. Fleming RYD, Rutan R, Jahoor F, Barrow RE, Wolfe RR, and Herndon DN (1992). Effect of recombinant human insulin-like growth hormone on catabolic hormones and free fatty acids following thermal injury. J. Traumatol. 32: 698-703.
65. Lieberman S, Butterfield GE, Harrison D and Hoffman AR (1994). Anabolic effects of recombinant insulin-like growth factor I in cachetic patients with the acquired immunodeficiency syndrome. J. Clin. Endocrinol. Metab. 78: 404-410.
66. Gellerfors P, Axelsson K, Helander A, Johansson S, Kenne L, Lindqvist B, Pavlu P, Skottner A, and Fryklund L (1989). Isolation and characterization of a glycosylated form of human insulin- like growth factor I produced in Saccharomyces cerevisiae. J. Biol. Chem. 264: 11444-11449.
67. Wong E, Seetharam R, Kotts C, Heeren R, et al. (1988). Expression of insulin like growth factor-I in E. coli. Gene 68: 193-203.
68. Moks T, Abrahamsen L, Osterlof B, Josephson S, Ostling M, et al. (1987). Large scale affinity purification of human insulin like growth factor I from culture medium of E. coli. Biotechnology 5: 379-382.
69. Schultz M, Buell G, Schmid E, Movra R and Selzer G (1987). Increased expression in E. coli of a synthetic gene encoding human somatomedin C after gene duplication and fusion. J Bacteriol. 169: 5385-5392.
70a.Sun-Ok Kim, Young Ik Lee (1996). High level expression and simple purification of recombinant insulin-like growth factor L Journal of Biotechnology 48: 97-105.
70b.Zinovieva N, Lassnig C, Schams D, Besenfelder U, Wolf E, Muller S, Frenyo L, Seregi J, M, Brem G (1998). Stable production of human insulin-like growth factor 1 (IGF-1) in the milk of hemi- and transgenic rabbits over several generations. Transgenic Res. 7(6): 437-47.
71. Nilsson B, Forsberg G and Hartmanis M (1991). Expression and Purification of Recombinant Insulin-like Growth Factors from Escherichia coli. Met. Enzymol. 198: 3-16.
72. Daniell H, McFadden BA (1988). Genetic Engineering of plant chloroplasts. United States Patents 5,932,479; 5,693,507.
73. Daniell H (1999). Universal chloroplast integration and expression vectors, transformed plants and products thereof. World Intellectual Property Organization WO 99/10513.
74. Carlson PS (1973). The use of protoplasts for genetic research. Proc. Natl. Acad. Sci. USA 70: 598-602.
75. Daniell H, Rebeiz CA (1982). Chloroplast culture IX: Chlorphyll(ide) A biosynthesis in vitro at rates higher than in vivo. Biochem. Biophys. Res. Comun. 106: 466-471.
76. Daniell H, Ramanujan P, Krishnan M, Gnanam A, Rebeiz CA (1983). In vitro synthesis of photosynthetic membranes: I. Development of photosystem I activity and cyclic phosphorylation. Biochem. Biophys. Res. Comun. 111: 740-749.
77. Daniell H, Krishnan M, Umabai U, Gnanam A (1986). An efficient and prolonged in vitro translational system from cucumber etioplasts. Biochem. Biophys. Res. Comun 135: 48-255.
78. Daniell H, McFadden BA (1987). Uptake and expression of bacterial and cyanobacterial genes by isolated cucumber etioplasts. Proc. Natl. Acad. Sci. USA 84: 6349-6353.
79. Daniell H (1993). Foreign gene expression in chloroplasts of higher plants mediated by tungsten particle bombardment. Methods Enzymol 217: 536-556.
80. Daniell H, Vivekananda J, Neilsen B, Ye GN, Tewari KK, Sanford JC (1990). Transient foreign gene expression in chloroplasts of cultured tobacco cells following biolistic delivery of chloroplast vectors. Proc. Natl. Acad. Sci. USA 87: 88-92.
81. Ye X, et al (2000). Engineering the provitamin A (β-carotene) biosynthetic pathway into (carotenoid-free) rice endosperm. Science 287: 303-305.
82. Daniell H, Krishman M, McFadden BA (1991). Expression of B-glucuronidase gene in different cellular compartments following biolistic delivery of foreign DNA into wheat leaves and calli. Plant Cell Reports 9: 615-619.
83. Svab Z, Maliga P (1993). High frequency plastid transformation in tobacco by selection for a chimeric aadA gene. Proc. Natl. Acad. Sci. USA 90: 913-917.
KE, Svab Z, Schaaf DJ, Hogen PS, Stalker DM, Maliga P (1995). Amplification of a chimeric Bacillus gene in chloroplasts leads to extraordinary level of an insecticidal protein in tobacco. Bio/technology 13: 362-365.
85. Kota M, Daniell H, Varma S, Garczynski F, Gould F, Moar WJ (1999). Overexpression of the Bacillus thuringiensis Cry2A protein in chloroplasts confers resistance to plants against susceptible and Bt-resistant insects. Proc. Natl. Acad. Sci. USA 96: 1840-1845.
86. Daniell H, Datta R, Varma S, Gray S, Lee SB (1998). Containment of herbicide resistance through genetic engineering of the chloroplast genome. Nature Biotechnology 16: 345-348.
87. Sidorov VA, Kasten D, Pang SZ, Hajdukiewicz PTJ, Staub JM, Nehra NS (1999). Stable chloroplast transformation in potato: use of green fluorescent protein as a plastid marker. Plant Journal 19: 209-216.
88. Guda C, Lee SB, Daniell H (2000). Stable expression of biodegradable protein based polymer in tobacco chloroplasts. Plant Cell Rep. 19: 257-262.
89. Vaucheret H, Beclin C, Elmayan T, Feuerbach F, Godon C, Morel JB, Mourrain P, Palauqui JC, Vernhettes S. (1998). Transgene induced gene silencing in plants. Plant J. 16: 651-659.
90. De Neve M, et al. (1999). Gene silencing results in instability of antibody production in transgenic plants. Mol. Gen. Genetics 260: 582-592.
91. Bogorad L (2000). Engineering chloroplasts: an alternative site for foreign genes, proteins, reactions and products. Trends in Biotechnology 18: 257-263.
92. Navrath C, Poirier Y, Somerville C (1994). Targeting of the polyhydroxybutyrate biosynthetic pathway to the plastis of Arabidopsis thaliana results in high levels of polymer accumulation. Proc. Natl. Acad. Sci. 91: 12760-12764.
93. Ma J, et al (1995). Generation and assembly of secretory antibodies in plants. Science 268: 716-719.
94. Ge B et al (1998). Differential effects of helper proteins encoded by the cry2A and cry11A operons on the formation of Cry2A inclusions in Bacillus thuringiensis. FEMS Microbiol. Lett. 165: 35-41.
95. Hancock R, Lehrer R (1998). Cationic peptides: a new source of antibiotics. TIBTECH 16: 82-88.
96. Biggin P, Sansom M (1999). Interactions of α-helices with lipid bilayers: a review of simulation studies. Biophysical Chemistry 76: 161-183.
97. Jacob L, Zasloff M (1994). Potential therapeutic applications of megainins and other antimicrobial agents of animal origin. Ciba Foundation Symposium 186: 3.
98. DeGray G, Smith F, Sanford J, Daniell H (2000). Hyper-expression of an antimicrobial peptide via the chloroplast genome to confer resistance against phytopathogenic bacteria. In review.
99. Lebens M, Holmgren J (1994). Mucosal vaccines based on the use of Cholera Toxin B subunit as immunogen and antigen carrier. Recombinant Vectors in Vaccine Development [Brown F (ed)]. 82:215-227.
100. Mor TS, Palmer KE, et al. (1998). Perspective: edible vaccines- a concept coming of age. Trends in Microbiology 6: 449-453.
101. Lipscombe M, Charles IG, Roberts M, Dougan G, Tite J, Fairweather NF (1991). Intranasal immunization using the B subunit of the Escherichia coli heat-labile toxin fused to an epitope of the Bordetella pertussis P.69 antigen. Mol. Microbiol. 5(6): 1385-1392.
102. Dertzbaugh MT, Elson CO (1993). Comparitive effectiveness of the cholera toxin B subunit and alkaline phosphatase as carriers for oral vaccines. Infect. Immun. 61: 48-55.
103. Holmgren J, Lycke N, Czerkinsky C (1993). Cholera toxin and cholera B subunit as oral-mucosal adjuvant and antigen vector systems. Vaccine. 11(12): 1179-84. Review.
104. Nashar TO, Amin T, Marcello A, Hirst TR (1993). Current progress in the development of the B subunits of cholera toxin and Escherichia coli heat-labile enterotoxin as carriers for the oral delivery of heterologous antigens and epitopes. Vaccine. 11(2): 235-40.
105. Sun JB, Holmgren J, Czerkinsky C (1994). Cholera toxin B subunit: an efficient transmucosal carrier-delivery system for induction of peripheral immunological tolerance. Proc. Natl. Acad. Sci. USA 91: 10795-10799.
106a.Hotz P, Guggenheim B, Schmid R (1972). Carbohydrates in pooled dental plaque. Caries Res. 6(2): 103-21.
106b.Ma J, Hitmak B, Wycoff K, Vine N, Charlegue D, Yu L1, Hein M, Lehner T. (1998). Characterization of a recombinant plant monoclonal secretory antibody and preventive immunotherapy in humans. Nature Medicine. 4(5): 601-606.
107. Twell D, y Ooms G (1987). The 5' flanking DNA of a patatin gene directs tuber specific expression of a chimaeric gene in potato. Plant Mol. Biol. 9: 365-375.
108. Sánchez-Serrano JJ, Schmidt R, Schell J, y Willmitzer L (1986). Nucleotide sequence of proteinase inhibitor II encoding cDNA of potato (Solanum tuberosum) and its mode of expression. Mol. Gen. Genet. 203: 15-20.
109. Brixey J, Guda C, Daniell H (1997). The chloroplast psbA promoter is more efficient in E. coli e T7 promoter for hyper expression of a foreign protein. Biotechnology Letters 19: 395-400.
110. Daniell H (1997). Transformation and foreign gene expression in plants mediated by microprojectile bombardment. Meth. Mol. Biol. 62: 453-488.
111. Berry-Lowe S and Schmidt G (1991). In The Molecular Biology of Plastids. Bogorad & Vasil Eds., Academic Press 10: 257-302.
112. Keegstra K and Cline K (1999) Protein Import and Routing Systems of Chloroplasts. The Plant Cell 11: 557-570.
113. Gregory WS and Mishkind ML (1986). The transport of proteins into chloroplasts. Ann. Rev. Biochem. 55: 879-912.
114. Keegstra K (1989). Transport and routing of proteins into chloroplasts. Cell 56: 247-253. Intranasal immunization using the B subunit of the Escherichia coli heat-labile toxin fused to an epitope of the Bordetella pertussis P.69 antigen. Mol. Microbiol. 5(6):1385-1392.
115. Smeekens S, Weisbeck P, Robinson C (1990). Protein transport into and within chloroplasts. Trends Biochem. Sci. 15(2): 73-6.
116. Rangasamy D, Ratledge C, Woolston C. (1997). Plastid targeting and transient expression of rat liver ATP: citrate lyase in pea protoplasts. Plant Cell Reports 16: 700-704.
117. Rangasamy D and Ratledge C. (2000). Genetic enhancement of Fatty acid synthesis by targeting Rat Liver ATP:Cytrate Lyase into plastids of tobacco. Plant Physiology 122:1231-1238.
118. Uhlen M, Nilsson B, Guss B, Lindberg M, Gatenbeck S, Philipson L (1983). Gene fusion vectors based on the gene for staphylococcal protein A. Gene. 23(3): 369-378.
119. Uhlen M, Forsberg G, Moks T, Hartmanis M, Nilsson B (1992). Fusion proteins in biotechnology. Curr. Opin. Biotechnol. 3(4): 363-369.
120. Nygren PA, Stahl S, Uhlen M (1994). Engineering proteins to facilitate bioprocessing. Trends in Biotechnol. 12(5): 184-8.
121. M Wang, WA Scott, KR Rao, J Udey, GE Conner, and Brew K (1989). Recombinant bovine alpha-lactalbumin obtained by limited proteolysis of a fusion protein expressed at high levels in Escherichia coli. J. Biol. Chem. 264: 21116-21121.
122. Gonzales T, Robert-Baudouy J (1996). Bacterial aminopeptidases: properties and functions. FEMS Microbiol. Rev. 18(4): 319-44.
123. Cohen A, Mayfield (1997). Translational regulation of gene expression in plants. Current Opinion in Biotechnology 8:189-194.
124. Lee SB, Kwon H, Kwon S, Park S, Jeong M, Han S, Daniell H, Byun H (2000). Drought ice conferred by the yeast trebalose-6 phosphate synthase gene engineered via the chloroplast genome. In review.
125a. Eibl C, Zou Z, Beck A, Kim M, Mullet J, Koop UH (1999). In vivo analysis of plastid psbA, rbcL and rpl32 UTR elements by chloroplast transformation: tobacco plastid gene expression is controlled by modulation of transcript levels and translation efficiency. The Plant Journal 19: 333-345.
125b. Morton B. (1993). Chloroplast DNA Codon Use:Evidence for Selection at the psbALocus Based on tRNA Availability. J Mol Evol 37: 273-280.
126. Morton B and Bernadette G. (2000). Codon usage in plastid genes is correlated with context, position within the gene, and amino acid content. J Mol Evol. 50: 184-193.
127. Prodromou C and Pearl LH (1992). Recursive PCR: a novel technique for total gene synthesis. Protein Engineering 5(8): 827-829.
128. Casimiro DR, Wright PE and Dyson HJ. (1997). PCR-based gene synthesis and protein NMR Spectroscopy. Structure 5 (11): 1407-1412.
129. Edwards K, Johnstone C, Thompson C (1991). A simple and rapid method for preparation of plant genomic DNA for PCR analysis. Nucleic Acids Res. 19: 1349.
130.Sambrook J, Fritch EF, Maniatis T (1989) Molecular cloning. Cold Spring Harbor Press, Cold Spring Harbor, New York.
131. Samuelsson E, Jonasson P, Viklund F, Nilsson B, Uhlen M. (1996). Affinity-assisted in vivo folding of a secreted human peptide hormone in Escherichia coli. Nat. Biotechnol. 14(6): 751-5.
132. Joly JC, Leung WS, Swartz JR (1998). Overexpression of Escherichia coli oxidoreductases increases recombinant insulin-like growth factor-I accumulation. Proc. Natl. Acad. Sci. USA 95(6):2773-7.
133. Nilsson B, Moks T, Jansson B, Abrahmsen L, Elmblad A, HolmgrenE, Henrichson C, Jones TA, Uhlen M (1987). A synthetic IgG-binding domain based on staphylococcal protein AProtein Eng. 1(2): 107-13.
134. Nygren PA, Eliasson M, Abrahmsen L, Uhlen M, Palmcrantz E (1988). Analysis and use of the serum albumin binding domains of streptococcal protein G. J. Mol. Recognit. 1(2): 69-74.
135. Federici M (1994). The quality control of biotechnology products. Biologicals 22(2): 151-9.
136. Ahmed N, Furth AJ (1991). A microassay for protein glycation based on the periodate method. Anal Biochem 192(1): 109-11.
-Santoro H, and Mukku Venkat AC (1998). A cell based potency assay for insulin like growth factor-L Biologicals 26: 61.
138. Clemens MJ, Morris AG, Gearing AJ, H eds. (1987). Lymphokines and Interferons- 0 practical approach. IRL Press.
139. Shani M, Barash I, Nathan M, Ricca G, Searfoss GH, Dekel I, Faerman A, Givol D, Hurwitz DR. (1992). Expression of human serum albumin in the milk of transgenic mice.Transgenic Res. Sep; 1(5):195-208.
140. Gines P, Arroyo V, Vargas V, Planas R, Casafont F, Panes J, Hoyos M, Viladomiu L, Rimola A, Morillas R, et al. (1991). Paracentesis with intravenous infusion of albumin as compared with peritoneovenous shunting in cirrhosis with refractory ascites. N Engl J Med. 19;325(12):829-35.
141. Bilbao R, Gerolami R, Bralet MP, Qian C, Tran PL, Tennant B, Prieto J, Brechot C. (2000). Transduction efficacy, antitumoral effect, and toxicity of adenovirus-mediated herpes simplex virus thymidine kinase/ ganciclovir therapy of hepatocellular carcinoma: the woodchuck animal model. Cancer Gene Ther. 7(5):657-62.

### a. SPECIFIC AIMS

Research on human proteins in the past years has revolutionized the use of these therapeutically valuable proteins in a variety of clinical situations. Since the demand for these proteins is expected to increase considerably in the coming years, it would be wise to ensure that in the future they will be available in significantly larger amounts, preferably on a cost-effective basis. Because most genes can be expressed in many different systems, it is essential to determine which system offers the most advantages for the manufacture of the recombinant protein. The ideal expression system would be one that produces a maximum amount of safe, biologically active material at a minimum cost. The use of modified mammalian cells with recombinant DNA techniques has the advantage of resulting in products, which are closely related to those of natural origin; however, culturing of these cells is intricate and can only be carried out on limited scale. The use of microorganisms such as bacteria permits manufacture on a larger scale, but introduces the disadvantage of producing products, which differ appreciably from the products of natural origin. For example, proteins that are usually glycosylated in humans are not glycosylated by bacteria. Furthermore, human proteins that are expressed at high levels in *E. coli* frequently acquire an unnatural conformation, accompanied by intracellular precipitation due to lack of proper folding and disulfide bridges. Production of recombinant proteins in plants has many potential advantages for generating biopharmaceuticals relevant to clinical medicine. These include the following: (I) plant systems are more economical than industrial facilities using fermentation systems; (ii) technology is available for harvesting and processing plants/ plant products on a large scale; (iii) elimination of the purification requirement when the plant tissue containing the recombinant protein is used as a food (edible vaccines); (iv) plants can be directed to target proteins into stable, intracellular compartments as chloroplasts, or expressed directly in chloroplasts; (v) the amount of recombinant product that can be produced approaches industrial-scale levels; and (vi) health risks due to contamination with potential human pathogens/toxins are minimized.

It has been estimated that one tobacco plant should be able to produce more recombinant protein than a 300-liter fermenter of *E. coli* (Crop Tech, VA). In addition, a tobacco plant produces a million seeds, facilitating large-scale production. Tobacco is also an ideal choice because of its relative ease of genetic manipulation and an impending need to explore alternate uses for this hazardous crop. However, with the exception of enzymes (e.g. phytase), levels of foreign proteins produced in nuclear transgenic plants are generally low, mostly less than 1% of the total soluble protein (Kusnadi et al. 1997). May et al. (1996) discuss this problem using the following examples. Although plant derived recombinant hepatitis B surface antigen was as commercial recombinant vaccine, the levels of expression in transgenic tobacco were low (0.0066% of total soluble protein). Even though Norwalk virus capsid protein expressed in potatoes caused oral immunization when consumed as food (edible vaccine), expression levels were low (0.3% of total soluble protein). In particular, expression of human proteins in nuclear transgenic plants has been disappointingly low: e.g. human Interferon-β 0.000017% of fresh weight, human serum albumin 0.02% and erythropoietin 0.0026% of total soluble protein (see table1 in Kusnadi et al. 1997). A synthetic gene coding for the human epidermal growth factor was expressed only up to 0.001% of total soluble protein in transgenic tobacco (May et al. 1996). The cost of producing recombinant proteins in alfalfa leaves was estimated to be 12-fold lower than in potato tubers and comparable with seeds (Kusnadi et al. 1997). However, tobacco leaves are much larger and have much higher biomass than alfalfa. Planet Biotechnology has recently estimated that at 50 mg/liter of mammalian cell culture or transgenic goat's milk or 50mg/kg of tobacco leaf expression, the cost of purified IgA will be $10,000, 1000 and 50/g, respectively (Daniell et al. 2000). The cost of production of recombinant proteins will be 50-fold lower than that of *E*.*coli* fermentation (with 20% expression levels in E.coli (Kusnadi et al. 1997). A decrease in insulin expression from 20% to 5% of biomass doubled the cost of production in *E. coli* (Petridis et al. 1995). Expression level less than 1% of total soluble protein in plants has been found to be not commercially feasible (Kusnadi et al. 1997). Therefore, it is important to increase levels of expression of recombinant proteins in plants in order to exploit plant production of pharmacologically important proteins.

An alternate approach is to express foreign proteins in chloroplasts of higher plants. We have recently integrated foreign genes (up to 10,000 copies per cell) into the tobacco chloroplast genome resulting in accumulation of recombinant proteins up to 46% of the total soluble protein (De Cosa et al. 2001). Chloroplast transformation utilizes two flanking sequences that, through homologous recombination, insert foreign DNA into the spacer region between the functional genes of the chloroplast genome, thus targeting the foreign genes to a precise location. This eliminates the "position effect" and gene silencing frequently observed in nuclear transgenic plants. Chloroplast genetic engineering is an environmentally friendly approach, minimizing concerns of out-cross of introduced traits via pollen to weeds or other crops (Bock and Hagemann 2000, Heifetz 2000). Also, the concerns of insects developing resistance to biopesticides are minimized by hyper-expression of single insecticidal proteins (high dosage) or expression of different types of insecticides in a single transformation event (gene pyramiding). Concerns of insecticidal proteins on non-target insects are minimized by lack of expression in transgenic pollen (De Cosa et al. 2001).

Most importantly, a significant advantage in the production of pharmaceutical proteins in chloroplasts is their ability to process eukaryotic proteins, including folding and formation of disulfide bridges (Drescher et al. 1998). Chaperonin proteins are present in chloroplasts (Roy, 1989; Vierling, 1991) that function in folding and assembly of prokaryotic/eukaryotic proteins. Also, proteins are activated by disulfide bond oxido/reduction cycles using the chloroplast thioredoxin system (Reulland and Miginiac-Maslow, 1999) or chloroplast protein disulfide isomerase (Kim and Mayfield, 1997). Accumulation of fully assembled, disulfide bonded form of human somatotropin via chloroplast transformation (Staub et al. 2000), oligomeric form of CTB (Henriques and Daniell, 2000) and the assembly of heavy/light chains of humanized Guy's 13 antibody in transgenic chloroplasts (Panchal et al. 2000) provide strong evidence for successful processing of pharmaceutical proteins inside chloroplasts. Such folding and assembly should eliminate the need for highly expensive *in vitro* processing of pharmaceutical proteins. For example, 60% of the total operating cost in the production of human insulin is associated with *in vitro* processing (formation of disulfide bridges and cleavage of methionine, Petridis et al. 1995).

Another major cost of insulin production is purification; chromatography accounts for 30% of operating expenses and 70% of equipment in production of insulin (Petridis et al. 1995). Therefore, new approaches are necessary to minimize or eliminate chromatography in insulin production. One such approach is the use of GVGVP as a fusion protein to facilitate single step purification without the use of chromatography. GVGVP is a Protein Based Polymer (PBP) made from synthetic genes; at lower temperatures this polymer exists as more extended molecules. Upon raising the temperature above the transition range, polymer hydrophobically folds into dynamic structures called β-spirals that further aggregate by hydrophobic association to form twisted filaments (Urry, 1991; Urry et al., 1994). Inverse temperature transition offers several advantages. It facilitates scale up of purification from grams to kilograms. Milder purification condition requires only a modest change in temperature and ionic strength. This should also facilitate higher recovery, faster purification and high volume processing; protein purification is generally the slow step (bottleneck) in pharmaceutical product development. Through exploitation of this reversible inverse temperature transition property, simple and inexpensive extraction and purification may be performed. The temperature at which the aggregation takes place can be manipulated by engineering biopolymers containing varying numbers of repeats and changing salt concentration in solution (McPherson et al., 1996). Chloroplast mediated expression of insulin-polymer fusion protein should eliminate the need for fermentation process as well as reagents needed for recombinant protein purification and downstream processing.

Oral delivery of insulin is yet another powerful approach that would eliminate 97% of the production cost of insulin (Petridis et al. 1995). For example, Sun et al. (1994) have shown that feeding a small dose of antigens conjugated to the receptor binding non-toxic B subunit moiety of the cholera toxin (CTB) suppressed systemic T cell-mediated inflammatory reactions in animals. Oral administration of a myelin antigen conjugated to CTB has been shown to protect animals against encephalomyelitis, even when given after disease induction (Sun et al. 1996). Bergerot et al. (1997) reported that feeding small amounts of human insulin conjugated to CTB suppressed beta cell destruction and clinical diabetes in adult non-obese diabetic (NOD) mice. The protective effect could be transferred by T cells from CTB-insulin treated animals and was associated with reduced insulitis. These results demonstrate that protection against autoimmune diabetes can indeed be achieved by feeding small amounts of a pancreas islet cell auto antigen linked to CTB (Bergerot et al. 1997). Conjugation with CTB facilitates antigen delivery and presentation to the Gut Associated Lymphoid Tissues (GALT) due to its affinity for the cell surface receptor GM₁-ganglioside located on GALT cells, for increased uptake and immunologic recognition (Arakawa et al. 1998). Transgenic potato tubers expressed up to 0.1% CTB-insulin fusion protein of total soluble protein, which retained GM₁-ganglioside binding affinity and native autogenicity for both CTB and insulin. NOD mice fed with transgenic potato tubers containing microgram quantities of CTB-insulin fusion protein showed a substantial reduction in insulitis and a delay in the progression of diabetes (Arkawa et al. 1998). However, for commercial exploitation, the levels of expression should be increased in transgenic plants. Therefore, we propose here expression of CTB-insulin fusion in transgenic chloroplasts of nicotine free edible tobacco to increase levels of expression adequate for animal testing.

Taken together, low levels of expression of human proteins in nuclear transgenic plants, and difficulty in folding, assembly/processing of human proteins in *E.coli* should make chloroplasts an alternate compartment for expression of these proteins; production of human proteins in transgenic chloroplasts should also dramatically lower the production cost. Large-scale production of insulin in tobacco in conjunction with an oral delivery system should be a powerful approach to provide treatment to diabetes patients at an affordable cost and provide tobacco farmers alternate uses for this hazardous crop. Therefore, the first objective of this project is to use poly(GVGVP) as a fusion protein to enable hyper-expression of insulin and accomplish rapid one step purification of the fusion peptide utilizing the inverse temperature erties of this polymer. The second objective is to develop insulin-CTB fusion protein for oral delivery in nicotine free edible tobacco (LAMD 605).
Both objectives will be accomplished as follows:
a) Develop recombinant DNA vectors for enhanced expression of Proinsulin as fusion proteins with GVGVP or CTB via chloroplast genomes of tobacco
b) Obtain transgenic tobacco (Petit Havana & LAMD 605) plants
c) Characterize transgenic expression of proinsulin polymer or CTB fusion proteins using molecular and Biochemical methods in chloroplasts
d) Employ existing or modified methods of polymer purification from transgenic leaves
e) Analyze Mendelian or maternal inheritance of transgenic plants
f) Large scale purification of insulin and comparison of current insulin purification methods with polymer-based purification method in *E.coli* and tobacco
g) Compare natural refolding in chloroplasts with *in vitro* processing
h) Characterization (yield and purity) of proinsulin produced in *E.coli* and transgenic tobacco
i) Assessment of diabetic symptoms in mice fed with edible tobacco expressing CTB-insulin fusion protein.

### b. BACKGROUND AND SIGNIFICANCE

**Diabetes and Insulin:** The most obvious action of insulin is to lower blood glucose (Oakly et al. 1973). This is a result of its immediate effect in increasing glucose uptake in tissues. In muscle, under the action of insulin, glucose is more readily taken up and either converted to glycogen and lactic acid or oxidized to carbon dioxide. Insulin also affects a number of important enzymes concerned with cellular metabolism. It increases the activity of glucokinase, which phosphorylates glucose thereby increasing the rate of glucose metabolism in the liver. Insulin also suppresses gluconeogenesis by depressing the function of liver enzymes, which operate the reverse pathway from proteins to glucose. Lack of insulin can restrict the transport of glucose into muscle and adipose tissue. This results in increases in blood glucose levels (hyperglycemia). In addition, the breakdown of natural fat to free fatty acids and glycerol is increased and there is a rise in the fatty acid content in the blood. Increased catabolism of fatty acids by the liver results in greater production of ketone bodies. They diffuse from the liver and pass to the muscles for further oxidation. Soon, ketone body production rate exceeds oxidation rate and ketosis results. Less amino acids are taken up by the tissues and protein degradation results. At the same time gluconeogenesis is stimulated and protein is used to produce glucose. Obviously, lack of insulin has serious consequences.

Diabetes is classified into types I and II. Type I is also known as insulin dependent diabetes mellitus (IDDM). Usually this is caused by a cell-mediated autoimmune destruction of the pancreatic β-cells (Davidson, 1998). Those suffering from this type are dependent on external sources of insulin. Type II is known as noninsulin-dependent diabetes mellitus (NIDDM). This usually involves resistance to insulin in combination with its underproduction. These prominent diseases have led to extensive research into microbial production of recombinant human insulin (rHI).
**Expression of Recombinant Human Insulin in *E.coli*:** In 1978, two thousand kilograms of insulin were used in the world each year, half of this was used in the United States (Steiner et al., 1978). At that time, the number of diabetics in the US were increasing 6% every year (Gunby, 1978). In 1997-98, 10% increase in sales of diabetes care products and 19% increase in insulin products have been reported by Novo Nordisk (world's leading supplier of insulin), making it a 7.8 billion dollar industry. Annually, 160,000 Americans are killed by diabetes, making it the fourth leading cause of death. Many methods of production of rHI have been developed. Insulin genes were first chemically synthesized for expression in *Esherichia coli* (Crea et al., 1978). These genes encoded separate insulin A and B chains. The genes were each expressed in *E. coli* as fusion proteins with the β-galactosidase (Goeddel et al., 1979). The first documented production of rHI using this system was reported by David Goeddel from Genentech (Hall, 1988). The genes were fused to the Trp synthase gene, which resulted is increased insulin yield, due to the smaller fusion peptide. This fusion protein was approved for commercial production by Eli Lilly in 1982 (Chance and Frank, 1993) with a product name of Humulin. As of 1986, Humulin was produced from proinsulin genes. Proinsulin contains both insulin chains and the C-peptide that connects them. Normal *in vitro* post-translational processing of proinsulin includes use of trypsin and carboxypeptidase B for maturation to insulin. Other data concerning commercial production of Humulin and other insulin products is now considered proprietary information and is not available to the public.
**Protein Based Polymers (PBP):** The synthetic gene that codes for a bioelastic PBP was designed after repeated amino acid sequences GVGVP, observed in all sequenced mammalian elastin proteins (Yeh et al. 1987). Elastin is one of the strongest known natural fibers and is present in skin, ligaments, and arterial walls. Bioelastic PBPs containing multiple repeats of this pentamer have remarkable elastic properties, enabling several medical and non-medical applications (Urry et al. 1993, Urry 1995, Daniell 1995). GVGVP polymers prevent adhesions ery, aid in reconstructing tissues and delivering drugs to the body over an extended period of time. North American Science Associates, Inc. reported that GVGVP polymer is non-toxic in mice, non-sensitizing and non-antigenic in guinea pigs, and non-pyrogenic in rabbits (Urry et al. 1993). Researchers have also observed that inserting sheets of GVGVP at the sites of contaminated wounds in rats reduces the number of adhesions that form as the wounds heal (Urry et al. 1993). In a similar manner, using the GVGVP to encase muscles that are cut during eye surgery in rabbits prevents scarring following the operation (Urry et al. 1993, Urry 1995). Other medical applications of bioelastic PBPs include tissue reconstruction (synthetic ligaments and arteries, bones), wound coverings, artificial pericardia, catheters and programmed drug delivery (Urry, 1995; Urry et al., 1993, 1996).

We have expressed the elastic PBP (GVGVP)₁₂₁ in *E*. *coli* (Guda et al. al 1995, Brixey et al. 1997), in the fungus *Aspergillus nidulans* (Herzog et al. 1997), in cultured tobacco cells (Zhang et al. 1995), and in transgenic tobacco plants (Zhang et al. 1996). In particular, (GVGVP)₁₂₁ has been expressed to such high levels in *E. coli* that polymer inclusion bodies occupied up to about 90% of the cell volume; also, inclusion bodies have been observed in chloroplasts of transgenic tobacco plants (see attached article, Daniell and Guda, 1997). Recently, we reported stable transformation of the tobacco chloroplasts by integration and expression the biopolymer gene (EG121), into the Large Single Copy region (5,000 copies per cell) or the Inverted Repeat region (10, 000 copies per cell) of the chloroplast genome (Guda et al., 2000).
**PBP as Fusion Proteins:** Several systems are now available to simplify protein purification including the maltose binding protein (Manna et al. 1988), glutothione S-transferase (Smith and Johnson, 1988), biotinylated (Tsao et al. 1996), thioredoxin (Smith et al. 1998) and cellulose binding (Ong et al. 1989) proteins. In order to effectively utilize aforementioned fusion proteins in the purification process, recombinant DNA vectors for fusion with short peptides are now available (Smith et al. 1988; Kim and Raines, 1993; Su et al. 1992). Recombinant proteins are generally purified by affinity chromatography, using ligands specific to carrier proteins (Nilsson et al. 1997). While these are useful techniques for laboratory scale purification, affinity chromatography for large-scale purification is time consuming and cost prohibitive. Therefore, economical and non-chromatographic techniques are highly desirable. In addition, a common solution to N-terminal degradation of small peptides is to fuse foreign peptides to endogenous *E*. *coli* proteins. Early in the development of this technique, β-galactosidase (β-gal) was used as a fusion protein (Goldberg and Goff, 1986). A drawback of this method was that the β-gal protein high molecular weight (MW 100,000). Therefore, the proportion of the peptide product in the total protein is low. Another problem associated with the large β-gal fusion is early termination of translation (Burnette, 1983; Hall, 1988). This occurred when β-gal was used to produce human insulin peptides because the fusion was detached from the ribosome during translation thus yielding incomplete peptides. In order to increase the peptide production, other proteins of lower molecular weight proteins have been used as fusion proteins. For example, better yields were obtained with the tryptophan synthase (190aa) fusion proteins (Hall, 1988, Bumett, 1983).

One of the primary goals of this study is to use poly(GVGVP) as a fusion protein to enable hyper-expression of insulin and accomplish rapid one step purification of the fusion peptide. At lower temperatures the polymers exist as more extended molecules which, on raising the temperature above the transition range hydrophobically fold into dynamic structures called β-spirals that further aggregate by hydrophobic association to form twisted filaments (Urry, 1991). Through exploitation of this reversible property, simple and inexpensive extraction and purification is performed. The temperature at which aggregation takes place (Tₜ) can be manipulated by engineering biopolymers containing varying numbers of repeats or changing salt concentration (McPherson et al., 1996). Another group has recently demonstrated purification of recombinant proteins by fusion with thermally responsive polypeptides (Meyer and Chilkoti, 1999). Polymers of different sizes have been synthesized and expressed in *E.coli* in the PI's laboratory. This approach would also eliminate the need for expensive reagents, equipment and time required for purification.
**Cholera Toxin β subunit as a fusion protein:** *Vibrio cholerae* causes diarrhea by colonizing the small intestine and producing enterotoxins, of which the cholera toxin (CT) is considered the main cause of toxicity. CT is a hexameric AB₅ protein having one 27KDa A subunit which has toxic ADP-ribosyl transferase activity and a non-toxic pentamer of 11.6 kDa B subunits that are non-covalently linked into a very stable doughnut like structure into which the toxic active (A) subunit is inserted. The A subunit of CT consists of two fragments - A1 and A2 which are linked by a disulfide bond. The enzymatic activity of CT is located solely on the A1 fragment (Gill, 1976). The A2 fragment of the A subunit links the A1 fragment and the B pentamer. CT binds via specific interactions of the B subunit pentamer with GM1 ganglioside, the membrane receptor, present on the intestinal epithelial cell surface of the host The A subunit is then translocated into the cell where it ADP-ribosylatcs the Gs subunit of adenylate cyclase bringing about the increased levels of cyclic AMP in affected cells that is associated with the electrolyte of clinical cholera (Lebens et al. 1994). For optimal enzymatic activity, the A1 fragment needs to be separated from the A2 fragment by proteolytic cleavage of the main chain and by reduction of the disulfide bond linking them (Mekalanos et aL 1979).

The Expression and assembly of CTB in transgenic potato tubers has been reported (Arakawa et al.1997). The CTB gene including the leader peptide was fused to an endoplasmic reticulum retention signal (SEKDEL) at the 3' end to sequester the CTB protein within the lumen of the ER. The DNA fragment encoding the 21-amino acid leader peptide of the CTB protein was retained in order to direct the newly synthesized CTB protein into the lumen of the ER. Immunoblot analysis indicated that the plant derived CTB protein was antigenically indistinguishable from the bacterial CTB protein and that oligomeric CTB molecules (Mr ~ 50 kDa) were the dominant molecular species isolated from transgenic potato leaf and tuber tissues. Similar to bacterial CTB, plant derived CTB dissociated into monomers (Mr~15 kDa) during heat/acid treatment.

Enzyme linked immunosorbent assay methods indicated that plant synthesized CTB protein bound specifically to GM1 gangliosides, the natural membrane receptors of Cholera Toxin. The maximum amount of CTB protein detected in auxin induced transgenic potato leaf and tuber tissues was approximately 0.3% of the total soluble protein. The oral immunization of CD-1 mice with transgenic potato tissues transformed with the CTB gene (administered at weekly intervals for a month with a final booster feeding on day 65) has also been reported. The levels of serum and mucosal anti-cholera toxin antibodies in mice were found to generate protective immunity against the cytopathic effects of CT holotoxin. Following intraileal injection with CT, the plant immunized mice showed up to a 60% reduction in diarrheal fluid accumulation in the small intestine. Systemic and mucosal CTB- specific antibody titers were determined in both serum and feces collected from immunized mice by the class-specific chemiluminescent ELISA method and the endpoint titers for the three antibody isotypes ( IgM,IgG and IgA) were determined. The extent of CT neutralization in both Vero cell and ileal loop experiments suggested that anti-CTB antibodies prevent CT binding to cellular GMI-gangliosides. Also, mice fed with 3 g of transgenic potato exhibited similar intestinal protection as mice gavaged with 30□g of bacterial CTB. Recombinant LTB [rLTB] (the heat labile enterotoxin produced by Enterotoxigenic *E.coli*) which is structurally, functionally and immunologically similar to CTB was expressed in transgenic tobacco (Arntzen et al. 1998; Haq et al. 1995). They have reported that, the rLTB retained its antigenicity as shown by immunoprecipitation of rLTB with antibodies raised to rLTB from *E.coli*. The rLTB protein was molecular weight and aggregated to form the pentamer as confirmed by gel permeation chromatography.
**Delivery of Human Insulin:** Insulin has been delivered intravenously in the past several years. However, more recently, alternate methods such as nasal spray, are also available. Oral delivery of insulin is yet another new approach (Mathiowitz et al., 1997). Engineered polymer microspheres made of biologically erodable polymers, which display strong interactions with gastrointestinal mucus and cellular linings, can traverse both mucosal absorptive epithelium and the follicle-associated epithelium, covering the lymphoid tissue of Peyer's patches. Polymers maintain contact with intestinal epithelium for extended periods of time and actually penetrate through and between cells. Animals fed with the poly(FA: PLGA)-encapsulated insulin preparation were able to regulate the glucose load better than controls, confirming that insulin crossed the intestinal barrier and was released from the microspheres in a biologically active form (Mathiowitz et al., 1997).

Besides, CTB has also been demonstrated to be an effective carrier molecule for the induction of mucosal immunity to polypeptides to which it is chemically or genetically conjugated (McKenzie et al. 1984; Dertzbaugh et al. 1993) The production of immunomodulatory transmucosal carrier molecules, such as CTB, in plants may greatly improve the efficacy of edible plant vaccines (Haq et al. 1995; Thanavala et al. 1995; Mason et al. 1996) and may also provide novel oral tolerance agents for prevention of such autoimmune diseases as Type I diabetes (Zhang et al. 1991), Rheumatoid arthritis (Trentham et al. 1993),multiple sclerosis( Khoury et al. 1990; Miller et al. 1992; Weiner et al. 1993) as well as the prevention of allergic and allograft rejection reactions (Sayegh et al. 1992; Hancock et al. 1993). Therefore, expressing a CTB-proinsulin fusion would be an ideal approach for oral delivery of insulin.
Chloroplast Genetic Engineering: When we developed the concept of chloroplast genetic engineering (Daniell and McFadden, 1988 U.S. Patents; Daniell, World Patent, 1999), it was possible to introduce isolated intact chloroplasts into protoplasts and regenerate transgenic plants (Carlson, 1973). Therefore, early investigations on chloroplast transformation focused on the development of *in organello* systems using intact chloroplasts capable of efficient and prolonged transcription and translation (Daniell and Rebeiz, 1982; Daniell et al., 1983, 1986) and expression of foreign *genes* in isolated chloroplasts (Daniell and McFadden, 1987). However, after the discovery of the gene gun as a transformation device (Daniell, 1993), it was possible to transform plant chloroplasts without the use of isolated plastids and protoplasts. Chloroplast ering was accomplished in several phases. Transient expression of foreign genes in plastids of dicots (Daniell et al., 1990; Ye et al., 1990) was followed by such studies in monocots (Daniell et al., 1991). Unique to the chloroplast genetic engineering is the development of a foreign gene expression system using autonomously replicating chloroplast expression vectors (Daniell et al., 1990). Stable integration of a selectable marker gene into the tobacco chloroplast genome (Svab and Maliga, 1993) was also accomplished using the gene gun. However, useful genes conferring valuable traits via chloroplast genetic engineering have been demonstrated only recently. For example, plants resistant to B.t. sensitive insects were obtained by integrating the *cryIAc gene* into the tobacco chloroplast genome (McBride et al., 1995). Plants resistant to B.t. resistant insects (up to 40,000 fold) were obtained by hyper-expression of the *cryllA* gene within the tobacco chloroplast genome (Kota et al., 1999). Plants have also been genetically engineered via the chloroplast genome to confer herbicide resistance and the introduced foreign genes were maternally inherited, overcoming the problem of out-cross with weeds (Daniell et al., 1998). Chloroplast genetic engineering has also been used to produce pharmaceutical products that are not used by plants (Staub et al. 2000, Guda et al. 2000). Chloroplast genetic engineering technology is currently being applied to other useful crops (Sidorov et al. 1999; Daniell, 1999).

### c. PRELIMINARY STUDIES

A remarkable feature of chloroplast genetic engineering is the observation of exceptionally large accumulation of foreign proteins in transgenic plants, as much as 46% of CRY protein in total soluble protein, even in bleached old leaves (DeCosa et aL 2001). Stable expression of a pharmaceutical protein in chloroplasts was first reported for GVGVP, a protein based polymer with varied medical applications (such as the prevention of post-surgical adhesions and scars, wound coverings, artificial pericardia, tissue reconstruction and programmed drug delivery (Guda et al. 2000). Subsequently, expression of the human somatotropin via the tobacco chloroplast genome (Staub et al. 2000) to high levels (7% of total soluble protein) was observed. The following investigations that are in progress in the Daniell lab illustrate the power of this technology to express small peptides, entire operons, vaccines that require oligomeric proteins with stable disulfide bridges and monoclonals that require assembly of heavy/light chains via chaperonins. In order for edible insulin approach to be successful, it is essential to develop a selection system free of antibiotic resistant genes. One such marker free chloroplast transformation system has been accomplished in this laboratory (Daniell et al. 2000). Experiments are in progress to develop chloroplast transformation of edible leaves (alfalfa and lettuce) for the practical applications of this approach.
**Engineering novel pathways via the chloroplast genome:** In plant and animal cells, nuclear mRNAs are translated monocistronically. This poses a serious problem when engineering multiple genes in plants (Bogorad, 2000). Therefore, in order to express the polyhydroxybutyrate polymer or Guy's 13 antibody, single genes were first introduced into individual transgenic plants, then these plants were back-crossed to reconstitute the entire pathway or the complete protein (Navrath et al. 1994; Ma et al. 1995). Similarly, in a seven year long effort, Ye et al. (2000) recently introduced a set of three genes for a short biosynthetic pathway that resulted in β-carotene expression in rice. In contrast, most chloroplast genes of higher plants are cotranscribed (Bogorad, 2000). Expression of polycistroms via the chloroplast genome provides a unique opportunity to express entire pathways in a single transformation event. We have recently used the *Bacillus thuringiensis* (Bt) cry2Aa2 operon as a model system to demonstrate operon expression and crystal formation via the chloroplast genome (De Cosa et al. 2001). *Cry*2Aa2 is the distal gene of a three-gene operon. The *orf* immediately upstream of cry2Aa2 codes for a putative chaperonin that facilitates the folding of *cry*2Aa2 (and other proteins) to from proteolytically stable cuboidal crystals (Ge et al.1998).

Therefore, the cry2Aa2 bacterial operon was expressed in tobacco chloroplasts to test the resultant transgenic plants for increased expression and improved persistence of the accumulated insecticidal protein(s). Stable foreign gene integration was confirmed by PCR and Southern blot analysis in T₀ and T₁ transgenic plants. *Cry2Aa2* operon derived protein accumulated at 45.3% of the total soluble protein in mature leaves and remained stable even in old bleached leaves (46.1%) (FIG. 4). This is the highest level of foreign gene expression ever reported in transgenic plants. Exceedingly uncontrollable insects (10-day old cotton bollworm, beetarmy worm) were killed 100% after consuming transgenic leaves. Electron micrographs showed the presence of the insecticidal protein folded into cuboidal crystals similar in shape to *Cry2Aa2* crystals observed in *Bacillus thuringiensis* (FIG. 5). In contrast to currently marketed transgenic plants with soluble CRY proteins, folded protoxin crystals will be processed only by target insects that have alkaline gut pH; this approach should improve safety of Bt transgenic plants. Absence of insecticidal proteins in transgenic pollen eliminates toxicity to non-target insects via pollen. In addition to these environmentally friendly approaches, this observation should serve as a model system for large-scale production of foreign proteins within chloroplasts in a folded configuration enhancing their stability and facilitating single step purification. This is the first demonstration of expression of a bacterial oporon in transgenic plants and opens the door to engineer novel pathways in plants in a single transformation event.
**Expressing small peptides via the chloroplast genome:** It is common knowledge that the medical community has been fighting a vigorous battle against drug resistant pathogenic bacteria for years. Cationic antibacterial peptides from mammals, amphibians and insects have gained more attention over the last decade (Hancock and Lehrer, 1998). Key features of these cationic peptides are a net positive change, an affinity for negatively-charged prokaryotic membrane phospholipids over neutral-charged eukaryotic membranes and the ability to form aggregates that disrupt the bacterial membrane (Biggin and Sansom, 1999).

There are three major peptides with α-helical structures, cecropin from *Hyalophora cecropia* (giant silk moth), magainins from *Xenopus laevis* (African frog) and defensins from mammalian neutrophils. Magainin and its analogues have been studied as a broad-spectrum topical agent, a systemic antibiotic; a wound-bealing stimulant; and an anticancer agent (Jacob and Zasloff, 1994). We have recently observed that a synthetic lytic peptide (MSI-99, 22 amino acids) can be successfully expressed in tobacco chloroplast (DeGray et al. 2000). The peptide retained its lytic activity against the phytopathogenic bacteria *Pseudomonas syringae* and multidrug resistant human pathogen, *Pseudomonas aeruginosa.* The anti-microbial peptide (AMP) used in this study was an amphipathic alpha-helix molecule that has an affinity for negatively charged phospholipids commonly found in the outer-membrane of bacteria. Upon contact with these membranes, individual peptides aggregate to form pores in the membane, resulting in bacterial lysis. Because of the concentration dependent action of the AMP, it was expressed via the chloroplast genome to accomplish high dose delivery at the point infection. PCR products and Southern blots confirmed chloroplast integration of the foreign genes and homoplasmy. Growth and development of the transgenic plants was unaffacted by hyper-expression of the AMP within chloroplasts. *In vitro* assays with T₀ and T₁ plants confirmed that the AMP was expressed at high levels (21.5 to 43% of the total soluble protein) and retained biological activity against *Pseudomonas syringae,* a major plant pathogen. *In situ* assays resulted in intense areas of necrosis around the point of infection in control leaves, While transformed leaves showed no signs of necrosis (200-800 µg of AMP at the site of infection)(FIG. 6). T₁ *in vitro* assays against *Pseudomonas aeruginosa* (a multi-drug resistant human pathogen) displayed a 96% inhibition of growth (FIG. 7). These results give a new option in the battle against phytopathogenic and drug-resistant human pathogenic bacteria. Small peptides (like insulin) are degraded in most organisms. However, stability of this AMP in chloroplasts opens up this compartment for expression of hormones and other small peptides.

**d. assembly of monoclonals in transgenic chloroplasts:** Dental caries (cavities) is probably the most present disease of humankind. Colonization of teeth by *S. mutans* is the single most important risk factor in the development of dental caries. *S*. *mutans* is a non-motile, gram positive coccus. It colonizes tooth surfaces and synthesizes glucans (insoluble polysaccharide) and fructans from sucrose using the enzymes glucosyltransferase and fructosyltransferase respectively (Hotz et al. 1972). The glucans play an important role by allowing the bacterium to adhere to the smooth tooth surfaces. After its adherence, the bacterium *ferments sucrose and produces* lactic acid. Lactic *acid dissolves the minerals* of the tooth, producing a cavity.

A topical monoclonal antibody therapy to prevent adherence of *S. mutans* to teeth has recently been developed The incidence of cariogenic bacteria (in humans and animals) and dental caries (in animals) was dramatically reduced for periods of up to two years after the cessation of the antibody therapy. No adverse events were detested either in the exposed animals or in human volunteers (Ma et al. 1998). The annual requirement for this antibody in the US alone may eventually exceed 1 metric ton. Therefore, this antibody was expressed via the chloroplast genome to achieve higher levels of expression and proper folding (Panchal et al. 2000). The integration of antibody genes into the chloroplast genome was confirmed by PCR and Southern blot analysis. The expression of both heavy and light chains was confirmed by western blot analysis under reducing conditions (FIG. 15A,B). The expression of fully assembled antibody was confirmed by western blot analysis under non-reducing conditions (FIG. 15C). This is the first report of successful assembly of a multi-subunit human protein in transgenic chloroplasts. Production of monoclonal antibodies at agricultural level should reduce their cost and create new applications of monoclonal antibodies.
**Marker free chloroplast transgenic plants** Most transformation techniques co-introduce a gene that confers antibiotic resistance, along with the gene of interest to impart a desired trait. Regenerating transformed cells in antibiotic containing growth media permits selection of only those cells that have incorporated the foreign genes. Once transgenic plants are regenerated, antibiotic resistance genes serve no useful purpose but they continue to produce their gene products. One among the primary concerns of genetically modified (GM) crops is the presence of clinically important antibiotic resistance gene products in transgenic plants that could inactivate oral doses of the antibiotic (reviewed by Puchta 2000; Daniell 1999A). Alternatively, the antibiotic resistant genes could be transferred to pathogenic microbes in the gastrointestinal tract or soil rendering them resistant to treatment with such antibiotics. Antibiotic resistant e of the major challenges of modem medicine. In Germany, GM crops containing antibiotic resistant genes have been banned from release (Pecrenboom

Chloroplast genetic engineering offers several advantages over nuclear transformation including high levels of gene expression and gene containment but utilizes thousands of copies of the most commonly used antibiotic resistance genes. Engineering genetically modified (GM) crops without the use of antibiotic resistance genes should eliminate Potential risk of their transfer to the environment or gut microbes. Therefore, betaine aldehyde dehydrogenase (BADH) gene from spinach is used in this study as a selectable marker (Daniell et al. 2000). The selection process involves conversion of toxic betaine aldehyde (BA) by the chloroplast BADH enzyme to nontoxic glycine betaine, which also serves as an osmoprotectant. Chloroplast transformation efficiency was 25 fold higher in BA selection than spectinomycin, in addiction to rapid regeneration (Table 1). Transgenic shoots appeared within 12 days in 80% of leaf discs (up to 23 shoots per disc) in BA selection compared to 45 days in 15% of discs (1 or 2 shoots per disc) on spectinomycin selection (FIG. 11). Southern blots confirm stable integration of foreign genes into all of the chloroplast genomes (-10,000 copies per cell) resulting in homoplasmy. Transgenic tobacco plants showed 1527-1816% higher BADH activity at different developmental stages than untrasformed controls. Transgenic plants were morphologically indistinguishable from untransformed plants and the introduced trait was stably inherited in the subsequent generation. This is the first report of genetic engineering of the chloroplast genome without the use of antibiotic selection. Use of genes that are naturally present in spinach for idition to gene containment, should ease public concerns or perception of GM crops. Also, this should be very helpful in the development of edible insulin.
**Expression of cholera toxin β subunit oligomers as a vaccine to chloroplasts:** CTB when administered orally (Lebens and Holmgren, 1994) is a potent mucosal immunogen, which can neutralize the toxicity of the CT holotoxin by preventing it from binding to the intestinal cells (Mor et al. 1998). This is believed to be a result of it binding to eukaryotic cell surfaces via the G_{M1} gangliosides, receptors present on the intestinal epithelial surface, thus eliciting a mucosal immune response to pathogens (Lipscombe et al. 1991) and enhancing the immune response when chemically coupled to other antigens (Dertzbaugh and Elson, 1993; Holmgren et al. 1993; Nashar et al. 1993; Sun et al. 1994).

Cholera toxin (CTB) has previously been expressed in nuclear transgenic plants at levels of 0.01 (leaves) to 03% (tubers) of the total soluble protein. To increase expression levels, we engineered the chloroplast genome to express the unmodified CTB gene (Henriques and Daniell, 2000). We observed expression of oligomeric CTB at levels of 4-5% of total soluble plant protein (FIG. 8A). PCR and Southern Blot analyses confirmed stable integration of the CTB gene into the chloroplast genome. Western blot analysis showed that transgenic chloroplast expressed CTB was antigenically identical to commercially available purified CTB antigen (FIG. 9). Also, G_{M1}-ganglioside binding assays confirm that chloroplast synthesized CTB binds to the intestinal membrane receptor of cholera toxin (FIG. 8B). Transgenic tobacco plants were morphologically indistinguishable from untransformed plants and the introduced gene was found to be stably inherited in the subsequent generation as confirmed by PCR and Southern Blot analyses. The increased production of an efficient transmucosal carrier molecule and delivery system, like CTB, in chloroplasts of plants makes plant based oral vaccines and fusion proteins with CTB needing oral administration, a much more feasible approach. These observations establish unequivocally that chloroplasts are capable of forming disulfide bridges to assemble foreign proteins, and ideal for expression of CTB fusion proteins.
**Polymer-proinsulin Recombinant DNA Vectors:** One possible insulin expression system involves independent expression of insulin chains A and B, as it has been produced in *E.coli* for commercial purposes in the past. The disadvantage of this method is that *E.coli* does not form disulfide bridges in the cell unless the protein is targeted to the periplasm. Expensive *in vitro* assembly after purification is necessary for this approach. Therefore, a better approach would be to express the human proinsulin as a polymer fusion protein. This method is ideal because e capable of forming disulfide bridges. Using a single gene, as opposed to the individual chains, would eliminate the necessity of conducting two parallel vector construction processes, as is required for the individual chains. In addition, the need for individual fermentations and purification procedures is eliminated by the single gene method. In addition, proinsulin requires less processing following extraction.

Recently, the human pre-proinsulin gene was obtained from Genentech, Inc. First the pre-proinsulin was sub-cloned into pUC19 to facilitate further manipulations. The next step was to design primers to make chloroplast expression vectors. Since we are interested in proinsulin expression, the 5' primer was designed to land on the proinsulin sequence. This FW primer excluded the 69 bases or 23 coded amino acids of the leader or pre-sequence of preproinsulin. Also, the forward primer included the enzymatic cleavage site for the protease factor Xa to avoid the use of cyanogen bromide. Besides the Xa-factor, a Smal site was introduced to facilitate subsequent subcloning. The order of the FW primer sequence is SmaI - Xa-factor - Proinsulin gene. The reverse primer included BamHI and XbaI sites, plus a short sequence with homology

9 sequence following the proinsulin gene. The 297bp PCR product (Xa Pris) was cloned into pCR2.1. A GVGVP 50-mer was generated as describe previously (Daniell et al. 1997) along with the RBS sequence GAAGGAG. Another SmaI partial digestion was performed to eliminate the stop codon of the biopolymer gene, decrease the 50mer to a 40mer, and fuse the 40mer to the Xa-proinsulin sequence. Once the correct fragment was obtained by the partial digestion of Smal (eliminating the stop codon but including the RBS site), it was ligated to the Xa-proinsulin fusion gene resulting in the construct pCR2.1-40-XaPris. Finally, the biopolymer (40mer) - proinsulin fusion gene was subcloned into the chloroplast vector pLD-CtV or pSBL CtV and the orientation was checked in the final vector using suitable restriction sites.
**Expression and Purification of the Biopolymer-proinsilin fusion protein:** XL-1 Blue strain of *E. coli* containing pLD-OC-XaPris and the negative controls, which included a plasmid containing the gene in the reverse orientation and the *E. coli* strain without any plasmid were grown in TB broth. Cell pellets were resuspended in 500µl of autoclaved dH₂O or 6M Guanidine hydrochloride phosphate buffer, pH 7.0 were sonicated and centrifuged at 4°C at 10,000g for 10min. After centrifugation, the supernatants were mixed with an equal volume of 2XTN buffer (100 mM Tris-HCl, pH 8, 100 mM NaCl). Tubes were warmed at 42°C for 25min to induce biopolymer aggregation. Then the fusion protein was recovered by centrifuging at 2,500rpm at 42°C for 3min. Samples were run in a 16.5% Tricine gel, transferred to the nitrocellulose membrane, and immunoblotting was performed. When the sonic extract is in 6M Guanidine Hydrochloride Phosphate Buffer, pH 7.0, the molecular weight changes from its original and correct MW 24 kD to a higher MW of approximately 30 kDa (FIG. 12A,B). This is probably due to the conformation of the biopolymer in this buffer.

The gel was first stained with 0.3M CuCl₂ and then the same gel was stained with Commassie R-250 Staining Solution for an hour and then destained for 15min first, and then overnight CuCl₂ creates a negative stain (Lee et al. 1987). Polymer proteins (without fusion) appear as clear bands against a blue background in color or dark against a light semiopaque background (FIG. 12A). This stain was used because other protein stains such as Coomassie Blue R250 does not stain the polymer protein due to the lack of aromatic side chains (McPherson et al., 1992). Therefore, the observation of the 24 kDa protein in R250 stained gel (FIG. 12B) is due to the insulin fusion with the polymer. This observation was further confirmed by probing these blots with the anti-human proinsulin antibody. As anticipated, the polymer insulin fusion protein was observed in western blots (FIG. 13A,B). Larger proteins observed (FIG 13A-C) are tetramer and hexamer complexes of proinsulin. It is evident that the insulin-polymer fusion ble in E-coli. Confirming this observation, recently another lab has shown that the PBP polymer protein conjugates (with thioredoxin and tendamistat) undergo thermally reversible phase transition, retaining the transition behavior of the free polymer (Meyer and Chilkoti, 1999). These results clearly demonstrate that insulin fusion has not affected the inverse temperature transition property of the polymer. One of the concerns is the stability of insulin at temperatures used for thermally reversible purification. Temperature induced production of human insulin has been in commercial use (Schmidt et al. 1999). Also, the temperature transition can be lowered by increasing the ionic strength of the solution during purification of this PBP (McPherson et al. 1996). Thus, GVGVP-fusion could be used to purify a multitude of economically important proteins in a simple inexpensive step.
**Biopolymer-proinsulin fusion gene expression in chloroplast:** As described in section d, chloroplast vector was bombarded into the tobacco chloroplast genome via particle bombardment (Daniell, 1997). PCR and Southern Blots were performed to confirm biopolymer-proinsulin fusion gene integration into chloroplast genome. Southern blots show homoplasmy in most T₀ lines but a few showed some heteroplasmy (FIG. 14). Western blots show the polymer proinsulin fusion protein in all transgenic lines (Figure IOC). Quantification using ELISA is in progress.
**Protease Xa Digestion of the Biopolymer-proinsulin fusion protein and Purification of Proinsulin:** The enzymatic cleavage of the fusion protein to release the proinsulin protein from the (GVGVP)₄₀ was initiated by adding the factor 10A protease to the purified fusion protein at a ratio (w/w) of approximately 1:500. Cleavage of the fusion protein was monitored by SDS-PAGE analysis. We detected cleaved proinsulin in the extracts isolated in 6M guanidine hydrochloride buffer (FIG. 13A,B). Conditions are now being optimized for complete cleavage. The Xa protease has been successfully used previously to cleave (GVGVP)₂₀-GST fusion (McPherson et al. 1992).

### d. RESEARCH DESIGN AND METHODS

**Evaluation of chloroplast gene expression:** A systematic approach to identify and overcome potential limitations of foreign gene expression in chloroplats of transgenic plants is essential. Information gained in this study should increase the utility of chloroplast transformation system by scientists interested in expressing other foreign proteins. Therefore, it is important to systematically analyze transcription, RNA abundance, RNA stability, rate of protein synthesis and degradation, proper folding and biological active. For example, the rate of transcription of the introduced insulin gene will be compared with the highly expressing endogenous chloroplast genes (rbcL, psbA, 16S rRNA), using run on transcription assays to determine if the 16SrRNA promoter is operating as expected. Transgenic chloroplast containing each of the three constructs with different 5' regions will be investigated to test their transcription efficiency. Similarly, transgene RNA levels will be monitored by northerns, dot blots and primer extension relative to endogenous rbcL, 16S RNA, or psbA. These results along with run on transcription assays should provide valuable information of RNA stability, processing, etc. With our past experience in expression of several Foreign genes, foreign transcripts appear to be extremely stable based on northern blot analysis. However, a systematic study would be valuable to advance utility of this system by other scientists. Most importantly, the efficiency of translation will be tested in isolated chloroplasts and compared with the highly translated chloroplast protein (psbA). Pulse chase experiments would help assess if translational pausing, premature termination occurs. Evaluation of percent RNA loaded on polysomes or in constructs with or without 5'UTRs would help determine the efficiency of the ribosome binding site and 5' stem-loop translational enhancers. Codon optimized genes will also be compared with unmodified genes to investigate the rate of translation, pausing and termination. In our recent experience, we observed a 200-fold ccumulation of foreign proteins due to decreases in proteolysis conferred by a putative chaperonin (De Cosa et al. 2001). Therefore, proteins from constructs expressing or not expressing the putative chaperonin (with or without ORF1+2) should provide valuable information on protein stability. Thus, all of this information will be used to improve the next generation of chloroplast vectors. The PI has extensive experience in analysis of chloroplast gene expression (Relevent publications are included in resume).
**Optimization of gene expression:** We have reported that foreign genes are expressed between 3% (*cry2Aa2*) and 46% (*cry2Aa2* operon) in transgenic chloroplasts (Kota et al. 1999; De Cosa et al. 2001). Several approaches will be used to enhance translation of the recombinant proteins. In chloroplasts, transcriptional regulation as a bottle-neck in gene expression has been overcome by utilizing the strong constituitive promoter of the 16s rRNA (Prm). One advantage of Prm is that it is recognized by both the chloroplast encoded RNA polymerase and the nuclear encoded chloroplast RNA polymerase in tobacco (Allison et al. 1996). Several investigators have utilized Prm in their studies to overcome the initial hurdle of gene expression, transcription (De Cosa et al. 2001, Eibl et al. 1999, Staub et al. 2000). RNA stability appears to be one among the least problems because of observation of excessive accumulation of foreign transcripts, at times 16,966-fold higher than the highly expressing nuclear transgenic plants (Lee et al. 2000). Also, other investigations regarding RNA stability in chloroplasts suggest that efforts for optimizing gene expression need to be addressed at the post-transcriptional level (Higgs et al. 1999, Eibl et al. 1999). We intend to focus our investigation to address protein expression post-transcriptionally. For example, 5' and 3' UTRs are necessary for optimal translation and mRNA stablility of chloroplast mRNAs (Zerges 2000). Optimal ribosomal binding sites (RBS's) as well as a stem-loop structure located 5' adjacent to the RBS are required for efficient translation. A recent study has shown that replacement of the Shine-Delgarno (GGAGG) with the psbA 5' UTR downstream of the 16S rRNA promoter enhanced translation of a foreign gene (GUS) hundred-fold (Eibl et al. 1999). Therefore, the 200-bp tobacco chloroplast DNA fragment (1680-1480) containing 5' psbA UTR will be used. This PCR product will be inserted downstream of the 16S rRNA promoter to enhance translation of the recombinant proteins.

Yet another approach for enhancement of translation would be to optimize codon compositions. We have compared A+T% content of all foreign genes that had been expressed in transgenic chloroplasts in our laboratory with the percentage of chloroplast expression. We found that higher levels of A+T always correlated with high expression levels (see table 2). It is also potentially possible to modify chloroplast protease recognition sites while modifying it affecting their biological functions. Therefore, optimizing codon compositions of insulin and polymer genes to match the psbA gene should enhance the level of translation. Although rbcL (RuBisCO) is the most abundant protein on earth, it is not translated as highly as the psbA gene due to the extremely high turnover of the psbA gene product. The psbA gene is under stronger selection for increased translation efficiency and is the most abundant thylakoid protein. In addition, the codon usage in higher plant chloroplasts is biased towards the NNC codon of 2-fold degenerate groups (i.e. TTC over TTT, GAC over GAT, CAC over CAT, AAC over AAT, ATC over ATT, ATA etc.). This is in addition to a strong bias towards T at third position of 4-fold degenerate groups. There is also a context effect that should be taken into consideration while modifying specific codons. The 2-fold degenerate sites immediately upstream from a GNN codon do not show this bias towards NNC. (TTT GGA is preferred to TTC GGA while TTC CGT is preferred to TTT CGT, TTC AGT to TTT AGT and TTC TCT to TTT TCT, Morton, 1993; Morton and Bernadette, 2000). In addition, highly expressed chloroplast genes use GNN more frequently that other genes. The web site http.//www.kazusa.or.jp/codon and http://www.ncbi.nlm.nih.gov will be used to optimize codon composition by comparing codon usage of different plant species' genomes and PsbA's genes. Abundance of amino acids in chloroplasts and tRNA anticodons present in chloroplast will be taken into consideration. Optimization of polymer and proinsulin will be done using a novel PCR approach (Prodromou and Pearl, 1992; Casimiro et al. 1997), which has been successfully used in our laboratory to optimize codon composition of other human proteins.
**Vector constructions:** For all the constructs pLD vector will be used. This vector was developed in this laboratory for chloroplast transformation. It contains the 16S rRNA promoter (Prm) driving the selectable marker gene *aadA* (aminoglycoside adenyl transferase conferring resistance to spectinomycin) followed by the multiple cloning site and then the *psbA* 3' region (the terminator from a gene coding for photosystem II reaction center components) from the tobacco chloroplast genome. The pLD vector is a universal chloroplast expression /integration vector and can be used to transform chloroplast genomes of several other plant species (Daniell et al. 1998, Daniell 1999) because these flanking sequences are highly conserved among higher plants. The universal vector uses *trnA* and *trnI* genes (chloroplast transfer RNAs coding for Alanine and Isoleucine) from the inverted repeat region of the tobacco chloroplast genome as flanking sequences for homologous recombination. Because the universal vector integrates foreign genes within the Inverted Repeat region of the chloroplast genome, it should double the copy number of the transgene (from 5000 to 10,000 copies per cell in tobacco). Furthermore, it has been demonstrated that homoplasmy is achieved even in the first round of selection in bly because of the presence of a chloroplast origin of replication within the flanking sequence in the universal vector (thereby providing more templates for integration). These, and several other reasons, foreign gene expression was shown to be much higher when the universal vector was used instead of the tobacco specific vector (Guda et al. 2000).
**CTB-Proinsulin Vector Construction:** The chloroplast expression vector pLD-CTB-Proins will be constructed as follows. First, both proinsulin and cholera toxin B-subunit genes were amplified from suitable DNA using primer sequences. Primer 1 will contain the GGAGG chloroplast preferred ribosome binding site five nucleotides upstream of the start codon (ATG) for the CTB gene and a suitable restriction enzyme site (Spel) for insertion into the chloroplast vector. Primer 2 will eliminate the stop codon and add the first two amino acids of a flexible hinge tetrapeptide GPGP as reported by Bergerot et al. (1997), in order to facilitate folding of the CTB-proinsulin fusion protein. Primer 3 will add the remaining two amino acids for the hinge tetra-peptide and eliminate the pre-sequence of the native pre-proinsulin. Primer 4 will add a suitable restriction site (speI) for subcloning into the chloroplast vector. Amplified PCR products will be inserted into the TA cloning vector. Both the CTB and proinsulin PCR fragments will be excised at the SmaI and XbaI restriction sites. Eluted fragments will be ligated into the TA cloning vector. The CTB-proinsulin fragment will be excised at the EcoRI sites and inserted into EcoRI digested dephosphorolated pLD vector.
We will design the following vectors to optimize protein expression, purification and production 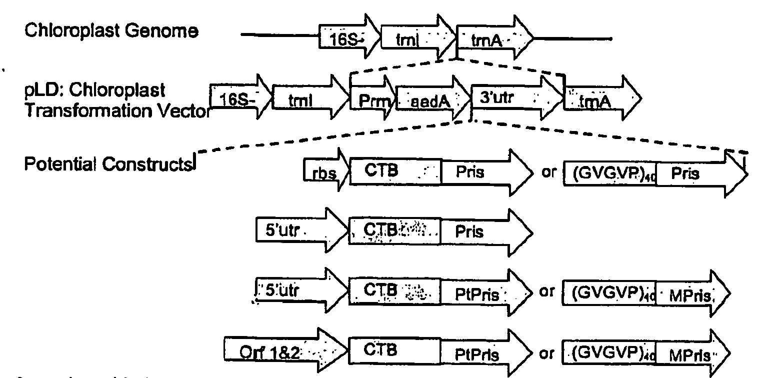 of proteins with the same amino acid composition as in human insulin. tcco plants, Eibl (1999) demonstrated, in vivo, the differences in translation efficiency and mRNA stability of a GUS reporter gene due to various 5' and 3' untranslated regions (UTR's). We intend to implement this already described systematic transcription and translation analysis in our practical endeavor of insulin production. Consistent with Eibl's (1999) data for increased translation efficiency and mRNA stability, we will use the psbA 5' UTR in addition with the psbA 3'UTR already in use. The 200 bp tobacco chloroplast DNA fragment containing 5' psbA UTR will be amplified by PCR using tobacco chloroplast DNA as template. This fragment will be cloned directly in the pLD vector multiple cloning site downstream of the promoter and the aadA gene. The cloned sequence will be exactly the same as in the psbA gene.
b) Another approach of protein production in chloroplasts involves potential insulin crystallization for facilitating purification. The *cry2Aa2 Bacillus thuringiensis* operon derived putative chaperonin will be used. Expression of the *cry2Aa2* operon in chloroplasts provides a model system for hyper-expression of foreign proteins (46% of total soluble protein) in a folded configuration enhancing their stability and facilitating purification (De Cosa et al. 2001). This justifies inclusion of the putative chaperonin from the *cry2Aa2* operon in one of the newly designed constructs. In this region there are two open reading frames (ORF1 and ORF2) and a ribosomal binding site (rbs). This sequence contains elements necessary for *Cy2Aa2* crystallization, which may help to crystallize insulin and aid in subsequent purification. Successful crystallization of other proteins using this putative chaperonin has been demonstrated (Ge et al. 1998). We will amplify the ORF1 and ORF2 of the Bt *Cry2Aa2* operon by PCR using the complete operon as template. Subsequent cloning, using a novel PCR technique, will allow for direct fusion of this sequence immediately upstream of the proinsulin fusion protein without altering the nucleotide sequence, which is normally necessary to provide a restriction enzyme site (Horton et al. 1988).
c) To address codon optimization the proinsulin gene will be subject to a certain modifications in subsequent constructs. The plastid modified proinsulin (PtPris) will have its nucleotide sequence modified such that the codons are optimized for plastid expression, yet its amino acid sequence will remain identical to human proinsulin. PtPris is an ideal substitute for human proinsulin in the CTB fusion peptide. We intend to compare the expression of this construct to the native human proinsulin to determine the affects to codon optimization, which will serve to address, in a case study format, one relevant mechanistic parameter of translation. Analysis of human proinsulin gene showed that 48 of its 87 codons were the uency codons in the chloroplast for the amino acid for which they encode. For example, there are six different codons for leucine. Their frequency within the chloroplast genome ranges from 7.3 to 30.8 per thousand codons. There are 12 leucines in proinsulin, 8 have the lowest frequency codons (7.3), and none code for the highest frequency codons (30.8). In the plastid optimized proinsulin gene all the codons will code for the most frequent, whereas in human proinsulin over half of the codons are the least frequent. Human proinsulin nucleotide sequence contains 62% C+G, whereas plastid optimized proinsulin gene will contain 24% C+G. Generally, lower C+G content of foreign genes correlates with higher levels of expression (Table 2).
d) Another version of the proinsulin gene, mini-proinsulin (Mpris), will also have its codons optimized for plastid expression, and its amino acid sequence will not differ from human proinsulin (Pris). Pris' sequence is B Chain-RR-C Chain-KR-A Chain, whereas MPris' sequence is B Chain-KR-A Chain. The MPris sequence excludes the RR-C Chain, which is normally excised in proinsulin maturation to insulin. The C chain of proinsulin is an unnecessary part of *in vitro* production of insulin. Proinsulin folds properly and forms the appropriate disulfide bonds in the absence of the C chain. The remaining KR motif that exists between the B chain and the A chain in MPris allows for mature insulin production upon cleavage with trypsin and carboxypeptidase B. This construct will be used for our proposed biopolymer fusion protein. It's codon optimization and amino acid sequence is ideal for mature insulin production.
e) Our current human proinsulin-biopolymer fusion protein contains a factor Xa proteolytic cut site, which serves as a cleavage point between the biopolymer and the proinsulin. Currently, cleavage of the polymer-proinsulin fusion protein with the factor Xa has been inefficient in our hands. Therefore, we will replace this cut site with a trypsin cut site. This will eliminate the need for the expensive factor Xa in processing proinsulin. Since proinsulin is currently processed by trypsin in the formation of mature insulin, insulin maturation and fusion peptide cleavage can be achieved in a single step with trypsin and carboxypeptidase B.
f) We observed incomplete translation products in plastids when we expressed the 120mer gene (Guda et al. 2000). Therefore, while expressing the polymer-proinsulin fusion protein, we have decreased the length of the polymer protein to 40mer, without losing the thermal responsive property. In addition, optimal codons for glycine (GGT) and valine (GTA), which constitute 80% of the total amino acids of the polymer, have been used. In all nuclear enes, glycine makes up 147/1000 amino acids while in tobacco chloroplasts it is 129/1000. Highly expressing genes like psbA and rbcL of tobacco make up 192 and 190 gly/1000. Therefore, glycine may not be a limiting factor. Nuclear genes use 52/1000 proline as opposed to 42/1000 in chloroplasts. However, currently used codon for proline (CCG) could be modified to CCA or CCT to further enhance translation. It is known that pathways for proline and valine are compartmentalized in chloroplasts (Guda et al. 2000). Also, proline is known to accumulate in chloroplasts as an osmoprotectant (Daniell et al. 1994).
g) Codon comparison of the CTB gene with psbA, showed 47% homology with the most frequent codons of the psbA gene. Codon analysis showed that 34% of the codons of CTB are complimentary to the tRNA population in the chloroplasts in comparison with 51% of psbA codons that are complimentary to the chloroplast tRNA population. Because of the high levels of CTB expression in transgenic chloroplasts (Henriques and Daniell, 2000), there will be no need to modify the CTB gene.

DNA sequence of all constructs will be determined to confirm the correct orientation of genes, in frame fusion, and accurate sequences in the recombinant DNA constructs. DNA sequencing will be done using a Perkin Elmer ABI prism 373 DNA sequencing system using a ABI Prism Dye Termination Cycle Sequencing kit. By using primers for each strand, insertion sites at both ends will be sequenced. Because of the similarity of protein synthetic machinery (Brixey et al. 1997), expression of all chloroplast vectors will be first tested in *E.coli* before their use in tobacco transformation. For *Escherichia coli* expression XL-1 Blue strain was used. *E. coli* will be transformed by standard CaCl₂ method.
**Bombardment and Regeneration of Chloroplast Transgenic Plants:** Tobacco (*Nicotiana tabacum* var. Petit Havana) and nicotine free edible tobacco (LAMD 605, gift from Dr. Keith Wycoff, Planet Biotechnology) plants will be grown aseptically by germination of seeds on MSO medium (Daniell 1993). Fully expanded, dark green leaves of about two month old plants will be used for bombardment.

Leaves will be placed abaxial side up on a Whatman No. 1 filter paper laying on the RMOP medium (Daniell, 1993) in standard petri plates (100x15 mm) for bombardment. Gold (0.6 µm) microprojectiles will be coated with plasmid DNA (chloroplast vectors) and will be carried out with the biolistic device PDS1000/He (Bio-Rad) as described by Daniell (1997). Following bombardment, petri plates will be sealed with parafilm and incubated at 24°C under 12 h photoperiod. Two days after bombardment, leaves will be chopped into small pieces of ∼5 mm² in size and placed on the selection medium (RMOP containing 500 µg/ml of spectinomycin dihydrochloride) with abaxial side touching the medium in deep (100x25 mm) petri plates (∼10 pieces per plate). The regenerated spectinomycin resistant shoots will be chopped into small pieces (∼2mm²) and subcloned into fresh deep petri plates (∼5 pieces per plate) containing the same selection medium. Resistant shoots from the second culture cycle will be transferred to the rooting medium (MSO medium supplemented with IBA, 1 mg/liter and spectinomycin dihydrochloride, 500 mg/liter). Rooted plants will be transferred to soil and grown at 26°C under continuous lighting conditions for further analysis.
**Polymerase Chain Reaction:** PCR will be done using DNA isolated from control and transgenic plants in order to distinguish a) true chloroplast transformants from mutants and b) chloroplast transformants from nuclear transformants. Primers for testing the presence of the aadA gene (that confers spectinomycin resistance) in transgenic plants will be landed on the aadA coding sequence and 16S rRNA gene (primers 1P&1M,). In order to test chloroplast integration of the insulin gene, one primer will land on the aadA *gene* while another will land on the native chloroplast genome (primers 3P&3M). No PCR product will be obtained with nuclear transgenic plants using this set of primers. The primer set (2P & 2M) will be used to test integration of the entire gene cassette without any internal deletion or looping out during homologous recombination, by landing on the respective recombination sites. A Similar strategy has been used successfully by us to confirm chloroplast integration of foreign genes (Daniell et al., 1998; Kota et al., 1999; Guda et al., 2000). This screening is essential to eliminate mutants and nuclear transformants. In order to conduct PCR analyses in transgenic plants, total DNA from unbombarded and transgenic plants will be isolated as described by Edwards et aL (1991). Chloroplast transgenic plants containing the proinsulin gene will be moved to second round of selection in order to achieve homoplasmy.
**Southern Blot Analysis:** Southern blots will be done to determine the copy number of the introduced foreign gene per cell as well as to test homoplasmy. There are several thousand copies of the chloroplast genome present in each plant cell. Therefore, when foreign genes are inserted into the chloroplast genome, it is possible that some of the chloroplast genomes have foreign genes integrated while other remain as the wild type (heteroplasmy). Therefore, in order to ensure that only the transformed genome exists in cells of transgenic plants (homoplasmy), the ss will be continued. In order to confirm that the wild type genome does not exist at the end of the selection cycle, total DNA from transgenic plants should be probed with the chloroplast border (flanking) sequences (the trnI-trnA fragment, Figure 2A,3B). If wild type genomes are present (heteroplasmy), the native fragment size will be observed along with transformed genomes. Presence of a large fragment (due to insertion of foreign genes within the flanking sequences) and absence of the native small fragment should confirm homoplasmy (Daniell et al., 1998; Kota et aL, 1999; Guda et al., 2000).

The copy number of the integrated gene will be determined by establishing homoplasmy for the transgenic chloroplast genome. Tobacco Chloroplasts contain 5000∼10,000 copies of their genome per cell (Daniell et al. 1998). If only a fraction of the genomes are actually transformed, the copy number, by default, must be less than 10,000. By establishing that in the transgenics the insulin inserted transformed genome is the only one present, one could establish that the copy number is 5000~10,000 per cell. This is usually done by digesting the total DNA with a suitable restriction enzyme and probing with the flanking sequences that enable homologous recombination into the chloroplast genome. The native fragment present in the control should be absent in the transgenics. The absence of native fragment proves that only the transgenic chloroplast genome is present in the cell and there is no native, untransformed, chloroplast genome, without the insulin gene present. This establishes the homoplasmic nature of our transformants, simultaneously providing us with an estimate of 5000∼10,000 copies of the foreign genes per cell.
**Northern Blot Analysis:** Northern blots will be done to test the efficiency of transcription of the proinsulin gene fused with CTB or polymer genes. Total RNA will be isolated from 150 mg of frozen leaves by using the "Rneasy Plant Total RNA Isolation Kit" (Qiagen Inc., Chatsworth, CA). RNA (10-40 µg) will be denatured by formaldehyde treatment, separated on a 1.2% agarose gel in the presence of formaldehyde and transferred to a nitrocellulose membrane (MSI) as described in Sambrook et al. (1989). Probe DNA (proinsulin gene coding region) will be labeled by the random-primed method (Promega) with ³²P-dCTP isotope. The blot will be pre-hybridized, hybridized and washed as described above for southern blot analysis. Transcript levels will be quantified by the Molecular Analyst Program using the GS-700 Imaging Densitometer (Bio-Rad, Hercules, CA).

### Polymer-insulin fusion protein purification, quantitation and characterization:

ter insulin fusion proteins exhibit inverse temperature transition properties (Figure 9 and 10), they will be purified from transgenic plants essentially following the same method recently described by us for polymer purification from transgenic tobacco plants (Zhang et al., 1996). Polymer extraction buffer contains 50 mM Tris-HCl, pH 7.5, 1% 2-mecaptoethanol, 5mM EDTA and 2mM PMSF and 0.8 M NaCl. The homogenate will then be centrifuged at 10,000 g for 10 minutes (4°C), and the pellet will be discarded. The supernatant will be incubated at 42°C for 30 minutes and then centrifuged immediately for 3 minutes at 5,000 g (room temperature). If insulin is found to be sensitive to this temperature, Tₜ will be lowered by increasing salt concentration (McPherson et al., 1996). The pellet containing the insulin-polymer fusion protein will be resuspended in the extraction buffer and incubated on ice for 10 minutes. The mixture will be centrifuged at 12,000 g for 10 minutes (4°C). The supernatant will be collected and stored at -20°C. The purified polymer insulin fusion-protein will be electrophoresed in a SDS-PAGE gel according to Laemmli (1970) and visualized by either staining with 0.3 M CuCl₂ (Lee et al. 1987) or transferred to nitrocellulose membrane and probed with antiserum raised against the polymer or insulin protein as described below. Quantification of purified polymer proteins will be carried out by ELISA in addition to densitometry.

After electrophoresis, proteins will be transferred to a nitrocellulose membrane electrophoretically in 25 mM Tris, 192 mM glycine, 5% methanol (pH 8.3). The filter will be blocked with 2% dry milk in Tris-buffered saline for two hours at room temperature and stained with antiserum raised against the polymer AVGVP (kindly provided by the University of Alabama at Birmingham, monoclonal facility) overnight in 2% dry milk/Tris buffered saline. The protein bands reacting to the antibodies will be visualized using alkaline phosphatase-linked secondary antibody and the substrates nitroblue tetrazolium and 5-bromo-4-chloro-3-indolyl-phosphate (Bio-Rad). Alternatively, for insulin-polymer fusion proteins, a Mouse anti-human proinsulin (IgGI) monoclonal antibody will be used as a primary antibody. To detect the binding of the primary antibody to the recombinant proinsulin, a Goat anti-mouse IgG Horseradish Peroxidase Labeled monoclonal antibody (HPR) will be used. The substrate to be used for conjugation with HRP will be 3,3', 5,5'-Tetramethylbenzidine. All products will be purchased from American Qualex Antibodies in San Clemente, CA. As a positive control, human recombinant proinsulin from Sigma will be used. This human recombinant proinsulin was expressed in *E.coli* by a synthetic proinsulin gene. Quantification of purified polymer fusion proteins will be carried out by densitometry using Scanning Analysis software (BioSoft, Ferguson, MO). Total protein contents will be determined by the dye-binding assay using reagents supplied in kit from Bio-Rad, with bovine serum albumin as a standard.
**Characterization of CTB expression:** CTB protein levels in transgenic plant crude extract will be determined using quantitative ELISA assays. A standard curve will be generated using known concentrations of bacterial CTB. A 96-well microtiter plate loaded with 100µl/well of bacterial CTB (concentrations in the range of 10-1000ng) will be incubated overnight at 4°C. The plate will be washed thrice with PBST (phosphate buffered saline containing 0.05% Tween-20). The background will be blocked by incubation in 1% bovine serum albumin (BSA) in PBS (300µl/well) at 37°C for 2 h followed by washing 3 times with PBST. The plate will be incubated in a 1:8,000 dilution of rabbit anti-cholera toxin antibody (Sigma C-3062) (100µl/well) for 2 h at 37°C, followed by washing the wells three times with PBST. The plate will be incubated with a 1:80,000 dilution of anti-rabbit IgG conjugated with alkaline phoshatase (100µl/well) for 2 h at 37°C and washed thrice with PBST. Then, 100 µl alkaline phosphatase substrate (Sigma Fast p-nitrophenyl phosphate tablet in 5 ml of water will be added and the reaction will be stopped with 1M NaOH (50µl/well) when absorbancies in the mid-range of the titration reach about 2.0, or after 1 hour, whichever comes first. The plate will then be read at 405nm. These results will be used to generate a standard curve from which concentrations of plant protein can be extrapolated. Thus, total soluble plant protein (concentration previously determined using the Bradford assay) in bicarbonate buffer, pH 9.6 (15mM Na₂Co₃, 35mM NaHCO₃) will be loaded at 100 plant µl/welland the same procedure as above can be repeated. The absorbance values will be used to determine the ratio of CTB protein to total soluble plant protein, using the standard curve generated previously and the Bradford assay results.
**Inheritance of Introduced Foreign Genes:** While it is unlikely that introduced DNA would move from the chloroplast genome to nuclear genome, it is possible that the gene could get integrated in the nuclear genome during bombardment and remain undetected in Southern analysis. Therefore, in initial tobacco transformants, some will be allowed to self-pollinate, whereas others will be used in reciprocal crosses with control tobacco (transgenics as female accepters and pollen donors; testing for maternal inheritance). Harvested seeds (T1) will be germinated on media containing spectinomycin.. Achievement of homoplasmy and mode of inheritance can be classified by looking at germination results. Homoplasmy should be indicated by totally green seedlings (Daniell et al., 1998) while heteroplasmy is displayed by variegated leaves (lack of pigmentation, Svab & Maliga, 1993). Lack of variation in chlorophyll pigmentation among progeny should also underscore the absence of position effect, an artifact of nuclear transformation. Maternal inheritance will be demonstrated by sole transmission of introduced genes via seed generated on transgenic plants, regardless of pollen source (green elective media). When transgenic pollen is used for pollination of control plants, resultant progeny would not contain resistance to chemical in selective media (will appear bleached; Svab and Maliga, 1993). Molecular analyses will confirm transmission and expression of introduced genes, and T2 seed will be generated from those confirmed plants by the analyses described above.
**Comparison of Current Purification with Polymer-based Purification Methods**: It is important to compare purification methods by testing yield and purity of insulin produced in *E.coli* and tobacco. Three methods will be compared: a standard fusion protein in *E. coli,* polymer proinsulin fusion protein in *E coli,* and polymer proinsulin fusion in tobacco. Polymer proinsulin fusion peptide from transgenic tobacco will be purified by methodology described in section c) and Daniell (1997). *E. coli* purification will be performed as follows. One liter of each pLD containing bacteria will be grown in LB/ampicillin (100 µg/ml) overnight and the fusion protein, either polymer-proinsulin or the control fusion protein (Cowley and Mackin 1997), will be expressed. Cells will be harvested by centrifugation at 5000 X g for 10 min at 4°C, and the bacterial pellets will be resuspended in 5 ml/g (wet wt. Bacteria) of 100 mM Tris-HCl, pH 7.3. Lysozyme will be added at a concentration of 1 mg/ml and placed on a rotating shaker at room temperature for 15 min. The lysate will be subjected to probe sonication for two cycles of 30 s on/30 s off at 4°C. Cellular debris will be removed by centrifugation at 1000 X g for 5 min at 4°C. The E. coli produced proinsulin polymer fusion protein will be purified by inverse temperature transition properties (Daniell et al., 1997). After Factor Xa cleavage (as described in section c)) the proinsulin will be isolated from the polymer using inverse temperature transition properties (Daneill et al., 1997) and subject to oxidative sulfitolysis as described below. Alternatively, the control fusion protein will be purified according to Cowley and Mackin (1997) as follows. The supernatant will be retained and centrifuged again at 27000 X g for 15 min at 4°C to pellet the inclusion bodies. The supernatant will be discarded and the pellet resuspended in 1 ml/g (original wt. Bacteria) of dH₂O, aliquoted into microcentrifuge tubes as I ml fractions, and then centrifuged at 16000 X g for 5 min at 4°C. The pellets will be individually washed with 1 ml of 100 mM Tris-HCl, pH 8.5, 1M urea, 1-1 Triton X-100 and again washed with100 mM Tris HCl pH8.5, 2 M urea, 2 % Trinton X-100. The pellets will be resuspended in 1 ml of dH₂O and transferred to a pre-weighed 30 ml Corex centrifuge tube. The sample will be centrifuged at 15000 X g for 5 min at 4°C, and the pellet will be resuspended in 10 ml/g (wet wt. pellet) of 70% formic acid. Cyanogen bromide will be added to a final concentration of 400 mM and the sample will be incubated at room temperature in the dark for 16 h. The reaction will be stopped by transferring the sample to a round bottom flask and removing the solvent by rotary 50°C. The residue will be resuspended in 20ml/g (wet wt. pellet) of dH₂O, shell frozen in a dry ice ethanol bath, and then lyophilized. The lyophilized protein will be dissolved in 20 ml/g (wet wt. pellet) of 500 mM Tris-HCl, pH 8.2, 7 M urea. Oxidative sulfitolysis will be performed by adding sodium sulfite and sodium tetrathionate to final concentrations of 100 and 10 mM, respectively, and incubating at room temperature for 3 h. This reaction will be stopped by freezing on dry ice.
**Purification and folding of Human Proinsulin:** The S-sulfonated material will be applied to a 2 ml bed of Sephadex G-25 equilibrated in 20 mM Tris-HCl, pH 8.2, 7 M urea, and then washed with 9 vols of 7 M urea. The collected fraction will be applied to a Pharmacia Mono Q HR 5/5 column equilibrated in 20 mM Tris HCl, pH 8.2, 7 M urea at a flow rate of 1 ml/min. A linear gradient leading to final concentration of 0.5 M NaCl will be used to elute the bound material. 2 min (2 ml) fractions will be collected during the gradient, and protein concentration in each fraction will be determined. Purity and molecular mass of fractions will be estimated by Tricine SDS-PAGE (as shown in Fig. 2), where Tricine is used as the trailing ion to allow better resolution of peptides in the range of 1-1000 kDa. Appropriate fractions will be pooled and applied to a 1.6 X 20 cm column of Sephadex G-25 (superfine) equilibrated in 5 mM ammonium acetate pH 6.8. The sample will be collected based on UV absorbance and freeze-dried. The partially purified S-sulfonated material will be resuspended in 50 mM glycine/NaOH, pH 10.5 at a final concentration of 2 mg/ml. β-mercaptoethanol will be added at a ratio of 1.5 mol per mol of cysteine S-sulfonate and the sample will be stirred at 4°C in an open container for 16 h. The sample will be then analyzed by reversed-phase high-performance liquid chromatography (RP-HPLC) using a Vydac C₄ column (2.2 X 150 mm) equilibrated in 4% acetonitrile and 0.1 % TFA. Adsorbed peptides will be eluted with a linear gradient of increasing acetonitrile concentration (0.88% per min up to a maximum of 48%). The remaining refolded proinsulin will be centrifuged at 16000 X g to remove insoluble material, and loaded onto a semi-preparative Vydac C₄ column (10 X 250 mm). The bound material will be eluted as described above, and the proinsulin will be collected and lyophilized.
**Analysis and characterization of insulin expressed In *E. coli* and Tobacco:** The purified expressed proinsulin will be subjected to matrix-assisted laser desorption/ionization-time of flight (MALDI-TOF) analysis (as described by Cowley and Mackin, 1997), using proinsulin from Eli Lilly as both an internal and external standard. To determine if the disulfide bridges have formed correctly naturally inside chloroplasts or by *in vitro* processing, a proteolytic digestion will be performed using *Staphylococcus aureus* protease V8. Five µg of both the nsulin and Eli Lilly's proinsulin will be lyophilized and resuspended in 50 µl of 250 mM NaPO₄, pH 7.8. Protease V8 will be added at a ratio of 1:50 (w/w) in experimental samples and no enzyme will be added to the controls. All samples will then be incubated overnight at 37°C, the reactions will be stopped by freezing on dry ice, and samples will be stored at -20 °C until analyzed. The samples will be analyzed by RP-HPLC using a Vydac C₄ column (2.2 X 150 mm) equilibrated in 4% acetonitrile and 0.1% TFA. Bound material will be eluted using a linear gradient of increasing acetonitrile concentration (0.88% per min up to a maximum of 48%).
**CTB-GM1 ganglioside binding assay**: A GMI-ELISA assay will be performed as described by Arakawa et al (1997) to determine the affinity of plant-derived CTB for GMI-ganglioside. The microtiter plate will be coated with monosialoganglioside-GMI (Sigma G-7641) by incubating the plate with 100 µl/well of GM1 (3.0 µg/ml) in bicarbonate buffer, pH 9.6 at 4 °C overnight Alternatively, the wells will be coated with 100 µl/well of BSA (3.0 µg/ml) as control. The plates will be incubated with transformed plant total soluble protein and bacterial CTB (Sigma C-9903) in PBS (100 µl/well) overnight at 4°C. The remainder of the procedure will be identical to the ELISA described above.
**Induction of oral tolerance**: Four week old female NOD mice will be purchased from Jackson Laboratory (Bar Harbor, ME) and housed at the animal care facility located in the school of Biology at the University of Central Florida (UCF). The mice will be divided into three groups, each group consisting of ten mice. Each group will be fed one of the following nicotine free edible tobacco: untransformed, expressing CTB, or expressing CTB-proinsulin fusion protein. Beginning at 5 weeks of age, each mouse will be fed 3 g of nicotine free edible tobacco once per week until reaching 9 weeks of age (a total of five feedings).
**Antibody titer:** At ten weeks of age, the serum and fecal material will be assayed for anti-CTB and anti-proinsulin antibody isotypes using the ELISA method described above.
**Assessment of diabetic symptoms In NOD mice**: The incidence of diabetic symptoms will be compared among mice fed with control nicotine free edible tobacco that expresses CTB and those that express the CTB-proinsulin fusion protein. Starting at 10 weeks of age, the mice will be monitored on a biweekly basis with urinary glucose test strips (Clinistix and Diastix, Bayer) for development of diabetes. Glycosuric mice will be bled from the tail vein to check for glycemia using a glucose analyzer (Accu-Check, Boehringer Mannheim). Diabetes will be lyperglycemia (>250 mg/dl) for two consecutive weeks (Ma et al. 1997). The plant tissue of control and transgenic plants to be fed to mice will be provided for a collaborator (Dr. Ali Amirkhosravi, Florida Hospital) to perform these studies. A letter of collaboration is provided documenting this arrangement.

### Tentative Proposed Schedule

### Year I:

a) Develop recombinant DNA vectors for enhanced translation of proinsulin as fusion protein with protein based polymers or CTB via chloroplast genomes of tobacco
b) Obtain transgenic tobacco plants using the transformation vectors
c) Assay transgenic expression of insulin-polymer fusion protein and CTB in chloroplasts using molecular and biochemical methods

### Year II:

d) Employ existing methods of polymer purification from transgenic leaves or develop new approaches for the fusion protein and estimate levels of expression
e) Analyze genetic composition of transgenic plants (Mendelian or maternal inheritance)
f) Large scale purification of insulin from green house grown transgenic plants and comparison of current insulin purification methods with polymer-based purification method

### Year III

g) Refolding and characterization (yield and purity) of proinsulin produced in *E.coli* and transgenic tobacco
h) Assessment of diabetic symptoms in NOD mice fed with leaves expressing CTB-proinsulin fusion protein
j) Assessment of immune response in mice fed with leaves expressing CTB
k) Continue to characterize subsequent transgenic generations (T1, T2, T3).

### f. Vertebrate Animals

Discription of proposed work: Oral tolerance and the incidence of diabetic symptoms will be compared among mice fed with nontransgenic tobacco (negative control), CTB expressing nicotine free edible tobacco (LAMD 605) and those that express the CTB-proinsulin fusion protein. Thirty, female nonobese diabetic (NOD) mice, four weeks of age, will be purchased from Jackson Laboratory (Bar Harbor, ME), and housed at the animal care facility located in the school of Biology at the University of Central Florida (UCF).
**Experimental groups:** The mice will be divided into the following groups, each group consisting of ten mince: group 1, fed untransformed LAMD 605; group 2, fed transgenic LAMD 605 synthesizing CTB; and group 3, fed transgenic LAMD 605 synthesizing CTB-proinsulin fusion protein. Beginning at five weeks of age, each mouse will be fed 3 g of LAMD 605 once per week until reaching 9 weeks of age (a total of five feedings). At ten weeks, serum and fecal material will be assayed for anti-CTB and anti-insulin antibody isotyypes using ELISA as described above.
The incidence of diabetic symptoms will be compared among mice fed transgenic LAMD 605 synthesizing CTB and LAMD 605 synthesizing CTB-proinsulin fusion protein. Starting at 10 weeks of age, the mice will be monitored on a biweekly basis with urinary glucose test strips (Clinistix and Diastix, Bayer) for development of diabetes. Glycosuric mice will be bled from the tail vein to check for glycemia using a glucose analyzer (Accu-Check, Boehringer Mannheim). Diabetes will be confirmed by hyperglycemia (>250 mg/dl) for two consecutive weeks (Ma et al. 1997).
**Investigator:** The plant tissue of control and transgenic plants to be fed to mice will be provided for a collaborator, Ali Amirkhosravi PhD. (Florida Hospital), to perform these studies. A letter of collaboration is provided documenting this arrangement. Dr. Amirkhosravi's expertise for performing scientific investigations involving animals is demonstrated by his experience and publications provided in his resume.
**Justification of species selection**: Female NOD mice have a high incidence of developing autoimmune diabetes after 12 weeks of age (Gaskins et al. 1992). Therefore, they are the appropriate model for our study for the prevention of autoimmune diabetes. According to the previous experience and Arakawa et al. (1998), ten mice for each group, thirty mice total, will provide our study with the amount necessary for reliable data.
**Veternary care**: Farol Tomson DVM is the veterinary consultant for the UCF animal care facility.
**Discomfort, distress, pain, and injury:** According to the investigator's experience, the mice involved in this investigation will not experience discomfort or severe symptoms, including severe diabetic symptoms, Furthermore, the specific diet for the mice is well tolerated. Animals will be checked regularly, and in case of any visible signs of distress or pain, animals will be removed from the study, however this is unlikely.
**Euthanasia:** Upon completion of the study mice will be euthanized by an overdose of the inhalent anesthetic halothane, which is a standard method of euthanasia. This method is consistent with the recommendations of the Panel on Euthanasia of the American Veterinary Medical Association.

### g. Literature Cited

Allison LA, Maliga P (1995) Light-responsive and transcription-enhancing elements regulate the plastid psbD core promotor. EMBO J 14: 3721-3730
Arakawa T, Yu J, Chong, DKX, Hough J, Engen PC, Langridge WHR (1998) A plant-basedcholera toxin B subunit-insulin fusion protein protects against the development of autoimmune diabetes. Nature Biotechnology. 16: 934-938.
Arakawa T, Chong DKX, Merritt JL, Langridge WHR (1997) Expression of cholera toxin B subunit oligomers in transgenic potato plants. Transgenic Research 6:403-413.
Arntzen CJ., Mason H.S, et al (1998) Edible vaccine protects mice against E.coli heat labile enterotoxin :potatoes expressing a synthetic LTB gene.Vaccine. 16(13):1336-1343
Bergerot I, Ploix C, Peterson J, Moulin V, Rask C, Fabien N, et al. (1997) A cholera toxoid-insulin conjugate as an oral vaccine against spontaneous autoimmune diabetes. Proc. Natl. Acad. Sci. USA. 94: 4610-4614.
Biggin P, Sansom M (1999). Interactions of α-helices with lipid bilayers: a review of simulation studies. Biophysical Chemistry 76: 161-183.
Bock R, Hagemann R (2000) Extracellular inheritance: Plastid genomics: Manipulation of Plastid genomes and biotechnological applications. Progress in Botany 6: 76-90.
Bogorad L (2000). Engineering chloroplasts: an alternative site for foreign genes, proteins, reactions and products. Trends in Biotechnology 18: 257-263.
Brixey J, Guda C, Daniell H (1997) The chloroplast psbA promoter is more efficient in E.coli than the T7 promoter for hyper expression of a foreign protein. Biotechnology Letters 19: 395-400.
Burnette JP (1983) Experimental Manipulation of Gene Expression. Oxender, D.L., Fox. C.F. eds. pp. 71-82, Alan R, Liss, Inc., New York, NY.
Carlson PS (1973) The use of protoplasts for genetic research. Proc. Natl. Acad. Sci USA 70:598-602.
Casimiro DR, Wright PE and Dyson HJ. (1997). PCR-based gene synthesis and protein NMR Spectroscopy. Structure 5 (11): 1407-1412.
Chance RE, Frank BH (1993) Research, development, production and safety of biosynthetic human insulin. Diabetes Care. 16(3): 133-142.
Cohen A, Mayfield (1997) Translational regulation of gene expression in plants. Current Opinion in Biotechnology 8: 189-194.
Cowley DJ, Mackin RB (1997) Expression, purification and characterization of recombinant human proinsulin. FEBS Letts. 402: 124-130.
wski A, Hirose T, Itakura K (1978) Chemical synthesis of genes for human insulin. Proc. Natl. Acad. Sci. 75(12): 5765-5769.
Daniell H. (1995) Producing polymers in plants and bacteria. Inform 6:1365-1370.
Daniell H (1997) Transformation and foreign gene expression in plants mediated by microprojectile bombardment. Meth Mol Biol 62:453-488.
Daniell H (1999) Universal chloroplast integration and expression vectors, transformed plants and products thereof, World Intellectual Property Organization WO 99/10513.
Daniell H, Datta R, Varma S Gray S Lee SB (1998) Containment of herbicide resistance through genetic engineering of the chloroplast genome. Nature Biotechnology 16: 345-348.
Daniell H, Guda C (1997) Biopolymer production in microorganisms and plants. Chemistry and Industry, 14: 555-560.
Daniell H, Guda C, McPherson DT, Xu J, Zhang X, Urry DW (1997) Hyper expression of an environmentally friendly synthetic polymer gene. Meth Mol Biol 63:359-371.
Daniell H, Krishnan M, McFadden BA (1991) Expression of B-glucuronidase gene in different cellular compartments following biolistic delivery of foreign DNA into wheat leaves and calli. Plant Cell Reports 9:615-619.
Daniell H, Krishnan M, Umabai U, Gnanam A (1986) An efficient and prolonged in vitro translational system from cucumber etioplasts. Biochem. Biophys. Res. Comun 135: 48-255.
Daniell H, McFadden BA (1987) Uptake and expression of bacterial and cyanobacterial genes by isolated cucumber etioplasts. Proc Natl Acad Sci USA 84:6349-6353.
Daniell H, McFadden BA (1988) Genetic Engineering of plant chloroplasts. United States Patents 5,932,479; 5,693,507.
Daneill H, Muthukumar B, Lee SB (2000) Engineering chloroplast genome without the use of antibiotic resistance genes. Current Genetics, in press.
Daniell H, Ramanujan P, Krishnan M, Gnanam A, Rebeiz CA (1983) In vitro synthesis of photosynthetic membranes: 1. Development of photosystem I activity and cyclic phosphorylation. Biochem. Biophys. Res. Comun 111:740-749.
Daniell H, Rebeiz CA (1982) Chloroplast culture IX: Chlorphyll(ide) A biosynthesis in vitro at rates higher than in vivo. Biochem. Biophys. Res. Comun 106:466-471.
Daniell H, Vivekananda J, Neilsen B, Ye GN, Tewari KK, Sanford JC (1990) Transient foreign gene expression in chloroplasts of cultured tobacco cells following biolistic delivery of chloroplast vectors. Proc Natl Acad Sci USA 87:88-92.
coff K, Stratfield S (2000) Production of vaccines, monoclonals, and pharmaceutical proteins in plants. Trends in Plant Science, in press
Davidson, MB (1998) Diagnosis and classification of diabetes mellituus. in "Diabetes Mellitus-Diagnosis and Treatment. pp.1-16, 4th edition., W.B. Saunders Co., Philidelphia, PA.
De Cosa B, Moar W, Lee SB, Miller M, Daniell H (2001). Hyper-expression of the Bt Cry2Aa2 operon in chloroplasts leads to formation of insecticidal crystals. Nature Biotechnology, in press.
DeGray G, Smith F, Sanford J, Daniell H (2000). Hyper-expression of an antimicrobial peptide via the chloroplast genome to confer resistance against phytopathogenic bacteria. In review.
Dertzbaugh MT, Elson CO (1993) Comparitive effectiveness of the cholera toxin B subunit and alkaline phosphatase as carriers for oral vaccines.Infect.Immun.61:48-55
Drescher DF, Follmann H, Haberlein I (1998). Sulfitolysis and thioredoxin-dependent reduction reveal the presence of a structural disulfide bridge in spinach chloroplast fructose-1, 6-bisphosphate. FEBS Letters 424: 109-112.
Edwards K, Johnstone C, Thompson C (1991) A simple and rapid method for preparation of plant genomic DNA for PCR analysis. Nucleic Acids Res 19:1349.
Eibl C, Zou Z, Beck A, Kim M, Mullet J, Koop UH (1999) In vivo analysis of plastid psbA, rbcL and rp132 UTR elements by chloroplast transformation: tobacco plastid gene expression is controlled by modulation of transcript levels and translation efficiency. The Plant Journal 19: 333-345.
Gaskins HR, Prochazka M, Hamaguchi K, Serreze DV, Leiter EH. (1992) Beta cell expression of endogenous xenotropic retrovirus distinguishes diabetes-susceptible NOD/Lt from resistant NON/Lt mice. J Clin Invest. 90(6):2220-7
Ge B et al (1998). Differential effects of helper proteins encoded by the cry2A and cry11A operons on the formation of Cry2A inclusions in Bacillus thuringiensis. FEMS Microbiol. Lett. 165: 35-41.
Gill D M (1976).The arrangement of subunits in cholera toxin.Biochemistry. 15;1242-1248
Goeddel DV, Kleid DG, Bolivar F, Heyneker HL, Yansura DG, Crea R, Hirose T, Kraszewski A, Italkura K, Riggs AD (1979) Expression in Escherichia coli of chemically synthesized genes for human insulin. Proc. Natl. Acad. Sci. 76: 106-110.
Goldberg AL, Goff SA (1986) Maximizing Gene Expression Reznikoff and Gold, eds.pp. 287 311, Butterworth Publishers, Stoneham, MD.
Guda C, Zhang X, McPherson DT, Xu J, Cherry J, Urry DW, Daniell H (1995) Hyperexpression of an environmentally friendly synthetic gene. Biotechnol Lett 17:745-750.
B, Daniell H (2000) Stable expression of biodegradable protein based polymer in tobacco chloroplasts. Plant Cell Rep. 19: 257-262.
Gunby P (1978) Bacteria directed to produce insulin in test application of genetic code. J. Am. Med. Assoc. 240(16): 1697-1698.
Hall SS (1988) Invisible Frontiers-The Race to Synthesize a Human Gene. Atlantic Monthly Press, New York, NY.
Hancock R, Lehrer R (1998). Cationic peptides: a new source of antibiotics. TIBTECH 16: 82-88.
Hancock WW., Sayegh MH., Weiner HL et al. (1993) Oral,but not intravenous , alloantigen prevents accelerated allograft rejection by selective intragraft Th2 cell cativation.Transplantation 55:1112-18.
Haq T A, Mason HS, Clements JD, Arntzen C.et al. (1995) Oral immunization with a recombinant bacterial antigen produced in Transgenic plants. Science.268:714-716
Heifetz P. (2000) Genetic engineering of the chloroplast. Biochimie. 82: 655-666.
Henriques L and Daniell H. (2000) Expression of cholera toxin B subunit oligomers in transgenic tobacco chloroplasts. In review.
Herzog RW, Singh NK, Urry DW, Daniell H (1997) Synthesis of a protein based polymer (elastomer) gene in Aspergillus nidulans. Applied Microbiology & Biotechnology 47:368-372.
Higgs DC, Shapiro RS, Kindle KL, Stem DB (1999) Small cis-acting sequences that specify secondary structures in chloroplast mRNA are essential for RNA stability and translation 19(12): 8479-8491.
Holmgren J, Lycke N, Czerkinsky C (1993). Cholera toxin and cholera B subunit as oral-mucosal adjuvant and antigen vector systems. Vaccine. 11(12): 1179-84. Review.
Horton RM, Hunt HD, Ho SN, Pullen JK, Pease LR (1989) Engineering hybrid genes without the use of restriction enzymes: gene splicing by overlap extension. Gene 77: 61-68.
Hotz P, Guggenheim B, Schmid R (1972). Carbohydrates in pooled dental plaque. Caries Res. 6(2): 103-21.
Jacob L, Zasloff M (1994). Potential therapeutic applications of megainins and other antimicrobial agents of animal origin. Ciba Foundation Symposium 186: 197-223.
Khoury SJ.,Lider O, Weiner HL et al. (1990) Suppression of experimental auto immune encephalomyelitis by oral administration of myelin basic protein. Cell Immunol. 131:302-10
Id PS (1997). Protein disulfide isomerase as a regulator of chloroplast translational activation. Science 278: 1954-1957.
Kim J-S, Raines RT (1993) Ribonuclease S-peptide as a carrier in fusion proteins. Protein. Sci. 2:348-356.
Kota M, Daniell H, Varma S, Garczynski F, Gould F, Moar WJ (1999) Overexpression of the Bacillus thuringiensis Cry2A protein in chloroplasts confers resistance to plants against susceptible and Bt-resistant insects. Proc. Natl. Acad. Sci. USA, 96:1840.1845.
Kusnadi A, Nikolov Z, Howard J (1997). Porduction of Recombinant proteins in Transgenic plants: Practical considerations. Biotechnology and Bioengineering. 56 (5): 473-484
Laemmli UK (1970) Cleavage of structural proteins during the assembly of the head of bacteriophage T4. Nature 227:680-685.
Lebens M, Holmgren J.(1994) Mucosal vaccines based on the use of Cholera Toxin B subunit as immunogen and antigen carrier. Recombinant Vectors in Vaccine Development [Brown F (ed)]. 82: 215-227
Lee C, Levin A, Branton D (1987) Copper staining: A five-minute protein stain for sodium dodecyl sulfate-polyacrylamide gels. Anal Biochem 166:308-312.
Lee SB, Kwon H, Kwon S, Park S, Jeong M, Han S, Daniell H, Byun H (2000). Drought tolerance conferred by the yeast trehalose-6 phosphate synthase gene engineered via the chloroplast genome. In review.
Lipscombe M, Charles IG, Roberts M, Dougan G, Tite J, Fairweather NF (1991). Intranasal immunization using the B subunit of the Escherichia coli heat-labile toxin fused to an epitope of the Bordetella pertussis P.69 antigen. Mol. Microbiol. 5(6): 1385-1392.
Ma J, et al (1995) Generation and assembly of secretory antibodies in plants. Science 268: 716-719.
Ma J, Hitmak B, Wycoff K, Vine N, Charlegue D, Yu LI, Hein M, Lehner T (1998) Charaterization of a recombinant plant monoclonal secretory antibody and preventive immunotherapy in humans. Nature Medicine. 4(5): 601-606.
Ma S-W, Zhao D-L, Yin Z-Q, Mukherjee R, Singh B, Qin H-Y et al. (1997) Transgenic plants expressing autoantigens fed to mice to induce oral tolerance. Transgenic Res. 3:793-796
Marina CV et al. (1988) An Escherichia coli vector to express and purify foreign proteins by fusion to and separation from maltose binding protein. Gene 74:365-373.
Mason HS, Ball JM, Arntzen CJ. et al. (1996).Expression of Norwalk virus capsid protein in transgenic tobacco and potato and its oral immunogenicity in mice. Proc. Nat. Acad. Sci. USA; 93 :5335-40.
on HS, Lyons PC (1996). Application of transgenic plants as production systems for pharmaceuticals in ACS symposium series 647. Fuller et al eds., chapter 13, 196-204.
Mathiowitz E, Jacob JS, long YS, Carino GP, Chickering DE, Chaturvedi P, Santos CA, Vijayarahauau K, Montgomery S, Bassett M, Morrell C. (1997) Biologically erodable microspheres as potential oral drug delivery systems. Nature 386: 410-414.
McBride KE, Svab Z, Schaaf DJ, Hogen PS, Stalker DM, Maliga P (1995) Amplification of a chimeric Bacillus gene in chloroplasts leads to extraordinary level of an insecticidal protein in tobacco. Bio/technology 13:362-365.McKenzie SJ and Halsey JF.(1984) Cholera toxin B subunit as a carrier protein to stimulate a mucosal immune response.Journal of Immunology.133: 1818-24
McPherson DT, Morrow C., Mineham DJ, Wu J, Hunter E, Urry DW (1992) Production and purification of a recombinant elastomeric polypeptide, G-(VPGVG) 19-VPGV from Escherichia coli. Biotechnology Prog. 8:317-322.
McPherson DT, Xu J, Urry DW (1996) Product purification by reversible phase transition following Escherichia coli expression of genes encoding up to 251 repeats of the elastomeric pentapeptide GVGVP. Protein Expression and Purification 7:51-57.
Mekalanos JJ, SadoffJC (1979) Cholera vaccines :Fighting an ancient scourge. Science 265:1387-1389.
Meyer DE, Chilkoti A (1999) Purificaiton of recombinant protiens by fusion with thermally-responsive polypeptides. Nature Biotechnology 17:1112-1115.
Miller A, Weiner HL et al. (1992) Suppressor T cells generated by oral tolerization to myelin basic protein suppress both in vitro and in vivo immune responses by the release of transforming growth factor B after antigen specific triggering. Proc. Nat. Acad. Sci. USA 89: 421-5.
Mor TS, Palmer KE et al.(1998) Perspective: edible vaccines- a concept coming of age. Trends in Microbiology.6:449-453
Morton B. (1993). Chloroplast DNA Codon Use:Evidence for Selection at the psbA Locus Based on tRNA Availability. J Mol Evol 37: 273-280.
Morton B and Bernadette G. (2000). Codon usage in plastid genes is correlated with context, position within the gene, amino acid content. J Mol EvoL 50: 184-193.
Nashar TO, Amin T, Marcello A, Hirst TR (1993). Current progress in the development of the B subunits of cholera toxin and Escherichia coli heat-labile enterotoxin as carriers for the oral delivery of heterologous antigens and epitopes. Vaccine. 11(2): 235-40.
rier Y, Somerville C (1994). Targeting of the polyhydroxybutyrate biosynthetic pathway to the plastis of Arabidopsis thaliana results in high levels of polymer accumulation. Proc. Natl. Acad. Sci. 91: 12760-12764.
Nilsson J, Stahl S, Lundeberg J, Uhlen M, Nygren PA (1997) Affinity fusion strategies for detection, purification, and immobilization of recombinant proteins. Protein Expr. Purif. 11:1-16.
Oakly WG, Pyke DA, Taylor KW (1973) Biochemical basis of Diabetes. In "Diabetes and It's Management", pp. 1-14, 2nd. edition., Blackwell Scientific Publications, Osney Mead, Oxford.
Ong E et al. (1989) The cellulose-binding domains of celulases: tools for biotechnology. Trends Biotechnol. 7:239-243.
Peerenboom E (2000) Geman health minister calls time out for B. T. maize. Nature Biotechnology. 18: 374
Petridis D, Sapidou E, Calandranis J (1995). Computer-Aided process analysis and economic evaluation for biosynthetic human insulin production-A case study. Biotechnology and Bioengineering 48: 529-541.
Panchal T, Wycoff K and Daniell H. (2000). Expression of humanized antibody in transgenic tobacco chloroplasts. In review.
Prodromou C and Pearl LH (1992). Recursive PCR: a novel technique for total gene synthesis. Protein Engineering 5(8): 827-829.
Puchta H (2000) Removing selectable marker genes: taking the shortcut. Trends in Plant Science 5: 273-274.
Reulland E, Miginiac-Maslow M (1999). Regulation of chloroplast enzyme activities by thioredoxins: activation or relief from inhibition. Trends in Plant Science 4: 136-141.
Roy H (1989). Rubisco assembly: a model system for studying the mechanism of chaperonin action. Plant Cell. 1: 1035-1042.
Sambrook J, Fritch EF, Maniatis T (1989) Molecular cloning. Cold Spring Harbor Press, Cold Spring Harbor, New York.
Sayegh MH, Khoury SJ.,Weiner HL et al (1992) Induction of immunity and oral tolerance with polymorphic classII major histocompatibility complex allopeptides in the rat. Proc. Nat. Acad. Sci. USA; 89:7762-6
Schmidt M, Babu KR, Khanna N, Marten S, Rinas U (1999) Temperature induced production of recombinant human insulin in high density cultures of recombinant E.coli. Biotechnology 68: 71-83.
lasten D, Pang SZ, Hajdukiewicz PTJ, Staub JM, Nehra, NS (1999) Stable chloroplast transformation in potato: use of green fluorescent protein as a plastid marker. Plant Journal 19:209-216.
Smith DB, Johnson KS (1988) Single-step purification of polypeptides expressed in Escherichia coli as fusion with glutathione S-transferase. Gene 67:31-40.
Smith PA et al. (1998) A plasmid expression system for quantitative in vivo biotinylation of thioredoxin fusion proteins in Escherichia coli. Nucleic Acids Res. 26:1414-1420.
Smith MC, Furman TC, Ingolia TD, Pidgeon C (1988) Chelating peptide-immobilized metal ion affinity chromatography. J. Biol. Chem. 263:7211-7215.
Staub JM, Garcia B, Graves J, Hajdukiewicz PT, Hunter P, Nehra N, Paradkar V, Schlittler M, Carroll JA, Spatola L, Ward D, Ye G, Russell DA (2000). High-yield production of a human therapeutic protein in tobacco chloroplasts. Nat. Biotechnol.18(3): 333-338.
Steiner DF, Arquila ER, Lerner J, Martin DB (1978) Recombinant DNA Research. Diabetes. 27: 877-878.
Su X, Prestwood AK, McGraw RA (1992) Production of recombinant porcine tumor necrosis factor alpha in a novel E. coli expression system. Biotechniques 13:756-762.
Sun JB, Holmgren J, Czerkinsky C (1994) Cholera toxin B subunit: an efficient transmucosal carrier-delivery system for induction of peripheral immunological tolerance. Proc. Natl. Acad. Sci. USA. 91:10795-10799.
Sun JB, Rask C, Olsson T, Holmgren J, Czerkinsky C (1996) Treatment of experimental autoimmune encephalomyelitis by feeding myelin basic protien conjugated to cholera toxin B subunit. Proc. Natl. Acad. Sci. USA. 93:7196-7201.
Svab Z, Maliga P (1993) High frequency plastid transformation in tobacco by selection for a chimeric aadA gene. Proc Natl Acad Sci USA 90:913-917.
Thanavala,Y ,Yang Y, Lyons P. et al(1995) Immunogenicity of transgenic plant derived hepatitis B surface antigen.Proc. Nat. Acad. Sci. USA ; 92:3358-3361
Trentham DE., Weiner HL et al. (1993) Effects of oral administration of Type II collagen on rheumatoid arthritis. Science 261:1727-30
Tsao KW, deBarbieri B, Hanspeter M, Waugh DW (1996) A versatile plasmid expression vector for the production of biotinylated proteins by site-specific enzymatic modification in Escherichia coli. Gene 69:59-64.
Urry DW (1995) Elastic biomolecular machines. Scientific American. 272: 64-69.
Urry DW, Nicol A, Gowda DC, Hoban LD, McKee A, Williams T, Olsen DB, Cox BA (1993) Medical applications of bioelastic materials. In: Gebelein CG (ed), Biotechnological ers: Medical, Pharmaceutical and Industrial Applications, Technomic Publishing Co., Inc., Atlanta, GA, pp. 82-103.
Urry DW, McPherson J, Xu J, Gowda DC, Jing N, Parker TM, Daniell H, Guda C (1996) Protein Based Polymeric Materials (Synthesis and Properties) in "Polymeric Materials Encyclopedia", (Solomone ed.), vol. 9, pp. 2645-2699, CRC Press.
Urry DW, Nichol A, McPherson DT, Xu J, Shewry PR, Harris CM, Parker TM, Gowda DC (1994) Properties, preparations and applications of bioelastic materials. in "Handbook of Biomaterials and Applications", Merck Dekker, New York, NY.
Urry DW (1991) Thermally Driven Self-assembly, Molecular Structuring and Entropic Mechanisms in Elastomeric Polypeptides in "Molecular Conformation and BiologicalInteractions" (Balaram, P., and Ramasashan, S., Eds.), pp. 555-583, Indian Acad.of Sci., Bangalore, India.
Vierling E (1991). The roles of heat shock proteins in plants. Annu. Rev. Plant Physiol. Plant Mol. Biol. 42: 579-620.
Weiner HL., Mackin GA, Hafler DA et at (1993) Double blind plot trial of oral tolerization with myelin antigens in multiple sclerosis.Science 259: 1321-4.
Ye GN, Daniell H, Sanford JC (1990) Optimization of delivery of foreign DNA into higher plant chloroplasts. Plant Mol. Biol. 15:809-819.
Ye X, et al (2000). Engineering the provitamin A (β-carotene) biosynthetic pathway into (carotenoid-free) rice endosperm. Science 287: 303-305.
Yeh H, Ornstein-Goldstein N, Indik Z, Sheppard P, Anderson N, Rosenbloom J, Cicilia G, Yoon K, Rosenbloom J (1987) Sequence variation of bovine elastin mRNA due to alternative splicing. Collagen Related Res 7:235-247.
Zerges W (2000) Translation in chloroplasts. Biochimie 82: 583-601
Zhang X, Guda C, Datta R, Dute R. Urry DW, Daniell H (1995) Nuclear expression of an environmentally friendly synthetic protein-based polymer gene in tobacco cells. Biotechnol Lett 17:1279-1284.
Zhang X, Urry DW, Daniell H (1996) Expression of an environmentally friendly synthetic protein-based polymer gene in transgenic tobacco plants. Plant Cell Rep 16:174-179.
Zhang ZJ, Davidson L, Weiner HL.et al.(1991) Supression of diabetes in nonobese diabetic mice by oral administration of porcine insulin. Proc. Nat. Acad. Sci.USA 88:10252-10256.

### PRODUCTION OF HUMAN INSULIN IN TRANSGENIC TOBACCO

As the value of molecular farming is realized, investigations are being performed to develop plant based expression systems. Aside from being an environmentally friendly approach, chloroplast genetic engineering continually exceeds nuclear genetic engineering in total production of foreign proteins in plants. A recent publication from our lab (featured on the cover of Nature Biotechnology, January 2001) demonstrated foreign gene expression up to 46% of the total soluble protein in chloroplast transgenic plants. However, the full potential of this technology is yet to be realized for biopharmaceutical production. Our proposed investigation entails both maximizing proinsulin production in chloroplast transgenic plants and evaluating proposed modifications for future chloroplast foreign gene expression.

Vector construction to synthesize the Cholera toxin B (CTB) subunit fused to native human proinsulin was first completed. As described on page 36 of this proposal, standard molecular biological techniques were used to create the sequence for the fusion protein DNA from individual genes encoding CTB and native human proinsulin. The DNA encoding this fusion protein was then sub cloned into the chloroplast transformation vector (pLD).

Although the pLD vector contains all the necessary elements for chloroplast expression of the CTB-proinsulin fusion protein, an important goal of this investigation is to evaluate the elements of translation that maximize foreign protein production. Therefore, as proposed on pages 35-38, we have developed additional constructs, each with a different modification, to allow for both the optimization of CTB-proinsulin gene expression and evaluation of these modifications.

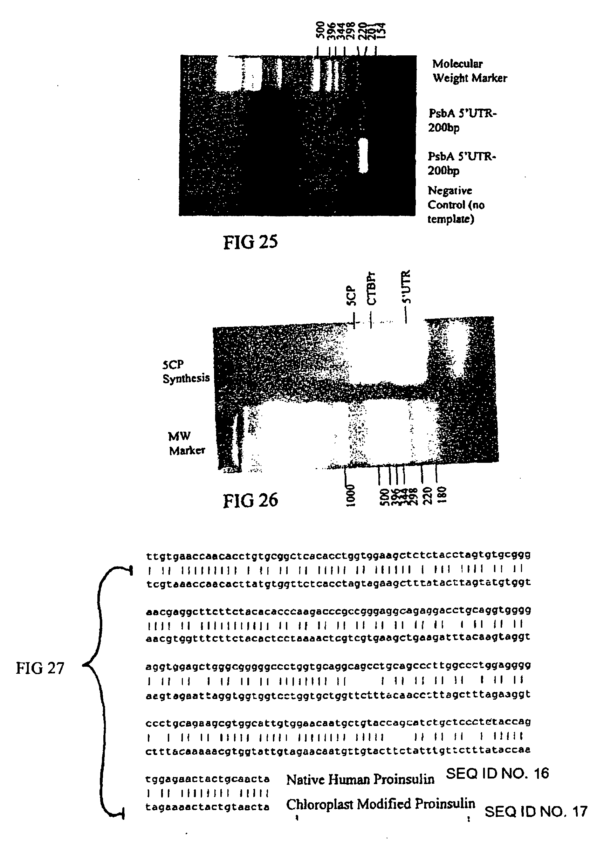

We have cloned the 5' untranslated region of the tobacco psbA gene including the promoter (5'UTR), shown in FIG. 25 and as proposed on page 36-37. We performed PCR using the primers CCQTCGACGTAGAGAAGTCCGTATT and GCCCATGGTAAAATCTTGG TTTATTTA, which resulted in a 200 base pair product, as expected. We inserted this PCR product into a TA cloning vector. Since restriction enzyme sites were not available to subclone the 5'UTR immediately upstream of the gene coding for the CTB-proinsulin fusion protein, we used the "SOEing" PCR technique, described on page 37, to create the DNA sequence with the 5'UTR immediately upstream of the CTB-proinsulin gene (FIG. 26). The products of this PCR include both the 5'UTR (200bp) and the gene for CTB-proinsulin (600bp) as additional products as well as the desired 3'UTR CTB-proinsulin (5CP) at 800 bp. 5CP was eluted and then inserted into the TA cloning vector where DNA sequencing was performed to confirm accuracy of nucleotide sequence before it was subcloned into the pLD vector.

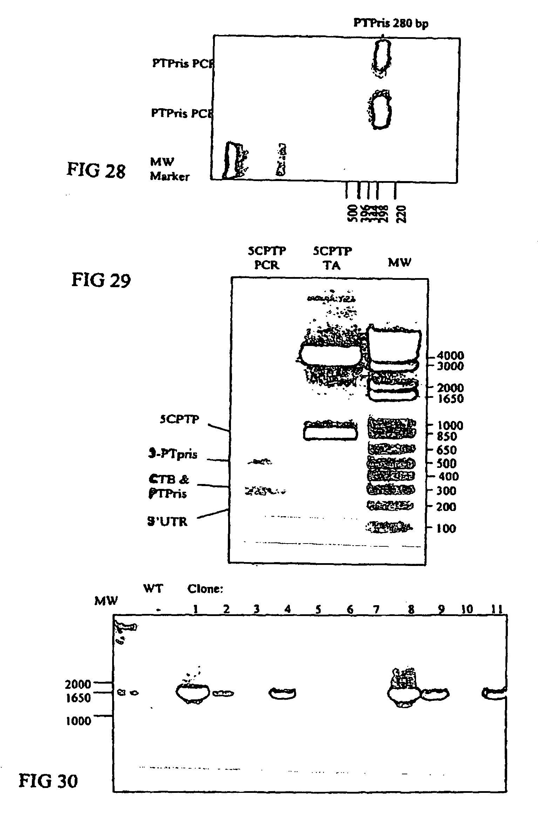

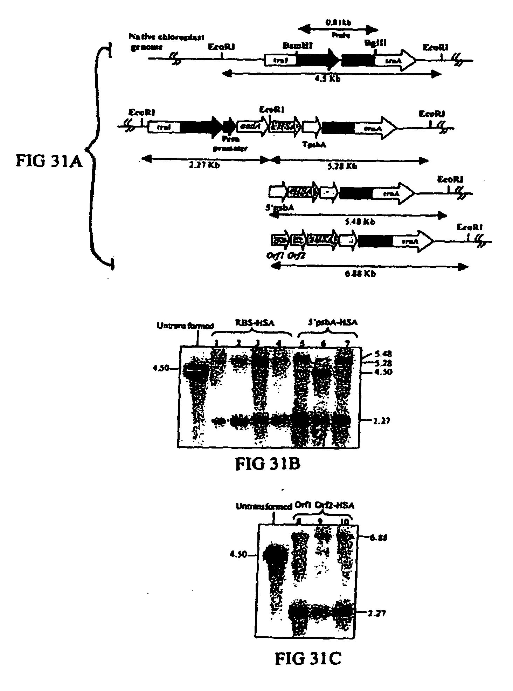

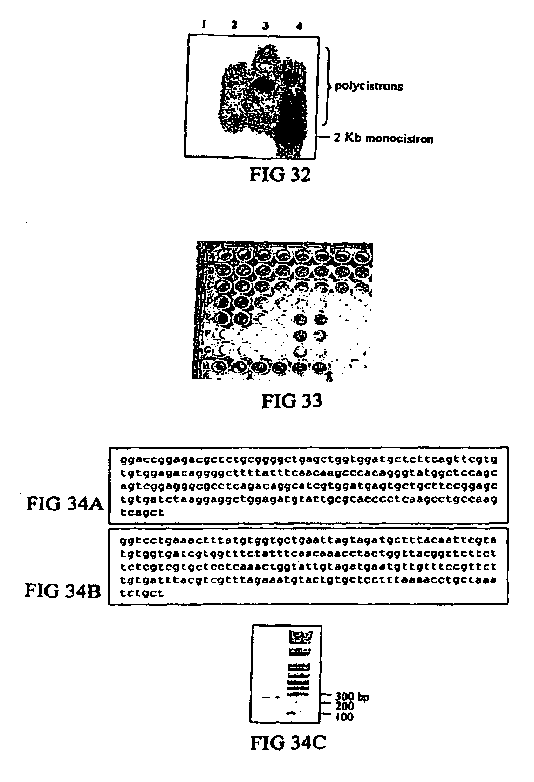

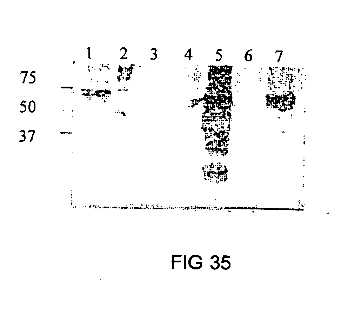

As discussed on page 35, chloroplast foreign gene expression correlates well with %AT of the gene coding sequence. The native human proinsulin sequence is 38% AT, while the newly synthesized chloroplast optimized proinsulin is 64% AT. We determined the optimal chloroplast coding sequence for the proinsulin (PTpris) gene by using a codon composition that is equivalent to the highest translated chloroplast gene, psbA. The preferred codon composition of psbA in tobacco is conserved within 20 vascular plant species. We have compared it to the native human proinsulin DNA sequence FIG. 27. Since there are too many changes for conventional mutagenesis, we employed the Recursive PCR method for total gene synthesis, as described on page 35. FIG. 28 shows the product of this gene synthesis corresponding to the 280 bp expected size.

This product, PTpria, then used as a template with CTB and 5'UTR to create a fusion of these sequences using the SOEing PCR technique described on page 37. The products of this reaction can be seen in FIG. 29. These include 5'UTR (200 bp), CTB (320 bp), Proinsulin (280 bp), and CTB-Proinsulin (600 bp) as side products, and also the desired 5'UTR CTB-PTpris bp), and CTB-Proinslin (600 bp) as side products, and also the desired 5'UTR CTB-PTpris (5CPTP) at 800 bp. This was then inserted into the TA cloning vector where the sequence was verified before being subcloned into the pLD vector.

Another parameter of foreign protein production to be investigates is post-translational. As discussed on page 37, the DNA for the putative chaperonin in the *Bacillus thuringiensis* Cry 2A2 operon encodes a protein that could potential fold and crystallize CTB-Proinsulin, which would allow it to accumulate in large quantities protected from chloroplast proteases and facilitate in subsequent purification. Standard molecular biology techniques were used to inset this DNA fragment immediately upstream of the 5'UTR of the construct containing the chloroplast optimized proinsulin. Additionally, another vector was constructed to contain only Shine-Dalgarno sequence (GGAGG) followed by the sequence encoding for the Cholera toxin B subunit and synthetic chloroplast optimized proinsulin fusion (CTB-PTpris). This construct will allow us to determine the value of the proinsulin sequence modification both with and without the 5'UTR*.*

All of the resulting vectors, containing the desired constructs, were used to transform both of the tobacco. cultivars, Petit Havana and LAMD 605 (edible tobacco). Transformation was performed using the particle bombardment method, as described on page 38-39. Bombarded leaves are currently being regenerated into transgenic plants under spectinomycin selection. Several clones have begun to form shoots. The clones of Petit Havana bombarded with the initial CTB-human proinsulin construct have regenerated large enough for us to extract DNA. Extracted DNA was used as a template in a PCR reaction to confirm integration of the cassette into the chloroplast genome by homologous recombination, as described on page 39. We used two primers in this reaction, 3P and 3M 3P anneals with the native chloroplast genome, while 3M anneals with the gene for spectinomycin resistance, *aadA*. The 1600 bp product of this reaction is indicative of integration of the construct into the genome (FIG. 30). The experiment demonstrated that 7 of the 11 analyzed clones were the desired chloroplast transgenic plants. Western blots are currently underway to confirm expression of various CTB-proinsulin fusion proteins in B coli. Because of the similarity of chloroplast and E. coli protein synthetic machinery, chloroplast vectors are routinely tested in our lab before bombardment. Membranes have been immunoblotted with antibodies to both CTB and Proinsulin. Results remonstrate the presence of the desired fusion proteins.

We eagerly await the regeneration of the remaining chloroplast transgenic plants. Our analysis of these plants will provide essential information to develop this technology for future biopharmaceutical production. Our investigation will also establish a method for production and delivery of orally administered protein therapies. With adequate production of the CTB-proinsulin fusion protein in the edible tobacco. plants, direct consumption of the plant tissue, as described on page 43, by NOD mice will prolong or prevent the onset of the autoimmune diabetes.

This project has already overcome initial experimental challenges by successfully constructing chloroplast vectors with different regulatory regions utilizing most challenging recombinant DNA techniques. Both native and codon optimized synthetic proinsulin genes have been inserted into chloroplast vectors. Our laboratory is quite efficient in carrying out subsequent steps to take this project to a successful completion. We look forward to NIH funding of this proposal to make rapid progress in proposed objectives.

### a. SPECIFIC AIMS

Research on human proteins in the past years has revolutionized the use of these therapeutically valuable proteins in a variety of clinical situations. Since the demand for these proteins is expected to increase considerably in the coming years, it would be wise to ensure that in the future they will be available in significantly larger amounts, preferably on a cost-effective basis. Because most genes can be expressed in many different systems, it is essential to determine which system offers the most advantages for the manufacture of the recombinant protein. The ideal expression system would be one that produces a maximum amount of safe, biologically active material at a minimum cost. The use of modified mammalian cells with recombinant DNA techniques has the advantage of resulting in products which are closely related to those of natural origin; however, culturing of these cells is intricate and can only be carried out on limited scale. The use of microorganisms such as bacteria permits manufacture on a larger scale, but introduces the disadvantage of producing products, which differ appreciably from the products of natural origin. For example, proteins that are usually glycosylated in humans are not glycosylated by bacteria. Furthermore, human proteins that are expressed at high levels in *E*. *coli* frequently acquire an unnatural conformation, accompanied by intracellular precipitation due to lack of proper folding and disulfide bridges. Production of recombinant proteins in plants has many potential advantages for generating biopharmaceuticals relevant to clinical medicine. These include the following: (I) plant systems are more economical than industrial facilities using fermentation systems; (ii) technology is available for harvesting and processing plants/ plant products on a large scale; (iii) elimination of the purification requirement when the plant tissue containing the recombinant protein is used as a food (edible vaccines); (iv) plants can be directed to target proteins into stable, intracellular compartments as chloroplasts, or expressed directly in chloroplasts; (v) the amount of recombinant product that can be produced approaches industrial-scale levels; and (vi) health risks due to contamination with potential human pathogens/toxins are minimized.

It has been estimated that one tobacco plant should be able to produce more recombinant protein than a 300-liter fermenter of *E*. *coli.* In addition, a tobacco plant produces a million seeds, facilitating large-scale production. Tobacco is also an ideal choice because of its relative ease of genetic manipulation and an impending need to explore alternate uses for this hazardous crop. However, with the exception of enzymes (e.g. phytase), levels of foreign proteins produced in nuclear transgenic plants are generally low, mostly less than 1% of the total soluble protein (1). May et al. (2a) discuss this problem using the following examples. Although plant derived epatitis B surface antigen was as effective as a commercial recombinant vaccine, the levels of expression in transgenic tobacco were low (0.0066% of total soluble protein). Even though Norwalk virus capsid protein expressed in potatoes caused oral immunization when consumed as food (edible vaccine), expression levels were low (0.3% of total soluble protein). In particular, expression of human proteins in nuclear transgenic plants has been disappointingly low: e.g. human Interferon-□ 0.000017% of fresh weight, human serum albumin 0.02% and erythropoietin 0.0026% of total soluble protein (see table1 in refl). A synthetic gene coding for the human epidermal growth factor was expressed only up to 0.001 % of total soluble protein in transgenic tobacco (2a). The cost of producing recombinant proteins in alfalfa leaves was estimated to be 12-fold lower than in potato tubers and comparable with seeds (1). However, tobacco leaves are much larger and have much higher biomass than alfalfa. The cost of production of recombinant proteins will be 50-fold lower than that of *E.coli* fermentation (with 20% expression levels, 1). A decrease in insulin expression from 20% to 5% of biomass doubled the cost of production (2b). Expression level less than 1% of total soluble protein in plants has been found to be not commercially feasible (1). Therefore, it is important to increase levels of expression of recombinant proteins in plants in order to exploit plant production of pharmacologically important proteins.

An alternate approach is to express foreign proteins in chloroplasts of higher plants. We have recently integrated foreign genes (up to 10,000 copies per cell) into the tobacco chloroplast genome resulting in accumulation of recombinant proteins up to 47% of the total cellular protein (3). Chloroplast transformation utilizes two flanking sequences that, through homologous recombination, insert foreign DNA into the spacer region between the functional genes of the chloroplast genome, thus targeting the foreign genes to a precise location. This eliminates the "position effect" and gene silencing frequently observed in nuclear transgenic plants. Chloroplast genetic engineering is an environmentally friendly approach, minimizing concerns of out-cross of introduced traits via pollen to weeds or other crops. Also, the concerns of insects developing resistance to biopesticides are minimized by hyper-expression of single insecticidal proteins (high dosage) or expression of different types of insecticides in a single transformation event (gene pyramiding). Concerns of insecticidal proteins on non-target insects are minimized by lack of expression in transgenic pollen. Most importantly, a significant advantage in the production of pharmaceutical proteins in chloroplasts is their ability to process eukaryotic proteins, including folding and formation of disulfide bridges (4). Chaperonin proteins are present in chloroplasts (5,6) that function in folding and assembly of prokaryotic/eukaryotic proteins. Also, proteins are activated by disulfide bond oxido/reduction cycles using the oredoxin system (7) or chloroplast protein disulfide isomerase (8). Accumulation of fully assembled, disulfide bonded form of human somatotropin via chloroplast transformation (9) and oligomeric form of CTB (10) and assembly of heavy and light chains of humanized Guy's 13 antibody in transgenic chloroplasts (11) provide strong evidence for successful processing of pharmaceutical proteins inside chloroplasts. Such folding and assembly should eliminate the need for highly expensive *in vitro* processing of pharmaceutical proteins. For example, 60% of the total operating cost in the production of human insulin is associated with in *vitro* processing (formation of disufide bridges and cleavage of methionine)(2b).

Taken together, low levels of expression of human proteins in nuclear transgenic plants, and difficultly in folding, assembly/processing of human proteins in *E.coli* should make chloroplasts an ideal compartment for expression of these proteins; production of human proteins in transgenic chloroplasts should also dramatically lower the production cost. Large-scale production of these proteins in plants should be a powerful approach to provide treatment to patients at an affordable cost and provide tobacco farmers alternate uses for this hazardous crop. Therefore, we propose here expression of therapeutic proteins in transgenic tobacco chloroplasts to increase levels of expression and accomplish *in vivo* processing.

### Objectives

a) Develop recombinant DNA vectors for enhanced expression of Human Serum Albumin, Insulin like growth factor I and Interferon-□ 2 and 5, via chloroplast genomes of tobacco
b) Optimize processing and purification of pharmaceutical proteins using chloroplast vectors in *E. coli*
c) Obtain transgenic tobacco plants
d) Characterize transgenic expression of proteins or fusion proteins using molecular and biochemical methods in chloroplasts
e) Employ existing or modified methods of purification from transgenic leaves
f) Analyze Mendelian or maternal inheritance of transgenic plants
g) Large scale purification of therapeutic proteins from transgenic tobacco and comparison of current purification methods in *E.coli* or yeast
h) Compare natural refolding in chloroplasts with existing *in vitro* processing methods
i) Comparison/characterization (yield and purity) of therapeutic proteins produced in yeast or *E.coli* with transgenic tobacco chloroplasts
j) *In vitro* and *in vivo* (pre-clinical trials) studies of protein biofunctionality.

### CHLOROPLAST GENETIC ENGINEERING

When we developed the concept of chloroplast genetic engineering (72,73), it was possible to introduce isolated intact chloroplasts into protoplasts and regenerate transgenic plants (74). Therefore, early investigations on chloroplast transformation focused on the development of in organello systems using intact chloroplasts capable of efficient and prolonged transcription and translation (75-77) and expression of foreign *genes* in isolated chloroplasts (78). However, after the discovery of the gene gun as a transformation device (79), it was possible to transform plant chloroplasts without the use of isolated plastids and protoplasts. Chloroplast genetic engineering was accomplished in several phases. Transient expression of foreign genes in plastids of dicots (80,81) was followed by such studies in monocots (82). Unique to the chloroplast genetic engineering is the development of a foreign gene expression system using autonomously replicating chloroplast expression vectors (80). Stable integration of a selectable marker gene into the tobacco chloroplast genome (83) was also accomplished using the gene gun. However, useful genes conferring valuable traits via chloroplast genetic engineering have been demonstrated only recently. For example, plants resistant to B.t. sensitive insects were obtained by integrating the *cryIAc* gene into the tobacco chloroplast genome (84). Plants resistant to B.t. resistant insects (up to 40,000 fold) were obtained by hyper-expression of the *cry2A* gene within the tobacco chloroplast genome (85). Plants have also been genetically engineered via the chloroplast genome to confer herbicide resistance and the introduced foreign genes were maternally inherited, overcoming the problem of out-cross with weeds (86). Chloroplast genetic engineering technology is currently being applied to other useful crops (73,87).

### c. PRELIMINARY STUDIES

A remarkable feature of chloroplast genetic engineering is the observation of exceptionally large accumulation of foreign proteins in transgenic plants, as much as 46% of CRY protein in total soluble protein, even in bleached old leaves (3). Stable expression of a pharmaceutical protein in chloroplasts was first reported for GVGVP, a protein based polymer with varied medical applications (such as the prevention of post-surgical adhesions and scars, wound coverings, artificial pericardia, tissue reconstruction and programmed drug delivery (88)). Subsequently, expression of the human somatotropin via the tobacco chloroplast genome (9) to of total soluble protein) was observed. The following investigations that are in progress in the Daniell laboratory illustrate the power of this technology to express small peptides, entire operons, vaccines that require oligomeric proteins with stable disulfide bridges and monoclonals that require assembly of heavy/light chains via chaperonins.

**Engineering novel pathways via the chloroplast genome:** In plant and animal cells, nuclear mRNAs are translated monocistronically. This poses a serious problem when engineering multiple genes in plants (91). Therefore, in order to express the polyhydroxybutyrate polymer or Guy's 13 antibody, single genes were first introduced into individual transgenic plants, then these plants were back-crossed to reconstitute the entire pathway or the complete protein (92,93). Similarly, in a seven year long effort, Ye et al. (81) recently introduced a set of three genes for a short biosynthetic pathway that resulted in β-carotene expression in rice. In contrast, most chloroplast genes of higher plants are cotranscribed (91). Expression of polycistrons via the chloroplast genome provides a unique opportunity to express entire pathways in a single transformation event. We have recently used the *Bacillus thuringiensis* (Bt) *cry*2Aa2 operon as a model system to demonstrate operon expression and crystal formation via the chloroplast genome (3). *Cry*2Aa2 is the distal gene of a three-gene operon. The *orf* immediately upstream of *cry*2Aa2 codes for a putative chaperonin that facilitates the folding of *cry2*Aa*2* (and other proteins) to form proteolytically stable cuboidal crystals (94).

, the *cry2*Aa2 bacterial operen was expressed in tobacco chloroplasts to test the resultant transgenic plants for increased expression and improved persistence of the accumulated insecticidal proteins(s). Stable foreign gene integration was confirmed by PCR and Southern blot analysis in T₀ and T₁ transgenic plants. *Cry2Aa2* operon derived protein accumulated at 45.3% , of the total soluble protein in mature leaves and remained stable even in old bleached leaves (46.1%) (FIG. 4). This is the highest level of foreign gene expression ever reported in transgenic plants. Exceedingly difficult to control insects (10-day old cotton bollworm, beetarmy worm) were killed 100% after consuming transgenic leaves. Electron micrographs showed the presence of the insecticidal protein folded into cuboidal cuboidal crystals similar shape to *Cry2Aa*2 crystals observed in *Bacilllus thuringiensis* (FIG. 5). In contrast to currently marketed transgenic plants with soluble CRY proteins, folded protoxin crystals will be processed only by target insects that have alkaline gut pH; this approach should improve safety of Bt transgenic plants. Absence of insecticidal proteins in transgenic pollen eliminate toxicity to non-target insects via pollen, In addition to these environmentally friendly approaches, this observation should serve as a model system for large-scale production of foreign proteins within chloroplasts in a folded configuration enhancing their stability and facilitating single step purification. This is the first demonstration of expression of a bacterial operon in transgenic plants and opens the door to engineer novel pathways in plants in a single transformation event.
Expressing small peptides via the chloroplast genome: It is common knowledge that the medical community has been fighting a vigorous battle against drug resistant pathogenic bacteria for years. Cationic antibacterial peptides from mammals, amphibians and insects have gained more attention over the last decade (95). Key features of these cationic peptides are a net positive charge, an affinity for negatively-charged prokaryotic membrane phospholipids over neutral-charged eukaryotic membranes and the ability to form aggregates that disrupt the bacterial membrane (96).

There are three major peptides with α-helical structures, cecropin from *Hyalophora cecropia* (giant silk moth), magainins from *Xenopus laevis* (African frog) and defensins from mammalian neutrophils. Magainin and its analogues have been studied as a broad-spectum topical agent, a systemic antibiotic; a wound-healing stimulant; and an anticancer agent (97). We have recently observed that a synthetic lytic peptide (MSI.99, 22 amino acids) can be successfully expressed in tobacco chloroplast (98). The peptide retained its lytic activity against the phytopathogenic bacteria *Pseudomonas syringae* and multidrug resistant human pathogen, *aeruginosa.* The anti-microbial peptide (AMP) used in this study was an amphipathic alpha-helix molecule that has an affinity for negatively charged phospholipids commonly found in the outer-membrane of bacteria. Upon contact with these membranes, individual peptides aggregate to form pores in the membrane, resulting in bacterial lysis. Because of the concentration dependent action of the AMP, it was expressed via the chloroplast genome to accomplish high dose delivery at the point of infection. PCR products and Southern blots confirmed chloroplast integration of the foreign genes and homoplasmy. Growth and development of the transgenic plants was unaffected by hyper-expression of the AMP within chloroplasts. *In* vitro assays with T₀ and T₁ plants confirmed that the AMP was expressed at high levels (21.5 to 43% of the total soluble protein) and retained biological activity against *Pseudomonas syringae,* a major plant pathogen. *In situ* assays resulted in intense areas of necrosis around the point of infection in control leaves, while transformed leaves showed no signs of necrosis (200-800 µg of AMP at the site of infection)(FIG. 6) T₁ *in vitro* assays against *Pseudomonas aeruginosa* (a multi-drug resistant human pathogen) displayed a 96% inhibition of growth (FIG. 7). These results give a new option in the battle against phytopathogenic and drug-resistant human pathogenic bacteria. Small peptides (like insulin) are degraded in most organisms. However, stability of this AMP in chloroplasts opens up this compartment for expression of hormones and other small peptides.

**is that are cleaved in chloroplast:** Staub et al. (9) reported chloroplast expression of human somatotropin similar to the native human protein by using ubiquitin fusions that were cleaved in the stroma by an ubiquitin protease. However, the processing efficiency ranged from 30-80% and the cleavage site was not accurate. In order to process chloroplast expressed proteins a peptide which is cleaved in the stroma is essential. The transit peptide sequence of the RuBisCo (ribulose 1,5-bisphosphate carboxylase) small subunit would be an ideal choice. This transit peptide has been studied in depth (111). RuBisCo is one of the proteins that is synthesized in cytoplasm and transported postranslationally into the chloroplast in an energy dependent process. The transit peptide is proteolytically removed upon transport in the stroma by the stromal processing peptidase (112). There are several sequences described for different species (113). A transit peptide consensus sequence for the RuBisCo small subunit of vascular plants is published by Keegstra et al. (114). The amino acids that are proximal to the C-terminal (41-59) are highly conserved in the higher plant transit sequences and belong to the domain which is involved in enzymatic cleavage (111). The RuBisCo small subunit transit peptide has been fused with various marker proteins (114,115), even with animal proteins (116,117), to target proteins to the chloroplast. Prior to transformation studies, the cleavage efficiency and accuracy will be tested by *in vitro* translation of the fusion proteins and *in organello* import studies using intact chloroplasts. Once we know the correct fusion sequence for producing the mature protein, such sequence encoding the amino terminal portion of tobacco chloroplast transit peptide will be linked with the mature sequence of each protein. Codon composition of the tobacco RuBisCo small subunit transit peptide appears to be compatible with chloroplast optimal translation (see section d3 and table 1 on page 30). Additional transit peptide sequences for targeting and cleavage in the chloroplast have been described (111). If we found that the RuBisCo small subunit transit peptide is not suitable, other transit peptides with cleavage in stroma will be studied. The lumen of thylakoids could be a good target because thylakoids are easy to purify. It is relatively easy to free lumenal proteins either by sonication or with a very low triton X100 concentration. However, this may require insertion of additional amino acid sequences for efficient import (111).
**Use of chemical or enzymatic cleavage:** The strategy of fusing a protein to a tag with affinity for a certain ligand has been used extensively for more than a decade to enable affinity purification of recombinant products (118-120). A vast number of cleavage methods, both chemical and enzymatic, have been investigated for this purpose (120). Chemical cleavage methods have low specificity and the relatively harsh cleavage conditions can result in chemical modifications of the released products (120). Some of the enzymatic methods offer significantly specificities together with high efficiency, e. g. H64A subtilisin, IgA protease and factor Xa (119,120), but these enzymes have the drawback of being quite expensive.

Trypsin, which cleaves C-terminal of basic amino-acid residues, has been used for a long time to cleave fusion proteins (14,121). Despite expected low specificity, trypsin has been shown to be useful for specific cleavage of fusion proteins, leaving basic residues within folded protein domains uncleavaged (121). The use of trypsin only requires that the N-terminus of the mature protein be accessible to the protease and that the potential internal sites are protected in the native conformation. Trypsin has the additional advantage of being inexpensive and readily available. In the case of HSA, when it was expressed in *E*. *coli* with 6 additional codons coding for a trypsin cleavage site, HSA was processed successfully into the mature protein after treatment with the protease. In addition, the N-terminal sequence was found to be unique and identical to the sequence of natural HSA, the conversion was complete and no degradation products were observed (14). This *in vitro* maturation is selective because correctly folded albumin is highly resistant to trypsin cleavage at inner sites (14). This system could be tested for chloroplasts HSA vectors using protein expressed in *E*. *coli.*

Staub et al; (9) demonstrated that the chloroplast methionine aminopeptidase is active and they found 95% of removal of the first methionine of an ATG-somatotropin protein that was expressed via the chloroplast genome. There are several investigations that have shown a very strict pattern of cleavage by this peptidase (122). Methionine is only removed when second residues are glycine, alanine, serine, cysteine, threonine, proline or valine, but if the third amino acid is proline the cleavage is inhibited. In the expression of our three proteins we could use this approach to obtain the mature protein in the case of Interferon because the penultimate aminoacid is cystein followed by aspartic acid. For HSA the second aminoacid is aspartic acid and for IGF-I glycine but it is followed by proline, so the cleavage may not be possible.

For IGF-I expression, the use of the TEV protease (Gibco cat n 10127-017) would be ideal. The cleavage site that is recognized for this protease is Glu-Asn-Leu-Tyr-Phe-Gln-Gly and it cuts between Gln-Gly. This strategy would allow the release of the mature protein by incubation with TEV protease leaving a glycine as the first amino acid consistent with human mature IGF-I protein.

In the *E. coli* Interferon-□5 expression method developed in Dr. Prieto's laboratory (sec section C), the purification system was based on 6 Histidine-tags that bind to a nickel column and biotinylated thrombin to eliminate the tag on IFN-□5. Thrombin recognizes Leu-Val-Pro-Arg-Gly-Ser and cuts between Arg and Gly. This would leave two extra amino acids in the mature protein, but antiviral activity studies have been done showing that this protein is at least as active as commercial IFN-□2 (unpublished data in Dr. Prieto's laboratory).
**d.3 Optimization of gene expression:** We have reported that foreign genes are expressed between 3% *(cry2Aa2)* and 47% *(cry2Aa2* operon) in transgenic chloroplasts (3,85). Based on the outcome of the evaluation of HSA chloroplast transgenic plants, several approaches will be used to enhance translation of the recombinant proteins. In chloroplasts, transcriptional regulation of gene expression is less important, although some modulations by light and developmental conditions are observed (123). RNA stability appears to be one among the least problems because of observation of excessive accumulation of foreign transcripts, at times 16,966-fold higher than the highly expressing nuclear transgenic plants (124). Chloroplast gene expression is regulated to a large extent at the post-transcriptional level. For example, 5' UTRs are necessary for optimal translation of chloroplast mRNAs. Shine-Dalgarno (GGAGG) sequences as well as a stem-loop structure located 5' adjacent to the SD sequence are required for efficient translation. A recent study has shown that insertion of the psbA 5' UTR downstream of the 16S rRNA promoter enhanced translation of a foreign gene (GUS) hundred-fold (125a). Therefore, the 200-bp tobacco chloroplast DNA fragment (1680-1480) containing 5' psbA UTR will be used. This PCR product will be inserted downstream of the 16S rRNA promoter to enhance translation of the recombinant proteins.

Yet another approach for enhancement of translation would be to optimize codon compositions. Since all the three proteins are translated in *E. coli* (see section b), it would be reasonable to expect efficient expression in chloroplasts. However, optimizing codon compositions to match the psbA gene could further enhance the level of translation. Although rbcL (RuBisCO) is the most abundant protein on earth, it is not translated as highly as the psbA gene due to the extremely high turnover of the psbA gene product. The psbA gene is under stronger selection for increased translation efficiency and is the most abundant thylakoid protein. In addition, the codon usage in higher plant chloroplasts is biased towards the NNC codon of 2-fold degenerate groups (i.e. TTC over TTT, GAC over GAT, CAC over CAT, AAC over AAT, ATC over ATT, ATA etc.). This is in addition to a strong bias towards T at third position of 4-fold degenerate groups. There is also a contest effect that should be taken into consideration while modifying specific codons. The 2-fold degenerate sites immediately upstream from a GNN codon do not show this bias towards NNC. (TTT GGA is preferred to TTC GGA while TTC CGT is preferred to TTT CGT, TTC AGT to TTT AGT and TTC TCT to TTT TCT)(125b,126). In addition, highly expressed chloroplast genes use GNN more frequently that other genes. The web site http://www.kazusa.or.ip/codon will be used to optimize codon composition by comparing different species. Abundance of amino acids in chloroplasts and tRNA anticodons present in chloroplast will be taken into consideration. We also compared of all foreign genes that had been expressed in transgenic chloroplasts in our ' laboratory with the percentage of chloroplast expression. We found that higher levels of A+T always correlated with high expression levels (see table I on page 30). It is also possible to modify chloroplast protease recognition sites while modifying codons, without affecting their biological functions.

The study of the sequences of HSA, IGF-I and Interferon-□5 was done. The HSA sequence showed 57% of A+T content and 40% of the total codons matched with the psbA most translated codons. According to the data of table 1, we should expect good chloroplast expression of the HSA gene without any modifications in its codon composition. IFN-□5 has 54% of A+T content and 40% of matching with psbA codons. The composition seems to be good but this protein is small (166 amino acids) and it would be easy to optimize the sequence to achieve A+T levels close to 65%. Finally, the analysis of the IGF-I sequence showed that the A+T content was 40% and only 20% of the codons are the most translated in psbA. Therefore, this gene needs to be optimized. Optimization of these two genes will be done using a novel PCR approach (127,128) which has been successfully used in our laboratory to optimize codon composition of other human proteins.
d.4 Vector constructions: For all the constructs pLD vector will be used. This vector was developed in this laboratory for chloroplast transformation. It contains the 16S rRNA promoter (Prm) driving the selectable marker gene *aadA* (aminoglycoside adenyl transferase conferring resistance to spectinomycin) followed by the *psbA* 3' region (the terminator from a gene coding for photosystem **II** reaction center components) from the tobacco chloroplast genome. The pLD vector is a universal chloroplast expression /integration vector and can be used to transform chloroplast genomes of several other plant species (73,86) because these flanking sequences are highly conserved among higher plants. The universal vector uses *trnA* and *trnI* genes (chloroplast transfer RNAs coding for Alanine and Isoleucine) from the inverted repeat region of the tobacco chloroplast genome as flanking sequences for homologous recombination. Because the universal vector integrates foreign genes within the Inverted Repeat region of the chloroplast genome, it should double the copy number of the transgene (from 5000 to 10,000 copies per cell in tobacco). Furthermore, it has been demonstrated that homoplasmy is achieved even in the first round of selection in tobacco probably because of the presence of a chloroplast origin of replication within the flanking sequence in the universal vector (thereby providing more templates for integration). Because of these and several other reasons, foreign gene expression was shown to be much higher when the universal vector was used instead of the tobacco specific vector (88).
We will design the following vectors to optimize protein expression, purification and production of proteins with the same amino acid composition as in human proteins.
a) In order to optimize expression we will increase translation using the psbA 5'UTR (see section d.3) and optimizing the codon composition for protein expression in chloroplasts according to criteria discussed in section d.3. The 200 bp tobacco chloroplast DNA fragment containing 5' psbA UTR will be amplified by PCR using tobacco chloroplast DNA as template. This fragment will be cloned directly in the pLD vector multiple cloning site (EcoRl-Ncol) downstream of the promoter and the aadA gene. The cloned sequence will be exactly the same as in the psbA gene.
b) For enhancing protein stability and facilitating purification, the *cry2Aa2 Bacillus thuringiensis* operon derived putative chaperonin will be used. Expression of the *cry2Aa2* operon in chloroplasts provides a model system for hyper-expression of foreign proteins (46% of total soluble protein) in a folded configuration enhancing their stability and facilitating purification (3). This justifies inclusion of the putative chaperonin from the *cry2Aa2* operon in one of the newly designed constructs. In this region there are two open reading frames (ORF1 and ORF2) and a ribosomal binding site (rbs). This sequence contains elements necessary for *Cry2Aa2* crystallization which may help to crystallize the HSA, IGF-I and IFN-□ proteins aiding in the subsequent purification. Successful crystallization of other proteins using this putative chaperonin has been demonstrated (94). We will amplify the ORF1 and ORF2 of the Bt *Cry2Aa2* operon by PCR using the complete operon as template. The fragment will be cloned into a PCR 2.1 vector and excised as an EcoRI-EcoRV product. This fragment will be cloned directly into the pLD vector multiple cloning site (EcoRI-EcoRV) downstream of the promoter and the aadA gene.
c) To obtain proteins with the same amino acid composition as mature human proteins, we will first fuse all three genes (codon optimized and native sequence) with the RuBisCo small subunit transit peptide. Also other constructions will be done to allow cleavage of the protein after isolation from chloroplast (see section d.2). These strategies would also allow affinity purification of the proteins.

The first set of constructs will include the sequence of each protein beginning with an ATG, introduced by PCR using primers. Once we achieve optimal expression levels, and if the ATG is problem (determined by mice immunological assays), processing to get the mature protein will be addressed. The first attempt will be the use of the RuBisCo small subunit transit peptide. This transit peptide will be amplified by PCR using tobacco DNA as template and cloned into the PCR 2.1 vector. All genes will be fused with the transit peptide using a MluI restriction site that will be introduced in the PCR primers for amplification of the transit peptide and genes coding for three proteins. The gene fusions will be inserted into the pLD vectors downstream of the 5'UTR or ORF1+2 using the restriction sites NcoI and EcoRV respectively. If use of tags or protease sequences is necessary, such sequences will be introduced by designing primers including these sequences and amplifying the gene with PCR After completing vector constructions, all the vectors will be sequenced to confirm correct nucleotide sequence and in frame fusion. DNA sequencing will be done using a Perkin Elmer ABI prism 373 DNA sequencing system.

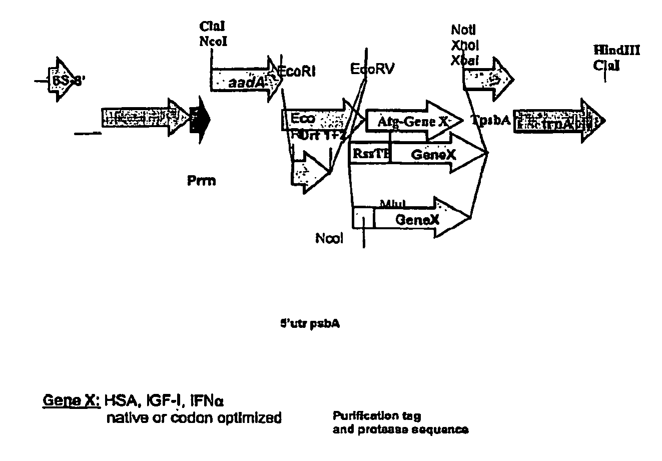

Because of the similarity of protein synthetic machinery (109), expression of all chloroplast vectors will be first tested in *E.coli* before their use in tobacco transformation. For *Escherichia coli* expression XL-1 Blue strain will be used. *E coli* will be transformed by standard CaCl₂ transformation procedures and grown in TB culture media. Purification, biological and immunogenic assays will be done using *E. coli* expressed proteins.
**d.5 Bombardment, Regeneration and Characterization of Chloroplast Transgenic Plants:** Tobacco *(Nicotiana tabacum* var. Petit Havana) plants will be grown aseptically by germination SO medium. This medium contains MS salts (4.3 g/liter), B5 vitamin mixture (myo-inositol, 100 mg/liter, thiamine-HCl, 10 mg/liter, nicotinic acid, 1 mg/liter; pyridoxine-HCl, 1 mg/liter), sucrose (30 g/liter) and phytagar (6 g/liter) at pH 5.8. Fully expanded, dark green leaves of about two month old plants will be used for bombardment

Leaves will be placed abaxial side up on a Whatman No. 1 filter paper laying on the RMOP medium (79) in standard petri plates (100x15 mm) for bombardment. Gold (0.6 µm) microprojectiles will be coated with plasmid DNA (chloroplast vectors) and bombardments will be carried out with the biolistic device PDS1000/He (Bio-Rad) as described by Daniell (110). Following bombardment, petri plates will be sealed with parafilm and incubated at 24°C under 12 h photoperiod. Two days after bombardment, leaves will be chopped into small pieces of ∼5 mm² in size and placed on the selection medium (RMOP containing 500 µg/ml of spectinomycin dihydrochloride) with abaxial side touching the medium in deep (100x25 mm) petri plates (-10 pieces per plate). The regenerated spectinomycin resistant shoots will be chopped into small pieces (∼2mm²) and subcloned into fresh deep petri plates (∼5 pieces per plate) containing the same selection medium. Resistant shoots from the second culture cycle will be transferred to the rooting medium (MSO medium supplemented with IBA, 1 mg/liter and spectinomycin dihydrochloride, 500 mg/liter). Rooted plants will be transferred to soil and grown at 26°C under 16 hour photoperiod conditions for further analysis.
**PCR analysis of putative transformants**: PCR will be done using DNA isolated from control and transgenic plants in order to distinguish a) true chloroplast transformants from mutants and b) chloroplast transformants from nuclear transformants. Primers for testing the presence of the aadA gene (that confers spectinomycin resistance) in transgenic plants will be landed on the aadA coding sequence and 16S rRNA gene. In order to test chloroplast integration of the genes, one primer will land on the aadA gene while another will land on the native chloroplast genome. No PCR product will be obtained with nuclear transgenic plants using this set of primers. The primer set will be used to test integration of the entire gene cassette without any internal deletion or looping out during homologous recombination. Similar strategy has been used successfully by us to confirm chloroplast integration of foreign genes (3,85-88). This screening is essential to eliminate mutants and nuclear transformants. In order to conduct PCR analyses in transgenic plants, total DNA from unbombarded and transgenic plants will be isolated as described by Edwards et al. (129). Chloroplast transgenic plants containing the desired gene will be moved to second round of selection in order to achieve homoplasmy.
**Southern Analysis for homoplasmy and copy number:** Southern blots will be done to copy number of the introduced foreign gene per cell as well as to test homoplasmy. There are several thousand copies of the chloroplast genome present in each plant cell. Therefore, when foreign genes are inserted into the chloroplast genome, it is possible that some of the chloroplast genomes have foreign genes integrated while others remain as the wild type (heteroplasmy). Therefore, in order to ensure that only the transformed genome exists in cells of transgenic plants (homoplasmy), the selection process will be continued. In order to confirm that the wild type genome does not exist at the end of the selection cycle, total DNA from transgenic plants should be probed with the chloroplast border (flanking) sequences (the trnI-trnA fragment). If wild type genomes are present (heteroplasmy), the native fragment size will be observed along with transformed genomes. Presence of a large fragment (due to insertion of foreign genes within the flanking sequences) and absence of the native small fragment should confirm homoplasmy (85,86,88).

The copy number of the integrated gene will be determined by establishing homoplasmy for the transgenic chloroplast genome. Tobacco chloroplasts contain 5000∼10,000 copies of their genome per cell (86). If only a fraction of the genomes are actually transformed, the copy number, by default, must be less than 10,000. By establishing that in the transgenics the gene inserted transformed genome is the only one present, one could establish that the copy number is 5000~10,000 per cell. This is usually done by digesting the total DNA with a suitable restriction enzyme and probing with the flanking sequences that enable homologous recombination into the chloroplast genome. The native fragment present in the control should be absent in the transgenics. The absence of native fragment proves that only the Transgenic chloroplast genome is present in the cell and there is no native, untransformed, chloroplast genome, without the foreign gene present This establishes the homoplasmic nature of our transformants, simultaneously providing us with an estimate of 5000∼10,000 copies of the foreign genes per cell.
**Northern Analysis for transcript stability**: Northern blots will be done to test the efficiency of transcription of the genes. Total RNA will be isolated from 150 mg of frozen leaves by using the "Rneasy Plant Total RNA Isolation Kit" (Qiagen Inc., Chatsworth, CA). RNA (10-40 µg) will be denatured by formaldehyde treatment, separated on a 1,2% agarose gel in the presence of formaldehyde and transferred to a nitrocellulose membrane (MSI) as described in Sambrook et al. (130). Probe DNA (proinsulin gene coding region) will be labeled by the random-primed method (Promega) with ³²P-dCTP isotope. The blot will be pre-hybridized, hybridized and washed as described above for southern blot analysis. Transcript levels will be quantified by the Molecular Analyst Program using the GS-700 Imaging Densitometer (Bio-Rad, Hercules, CA).
**Expression and quantification of the total protein expressed in chloroplast:** Chloroplast expression assays will be done for each protein by Western Blot. Recombinant protein levels in transgenic plants will be determined using quantitative ELISA assays. A standard curve will be generated using known concentrations and serial dilutions of recombinant and native proteins. Different tissues will be analyzed using young, mature and old leaves against these primary antibodies: goat anti-HSA (Nordic Immunology), anti-IGF-I and anti-Interferon alpha (Sigma). Bound IgG will be measured using horseradish peroxidase-labelled anti-goat IgG.
**Inheritance of Introduced Foreign Genes:** While it is unlikely that introduced DNA would move from the chloroplast genome to nuclear genome, it is possible that the gene could get integrated in the nuclear genome during bombardment and remain undetected in Southern analysis. Therefore, in initial tobacco transformants, some will be allowed to self-pollinate, whereas others will be used in reciprocal crosses with control tobacco (transgenics as female accepters and pollen donors; testing for maternal inheritance). Harvested seeds (T1) will be germinated on media containing spectinomycin. Achievement of homoplasmy and mode of inheritance can be classified by looking at germination results. Homoplasmy should be indicated by totally green seedlings (86) while heteroplasmy is displayed by variegated leaves (lack of pigmentation, 83). Lack of variation in chlorophyll pigmentation among progeny should also underscore the absence of position effect, an artifact of nuclear transformation. Maternal inheritance will be demonstrated by sole transmission of introduced genes via seed generated on transgenic plants, regardless of pollen source (green seedlings on selective media). When transgenic pollen is used for pollination of control plants, resultant progeny would not contain resistance to chemical in selective media (will appear bleached; 83). Molecular analyses will confirm transmission and expression of introduced genes, and T2 seed will be generated from those confirmed plants by the analyses described above.
**d.6 Purification methods**: The standard method of purification will employ classical biochemical techniques with the crystallized proteins inside the chloroplast In this case, the homogenates will be passed through miracloth to remove cell debris. Centrifugation at 10,000 xg would pellet all foreign proteins (3). Proteins will be solubilized using pH, temperature gradient, etc. This is possible if the ORF1 and 2 of the *cry2Aa2* operon (see section c) can fold and crystallize the recombinant proteins as expected. If there is no crystal formation, other purification methods will be done (classical biochemistry techniques and affinity columns with protease cleavage).
**d.7 Characterization of the recombinant proteins:** For the safe use of recombinant proteins as a replacement in any of the current applications, these proteins must be structurally equivalent and must not contain abnormal host-derived modifications. To confirm compliance with these I compare human and recombinant proteins using the currently highly sensitive and highly resolving techniques expected by the regulatory authorities to characterize recombinant products (135).
1- Amino acid analysis: Amino acid analysis to confirm the correct sequence will be performed following off-line vapour phase hydrolysis using ABI 420A amino acid derivatizer with an on line 130A phenylthiocarbamyl-amino acid analyzer (Applied Biosystems/ABI). N-terminal sequence analysis will be performed by Edman degradation using ABI 477A protein sequencer with an on-line 120A phenylthiohydantoin-amino acid analyzer. Automated C-terminal sequence analysis will use a Hewlett-Packard G1009A protein sequencer. To confirm the C-terminal sequence to a greater number of residues, the C-terminal tryptic peptide will be isolated from tryptic digests by reverse-phase HPLC.
2- Protein folding and disulfide bridges formation: Western blots with reducing and non-reducing gels will be done to check protein folding. PAGE to visualize small proteins will be done in the presence of tricine. Protein standards (Sigma) will be loaded to compare the mobility of the recombinant proteins. PAGE will be performed on PhastGels (Pharmacia Biotech). Proteins will be blotted and then probed with goat anti-HSA, interferon alpha and IGF-I polyclonal antibodies. Bound IgG will be detected with horseradish peroxidase-labelled anti goat IgG and visualized on X-ray film using ECL detection reagents (Amersham).
3- Tryptic mapping: To confirm the presence of chloroplast expressed proteins with disulfide linkages identical to native human proteins, the samples will subjected to tryptic digestion followed by peptide mass mapping using matrix-assisted laser desorption ionization mass spectrometry (MALDI-MS). Samples will be reduced with dithiothreitol, alkylated with iodoacetamide and then digested with trypsin comprising three additions of 1:100 enzyme/substrate over 48h at 37°C. Subsequently tryptic peptides will be separated by reverse-phase HPLC on a Vydac C18 column.
4- Mass analysis: Electrospray mass spectrometry (ESMS) will be performed using a VG Quattro electrospray mass spectrometer. Samples will be desalted prior to analysis by reverse-phase HPLC using an acetonitrile gradient containing trifluoroacetic acid.
5- CD: Spectra will be measured in a nitrogen atmosphere using a Jasco J600 spectropolarimeter.
6- Chromatographic techniques: For HSA, analytical gel-permeation HPLC will be performed using a TSK G3000 SWx1 column. Preparative gel permeation chromatography of HSA will be performed using a Sephacryl S200 HR column. The monomer fraction, identified by at 280 nm, will be dialyzed and reconcentrated to its starting concentration. For IGF-I, the reversed-phase chromatography the SMART system (Pharmacia Biotech) will be used with the mRPC C2/18 SC 2.1110 column.
7- Viscosity: This is a classical assay for recombinant HSA Viscosity is a characteristic of proteins related directly to their size, shape, and conformation. The viscosities of HSA and recombinant HSA will be measured at 100 mg. M1-1 in 0.15 M NaCl using a U-tube viscosimeter (M2 type, Poulton, Selfe and Lee Ltd, Essex, UK) at 25°C.
8- Glycosylation: Chloroplast proteins are not known to be glycosylated. However there are no publications to confirm or refute this assumption. Therefore glycosylation will be measured using a scaled-up version of the method of Ahmed and Furth (136).

## Claims

1. A plastid transformation and expression vector which comprises an expression cassette comprising as operably linked components, in the 5' to the 3' direction of translation,
- a promoter operative in said plastid,
- a selectable marker sequence,
- a heterologous DNA sequence coding for cholera toxin B-subunit fused to proinsulin,
- a transcription termination region functional in said plastid, and
- flanking, each side of the expression cassette, flanking DNA sequences which are homologous to a DNA sequence inclusive of a spacer sequence of the target plastid genome,
and, whereby stable integration of the heterologous coding sequence into the plastid genome of the target plant is facilitated throughout homologous recombination of the flanking sequence with the homologous sequences in the target plastid genome.

2. A plastid transformation and expression vector According to claim 1, wherein the heterologous DNA sequence coding for a plastid DNA fragment further comprises a 5'UTR sequence positioned upstream of the promoter to enhance translation of the active molecule.

3. A plastid transformation and expression vector according to claim 2, wherein said 5'UTR sequence is also positioned upstream ot the selectable marker sequence to further enhance translation of the active molecule.

4. A plastid transformation and expression vector according to claim 31, wherein said vector further comprises a heterologous DNA Sequence coding for a Cry2aA2 operon which comprises two open reading frames (ORF1 and ORF22), wherein the ORF immediately upstream of the Cry2aA2 codes for a putative chaperonin, which assists the crystallization of the insulin and aid in subsequent purification, and which operon is fused directly upstream of the promoter fusion protein.

5. A plastid transformation and expression vector according to any one of claims 1 or 4, wherein said vector further comprises a heterologous DNA sequence coding for a cholera toxin B-subunit-plastid modified proinsulin (PtPris) fusion wherein its nucleotide sequence modified such that the codons are optimized for plastid expression, while its amino acid sequence remains identical to native human proinsulin, a transcription termination region functional in said plastid.

6. A plastid transformation and expression vector according to any one of claims 1 or 4, wherein said vector further comprises a heterologous DNA sequence further coding for cholera toxin B-subunit-mini-proinsulin (Mpbis) fusion wherein its codons are optimized for plastid expression, while its amino acid sequence remains identical to native human proinsulin.

7. A plastid transformation and expression vector according to any one of claims 1 to 6, which comprises flanking each side of the expression cassette, flanking DNA sequences which are homologous to a DNA sequence inclusive of a spacer sequence of the target plastid genome, which sequence is conserved in the plastid genome of different plant species, whereby stable integration of the heterologous coding sequence into the plastid genome of the target plant is facilitated through homologous recombination of the flanking sequences with the homologous sequences in the target plastid genome

8. A plastid of a target plant species stably transformed with a plastid transformation and expression vector according to any one of claims 1 to 7.

9. A stable transformed plant which comprises a plastid stably transformed according to claim 8 or which is transformed by means of an expression vector according to any one of claims 1 to 7, or the transgenic progeny or the transgenic seed thereof.

10. A stable transformed plant according to claim 9, which is edible by humans.

11. A stable transformed plant according to any one of claims 9 or 10, which is either tobacco or alfalfa plant.

12. A process for stably transforming a higher target plant species, which comprises introducing into the plastid genome of the plant a vector according to any one of claims 1 to 7.

13. A process according to claim 12 wherein the transformed plastid shows the homoplasmic nature of the transformant plants.

14. A process according to claim 12 wherein the transformed plastid shows the heteroplasmic nature of the transformant plants.

## Patentansprüche

1. Plastidtransformations- und -expressionsvektor, der eine Expressionskassette umfasst, die als funktionell verknüpfte Komponenten in 5'- zu 3'-Translationsrichtung
- einen Promoter, der in dem Plastid funktionsfähig ist,
- eine selektierbare Markersequenz,
- eine heterologe DNA-Sequenz, die eine Choleratoxin-B-Untereinheit fusioniert an Proinsulin codiert,
- eine in dem Plastid funktionelle Transkriptionsterminationsregion, und
- flankierende DNA-Sequenzen, die jede Seite der Expressionskassette flankieren und homolog zu einer eine Spacersequenz einschließenden DNA-Sequenz des Zielplastidgenoms sind,
umfasst, und wobei die stabile Integration der heterologen codierenden Sequenz in das Plastidgenom der Zielpflanze durch homologe Rekombination der flankierenden Sequenz mit den homologen Sequenzen des Zielplastidgenoms vereinfacht ist.

2. Plastidtransformations- und -expressionsvektor nach Anspruch 1, wobei die heterologe DNA-Sequenz, die ein Plastid-DNA-Fragment codiert, weiterhin eine 5'-UTR-Sequenz umfasst, die stromaufwärts vom Promoter positioniert ist, um die Translation des aktiven Moleküls zu verbessern.

3. Plastidtransformations- und -expressionsvektor nach Anspruch 2, wobei die 5'-UTR-Sequenz auch stromaufwärts vom selektierbaren Markersequenz positioniert ist, um die Translation des aktiven Moleküls weiter zu verbessern.

4. Plastidtransformations- und -expressionsvektor nach Anspruch 1, wobei der Vektor weiterhin eine heterologe DNA-Sequenz umfasst, die ein Cry2aA2-Operon codiert, welches zwei offene Leseraster (ORF1 und ORF2) umfasst, wobei das ORF, das sich unmittelbar stromaufwärts von Cry2aA2 befindet, ein putatives Chaperonin codiert, welches die Kristallisation des Insulins unterstützt und bei der darauffolgenden Aufreinigung hilft, und wobei das Operon unmittelbar stromaufwärts des Promoterfusionsproteins fusioniert ist.

5. Plastidtransformations- und -expressionsvektor nach einem der Ansprüche 1 oder 4, wobei der Vektor weiterhin eine heterologe DNA-Sequenz, die ein Choleratoxin-B-Untereinheit-Plastid-modifiziertes Proinsulin-(PtPris)-Fusionsprotein codiert, wobei dessen Nucleotidsequenz so modifiziert ist, dass die Codons für Plastidexpression optimiert sind, während dessen Aminosäuresequenz identisch zu natürlichem humanem Proinsulin bleibt, und eine in dem Plastid funktionsfähige Transkriptionsterminationsregion umfasst.

6. Plastidtransformations- und -expressionsvektor nach einem der Ansprüche 1 oder 4, wobei der Vektor überdies eine heterologe DNA-Sequenz umfasst, die außerdem ein Cholera toxin-B-Untereinheit-Mini-Proinsulin-(Mpbis)-Fusionsprotein codiert, wobei dessen Codons für Plastidexpression optimiert sind, während dessen Aminosäuresequenz identisch zu natürlichem humanem Proinsulin bleibt.

7. Plastidtransformations- und -expressionsvektor nach einem der Ansprüche 1 bis 6, welcher flankierende DNA-Sequenzen, die jede Seite der Expressionskassette flankieren, umfasst, die homolog zu einer eine Spacersequenz einschließenden DNA-Sequenz des Zielplastidgenoms sind, deren Sequenz im Plastidgenom verschiedener Pflanzenarten konserviert ist, wobei die stabile Integration der heterologen codierenden Sequenz in das Plastidgenom der Zielpflanze durch homologe Rekombination der flankierenden Sequenzen mit den homologen Sequenzen in dem Zielplastidgenom vereinfacht ist.

8. Plastid einer Zielpflanzenart, das mit einem Plastidtransformations- und -expressionsvektor nach einem der Ansprüche 1 bis 7 stabil transformiert ist.

9. Stabil transformierte Pflanze, die ein stabil transformiertes Plastid nach Anspruch 8 umfasst oder die durch einen Expressionsvektor nach einem der Ansprüche 1 bis 7 transformiert ist, oder transgene Nachkommen oder transgene Samen davon.

10. Stabil transformierte Pflanze nach Anspruch 9, welche von Menschen verzehrt werden kann.

11. Stabil transformierte Pflanze nach einem der Ansprüche 9 oder 10, die entweder eine Tabak- oder Alfalfapflanze ist.

12. Verfahren für die stabile Transformation einer höheren Zielpflanzenart, welches das Einbringen eines Vektors nach einem der Ansprüche 1 bis 7 in das Plastidgenom der Pflanze umfasst.

13. Verfahren nach Anspruch 12, wobei das transformierte Plastid die homoplasmische Eigenschaft der transformierten Pflanzen zeigt.

14. Verfahren nach Anspruch 12, wobei das tranformierte Plastid die heteroplasmische Eigenschaft der transformierten Pflanzen zeigt.

## Revendications

1. Vecteur de transformation de plastide et d'expression, qui comprend une cassette d'expression comportant les composants suivants, opérationnellement raccordés dans la direction de traduction 5' → 3' :
- un promoteur opérationnel dans ledit plastide,
- une séquence marqueur pour sélection,
- une séquence d'ADN hétérologue, codant la sous-unité B de la toxine cholérique, fusionnée avec la pro-insuline,
- une région de fin de transcription, opérationnelle dans ledit plastide,
- et en flanc de chaque côté de la cassette d'expression, des séquences d'ADN flanquantes qui sont homologues à une séquence d'ADN
comprenant une séquence espaceur du génome de plastide cible, l'intégration stable de la séquence codante hétérologue dans le génome de plastide de la plante cible étant facilitée grâce à la recombinaison, par raison d'homologie, des séquences flanquantes avec les séquences homologues dans le génome de plastide cible.

2. Vecteur de transformation de plastide et d'expression, conforme à la revendication 1, dans lequel la séquence d'ADN hétérologue codant un fragment d'ADN de plastide comprend en outre une séquence de région 5' non-traduite, placée en amont du promoteur afin d'augmenter la traduction de la molécule active.

3. Vecteur de transformation de plastide et d'expression, conforme à la revendication 2, dans lequel ladite séquence de région 5' non-traduite est également placée en amont de la séquence marqueur pour sélection, afin d'augmenter encore davantage la traduction de la molécule active.

4. Vecteur de transformation de plastide et d'expression, conforme à la revendication 1, lequel vecteur comporte en outre une séquence d'ADN hétérologue codant un opéron Cry2aA2, qui comporte deux cadres ouverts de lecture, ORF1 et ORF2, parmi lesquels le cadre ouvert de lecture situé immédiatement en amont de l'opéron Cry2aA2 code une chaperonine putative qui concourt à la cristallisation de l'insuline et aide à la purification ultérieure de celle-ci, et lequel opéron est fusionné directement en amont des promoteur et protéine de fusion.

5. Vecteur de transformation de plastide et d'expression, conforme à la revendication 1 ou 4, lequel vecteur comporte en outre une séquence d'ADN hétérologue codant une protéine de fusion de la sous-unité B de la toxine cholérique et d'une pro-insuline modifiée pour expression en plastide (PtPris), la séquence de nucléotides qui la code étant modifiée de telle sorte que les codons sont optimisés en vue de l'expression en plastide alors que sa séquence d'acides aminés reste identique à celle de la pro-insuline humaine naturelle, ainsi qu'une région de fin de transcription fonctionnelle dans ledit plastide.

6. Vecteur de transformation de plastide et d'expression, conforme à la revendication 1 ou 4, lequel vecteur comporte en outre une séquence d'ADN hétérologue codant en outre une protéine de fusion de la sous-unité B de la toxine cholérique et d'une mini-pro-insuline (Mpbis), dont les codons sont optimisés en vue de l'expression en plastide alors que sa séquence d'acides aminés reste identique à celle de la pro-insuline humaine naturelle.

7. Vecteur de transformation de plastide et d'expression, conforme à l'une des revendications 1 à 6, qui comporte, en flanc de chaque côté de la cassette d'expression, des séquences d'ADN flanquantes qui sont homologues à une séquence d'ADN comprenant une séquence espaceur du génome de plastide cible, laquelle séquence est conservée dans le génome de plastide de différentes espèces végétales, l'intégration stable de la séquence codante hétérologue dans le génome de plastide de la plante cible étant facilitée grâce à la recombinaison, par raison d'homologie, des séquences flanquantes avec les séquences homologues dans le génome de plastide cible.

8. Plastide d'une espèce végétale cible, transformé de manière stable à l'aide d'un vecteur de transformation de plastide et d'expression, conforme à l'une des revendications 1 à 7.

9. Plante transformée de manière stable, qui contient un plastide transformé de manière stable, conforme à la revendication 8, ou qui a été transformée à l'aide d'un vecteur d'expression conforme à l'une des revendications 1 à 7, ou descendance transgénique ou semence transgénique d'une telle plante.

10. Plante transformée de manière stable, conforme à la revendication 9, qui est comestible pour les humains.

11. Plante transformée de manière stable, conforme à la revendication 9 ou 10, qui est du tabac ou de la luzerne.

12. Procédé permettant de transformer de manière stable une espèce cible de végétal supérieur, qui comporte le fait d'introduire, dans le génome de plastide du végétal, un vecteur conforme à l'une des revendications 1 à 7.

13. Procédé conforme à la revendication 12, dans lequel le plastide transformé montre le caractère homoplasmique des végétaux transformés.

14. Procédé conforme à la revendication 12, dans lequel le plastide transformé montre le caractère hétéroplasmique des végétaux transformés.
